(19) Europäisches Patentamt
European Patent Office
Office européen des brevets

(11) EP 1 328 287 B1

(12) # EUROPEAN PATENT SPECIFICATION

(45) Date of publication and mention of the grant of the patent:
**23.07.2008 Bulletin 2008/30**

(21) Application number: **01977493.4**

(22) Date of filing: **03.10.2001**

(51) Int Cl.:
***C07K 14/47*** *(2006.01)* ***C12N 15/63*** *(2006.01)*
***A61K 38/00*** *(2006.01)* ***A61K 39/00*** *(2006.01)*

(86) International application number:
**PCT/US2001/031139**

(87) International publication number:
**WO 2002/028414 (11.04.2002 Gazette 2002/15)**

(54) **COMPOSITIONS AND METHODS FOR WT1 SPECIFIC IMMUNOTHERAPY**

VERBINDUNGEN UND METHODEN ZUR WT1-SPEZIFISCHEN IMMUNTHERAPIE

COMPOSITIONS ET METHODES D'IMMUNOTHERAPIE SPECIFIQUE A WT1

(84) Designated Contracting States:
**AT BE CH CY DE DK ES FI FR GB GR IE IT LI LU MC NL PT SE TR**

(30) Priority:
**06.10.2000 US 684361**
**09.10.2000 US 685830**
**15.02.2001 US 785019**
**24.08.2001 US 938864**

(43) Date of publication of application:
**23.07.2003 Bulletin 2003/30**

(73) Proprietors:
- **CORIXA CORPORATION**
**Seattle, WA 98104 (US)**
- **Gaiger, Alexander**
**Seattle, WA 98112 (US)**

(72) Inventors:
- **GAIGER, Alexander**
**Seattle, WA 98112 (US)**
- **MCNEILL, Patricia, D.**
**Federal Way, WA 98003 (US)**
- **SMITHGALL, Molly**
**Seattle, WA 98115 (US)**
- **MOULTON, Gus**
**Seattle, WA 98115 (US)**
- **VEDVICK, Thomas, S.**
**Federal Way, WA 98003 (US)**
- **SLEATH, Paul, R.**
**Seattle, WA 98119 (US)**
- **MOSSMAN, Sally**
**Seattle, WA 98107 (US)**
- **EVANS, Lawrence**
**Seattle, WA 98126 (US)**
- **SPIES, A., Gregory**
**Shoreline, WA 98155 (US)**
- **BOYDSTON, Jeremy**
**Seattle, WA 98104 (US)**

(74) Representative: **Walker, Ross Thomson**
**Forrester & Boehmert**
**Pettenkoferstrasse 20-22**
**80336 München (DE)**

(56) References cited:
**WO-A-01/62920** **WO-A2-00/18795**

- **GAIGER A ET AL: "Immunity to WT1 in the animal model and patients with acute myeloid leukemia" BLOOD, W.B.SAUNDERS COMPANY, ORLANDO, FL, US, vol. 96, no. 4, 15 August 2000 (2000-08-15), pages 1480-1489, XP002965863 ISSN: 0006-4971**
- **TSUBOI AKIHIRO ET AL: "Cytotoxic T-lymphocyte responses elicited to Wilms' tumor gene WT1 product by DNA vaccination" JOURNAL OF CLINICAL IMMUNOLOGY, vol. 20, no. 3, May 2000 (2000-05), pages 195-202, XP002335291 ISSN: 0271-9142**
- **OKA Y ET AL: "HUMAN CYTOTOXIC T-LYMPHOCYTE RESPONSES SPECIFIC FOR PEPTIDES OF THEWILD-TYPE WILMS' TUMOR GENE (WT1) PRODUCT" IMMUNOGENETICS, SPRINGER VERLAG, BERLIN, DE, vol. 51, no. 2, 1 February 2000 (2000-02-01), pages 99-107, XP000884935 ISSN: 0093-7711**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 328 287 B1

SEQUENCE LISTING

<110> Corixa Corporation
Gaiger, Alexander
McNeill, Patricia D.
Smithgall, Molly
Moulton, Gus
Vedvick, Thomas S.
Sleath, Paul R.
Mossman, Sally
Evans, Lawrence
Spies, A. Gregory
Boydston, Jeremy

<120> COMPOSITIONS AND METHODS FOR WT1 SPECIFIC IMMUNOTHERAPY

<130> 210121.46501PC

<140> PCT
<141> 2001-10-03

<160> 413

<170> FastSEQ for Windows Version 3.0

<210> 1
<211> 17
<212> PRT
<213> Homo sapien

<400> 1

```
Arg Asp Leu Asn Ala Leu Leu Pro Ala Val Pro Ser Leu Gly Gly Gly
 1               5                  10                  15
Gly
```

<210> 2
<211> 23
<212> PRT
<213> Homo sapien

<400> 2

```
Pro Ser Gln Ala Ser Ser Gly Gln Ala Arg Met Phe Pro Asn Ala Pro
 1               5                  10                  15
Tyr Leu Pro Ser Cys Leu Glu
            20
```

<210> 3

<211> 23
<212> PRT
<213> Mus musculus

<400> 3

Pro Ser Gln Ala Ser Ser Gly Gln Ala Arg Met Phe Pro Asn Ala Pro
1 5 10 15
Tyr Leu Pro Ser Cys Leu Glu
20

<210> 4
<211> 19
<212> PRT
<213> Homo sapien

<400> 4

Gly Ala Thr Leu Lys Gly Val Ala Ala Gly Ser Ser Ser Ser Val Lys
1 5 10 15
Trp Thr Glu

<210> 5
<211> 22
<212> DNA
<213> Homo sapien

<400> 5
gagagtcaga cttgaaagca gt 22

<210> 6
<211> 20
<212> DNA
<213> Homo sapien

<400> 6
ctgagcctca gcaaatgggc 20

<210> 7
<211> 27
<212> DNA
<213> Homo sapien

<400> 7
gagcatgcat gggctccgac gtgcggg 27

<210> 8
<211> 25
<212> DNA
<213> Homo sapien

<400> 8
ggggtaccca ctgaacggtc cccga 25

<210> 9
<211> 18
<212> DNA
<213> Mus musculus

<400> 9
tccgagccgc acctcatg 18

<210> 10
<211> 18
<212> DNA
<213> Mus musculus

<400> 10
gcctgggatg ctggactg 18

<210> 11
<211> 27
<212> DNA
<213> Mus musculus

<400> 11
gagcatgcga tgggttccga cgtgcgg 27

<210> 12
<211> 29
<212> DNA
<213> Mus musculus

<400> 12
ggggtacctc aaagcgccac gtggagttt 29

<210> 13
<211> 17
<212> PRT
<213> Mus musculus

<400> 13

```
Arg Asp Leu Asn Ala Leu Leu Pro Ala Val Ser Ser Leu Gly Gly Gly
 1               5                  10                  15
Gly
```

<210> 14
<211> 19
<212> PRT
<213> Mus musculus

<400> 14

```
Gly Ala Thr Leu Lys Gly Met Ala Ala Gly Ser Ser Ser Ser Val Lys
 1               5                  10                  15
Trp Thr Glu
```

<210> 15
<211> 15

<212> PRT
<213> Homo sapien

<400> 15

```
Arg Ile His Thr His Gly Val Phe Arg Gly Ile Gln Asp Val Arg
1               5                   10                  15
```

<210> 16
<211> 15
<212> PRT
<213> Mus musculus

<400> 16

```
Arg Ile His Thr His Gly Val Phe Arg Gly Ile Gln Asp Val Arg
1               5                   10                  15
```

<210> 17
<211> 14
<212> PRT
<213> Mus musculus

<400> 17

```
Val Arg Arg Val Ser Gly Val Ala Pro Thr Leu Val Arg Ser
1               5                   10
```

<210> 18
<211> 14
<212> PRT
<213> Homo sapien

<400> 18

```
Val Arg Arg Val Pro Gly Val Ala Pro Thr Leu Val Arg Ser
1               5                   10
```

<210> 19
<211> 15
<212> PRT
<213> Homo sapien

<400> 19

```
Cys Gln Lys Lys Phe Ala Arg Ser Asp Glu Leu Val Arg His His
1               5                   10                  15
```

<210> 20
<211> 15
<212> PRT
<213> Mus musculus

<400> 20

Cys Gln Lys Lys Phe Ala Arg Ser Asp Glu Leu Val Arg His His
1 5 10 15

<210> 21
<211> 21
<212> DNA
<213> Mus musculus

<400> 21
cccaggctgc aataagagat a 21

<210> 22
<211> 21
<212> DNA
<213> Mus musculus

<400> 22
atgttgtgat ggcggaccaa t 21

<210> 23
<211> 20
<212> DNA
<213> Homo sapien

<400> 23
gtggggcgcc ccaggcacca 20

<210> 24
<211> 24
<212> DNA
<213> Homo sapien

<400> 24
gtccttaatg ctacgcacga tttc 24

<210> 25
<211> 21
<212> DNA
<213> Homo sapien

<400> 25
ggcatctgag accagtgaga a 21

<210> 26
<211> 21
<212> DNA
<213> Homo sapien

<400> 26
gctgtcccac ttacagatgc a 21

<210> 27
<211> 21
<212> DNA
<213> Homo sapien

<400> 27
tcaaagcgcc agctggagtt t 21

<210> 28
<211> 9
<212> PRT
<213> Homo sapien

<400> 28

Ala Ala Gly Ser Ser Ser Ser Val Lys
1 5

<210> 29
<211> 9
<212> PRT
<213> Homo sapien

<400> 29

Ala Ala Gln Phe Pro Asn His Ser Phe
1 5

<210> 30
<211> 9
<212> PRT
<213> Homo sapien

<400> 30

Ala Glu Pro His Glu Glu Gln Cys Leu
1 5

<210> 31
<211> 9
<212> PRT
<213> Homo sapien

<400> 31

Ala Gly Ala Cys Arg Tyr Gly Pro Phe
1 5

<210> 32
<211> 9
<212> PRT
<213> Homo sapien

<400> 32

Ala Gly Ser Ser Ser Ser Val Lys Trp
1 5

<210> 33
<211> 9
<212> PRT
<213> Homo sapien

<400> 33

Ala Ile Arg Asn Gln Gly Tyr Ser Thr
1 5

<210> 34
<211> 9
<212> PRT
<213> Homo sapien

<400> 34

Ala Leu Leu Pro Ala Val Pro Ser Leu
1 5

<210> 35
<211> 9
<212> PRT
<213> Homo sapien

<400> 35

Ala Leu Leu Pro Ala Val Ser Ser Leu
1 5

<210> 36
<211> 9
<212> PRT
<213> Homo sapien

<400> 36

Ala Gln Phe Pro Asn His Ser Phe Lys
1 5

<210> 37
<211> 9
<212> PRT
<213> Homo sapien

<400> 37

Ala Gln Trp Ala Pro Val Leu Asp Phe
1 5

<210> 38
<211> 9
<212> PRT

<213> Homo sapien

<400> 38

Ala Arg Met Phe Pro Asn Ala Pro Tyr
1               5

<210> 39
<211> 9
<212> PRT
<213> Homo sapien

<400> 39

Ala Arg Ser Asp Glu Leu Val Arg His
1               5

<210> 40
<211> 9
<212> PRT
<213> Homo sapien

<400> 40

Ala Ser Ser Gly Gln Ala Arg Met Phe
1               5

<210> 41
<211> 9
<212> PRT
<213> Homo sapien

<400> 41

Ala Tyr Gly Ser Leu Gly Gly Pro Ala
1               5

<210> 42
<211> 9
<212> PRT
<213> Homo sapien

<400> 42

Ala Tyr Pro Gly Cys Asn Lys Arg Tyr
1               5

<210> 43
<211> 9
<212> PRT
<213> Homo sapien

<400> 43

Cys Ala Leu Pro Val Ser Gly Ala Ala
1 5

<210> 44
<211> 9
<212> PRT
<213> Homo sapien

<400> 44

Cys Ala Tyr Pro Gly Cys Asn Lys Arg
1 5

<210> 45
<211> 9
<212> PRT
<213> Homo sapien

<400> 45

Cys His Thr Pro Thr Asp Ser Cys Thr
1 5

<210> 46
<211> 9
<212> PRT
<213> Homo sapien

<400> 46

Cys Lys Thr Cys Gln Arg Lys Phe Ser
1 5

<210> 47
<211> 9
<212> PRT
<213> Homo sapien

<400> 47

Cys Leu Glu Ser Gln Pro Ala Ile Arg
1 5

<210> 48
<211> 9
<212> PRT
<213> Homo sapien

<400> 48

Cys Leu Ser Ala Phe Thr Val His Phe
1 5

<210> 49
<211> 9
<212> PRT
<213> Homo sapien

<400> 49

Cys Met Thr Trp Asn Gln Met Asn Leu
1 5

<210> 50
<211> 9
<212> PRT
<213> Homo sapien

<400> 50

Cys Arg Trp Pro Ser Cys Gln Lys Lys
1 5

<210> 51
<211> 9
<212> PRT
<213> Homo sapien

<400> 51

Cys Arg Tyr Gly Pro Phe Gly Pro Pro
1 5

<210> 52
<211> 9
<212> PRT
<213> Homo sapien

<400> 52

Cys Thr Gly Ser Gln Ala Leu Leu Leu
1 5

<210> 53
<211> 9
<212> PRT
<213> Homo sapien

<400> 53

Asp Glu Leu Val Arg His His Asn Met
1 5

<210> 54
<211> 9
<212> PRT

<213> Homo sapien

<400> 54

Asp Phe Ala Pro Pro Gly Ala Ser Ala
1 5

<210> 55
<211> 9
<212> PRT
<213> Homo sapien

<400> 55

Asp Phe Lys Asp Cys Glu Arg Arg Phe
1 5

<210> 56
<211> 9
<212> PRT
<213> Homo sapien

<400> 56

Asp Gly Thr Pro Ser Tyr Gly His Thr
1 5

<210> 57
<211> 9
<212> PRT
<213> Homo sapien

<400> 57

Asp His Leu Lys Thr His Thr Arg Thr
1 5

<210> 58
<211> 9
<212> PRT
<213> Homo sapien

<400> 58

Asp Leu Asn Ala Leu Leu Pro Ala Val
1 5

<210> 59
<211> 9
<212> PRT
<213> Homo sapien

<400> 59

Asp Pro Met Gly Gln Gln Gly Ser Leu
1 5

<210> 60
<211> 9
<212> PRT
<213> Homo sapien

<400> 60

Asp Gln Leu Lys Arg His Gln Arg Arg
1 5

<210> 61
<211> 9
<212> PRT
<213> Homo sapien

<400> 61

Asp Ser Cys Thr Gly Ser Gln Ala Leu
1 5

<210> 62
<211> 9
<212> PRT
<213> Homo sapien

<400> 62

Asp Val Arg Asp Leu Asn Ala Leu Leu

1 5

<210> 63
<211> 9
<212> PRT
<213> Homo sapien

<400> 63

Asp Val Arg Arg Val Pro Gly Val Ala
1 5

<210> 64
<211> 9
<212> PRT
<213> Homo sapien

<400> 64

Glu Asp Pro Met Gly Gln Gln Gly Ser
1 5

<210> 65
<211> 9
<212> PRT
<213> Homo sapien

<400> 65

Glu Glu Gln Cys Leu Ser Ala Phe Thr
1 5

<210> 66
<211> 9.
<212> PRT
<213> Homo sapien

<400> 66

Glu Lys Pro Tyr Gln Cys Asp Phe Lys
1 5

<210> 67
<211> 9
<212> PRT
<213> Homo sapien

<400> 67

Glu Lys Arg Pro Phe Met Cys Ala Tyr
1 5

<210> 68
<211> 9
<212> PRT
<213> Homo sapien

<400> 68

Glu Pro His Glu Glu Gln Cys Leu Ser
1 5

<210> 69
<211> 9
<212> PRT
<213> Homo sapien

<400> 69

Glu Gln Cys Leu Ser Ala Phe Thr Val
1 5

<210> 70
<211> 9
<212> PRT
<213> Homo sapien

<400> 70

Glu Ser Asp Asn His Thr Ala Pro Ile
1 5

<210> 71
<211> 9
<212> PRT
<213> Homo sapien

<400> 71

Glu Ser Asp Asn His Thr Thr Pro Ile
1 5

<210> 72
<211> 9
<212> PRT
<213> Homo sapien

<400> 72

Glu Ser Gln Pro Ala Ile Arg Asn Gln
1 5

<210> 73
<211> 9
<212> PRT
<213> Homo sapien

<400> 73

Glu Thr Ser Glu Lys Arg Pro Phe Met
1 5 .

<210> 74
<211> 9
<212> PRT
<213> Homo sapien

<400> 74

Phe Ala Pro Pro Gly Ala Ser Ala Tyr
1 5

<210> 75
<211> 9
<212> PRT
<213> Homo sapien

<400> 75

Phe Ala Arg Ser Asp Glu Leu Val Arg
1 5

<210> 76
<211> 9
<212> PRT
<213> Homo sapien

<400> 76

Phe Gly Pro Pro Pro Pro Ser Gln Ala
1 5

<210> 77
<211> 9
<212> PRT
<213> Homo sapien

<400> 77

Phe Lys Asp Cys Glu Arg Arg Phe Ser
1 5

<210> 78
<211> 9
<212> PRT
<213> Homo sapien

<400> 78

Phe Lys Leu Ser His Leu Gln Met His
1 5

<210> 79
<211> 9
<212> PRT
<213> Homo sapien

<400> 79

Phe Pro Asn Ala Pro Tyr Leu Pro Ser
1 5

<210> 80
<211> 9
<212> PRT

<213> Homo sapien

<400> 80

Phe Gln Cys Lys Thr Cys Gln Arg Lys
1 5

<210> 81
<211> 9
<212> PRT
<213> Homo sapien

<400> 81

Phe Arg Gly Ile Gln Asp Val Arg Arg
1 5

<210> 82
<211> 9
<212> PRT
<213> Homo sapien

<400> 82

Phe Ser Gly Gln Phe Thr Gly Thr Ala
1 5

<210> 83
<211> 9
<212> PRT
<213> Homo sapien

<400> 83

Phe Ser Arg Ser Asp Gln Leu Lys Arg
1 5

<210> 84
<211> 9
<212> PRT
<213> Homo sapien

<400> 84

Phe Thr Gly Thr Ala Gly Ala Cys Arg
1 5

<210> 85
<211> 9
<212> PRT
<213> Homo sapien

<400> 85

Phe Thr Val His Phe Ser Gly Gln Phe
1 5

<210> 86
<211> 9
<212> PRT
<213> Homo sapien

<400> 86

Gly Ala Ala Gln Trp Ala Pro Val Leu
1 5

<210> 87
<211> 9
<212> PRT
<213> Homo sapien

<400> 87

Gly Ala Glu Pro His Glu Glu Gln Cys
1 5

<210> 88
<211> 9
<212> PRT
<213> Homo sapien

<400> 88

Gly Ala Thr Leu Lys Gly Val Ala Ala
1 5

<210> 89
<211> 9
<212> PRT
<213> Homo sapien

<400> 89

Gly Cys Ala Leu Pro Val Ser Gly Ala
1 5

<210> 90
<211> 9
<212> PRT
<213> Homo sapien

<400> 90

Gly Cys Asn Lys Arg Tyr Phe Lys Leu
1 5

<210> 91
<211> 9
<212> PRT
<213> Homo sapien

<400> 91

Gly Glu Lys Pro Tyr Gln Cys Asp Phe
1 5

<210> 92
<211> 9
<212> PRT
<213> Homo sapien

<400> 92

Gly Gly Gly Gly Cys Ala Leu Pro Val
1 5

<210> 93
<211> 9
<212> PRT
<213> Homo sapien

<400> 93

Gly Gly Pro Ala Pro Pro Pro Ala Pro
1 5

<210> 94
<211> 9
<212> PRT
<213> Homo sapien

<400> 94

Gly His Thr Pro Ser His His Ala Ala
1 5

<210> 95
<211> 9
<212> PRT
<213> Homo sapien

<400> 95

Gly Lys Thr Ser Glu Lys Pro Phe Ser
1 5

<210> 96
<211> 9

<212> PRT
<213> Homo sapien

<400> 96

Gly Pro Phe Gly Pro Pro Pro Pro Ser
1 5

<210> 97
<211> 9
<212> PRT
<213> Homo sapien

<400> 97

Gly Pro Pro Pro Pro Ser Gln Ala Ser
1 5

<210> 98
<211> 9
<212> PRT
<213> Homo sapien

<400> 98

Gly Gln Ala Arg Met Phe Pro Asn Ala
1 5

<210> 99
<211> 9
<212> PRT
<213> Homo sapien

<400> 99

Gly Gln Phe Thr Gly Thr Ala Gly Ala
1 5

<210> 100
<211> 9
<212> PRT
<213> Homo sapien

<400> 100

Gly Gln Ser Asn His Ser Thr Gly Tyr
1 5

<210> 101
<211> 9
<212> PRT
<213> Homo sapien

<400> 101

Gly Ser Asp Val Arg Asp Leu Asn Ala
1 5

<210> 102
<211> 9
<212> PRT
<213> Homo sapien

<400> 102

Gly Ser Gln Ala Leu Leu Leu Arg Thr
1 5

<210> 103
<211> 9
<212> PRT
<213> Homo sapien

<400> 103

Gly Val Phe Arg Gly Ile Gln Asp Val
1 5

<210> 104
<211> 9
<212> PRT
<213> Homo sapien

<400> 104

Gly Val Lys Pro Phe Gln Cys Lys Thr
1 5

<210> 105
<211> 9
<212> PRT
<213> Homo sapien

<400> 105

Gly Tyr Glu Ser Asp Asn His Thr Ala
1 5

<210> 106
<211> 9
<212> PRT
<213> Homo sapien

<400> 106

Gly Tyr Glu Ser Asp Asn His Thr Thr
1 5

<210> 107
<211> 9
<212> PRT
<213> Homo sapien

<400> 107

His Glu Glu Gln Cys Leu Ser Ala Phe
1 5

<210> 108
<211> 9
<212> PRT
<213> Homo sapien

<400> 108

His His Asn Met His Gln Arg Asn Met
1 5

<210> 109
<211> 9
<212> PRT
<213> Homo sapien

<400> 109

His Gln Arg Arg His Thr Gly Val Lys
1 5

<210> 110
<211> 9
<212> PRT
<213> Homo sapien

<400> 110

His Ser Phe Lys His Glu Asp Pro Met
1 5

<210> 111
<211> 9
<212> PRT
<213> Homo sapien

<400> 111

His Ser Arg Lys His Thr Gly Glu Lys
1 5

<210> 112
<211> 9
<212> PRT
<213> Homo sapien

<400> 112

His Thr Gly Glu Lys Pro Tyr Gln Cys
1 5

<210> 113
<211> 9
<212> PRT
<213> Homo sapien

<400> 113

His Thr His Gly Val Phe Arg Gly Ile
1 5

<210> 114
<211> 9
<212> PRT
<213> Homo sapien

<400> 114

His Thr Arg Thr His Thr Gly Lys Thr
1 5

<210> 115
<211> 9
<212> PRT
<213> Homo sapien

<400> 115

His Thr Thr Pro Ile Leu Cys Gly Ala
1 5

<210> 116
<211> 9
<212> PRT
<213> Homo sapien

<400> 116

Ile Leu Cys Gly Ala Gln Tyr Arg Ile
1 5

<210> 117
<211> 9
<212> PRT
<213> Homo sapien

<400> 117

Ile Arg Asn Gln Gly Tyr Ser Thr Val
1 5

<210> 118
<211> 9
<212> PRT
<213> Homo sapien

<400> 118

Lys Asp Cys Glu Arg Arg Phe Ser Arg
1 5

<210> 119
<211> 9
<212> PRT
<213> Homo sapien

<400> 119

Lys Phe Ala Arg Ser Asp Glu Leu Val
1 5

<210> 120
<211> 9
<212> PRT
<213> Homo sapien

<400> 120

Lys Phe Ser Arg Ser Asp His Leu Lys

1 5

<210> 121
<211> 9
<212> PRT
<213> Homo sapien

<400> 121

Lys His Glu Asp Pro Met Gly Gln Gln
1 5

<210> 122
<211> 9
<212> PRT
<213> Homo sapien

<400> 122

Lys Lys Phe Ala Arg Ser Asp Glu Leu
1 5

<210> 123
<211> 9
<212> PRT
<213> Homo sapien

<400> 123

Lys Pro Phe Ser Cys Arg Trp Pro Ser
1 5

<210> 124
<211> 9
<212> PRT
<213> Homo sapien

<400> 124

Lys Pro Tyr Gln Cys Asp Phe Lys Asp
1 5

<210> 125
<211> 9
<212> PRT
<213> Homo sapien

<400> 125

Lys Gln Glu Pro Ser Trp Gly Gly Ala
1 5

<210> 126
<211> 9
<212> PRT
<213> Homo sapien

<400> 126

Lys Arg His Gln Arg Arg His Thr Gly
1 5

<210> 127
<211> 9
<212> PRT

<213> Homo sapien

<400> 127

Lys Arg Tyr Phe Lys Leu Ser His Leu
1 5

<210> 128
<211> 9
<212> PRT
<213> Homo sapien

<400> 128

Lys Thr Cys Gln Arg Lys Phe Ser Arg
1 5

<210> 129
<211> 9
<212> PRT
<213> Homo sapien

<400> 129

Lys Thr Ser Glu Lys Pro Phe Ser Cys
1 5

<210> 130
<211> 9
<212> PRT
<213> Homo sapien

<400> 130

Leu Asp Phe Ala Pro Pro Gly Ala Ser
1 5

<210> 131
<211> 9
<212> PRT
<213> Homo sapien

<400> 131

Leu Glu Cys Met Thr Trp Asn Gln Met
1 5

<210> 132
<211> 9
<212> PRT
<213> Homo sapien

<400> 132

Leu Glu Ser Gln Pro Ala Ile Arg Asn
1 5

<210> 133
<211> 9
<212> PRT
<213> Homo sapien

<400> 133

Leu Gly Ala Thr Leu Lys Gly Val Ala
1 5

<210> 134
<211> 9
<212> PRT
<213> Homo sapien

<400> 134

Leu Gly Gly Gly Gly Gly Cys Ala Leu
1 5

<210> 135
<211> 9
<212> PRT
<213> Homo sapien

<400> 135

Leu Lys Gly Val Ala Ala Gly Ser Ser
1 5

<210> 136
<211> 9
<212> PRT
<213> Homo sapien

<400> 136

Leu Lys Arg His Gln Arg Arg His Thr
1 5

<210> 137
<211> 9
<212> PRT
<213> Homo sapien

<400> 137

Leu Lys Thr His Thr Arg Thr His Thr
1 5

<210> 138
<211> 9
<212> PRT
<213> Homo sapien

<400> 138

```
Leu Pro Val Ser Gly Ala Ala Gln Trp
 1               5
```

<210> 139
<211> 9
<212> PRT
<213> Homo sapien

<400> 139

```
Leu Gln Met His Ser Arg Lys His Thr
 1               5
```

<210> 140
<211> 9
<212> PRT
<213> Homo sapien

<400> 140

```
Leu Arg Thr Pro Tyr Ser Ser Asp Asn
 1               5
```

<210> 141
<211> 9
<212> PRT
<213> Homo sapien

<400> 141

```
Leu Ser His Leu Gln Met His Ser Arg
 1               5
```

<210> 142
<211> 9
<212> PRT
<213> Homo sapien

<400> 142

```
Met Cys Ala Tyr Pro Gly Cys Asn Lys
 1               5
```

<210> 143
<211> 9
<212> PRT

<213> Homo sapien

<400> 143

Met His Gln Arg Asn Met Thr Lys Leu
1 5

<210> 144
<211> 9
<212> PRT
<213> Homo sapien

<400> 144

Asn Ala Pro Tyr Leu Pro Ser Cys Leu
1 5

<210> 145
<211> 9
<212> PRT
<213> Homo sapien

<400> 145

Asn Lys Arg Tyr Phe Lys Leu Ser His
1 5

<210> 146
<211> 9
<212> PRT
<213> Homo sapien

<400> 146

Asn Leu Gly Ala Thr Leu Lys Gly Val
1 5

<210> 147
<211> 9
<212> PRT
<213> Homo sapien

<400> 147

Asn Leu Tyr Gln Met Thr Ser Gln Leu
1 5

<210> 148
<211> 9
<212> PRT
<213> Homo sapien

<400> 148

Asn Met His Gln Arg Asn Met Thr Lys
1 5

<210> 149
<211> 9
<212> PRT
<213> Homo sapien

<400> 149

Asn Met Thr Lys Leu Gln Leu Ala Leu
1 5

<210> 150
<211> 9
<212> PRT
<213> Homo sapien

<400> 150

Asn Gln Gly Tyr Ser Thr Val Thr Phe
1 5

<210> 151
<211> 9
<212> PRT
<213> Homo sapien

<400> 151

Asn Gln Met Asn Leu Gly Ala Thr Leu
1 5

<210> 152
<211> 9
<212> PRT
<213> Homo sapien

<400> 152

Pro Ala Ile Arg Asn Gln Gly Tyr Ser
1 5

<210> 153
<211> 9
<212> PRT
<213> Homo sapien

<400> 153

Pro Gly Ala Ser Ala Tyr Gly Ser Leu
1 5

<210> 154
<211> 9
<212> PRT
<213> Homo sapien

<400> 154

Pro His Glu Glu Gln Cys Leu Ser Ala
1 5

<210> 155
<211> 9
<212> PRT
<213> Homo sapien

<400> 155

Pro Ile Leu Cys Gly Ala Gln Tyr Arg
1 5

<210> 156
<211> 9
<212> PRT
<213> Homo sapien

<400> 156

Pro Pro Pro Pro His Ser Phe Ile Lys
1 5

<210> 157
<211> 9
<212> PRT
<213> Homo sapien

<400> 157

Pro Pro Pro Pro Pro His Ser Phe Ile
1 5

<210> 158
<211> 9
<212> PRT
<213> Homo sapien

<400> 158

Pro Pro Pro Pro Pro Pro His Ser Phe
1 5

<210> 159
<211> 9
<212> PRT

<213> Homo sapien

<400> 159

Pro Ser Cys Gln Lys Lys Phe Ala Arg
1 5

<210> 160
<211> 9
<212> PRT
<213> Homo sapien

<400> 160

Gln Ala Leu Leu Leu Arg Thr Pro Tyr
1 5

<210> 161
<211> 9
<212> PRT
<213> Homo sapien

<400> 161

Gln Ala Ser Ser Gly Gln Ala Arg Met
1 5

<210> 162
<211> 9
<212> PRT
<213> Homo sapien

<400> 162

Gln Cys Asp Phe Lys Asp Cys Glu Arg
1 5

<210> 163
<211> 9
<212> PRT
<213> Homo sapien

<400> 163

Gln Cys Lys Thr Cys Gln Arg Lys Phe
1 5

<210> 164
<211> 9
<212> PRT
<213> Homo sapien

<400> 164

Gln Asp Val Arg Arg Val Pro Gly Val
1 5

<210> 165
<211> 9
<212> PRT
<213> Homo sapien

<400> 165

Gln Phe Thr Gly Thr Ala Gly Ala Cys
1 5

<210> 166
<211> 9
<212> PRT
<213> Homo sapien

<400> 166

Gln Gly Ser Leu Gly Glu Gln Gln Tyr
1 5

<210> 167
<211> 9
<212> PRT
<213> Homo sapien

<400> 167

Gln Leu Glu Cys Met Thr Trp Asn Gln
1 5

<210> 168
<211> 9
<212> PRT
<213> Homo sapien

<400> 168

Gln Met Asn Leu Gly Ala Thr Leu Lys
1 5

<210> 169
<211> 9
<212> PRT
<213> Homo sapien

<400> 169

Gln Met Thr Ser Gln Leu Glu Cys Met
1 5

<210> 170
<211> 9
<212> PRT
<213> Homo sapien

<400> 170

Gln Pro Ala Ile Arg Asn Gln Gly Tyr
1 5

<210> 171
<211> 9
<212> PRT
<213> Homo sapien

<400> 171

Gln Gln Tyr Ser Val Pro Pro Pro Val
1 5

<210> 172
<211> 9
<212> PRT
<213> Homo sapien

<400> 172

Gln Arg Lys Phe Ser Arg Ser Asp His
1 5

<210> 173
<211> 9
<212> PRT
<213> Homo sapien

<400> 173

Gln Arg Asn Met Thr Lys Leu Gln Leu
1 5

<210> 174
<211> 9
<212> PRT
<213> Homo sapien

<400> 174

Gln Trp Ala Pro Val Leu Asp Phe Ala
1 5

<210> 175
<211> 9
<212> PRT
<213> Homo sapien

<400> 175

Gln Tyr Arg Ile His Thr His Gly Val
1 5

<210> 176
<211> 9
<212> PRT
<213> Homo sapien

<400> 176

Gln Tyr Ser Val Pro Pro Pro Val Tyr
1 5

<210> 177
<211> 9
<212> PRT
<213> Homo sapien

<400> 177

Arg Asp Leu Asn Ala Leu Leu Pro Ala
1 5

<210> 178
<211> 9
<212> PRT
<213> Homo sapien

<400> 178

Arg Phe Ser Arg Ser Asp Gln Leu Lys

1 5

<210> 179
<211> 9
<212> PRT
<213> Homo sapien

<400> 179

Arg Gly Ile Gln Asp Val Arg Arg Val
1 5

<210> 180
<211> 9
<212> PRT
<213> Homo sapien

<400> 180

Arg His His Asn Met His Gln Arg Asn
1 5

<210> 181
<211> 9
<212> PRT
<213> Homo sapien

<400> 181

Arg His Gln Arg Arg His Thr Gly Val
1 5

<210> 182
<211> 9
<212> PRT
<213> Homo sapien

<400> 182

Arg Ile His Thr His Gly Val Phe Arg
1 5

<210> 183
<211> 9
<212> PRT
<213> Homo sapien

<400> 183

Arg Lys Phe Ser Arg Ser Asp His Leu
1 5

<210> 184
<211> 9
<212> PRT
<213> Homo sapien

<400> 184

Arg Lys His Thr Gly Glu Lys Pro Tyr
1 5

<210> 185
<211> 9
<212> PRT

<213> Homo sapien

<400> 185

Arg Met Phe Pro Asn Ala Pro Tyr Leu
1 5

<210> 186
<211> 9
<212> PRT
<213> Homo sapien

<400> 186

Arg Asn Met Thr Lys Leu Gln Leu Ala
1 5

<210> 187
<211> 9
<212> PRT
<213> Homo sapien

<400> 187

Arg Arg Phe Ser Arg Ser Asp Gln Leu
1 5

<210> 188
<211> 9
<212> PRT
<213> Homo sapien

<400> 188

Arg Arg His Thr Gly Val Lys Pro Phe
1 5

<210> 189
<211> 9
<212> PRT
<213> Homo sapien

<400> 189

Arg Arg Val Pro Gly Val Ala Pro Thr
1 5

<210> 190
<211> 9
<212> PRT
<213> Homo sapien

<400> 190

Arg Ser Ala Ser Glu Thr Ser Glu Lys
1 5

<210> 191
<211> 9
<212> PRT
<213> Homo sapien

<400> 191

Arg Ser Asp Glu Leu Val Arg His His
1 5

<210> 192
<211> 9
<212> PRT
<213> Homo sapien

<400> 192

Arg Ser Asp His Leu Lys Thr His Thr
1 5

<210> 193
<211> 9
<212> PRT
<213> Homo sapien

<400> 193

Arg Ser Asp Gln Leu Lys Arg His Gln
1 5

<210> 194
<211> 9
<212> PRT
<213> Homo sapien

<400> 194

Arg Thr Pro Tyr Ser Ser Asp Asn Leu
1 5

<210> 195
<211> 9
<212> PRT
<213> Homo sapien

<400> 195

Arg Val Pro Gly Val Ala Pro Thr Leu
1 5

<210> 196
<211> 9
<212> PRT
<213> Homo sapien

<400> 196

Arg Trp Pro Ser Cys Gln Lys Lys Phe
1 5

<210> 197
<211> 9
<212> PRT
<213> Homo sapien

<400> 197

Ser Ala Ser Glu Thr Ser Glu Lys Arg
1 5

<210> 198
<211> 9
<212> PRT
<213> Homo sapien

<400> 198

Ser Cys Leu Glu Ser Gln Pro Ala Ile
1 5

<210> 199
<211> 9
<212> PRT
<213> Homo sapien

<400> 199

Ser Cys Leu Glu Ser Gln Pro Thr Ile
1 5

<210> 200
<211> 9
<212> PRT
<213> Homo sapien

<400> 200

Ser Cys Gln Lys Lys Phe Ala Arg Ser
1 5

<210> 201
<211> 9
<212> PRT

<213> Homo sapien

<400> 201

Ser Cys Arg Trp Pro Ser Cys Gln Lys
1 5

<210> 202
<211> 9
<212> PRT
<213> Homo sapien

<400> 202

Ser Cys Thr Gly Ser Gln Ala Leu Leu
1 5

<210> 203
<211> 9
<212> PRT
<213> Homo sapien

<400> 203

Ser Asp Glu Leu Val Arg His His Asn
1 5

<210> 204
<211> 9
<212> PRT
<213> Homo sapien

<400> 204

Ser Asp Asn His Thr Thr Pro Ile Leu
1 5

<210> 205
<211> 9
<212> PRT
<213> Homo sapien

<400> 205

Ser Asp Asn Leu Tyr Gln Met Thr Ser
1 5

<210> 206
<211> 9
<212> PRT
<213> Homo sapien

<400> 206

Ser Asp Val Arg Asp Leu Asn Ala Leu
1 5

<210> 207
<211> 9
<212> PRT
<213> Homo sapien

<400> 207

Ser Glu Lys Pro Phe Ser Cys Arg Trp
1 5

<210> 208
<211> 9
<212> PRT
<213> Homo sapien

<400> 208

Ser Glu Lys Arg Pro Phe Met Cys Ala
1 5

<210> 209
<211> 9
<212> PRT
<213> Homo sapien

<400> 209

Ser Glu Thr Ser Glu Lys Arg Pro Phe
1 5

<210> 210
<211> 9
<212> PRT
<213> Homo sapien

<400> 210

Ser Phe Ile Lys Gln Glu Pro Ser Trp
1 5

<210> 211
<211> 9
<212> PRT
<213> Homo sapien

<400> 211

Ser Gly Ala Ala Gln Trp Ala Pro Val
1 5

<210> 212
<211> 9
<212> PRT
<213> Homo sapien

<400> 212

Ser Gly Gln Ala Arg Met Phe Pro Asn
1 5

<210> 213
<211> 9
<212> PRT
<213> Homo sapien

<400> 213

Ser His His Ala Ala Gln Phe Pro Asn
1 5

<210> 214
<211> 9
<212> PRT
<213> Homo sapien

<400> 214

Ser Leu Gly Glu Gln Gln Tyr Ser Val
1 5

<210> 215
<211> 9
<212> PRT
<213> Homo sapien

<400> 215

Ser Leu Gly Gly Gly Gly Gly Cys Ala
1 5

<210> 216
<211> 9
<212> PRT
<213> Homo sapien

<400> 216

Ser Gln Ala Ser Ser Gly Gln Ala Arg
1 5

<210> 217
<211> 9
<212> PRT

<213> Homo sapien

<400> 217

Ser Ser Asp Asn Leu Tyr Gln Met Thr
1 5

<210> 218
<211> 9
<212> PRT
<213> Homo sapien

<400> 218

Ser Val Pro Pro Pro Val Tyr Gly Cys
1 5

<210> 219
<211> 9
<212> PRT
<213> Homo sapien

<400> 219

Thr Cys Gln Arg Lys Phe Ser Arg Ser
1 5

<210> 220
<211> 9
<212> PRT
<213> Homo sapien

<400> 220

Thr Asp Ser Cys Thr Gly Ser Gln Ala
1 5

<210> 221
<211> 9
<212> PRT
<213> Homo sapien

<400> 221

Thr Glu Gly Gln Ser Asn His Ser Thr
1 5

<210> 222
<211> 9
<212> PRT
<213> Homo sapien

<400> 222

Thr Gly Lys Thr Ser Glu Lys Pro Phe
1 5

<210> 223
<211> 9
<212> PRT
<213> Homo sapien

<400> 223

Thr Gly Ser Gln Ala Leu Leu Leu Arg
1 5

<210> 224
<211> 9
<212> PRT
<213> Homo sapien

<400> 224

Thr Gly Thr Ala Gly Ala Cys Arg Tyr
1 5

<210> 225
<211> 9
<212> PRT
<213> Homo sapien

<400> 225

Thr Gly Tyr Glu Ser Asp Asn His Thr
1 5

<210> 226
<211> 9
<212> PRT
<213> Homo sapien

<400> 226

Thr Leu Val Arg Ser Ala Ser Glu Thr
1 5

<210> 227
<211> 9
<212> PRT
<213> Homo sapien

<400> 227

Thr Pro Ile Leu Cys Gly Ala Gln Tyr
1 5

<210> 228
<211> 9
<212> PRT
<213> Homo sapien

<400> 228

Thr Pro Ser His His Ala Ala Gln Phe
1 5

<210> 229
<211> 9
<212> PRT
<213> Homo sapien

<400> 229

Thr Pro Ser Tyr Gly His Thr Pro Ser
1 5

<210> 230
<211> 9
<212> PRT
<213> Homo sapien

<400> 230

Thr Pro Thr Asp Ser Cys Thr Gly Ser
1 5

<210> 231
<211> 9
<212> PRT
<213> Homo sapien

<400> 231

Thr Pro Tyr Ser Ser Asp Asn Leu Tyr
1 5

<210> 232
<211> 9
<212> PRT
<213> Homo sapien

<400> 232

Thr Ser Glu Lys Pro Phe Ser Cys Arg
1 5

<210> 233
<211> 9
<212> PRT
<213> Homo sapien

<400> 233

Thr Ser Glu Lys Arg Pro Phe Met Cys
1 5

<210> 234
<211> 9
<212> PRT
<213> Homo sapien

<400> 234

Thr Ser Gln Leu Glu Cys Met Thr Trp
1 5

<210> 235
<211> 9
<212> PRT
<213> Homo sapien

<400> 235

Thr Val His Phe Ser Gly Gln Phe Thr
1 5

<210> 236
<211> 9
<212> PRT
<213> Homo sapien

<400> 236

Val Ala Ala Gly Ser Ser Ser Ser Val

1 5

<210> 237
<211> 9
<212> PRT
<213> Homo sapien

<400> 237

Val Ala Pro Thr Leu Val Arg Ser Ala
1 5

<210> 238

<211> 9
<212> PRT
<213> Homo sapien

<400> 238

Val Phe Arg Gly Ile Gln Asp Val Arg
1 5

<210> 239
<211> 9
<212> PRT
<213> Homo sapien

<400> 239

Val Lys Pro Phe Gln Cys Lys Thr Cys
1 5

<210> 240
<211> 9
<212> PRT
<213> Homo sapien

<400> 240

Val Lys Trp Thr Glu Gly Gln Ser Asn
1 5

<210> 241
<211> 9
<212> PRT
<213> Homo sapien

<400> 241

Val Leu Asp Phe Ala Pro Pro Gly Ala
1 5

<210> 242
<211> 9
<212> PRT
<213> Homo sapien

<400> 242

Val Pro Gly Val Ala Pro Thr Leu Val
1 5

<210> 243
<211> 9
<212> PRT
<213> Homo sapien

<400> 243

Val Arg His His Asn Met His Gln Arg
1 5

<210> 244
<211> 9
<212> PRT
<213> Homo sapien

<400> 244

Val Thr Phe Asp Gly Thr Pro Ser Tyr
1 5

<210> 245
<211> 9
<212> PRT
<213> Homo sapien

<400> 245

Trp Asn Gln Met Asn Leu Gly Ala Thr
1 5

<210> 246
<211> 9
<212> PRT
<213> Homo sapien

<400> 246

Trp Pro Ser Cys Gln Lys Lys Phe Ala
1 5

<210> 247
<211> 9
<212> PRT
<213> Homo sapien

<400> 247

Trp Thr Glu Gly Gln Ser Asn His Ser
1 5

<210> 248
<211> 9
<212> PRT
<213> Homo sapien

<400> 248

Tyr Phe Lys Leu Ser His Leu Gln Met
1 5

<210> 249
<211> 9
<212> PRT
<213> Homo sapien

<400> 249

Tyr Gly His Thr Pro Ser His His Ala
1 5

<210> 250
<211> 9
<212> PRT
<213> Homo sapien

<400> 250

Tyr Pro Gly Cys Asn Lys Arg Tyr Phe
1 5

<210> 251
<211> 9
<212> PRT
<213> Homo sapien

<400> 251

Tyr Gln Met Thr Ser Gln Leu Glu Cys
1 5

<210> 252
<211> 9
<212> PRT
<213> Homo sapien

<400> 252

Tyr Arg Ile His Thr His Gly Val Phe
1 5

<210> 253
<211> 9
<212> PRT
<213> Homo sapien

<400> 253

Tyr Ser Ser Asp Asn Leu Tyr Gln Met
1 5

<210> 254
<211> 9
<212> PRT
<213> Mus musculus

<400> 254

Ala Glu Pro His Glu Glu Gln Cys Leu
1 5

<210> 255
<211> 9
<212> PRT
<213> Mus musculus

<400> 255

Ala Leu Leu Pro Ala Val Ser Ser Leu
1 5

<210> 256
<211> 9
<212> PRT
<213> Mus musculus

<400> 256

Ala Tyr Gly Ser Leu Gly Gly Pro Ala
1 5

<210> 257
<211> 9
<212> PRT
<213> Mus musculus

<400> 257

Ala Tyr Pro Gly Cys Asn Lys Arg Tyr
1 5

<210> 258
<211> 9
<212> PRT
<213> Mus musculus

<400> 258

Cys Met Thr Trp Asn Gln Met Asn Leu
1 5

<210> 259
<211> 9
<212> PRT
<213> Mus musculus

<400> 259

Cys Thr Gly Ser Gln Ala Leu Leu Leu
1 5

<210> 260
<211> 9
<212> PRT
<213> Mus musculus

<400> 260

Asp Gly Ala Pro Ser Tyr Gly His Thr
1 5

<210> 261
<211> 9
<212> PRT
<213> Mus musculus

<400> 261

Asp Leu Asn Ala Leu Leu Pro Ala Val
1 5

<210> 262
<211> 9
<212> PRT
<213> Mus musculus

<400> 262

Asp Pro Met Gly Gln Gln Gly Ser Leu
1 5

<210> 263
<211> 9
<212> PRT
<213> Mus musculus

<400> 263

Asp Ser Cys Thr Gly Ser Gln Ala Leu
1 5

<210> 264
<211> 9
<212> PRT

<213> Mus musculus

<400> 264

Asp Val Arg Asp Leu Asn Ala Leu Leu
1 5

<210> 265
<211> 9
<212> PRT
<213> Mus musculus

<400> 265

Glu Gln Cys Leu Ser Ala Phe Thr Leu
1 5

<210> 266
<211> 9
<212> PRT
<213> Mus musculus

<400> 266

Glu Ser Asp Asn His Thr Ala Pro Ile
1 5

<210> 267
<211> 9
<212> PRT
<213> Mus musculus

<400> 267

Phe Pro Asn Ala Pro Tyr Leu Pro Ser
1 5

<210> 268
<211> 9
<212> PRT
<213> Mus musculus

<400> 268

Gly Cys Asn Lys Arg Tyr Phe Lys Leu
1 5

<210> 269
<211> 9
<212> PRT
<213> Mus musculus

<400> 269

Gly Gln Ala Arg Met Phe Pro Asn Ala
1 5

<210> 270
<211> 9
<212> PRT
<213> Mus musculus

<400> 270

Gly Val Phe Arg Gly Ile Gln Asp Val
1 5

<210> 271
<211> 9
<212> PRT
<213> Mus musculus

<400> 271

Gly Tyr Glu Ser Asp Asn His Thr Ala
1 5

<210> 272
<211> 9
<212> PRT
<213> Mus musculus

<400> 272

His Ser Phe Lys His Glu Asp Pro Met
1 5

<210> 273
<211> 9
<212> PRT
<213> Mus musculus

<400> 273

His Thr His Gly Val Phe Arg Gly Ile
1 5

<210> 274
<211> 9
<212> PRT
<213> Mus musculus

<400> 274

Ile Leu Cys Gly Ala Gln Tyr Arg Ile
1 5

<210> 275
<211> 9
<212> PRT
<213> Mus musculus

<400> 275

Lys Phe Ala Arg Ser Asp Glu Leu Val
1 5

<210> 276
<211> 9
<212> PRT
<213> Mus musculus

<400> 276

Lys Arg Tyr Phe Lys Leu Ser His Leu
1 5

<210> 277
<211> 9
<212> PRT
<213> Mus musculus

<400> 277

Lys Thr Ser Glu Lys Pro Phe Ser Cys
1 5

<210> 278
<211> 9
<212> PRT
<213> Mus musculus

<400> 278

Leu Glu Cys Met Thr Trp Asn Gln Met
1 5

<210> 279
<211> 9
<212> PRT
<213> Mus musculus

<400> 279

Leu Gly Gly Gly Gly Gly Cys Gly Leu
1 5

<210> 280
<211> 9
<212> PRT

<213> Mus musculus

<400> 280

Leu Gln Met His Ser Arg Lys His Thr
1 5

<210> 281
<211> 9
<212> PRT
<213> Mus musculus

<400> 281

Met His Gln Arg Asn Met Thr Lys Leu
1 5

<210> 282
<211> 9
<212> PRT
<213> Mus musculus

<400> 282

Asn Ala Pro Tyr Leu Pro Ser Cys Leu
1 5

<210> 283
<211> 9
<212> PRT
<213> Mus musculus

<400> 283

Asn Leu Gly Ala Thr Leu Lys Gly Met
1 5

<210> 284
<211> 9
<212> PRT
<213> Mus musculus

<400> 284

Asn Leu Tyr Gln Met Thr Ser Gln Leu
1 5

<210> 285
<211> 9
<212> PRT
<213> Mus musculus

<400> 285

Asn Met Thr Lys Leu His Val Ala Leu
1 5

<210> 286
<211> 9
<212> PRT
<213> Mus musculus

<400> 286

Asn Gln Met Asn Leu Gly Ala Thr Leu
1 5

<210> 287
<211> 9
<212> PRT
<213> Mus musculus

<400> 287

Pro Gly Ala Ser Ala Tyr Gly Ser Leu
1 5

<210> 288
<211> 9
<212> PRT
<213> Mus musculus

<400> 288

Gln Ala Ser Ser Gly Gln Ala Arg Met
1 5

<210> 289
<211> 9
<212> PRT
<213> Mus musculus

<400> 289

Gln Met Thr Ser Gln Leu Glu Cys Met
1 5

<210> 290
<211> 9
<212> PRT
<213> Mus musculus

<400> 290

Gln Gln Tyr Ser Val Pro Pro Pro Val
1 5

<210> 291
<211> 9
<212> PRT
<213> Mus musculus

<400> 291

Gln Tyr Arg Ile His Thr His Gly Val
1 5

<210> 292
<211> 9
<212> PRT
<213> Mus musculus

<400> 292

Gln Tyr Ser Val Pro Pro Pro Val Tyr
1 5

<210> 293
<211> 9
<212> PRT
<213> Mus musculus

<400> 293

Arg Met Phe Pro Asn Ala Pro Tyr Leu
1 5

<210> 294
<211> 9
<212> PRT
<213> Mus musculus

<400> 294

Arg Thr Pro Tyr Ser Ser Asp Asn Leu
1 5

<210> 295
<211> 9
<212> PRT
<213> Mus musculus

<400> 295

Arg Val Ser Gly Val Ala Pro Thr Leu
1 5

<210> 296
<211> 9
<212> PRT
<213> Mus musculus

<400> 296

Ser Cys Leu Glu Ser Gln Pro Thr Ile
1 5

<210> 297
<211> 9
<212> PRT
<213> Mus musculus

<400> 297

Ser Cys Gln Lys Lys Phe Ala Arg Ser
1 5

<210> 298
<211> 9
<212> PRT
<213> Mus musculus

<400> 298

Ser Asp Val Arg Asp Leu Asn Ala Leu
1 5

<210> 299
<211> 9
<212> PRT
<213> Mus musculus

<400> 299

Ser Leu Gly Glu Gln Gln Tyr Ser Val
1 5

<210> 300
<211> 9
<212> PRT
<213> Mus musculus

<400> 300

Thr Cys Gln Arg Lys Phe Ser Arg Ser
1 5

<210> 301
<211> 9
<212> PRT

<213> Mus musculus

<400> 301

Thr Glu Gly Gln Ser Asn His Gly Ile
1 5

<210> 302
<211> 9
<212> PRT
<213> Mus musculus

<400> 302

Thr Leu His Phe Ser Gly Gln Phe Thr
1 5

<210> 303
<211> 9
<212> PRT
<213> Mus musculus

<400> 303

Thr Leu Val Arg Ser Ala Ser Glu Thr
1 5

<210> 304
<211> 9
<212> PRT
<213> Mus musculus

<400> 304

Val Leu Asp Phe Ala Pro Pro Gly Ala
1 5

<210> 305
<211> 9
<212> PRT
<213> Mus musculus

<400> 305

Trp Asn Gln Met Asn Leu Gly Ala Thr
1 5

<210> 306
<211> 9
<212> PRT
<213> Mus musculus

<400> 306

Tyr Phe Lys Leu Ser His Leu Gln Met
1 5

<210> 307
<211> 9
<212> PRT
<213> Mus musculus

<400> 307

Tyr Gln Met Thr Ser Gln Leu Glu Cys
1 5

<210> 308
<211> 9
<212> PRT
<213> Mus musculus

<400> 308

Tyr Ser Ser Asp Asn Leu Tyr Gln Met
1 5

<210> 309
<211> 6
<212> PRT
<213> Homo sapien

<400> 309

Gly Ala Ala Gln Trp Ala
1 5

<210> 310
<211> 12
<212> PRT
<213> Homo sapien

<400> 310

Ala Ser Ala Tyr Gly Ser Leu Gly Gly Pro Ala Pro
1 5 10

<210> 311
<211> 15
<212> PRT
<213> Homo sapien

<400> 311

Ala Phe Thr Val His Phe Ser Gly Gln Phe Thr Gly Thr Ala Gly
1 5 10 15

<210> 312
<211> 5
<212> PRT
<213> Homo sapien

<400> 312

His Ala Ala Gln Phe
1 5

<210> 313
<211> 32
<212> PRT
<213> Homo sapien

<400> 313

Cys His Thr Pro Thr Asp Ser Cys Thr Gly Ser Gln Ala Leu Leu Leu
1 5 10 15
Arg Thr Pro Tyr Ser Ser Asp Asn Leu Tyr Gln Met Thr Ser Gln Leu
20 25 30

<210> 314
<211> 32
<212> PRT
<213> Homo sapien

<400> 314

Arg Ile His Thr His Gly Val Phe Arg Gly Ile Gln Asp Val Arg Arg
1 5 10 15
Val Pro Gly Val Ala Pro Thr Leu Val Arg Ser Ala Ser Glu Thr Ser
20 25 30

<210> 315
<211> 4
<212> PRT
<213> Homo sapien

<400> 315

Arg Tyr Phe Lys
1

<210> 316
<211> 14
<212> PRT
<213> Homo sapien

<400> 316

Glu Arg Arg Phe Ser Arg Ser Asp Gln Leu Lys Arg His Gln
1 5 10

<210> 317
<211> 22
<212> PRT
<213> Homo sapien

<400> 317

Gln Arg Lys Phe Ser Arg Ser Asp His Leu Lys Thr His Thr Arg Thr
1 5 10 15
His Thr Gly Lys Thr Ser
20

<210> 318
<211> 21
<212> PRT
<213> Homo sapien

<400> 318

Cys Gln Lys Lys Phe Ala Arg Ser Asp Glu Leu Val Arg His His Asn
1 5 10 15
Met His Gln Arg Asn
20

<210> 319
<211> 449
<212> PRT
<213> Homo sapien

<400> 319

```
Met Gly Ser Asp Val Arg Asp Leu Asn Ala Leu Leu Pro Ala Val Pro
 1               5                  10                  15
Ser Leu Gly Gly Gly Gly Gly Cys Ala Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Val His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Phe
                165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Val Ala Ala Gly Ser Ser Ser
                245                 250                 255
Ser Val Lys Trp Thr Glu Gly Gln Ser Asn His Ser Thr Gly Tyr Glu
            260                 265                 270
Ser Asp Asn His Thr Thr Pro Ile Leu Cys Gly Ala Gln Tyr Arg Ile
        275                 280                 285
His Thr His Gly Val Phe Arg Gly Ile Gln Asp Val Arg Arg Val Pro
    290                 295                 300
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
                325                 330                 335
Leu Ser His Leu Gln Met His Ser Arg Lys His Thr Gly Glu Lys Pro
            340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
                405                 410                 415
Arg Trp Pro Ser Cys Gln Lys Lys Phe Ala Arg Ser Asp Glu Leu Val
            420                 425                 430
Arg His His Asn Met His Gln Arg Asn Met Thr Lys Leu Gln Leu Ala
        435                 440                 445
Leu
```

<210> 320

<211> 449
<212> PRT
<213> Mus musculus

<400> 320

```
Met Gly Ser Asp Val Arg Asp Leu Asn Ala Leu Leu Pro Ala Val Ser
 1               5                  10                  15
Ser Leu Gly Gly Gly Gly Gly Cys Gly Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Leu His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Thr Ile
    130                 135                 140
Arg Asn Gln Gly Tyr Ser Thr Val Thr Phe Asp Gly Ala Pro Ser Tyr
145                 150                 155                 160
Gly His Thr Pro Ser His His Ala Ala Gln Phe Pro Asn His Ser Phe
                165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Met Ala Ala Gly Ser Ser Ser
                245                 250                 255
Ser Val Lys Trp Thr Glu Gly Gln Ser Asn His Gly Ile Gly Tyr Glu
            260                 265                 270
Ser Asp Asn His Thr Ala Pro Ile Leu Cys Gly Ala Gln Tyr Arg Ile
        275                 280                 285
His Thr His Gly Val Phe Arg Gly Ile Gln Asp Val Arg Arg Val Ser
    290                 295                 300
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
                325                 330                 335
Leu Ser His Leu Gln Met His Ser Arg Lys His Thr Gly Glu Lys Pro
            340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
```

```
                405                 410                 415
Arg Trp His Ser Cys Gln Lys Lys Phe Ala Arg Ser Asp Glu Leu Val
            420                 425                 430
Arg His His Asn Met His Gln Arg Asn Met Thr Lys Leu His Val Ala
        435                 440                 445
Leu
```

<210> 321
<211> 9
<212> PRT
<213> Homo sapien and Mus musculus

<400> 321

```
Pro Ser Gln Ala Ser Ser Gly Gln Ala
 1               5
```

<210> 322
<211> 9
<212> PRT
<213> Homo sapien and Mus musculus

<400> 322

```
Ser Ser Gly Gln Ala Arg Met Phe Pro
 1               5
```

<210> 323
<211> 9
<212> PRT
<213> Homo sapien and Mus musculus

<400> 323

```
Gln Ala Arg Met Phe Pro Asn Ala Pro
 1               5
```

<210> 324
<211> 9
<212> PRT
<213> Homo sapien and Mus musculus

<400> 324

```
Met Phe Pro Asn Ala Pro Tyr Leu Pro
 1               5
```

<210> 325
<211> 9
<212> PRT
<213> Homo sapien and Mus musculus

<400> 325

Pro Asn Ala Pro Tyr Leu Pro Ser Cys
1 5

<210> 326
<211> 9
<212> PRT
<213> Homo sapien and Mus musculus

<400> 326

Ala Pro Tyr Leu Pro Ser Cys Leu Glu
1 5

<210> 327
<211> 1029
<212> DNA
<213> Homo sapiens

<400> 327

```
atgcagcatc accaccatca ccacatgagc gataaaatta ttcacctgac tgacgacagt 60
tttgacacgg atgtactcaa agcggacggg gcgatcctcg tcgatttctg ggcagagtgg 120
tgcggtccgt gcaaaatgat cgccccgatt ctggatgaaa tcgctgacga atatcagggc 180
aaactgaccg ttgcaaaact gaacatcgat caaaaccctg gcactgcgcc gaaatatggc 240
atccgtggta tcccgactct gctgctgttc aaaaacggtg aagtggcggc aaccaaagtg 300
ggtgcactgt ctaaaggtca gttgaaagag ttcctcgacg ctaacctggc cggttctggt 360
tctggccata tgcagcatca ccaccatcac cacgtgtcta tcgaaggtcg tgctagctct 420
ggtggcagcg gtctggttcc gcgtggtagc tctggttcgg gggacgacga cgacaaatct 480
agtaggcaca gcacagggta cgagagcgat aaccacacaa cgcccatcct ctgcggagcc 540
caatacagaa tacacacgca cggtgtcttc agaggcattc aggatgtgcg acgtgtgcct 600
ggagtagccc cgactcttgt acggtcggca tctgagacca gtgagaaacg ccccttcatg 660
tgtgcttacc caggctgcaa taagagatat tttaagctgt cccacttaca gatgcacagc 720
aggaagcaca ctggtgagaa accataccag tgtgacttca aggactgtga acgaaggttt 780
tttcgttcag accagctcaa aagacaccaa aggagacata caggtgtgaa accattccag 840
tgtaaaactt gtcagcgaaa gttctcccgg tccgaccacc tgaagaccca caccaggact 900
catacaggtg aaaagccctt cagctgtcgg tggccaagtt gtcagaaaaa gtttgcccgg 960
tcagatgaat tagtccgcca tcacaacatg catcagagaa acatgaccaa actccagctg 1020
gcgctttga                                                         1029
```

<210> 328
<211> 1233
<212> DNA
<213> Homo sapiens

<400> 328

```
atgcagcatc accaccatca ccacatgagc gataaaatta ttcacctgac tgacgacagt 60
tttgacacgg atgtactcaa agcggacggg gcgatcctcg tcgatttctg ggcagagtgg 120
tgcggtccgt gcaaaatgat cgccccgatt ctggatgaaa tcgctgacga atatcagggc 180
aaactgaccg ttgcaaaact gaacatcgat caaaaccctg gcactgcgcc gaaatatggc 240
atccgtggta tcccgactct gctgctgttc aaaaacggtg aagtggcggc aaccaaagtg 300
ggtgcactgt ctaaaggtca gttgaaagag ttcctcgacg ctaacctggc cggttctggt 360
tctggccata tgcagcatca ccaccatcac cacgtgtcta tcgaaggtcg tgctagctct 420
ggtggcagcg gtctggttcc gcgtggtagc tctggttcgg gggacgacga cgacaaatct 480
agtaggggct ccgacgttcg tgacctgaac gcactgctgc cggcagttcc gtccctgggt 540
ggtggtggtg gttgcgcact gccggttagc ggtgcagcac agtgggctcc ggttctggac 600
ttcgcaccgc cgggtgcatc cgcatacggt tccctgggtg gtccggcacc gccgccggca 660
ccgccgccgc cgccgccgcc gccgccgcac tccttcatca aacaggaacc gagctggggt 720
ggtgcagaac cgcacgaaga acagtgcctg agcgcattca ccgttcactt ctccggccag 780
ttcactggca cagccggagc ctgtcgctac gggcccttcg gtcctcctcc gcccagccag 840
gcgtcatccg gccaggccag gatgtttcct aacgcgccct acctgcccag ctgcctcgag 900
agccagcccg ctattcgcaa tcagggttac agcacggtca ccttcgacgg gacgcccagc 960
tacggtcaca cgccctcgca ccatgcggcg cagttcccca accactcatt caagcatgag 1020
gatcccatgg gccagcaggg ctcgctgggt gagcagcagt actcggtgcc gcccccggtc 1080
tatggctgcc acacccccac cgacagctgc accggcagcc aggctttgct gctgaggacg 1140
ccctacagca gtgacaattt ataccaaatg acatcccagc ttgaatgcat gacctggaat 1200
cagatgaact taggagccac cttaaagggc tga                              1233
```

<210> 329
<211> 1776
<212> DNA
<213> Homo sapiens

<400> 329

```
atgcagcatc accaccatca ccacatgagc gataaaatta ttcacctgac tgacgacagt 60
tttgacacgg atgtactcaa agcggacggg gcgatcctcg tcgatttctg ggcagagtgg 120
tgcggtccgt gcaaaatgat cgccccgatt ctggatgaaa tcgctgacga atatcagggc 180
aaactgaccg ttgcaaaact gaacatcgat caaaaccctg gcactgcgcc gaaatatggc 240
atccgtggta tcccgactct gctgctgttc aaaaacggtg aagtggcggc aaccaaagtg 300
ggtgcactgt ctaaaggtca gttgaaagag ttcctcgacg ctaacctggc cggttctggt 360
tctggccata tgcagcatca ccaccatcac cacgtgtcta tcgaaggtcg tgctagctct 420
ggtggcagcg gtctggttcc gcgtggtagc tctggttcgg gggacgacga cgacaaatct 480
agtaggatgg gctccgacgt tcgtgacctg aacgcactgc tgccggcagt tccgtccctg 540
ggtggtggtg gtggttgcgc actgccggtt agcggtgcag cacagtgggc tccggttctg 600
gacttcgcac cgccgggtgc atccgcatac ggttccctgg gtggtccggc accgccgccg 660
gcaccgccgc cgccgccgcc gccgccgccg cactccttca tcaaacagga accgagctgg 720
ggtggtgcag aaccgcacga agaacagtgc ctgagcgcat tcaccgttca cttctccggc 780
cagttcactg gcacagccgg agcctgtcgc tacgggccct tcggtcctcc tccgcccagc 840
caggcgtcat ccggccaggc caggatgttt cctaacgcgc cctacctgcc cagctgcctc 900
gagagccagc ccgctattcg caatcagggt tacagcacgg tcaccttcga cgggacgccc 960
agctacggtc acacgccctc gcaccatgcg gcgcagttcc ccaaccactc attcaagcat 1020
gaggatccca tgggccagca gggctcgctg ggtgagcagc agtactcggt gccgcccccg 1080
gtctatggct gccacacccc caccgacagc tgcaccggca gccaggcttt gctgctgagg 1140
acgccctaca gcagtgacaa tttataccaa atgacatccc agcttgaatg catgacctgg 1200
aatcagatga acttaggagc caccttaaag ggccacagca cagggtacga gagcgataac 1260
cacacaacgc ccatcctctg cggagcccaa tacagaatac acacgcacgg tgtcttcaga 1320
ggcattcagg atgtgcgacg tgtgcctgga gtagccccga ctcttgtacg gtcggcatct 1380
gagaccagtg agaaacgccc cttcatgtgt gcttacccag gctgcaataa gagatatttt 1440
aagctgtccc acttacagat gcacagcagg aagcacactg gtgagaaacc ataccagtgt 1500
gacttcaagg actgtgaacg aaggtttttt cgttcagacc agctcaaaag acaccaaagg 1560
agacatacag gtgtgaaacc attccagtgt aaaacttgtc agcgaaagtt ctcccggtcc 1620
gaccacctga agacccacac caggactcat acaggtgaaa agcccttcag ctgtcggtgg 1680
ccaagttgtc agaaaaagtt tgcccggtca gatgaattag tccgccatca caacatgcat 1740
cagagaaaca tgaccaaact ccagctggcg ctttga                            1776
```

<210> 330
<211> 771
<212> DNA
<213> Homo sapiens

<400> 330

```
atgcagcatc accaccatca ccacggctcc gacgttcgtg acctgaacgc actgctgccg 60
gcagttccgt ccctgggtgg tggtggtggt tgcgcactgc cggttagcgg tgcagcacag 120
tgggctccgg ttctggactt cgcaccgccg ggtgcatccg catacggttc cctgggtggt 180
ccggcaccgc cgccggcacc gccgccgccg ccgccgccgc cgccgcactc cttcatcaaa 240
caggaaccga gctggggtgg tgcagaaccg cacgaagaac agtgcctgag cgcattcacc 300
gttcacttct ccggccagtt cactggcaca gccggagcct gtcgctacgg gcccttcggt 360
cctcctccgc ccagccaggc gtcatccggc caggccagga tgtttcctaa cgcgccctac 420
ctgcccagct gcctcgagag ccagcccgct attcgcaatc agggttacag cacggtcacc 480
ttcgacggga cgcccagcta cggtcacacg ccctcgcacc atgcggcgca gttccccaac 540
cactcattca agcatgagga tcccatgggc cagcagggct cgctgggtga gcagcagtac 600
tcggtgccgc ccccggtcta tggctgccac acccccaccg acagctgcac cggcagccag 660
gctttgctgc tgaggacgcc ctacagcagt gacaatttat accaaatgac atcccagctt 720
gaatgcatga cctggaatca gatgaactta ggagccacct taaagggctg a          771
```

<210> 331
<211> 567
<212> DNA
<213> Homo sapiens

<400> 331

```
atgcagcatc accaccatca ccaccacagc acagggtacg agagcgataa ccacacaacg 60
cccatcctct gcggagccca atacagaata cacacgcacg gtgtcttcag aggcattcag 120
gatgtgcgac gtgtgcctgg agtagccccg actcttgtac ggtcggcatc tgagaccagt 180
gagaaacgcc ccttcatgtg tgcttaccca ggctgcaata agagatattt taagctgtcc 240
cacttacaga tgcacagcag gaagcacact ggtgagaaac cataccagtg tgacttcaag 300
gactgtgaac gaaggttttt tcgttcagac cagctcaaaa gacaccaaag gagacataca 360
ggtgtgaaac cattccagtg taaaacttgt cagcgaaagt tctcccggtc cgaccacctg 420
aagacccaca ccaggactca tacaggtgaa aagcccttca gctgtcggtg gccaagttgt 480
cagaaaaagt ttgcccggtc agatgaatta gtccgccatc acaacatgca tcagagaaac 540
atgaccaaac tccagctggc gctttga                                     567
```

<210> 332
<211> 342
<212> PRT
<213> Homo sapiens

<400> 332

```
Met Gln His His His His His His Met Ser Asp Lys Ile Ile His Leu
                    5                   10                  15
Thr Asp Asp Ser Phe Asp Thr Asp Val Leu Lys Ala Asp Gly Ala Ile
            20                  25                  30
Leu Val Asp Phe Trp Ala Glu Trp Cys Gly Pro Cys Lys Met Ile Ala
        35                  40                  45
Pro Ile Leu Asp Glu Ile Ala Asp Glu Tyr Gln Gly Lys Leu Thr Val
    50                  55                  60
Ala Lys Leu Asn Ile Asp Gln Asn Pro Gly Thr Ala Pro Lys Tyr Gly
65                  70                  75                  80
Ile Arg Gly Ile Pro Thr Leu Leu Leu Phe Lys Asn Gly Glu Val Ala
                85                  90                  95
Ala Thr Lys Val Gly Ala Leu Ser Lys Gly Gln Leu Lys Glu Phe Leu
            100                 105                 110
Asp Ala Asn Leu Ala Gly Ser Gly Ser Gly His Met Gln His His His
        115                 120                 125
His His His Val Ser Ile Glu Gly Arg Ala Ser Ser Gly Gly Ser Gly
    130                 135                 140
Leu Val Pro Arg Gly Ser Ser Gly Ser Gly Asp Asp Asp Asp Lys Ser
145                 150                 155                 160
Ser Arg His Ser Thr Gly Tyr Glu Ser Asp Asn His Thr Thr Pro Ile
                165                 170                 175
Leu Cys Gly Ala Gln Tyr Arg Ile His Thr His Gly Val Phe Arg Gly
            180                 185                 190
Ile Gln Asp Val Arg Arg Val Pro Gly Val Ala Pro Thr Leu Val Arg
        195                 200                 205
Ser Ala Ser Glu Thr Ser Glu Lys Arg Pro Phe Met Cys Ala Tyr Pro
    210                 215                 220
Gly Cys Asn Lys Arg Tyr Phe Lys Leu Ser His Leu Gln Met His Ser
225                 230                 235                 240
Arg Lys His Thr Gly Glu Lys Pro Tyr Gln Cys Asp Phe Lys Asp Cys
                245                 250                 255
Glu Arg Arg Phe Phe Arg Ser Asp Gln Leu Lys Arg His Gln Arg Arg
```

```
                260                     265                     270
His Thr Gly Val Lys Pro Phe Gln Cys Lys Thr Cys Gln Arg Lys Phe
            275                     280                     285
Ser Arg Ser Asp His Leu Lys Thr His Thr Arg Thr His Thr Gly Glu
        290                     295                     300
Lys Pro Phe Ser Cys Arg Trp Pro Ser Cys Gln Lys Lys Phe Ala Arg
305                     310                     315                     320
Ser Asp Glu Leu Val Arg His His Asn Met His Gln Arg Asn Met Thr
                    325                     330                     335
Lys Leu Gln Leu Ala Leu
            340
```

<210> 333
<211> 410
<212> PRT
<213> Homo sapiens

<400> 333

```
Met Gln His His His His His His Met Ser Asp Lys Ile Ile His Leu
1               5                   10                  15
Thr Asp Asp Ser Phe Asp Thr Asp Val Leu Lys Ala Asp Gly Ala Ile
            20                  25                  30
Leu Val Asp Phe Trp Ala Glu Trp Cys Gly Pro Cys Lys Met Ile Ala
        35                  40                  45
Pro Ile Leu Asp Glu Ile Ala Asp Glu Tyr Gln Gly Lys Leu Thr Val
    50                  55                  60
Ala Lys Leu Asn Ile Asp Gln Asn Pro Gly Thr Ala Pro Lys Tyr Gly
65                  70                  75                  80
Ile Arg Gly Ile Pro Thr Leu Leu Leu Phe Lys Asn Gly Glu Val Ala
                85                  90                  95
Ala Thr Lys Val Gly Ala Leu Ser Lys Gly Gln Leu Lys Glu Phe Leu
            100                 105                 110
Asp Ala Asn Leu Ala Gly Ser Gly Ser Gly His Met Gln His His His
        115                 120                 125
His His His Val Ser Ile Glu Gly Arg Ala Ser Ser Gly Gly Ser Gly
    130                 135                 140
Leu Val Pro Arg Gly Ser Ser Gly Ser Gly Asp Asp Asp Asp Lys Ser
145                 150                 155                 160
Ser Arg Gly Ser Asp Val Arg Asp Leu Asn Ala Leu Leu Pro Ala Val
                165                 170                 175
Pro Ser Leu Gly Gly Gly Gly Gly Cys Ala Leu Pro Val Ser Gly Ala
            180                 185                 190
Ala Gln Trp Ala Pro Val Leu Asp Phe Ala Pro Pro Gly Ala Ser Ala
        195                 200                 205
Tyr Gly Ser Leu Gly Gly Pro Ala Pro Pro Pro Ala Pro Pro Pro Pro
    210                 215                 220
Pro Pro Pro Pro Pro His Ser Phe Ile Lys Gln Glu Pro Ser Trp Gly
225                 230                 235                 240
Gly Ala Glu Pro His Glu Glu Gln Cys Leu Ser Ala Phe Thr Val His
                245                 250                 255
Phe Ser Gly Gln Phe Thr Gly Thr Ala Gly Ala Cys Arg Tyr Gly Pro
            260                 265                 270
Phe Gly Pro Pro Pro Pro Ser Gln Ala Ser Ser Gly Gln Ala Arg Met
        275                 280                 285
Phe Pro Asn Ala Pro Tyr Leu Pro Ser Cys Leu Glu Ser Gln Pro Ala
    290                 295                 300
```

```
Ile Arg Asn Gln Gly Tyr Ser Thr Val Thr Phe Asp Gly Thr Pro Ser
305                 310                 315                 320
Tyr Gly His Thr Pro Ser His His Ala Ala Gln Phe Pro Asn His Ser
                325                 330                 335
Phe Lys His Glu Asp Pro Met Gly Gln Gln Gly Ser Leu Gly Glu Gln
            340                 345                 350
Gln Tyr Ser Val Pro Pro Pro Val Tyr Gly Cys His Thr Pro Thr Asp
        355                 360                 365
Ser Cys Thr Gly Ser Gln Ala Leu Leu Leu Arg Thr Pro Tyr Ser Ser
    370                 375                 380
Asp Asn Leu Tyr Gln Met Thr Ser Gln Leu Glu Cys Met Thr Trp Asn
385                 390                 395                 400
Gln Met Asn Leu Gly Ala Thr Leu Lys Gly
                405                 410
```

<210> 334
<211> 591
<212> PRT
<213> Homo sapiens

<400> 334

```
Met Gln His His His His His His Met Ser Asp Lys Ile Ile His Leu
                5                   10                  15
Thr Asp Asp Ser Phe Asp Thr Asp Val Leu Lys Ala Asp Gly Ala Ile
            20                  25                  30
Leu Val Asp Phe Trp Ala Glu Trp Cys Gly Pro Cys Lys Met Ile Ala
        35                  40                  45
Pro Ile Leu Asp Glu Ile Ala Asp Glu Tyr Gln Gly Lys Leu Thr Val
    50                  55                  60
Ala Lys Leu Asn Ile Asp Gln Asn Pro Gly Thr Ala Pro Lys Tyr Gly
65                  70                  75                  80
Ile Arg Gly Ile Pro Thr Leu Leu Leu Phe Lys Asn Gly Glu Val Ala
                85                  90                  95
Ala Thr Lys Val Gly Ala Leu Ser Lys Gly Gln Leu Lys Glu Phe Leu
            100                 105                 110
Asp Ala Asn Leu Ala Gly Ser Gly Ser Gly His Met Gln His His His
        115                 120                 125
His His His Val Ser Ile Glu Gly Arg Ala Ser Ser Gly Gly Ser Gly
    130                 135                 140
Leu Val Pro Arg Gly Ser Ser Gly Ser Gly Asp Asp Asp Asp Lys Ser
145                 150                 155                 160
Ser Arg Met Gly Ser Asp Val Arg Asp Leu Asn Ala Leu Leu Pro Ala
                165                 170                 175
Val Pro Ser Leu Gly Gly Gly Gly Gly Cys Ala Leu Pro Val Ser Gly
            180                 185                 190
Ala Ala Gln Trp Ala Pro Val Leu Asp Phe Ala Pro Pro Gly Ala Ser
        195                 200                 205
Ala Tyr Gly Ser Leu Gly Gly Pro Ala Pro Pro Pro Ala Pro Pro Pro
    210                 215                 220
Pro Pro Pro Pro Pro Pro His Ser Phe Ile Lys Gln Glu Pro Ser Trp
225                 230                 235                 240
Gly Gly Ala Glu Pro His Glu Glu Gln Cys Leu Ser Ala Phe Thr Val
                245                 250                 255
His Phe Ser Gly Gln Phe Thr Gly Thr Ala Gly Ala Cys Arg Tyr Gly
            260                 265                 270
Pro Phe Gly Pro Pro Pro Pro Ser Gln Ala Ser Ser Gly Gln Ala Arg
        275                 280                 285
```

```
Met Phe Pro Asn Ala Pro Tyr Leu Pro Ser Cys Leu Glu Ser Gln Pro
    290                 295                 300
Ala Ile Arg Asn Gln Gly Tyr Ser Thr Val Thr Phe Asp Gly Thr Pro
305                 310                 315                 320
Ser Tyr Gly His Thr Pro Ser His His Ala Ala Gln Phe Pro Asn His
                325                 330                 335
Ser Phe Lys His Glu Asp Pro Met Gly Gln Gln Gly Ser Leu Gly Glu
            340                 345                 350
Gln Gln Tyr Ser Val Pro Pro Pro Val Tyr Gly Cys His Thr Pro Thr
        355                 360                 365
Asp Ser Cys Thr Gly Ser Gln Ala Leu Leu Leu Arg Thr Pro Tyr Ser
    370                 375                 380
Ser Asp Asn Leu Tyr Gln Met Thr Ser Gln Leu Glu Cys Met Thr Trp
385                 390                 395                 400
Asn Gln Met Asn Leu Gly Ala Thr Leu Lys Gly His Ser Thr Gly Tyr
                405                 410                 415
Glu Ser Asp Asn His Thr Thr Pro Ile Leu Cys Gly Ala Gln Tyr Arg
            420                 425                 430
Ile His Thr His Gly Val Phe Arg Gly Ile Gln Asp Val Arg Arg Val
        435                 440                 445
Pro Gly Val Ala Pro Thr Leu Val Arg Ser Ala Ser Glu Thr Ser Glu
    450                 455                 460
Lys Arg Pro Phe Met Cys Ala Tyr Pro Gly Cys Asn Lys Arg Tyr Phe
465                 470                 475                 480
Lys Leu Ser His Leu Gln Met His Ser Arg Lys His Thr Gly Glu Lys
                485                 490                 495
Pro Tyr Gln Cys Asp Phe Lys Asp Cys Glu Arg Arg Phe Phe Arg Ser
            500                 505                 510
Asp Gln Leu Lys Arg His Gln Arg Arg His Thr Gly Val Lys Pro Phe
        515                 520                 525
Gln Cys Lys Thr Cys Gln Arg Lys Phe Ser Arg Ser Asp His Leu Lys
    530                 535                 540
Thr His Thr Arg Thr His Thr Gly Glu Lys Pro Phe Ser Cys Arg Trp
545                 550                 555                 560
Pro Ser Cys Gln Lys Lys Phe Ala Arg Ser Asp Glu Leu Val Arg His
                565                 570                 575
His Asn Met His Gln Arg Asn Met Thr Lys Leu Gln Leu Ala Leu
            580                 585                 590
```

<210> 335
<211> 256
<212> PRT
<213> Homo sapiens

<400> 335

```
Met Gln His His His His His His Gly Ser Asp Val Arg Asp Leu Asn
                    5                  10                  15
Ala Leu Leu Pro Ala Val Pro Ser Leu Gly Gly Gly Gly Gly Cys Ala
            20                  25                  30
Leu Pro Val Ser Gly Ala Ala Gln Trp Ala Pro Val Leu Asp Phe Ala
        35                  40                  45
Pro Pro Gly Ala Ser Ala Tyr Gly Ser Leu Gly Gly Pro Ala Pro Pro
    50                  55                  60
Pro Ala Pro Pro Pro Pro Pro Pro Pro Pro Pro His Ser Phe Ile Lys
65                  70                  75                  80
Gln Glu Pro Ser Trp Gly Gly Ala Glu Pro His Glu Glu Gln Cys Leu
                85                  90                  95
Ser Ala Phe Thr Val His Phe Ser Gly Gln Phe Thr Gly Thr Ala Gly
            100                 105                 110
Ala Cys Arg Tyr Gly Pro Phe Gly Pro Pro Pro Pro Ser Gln Ala Ser
        115                 120                 125
Ser Gly Gln Ala Arg Met Phe Pro Asn Ala Pro Tyr Leu Pro Ser Cys
    130                 135                 140
Leu Glu Ser Gln Pro Ala Ile Arg Asn Gln Gly Tyr Ser Thr Val Thr
145                 150                 155                 160
Phe Asp Gly Thr Pro Ser Tyr Gly His Thr Pro Ser His His Ala Ala
                165                 170                 175
Gln Phe Pro Asn His Ser Phe Lys His Glu Asp Pro Met Gly Gln Gln
            180                 185                 190
Gly Ser Leu Gly Glu Gln Gln Tyr Ser Val Pro Pro Pro Val Tyr Gly
        195                 200                 205
Cys His Thr Pro Thr Asp Ser Cys Thr Gly Ser Gln Ala Leu Leu Leu
    210                 215                 220
Arg Thr Pro Tyr Ser Ser Asp Asn Leu Tyr Gln Met Thr Ser Gln Leu
225                 230                 235                 240
Glu Cys Met Thr Trp Asn Gln Met Asn Leu Gly Ala Thr Leu Lys Gly
                245                 250                 255
```

<210> 336
<211> 188
<212> PRT
<213> Homo sapiens

<400> 336

```
Met Gln His His His His His His His Ser Thr Gly Tyr Glu Ser Asp
                5                   10                  15
Asn His Thr Thr Pro Ile Leu Cys Gly Ala Gln Tyr Arg Ile His Thr
            20                  25                  30
His Gly Val Phe Arg Gly Ile Gln Asp Val Arg Arg Val Pro Gly Val
        35                  40                  45
Ala Pro Thr Leu Val Arg Ser Ala Ser Glu Thr Ser Glu Lys Arg Pro
    50                  55                  60
Phe Met Cys Ala Tyr Pro Gly Cys Asn Lys Arg Tyr Phe Lys Leu Ser
65                  70                  75                  80
His Leu Gln Met His Ser Arg Lys His Thr Gly Glu Lys Pro Tyr Gln
                85                  90                  95
Cys Asp Phe Lys Asp Cys Glu Arg Arg Phe Phe Arg Ser Asp Gln Leu
            100                 105                 110
Lys Arg His Gln Arg Arg His Thr Gly Val Lys Pro Phe Gln Cys Lys
        115                 120                 125
Thr Cys Gln Arg Lys Phe Ser Arg Ser Asp His Leu Lys Thr His Thr
    130                 135                 140
Arg Thr His Thr Gly Glu Lys Pro Phe Ser Cys Arg Trp Pro Ser Cys
145                 150                 155                 160
Gln Lys Lys Phe Ala Arg Ser Asp Glu Leu Val Arg His His Asn Met
                165                 170                 175
His Gln Arg Asn Met Thr Lys Leu Gln Leu Ala Leu
            180                 185
```

<210> 337
<211> 324
<212> DNA
<213> Homo sapiens

<400> 337

```
atgcagcatc accaccatca ccacggttcc gacgtgcggg acctgaacgc actgctgccg 60
gcagttccat ccctgggtgg cggtggaggc tgcgcactgc cggttagcgg tgcagcacag 120
tgggctccag ttctggactt cgcaccgcct ggtgcatccg catacggttc cctgggtggt 180
ccagcacctc cgcccgcaac gcccccaccg cctccaccgc ccccgcactc cttcatcaaa 240
caggaaccta gctggggtgg tgcagaaccg cacgaagaac agtgcctgag cgcattctga 300
gaattctgca gatatccatc acac                                        324
```

<210> 338
<211> 462
<212> DNA
<213> Homo sapiens

<400> 338

```
atgcagcatc accaccatca ccaccacgaa gaacagtgcc tgagcgcatt caccgttcac 60
ttctccggcc agttcactgg cacagccgga gcctgtcgct acgggccctt cggtcctcct 120
ccgcccagcc aggcgtcatc cggccaggcc aggatgtttc ctaacgcgcc ctacctgccc 180
agctgcctcg agagccagcc cgctattcgc aatcagggtt acagcacggt caccttcgac 240
gggacgccca gctacggtca cacgccctcg caccatgcgg cgcagttccc caaccactca 300
ttcaagcatg aggatcccat gggccagcag ggctcgctgg gtgagcagca gtactcggtg 360
ccgcccccgg tctatggctg ccacaccccc accgacagct gcaccggcag ccaggctttg 420
ctgctgagga cgccctacag cagtgacaat ttatactgat ga                     462
```

<210> 339

<211> 405
<212> DNA
<213> Homo sapiens

<400> 339

```
atgcagcatc accaccatca ccaccaggct ttgctgctga ggacgcccta cagcagtgac 60
aatttatacc aaatgacatc ccagcttgaa tgcatgacct ggaatcagat gaacttagga 120
gccaccttaa agggccacag cacagggtac gagagcgata accacacaac gcccatcctc 180
tgcggagccc aatacagaat acacacgcac ggtgtcttca gaggcattca ggatgtgcga 240
cgtgtgcctg gagtagcccc gactcttgta cggtcggcat ctgagaccag tgagaaacgc 300
cccttcatgt gtgcttaccc aggctgcaat aagagatatt ttaagctgtc ccacttacag 360
atgcacagca ggaagcacac tggtgagaaa ccataccagt gatga                405
```

<210> 340
<211> 339
<212> DNA
<213> Homo sapiens

<400> 340

```
atgcagcatc accaccatca ccaccacagc aggaagcaca ctggtgagaa accataccag 60
tgtgacttca aggactgtga acgaaggttt tttcgttcag accagctcaa aagacaccaa 120
aggagacata caggtgtgaa accattccag tgtaaaactt gtcagcgaaa gttctcccgg 180
tccgaccacc tgaagaccca caccaggact catacaggtg aaaagccctt cagctgtcgg 240
tggccaagtt gtcagaaaaa gtttgcccgg tcagatgaat tagtccgcca tcacaacatg 300
catcagagaa acatgaccaa actccagctg gcgctttga                       339
```

<210> 341
<211> 1110
<212> DNA
<213> Homo sapiens

<400> 341

```
atgcagcatc accaccatca ccaccactcc ttcatcaaac aggaaccgag ctggggtggt 60
gcagaaccgc acgaagaaca gtgcctgagc gcattcaccg ttcacttctc cggccagttc 120
actggcacag ccggagcctg tcgctacggg cccttcggtc ctcctccgcc cagccaggcg 180
tcatccggcc aggccaggat gtttcctaac gcgccctacc tgcccagctg cctcgagagc 240
cagcccgcta ttcgcaatca gggttacagc acggtcacct tcgacgggac gcccagctac 300
ggtcacacgc cctcgcacca tgcggcgcag ttccccaacc actcattcaa gcatgaggat 360
cccatgggcc agcagggctc gctgggtgag cagcagtact cggtgccgcc cccggtctat 420
ggctgccaca cccccaccga cagctgcacc ggcagccagg ctttgctgct gaggacgccc 480
tacagcagtg acaatttata ccaaatgaca tcccagcttg aatgcatgac ctggaatcag 540
atgaacttag gagccacctt aaagggccac agcacagggt acgagagcga taaccacaca 600
acgcccatcc tctgcggagc ccaatacaga atacacacgc acggtgtctt cagaggcatt 660
caggatgtgc gacgtgtgcc tggagtagcc ccgactcttg tacggtcggc atctgagacc 720
agtgagaaac gccccttcat gtgtgcttac ccaggctgca ataagagata ttttaagctg 780
tcccacttac agatgcacag caggaagcac actggtgaga aaccatacca gtgtgacttc 840
aaggactgtg aacgaaggtt ttttcgttca gaccagctca aaagacacca aaggagacat 900
acaggtgtga aaccattcca gtgtaaaact tgtcagcgaa agttctcccg gtccgaccac 960
ctgaagaccc acaccaggac tcatacaggt gaaaagccct tcagctgtcg gtggccaagt 1020
tgtcagaaaa agtttgcccg gtcagatgaa ttagtccgcc atcacaacat gcatcagaga 1080
aacatgacca aactccagct ggcgctttga                                  1110
```

<210> 342
<211> 99
<212> PRT
<213> Homo sapiens

<400> 342

```
Met Gln His His His His His His Gly Ser Asp Val Arg Asp Leu Asn
                5                   10                  15
Ala Leu Leu Pro Ala Val Pro Ser Leu Gly Gly Gly Gly Gly Cys Ala
            20                  25                  30
Leu Pro Val Ser Gly Ala Ala Gln Trp Ala Pro Val Leu Asp Phe Ala
        35                  40                  45
Pro Pro Gly Ala Ser Ala Tyr Gly Ser Leu Gly Gly Pro Ala Pro Pro
    50                  55                  60
Pro Ala Pro Pro Pro Pro Pro Pro Pro Pro Pro His Ser Phe Ile Lys
65                  70                  75                  80
Gln Glu Pro Ser Trp Gly Gly Ala Glu Pro His Glu Glu Gln Cys Leu
                85                  90                  95
Ser Ala Phe
```

<210> 343
<211> 152
<212> PRT
<213> Homo sapiens

<400> 343

```
Met Gln His His His His His His His Glu Glu Gln Cys Leu Ser Ala
                5                   10                  15
Phe Thr Val His Phe Ser Gly Gln Phe Thr Gly Thr Ala Gly Ala Cys
            20                  25                  30
Arg Tyr Gly Pro Phe Gly Pro Pro Pro Pro Ser Gln Ala Ser Ser Gly
        35                  40                  45
Gln Ala Arg Met Phe Pro Asn Ala Pro Tyr Leu Pro Ser Cys Leu Glu
    50                  55                  60
Ser Gln Pro Ala Ile Arg Asn Gln Gly Tyr Ser Thr Val Thr Phe Asp
65                  70                  75                  80
Gly Thr Pro Ser Tyr Gly His Thr Pro Ser His His Ala Ala Gln Phe
                85                  90                  95
Pro Asn His Ser Phe Lys His Glu Asp Pro Met Gly Gln Gln Gly Ser
            100                 105                 110
Leu Gly Glu Gln Gln Tyr Ser Val Pro Pro Pro Val Tyr Gly Cys His
        115                 120                 125
Thr Pro Thr Asp Ser Cys Thr Gly Ser Gln Ala Leu Leu Leu Arg Thr
    130                 135                 140
Pro Tyr Ser Ser Asp Asn Leu Tyr
145                 150
```

<210> 344
<211> 133
<212> PRT
<213> Homo sapiens

<400> 344

```
Met Gln His His His His His His Gln Ala Leu Leu Leu Arg Thr Pro
                5                   10                  15
Tyr Ser Ser Asp Asn Leu Tyr Gln Met Thr Ser Gln Leu Glu Cys Met
            20                  25                  30
Thr Trp Asn Gln Met Asn Leu Gly Ala Thr Leu Lys Gly His Ser Thr
        35                  40                  45
Gly Tyr Glu Ser Asp Asn His Thr Thr Pro Ile Leu Cys Gly Ala Gln
    50                  55                  60
Tyr Arg Ile His Thr His Gly Val Phe Arg Gly Ile Gln Asp Val Arg
65                  70                  75                  80
Arg Val Pro Gly Val Ala Pro Thr Leu Val Arg Ser Ala Ser Glu Thr
                85                  90                  95
Ser Glu Lys Arg Pro Phe Met Cys Ala Tyr Pro Gly Cys Asn Lys Arg
            100                 105                 110
Tyr Phe Lys Leu Ser His Leu Gln Met His Ser Arg Lys His Thr Gly
        115                 120                 125
Glu Lys Pro Tyr Gln
    130
```

<210> 345
<211> 112
<212> PRT
<213> Homo sapiens

<400> 345

```
Met Gln His His His His His His His Ser Arg Lys His Thr Gly Glu
                5                   10                  15
Lys Pro Tyr Gln Cys Asp Phe Lys Asp Cys Glu Arg Arg Phe Phe Arg
            20                  25                  30
Ser Asp Gln Leu Lys Arg His Gln Arg Arg His Thr Gly Val Lys Pro
        35                  40                  45
Phe Gln Cys Lys Thr Cys Gln Arg Lys Phe Ser Arg Ser Asp His Leu
    50                  55                  60
Lys Thr His Thr Arg Thr His Thr Gly Glu Lys Pro Phe Ser Cys Arg
65                  70                  75                  80
Trp Pro Ser Cys Gln Lys Lys Phe Ala Arg Ser Asp Glu Leu Val Arg
                85                  90                  95
His His Asn Met His Gln Arg Asn Met Thr Lys Leu Gln Leu Ala Leu



            100                 105                 110
```

<210> 346
<211> 369
<212> PRT
<213> Homo sapiens

<400> 346

```
Met Gln His His His His His His His Ser Phe Ile Lys Gln Glu Pro
                    5                  10                  15
Ser Trp Gly Gly Ala Glu Pro His Glu Glu Gln Cys Leu Ser Ala Phe
                20                  25                  30
Thr Val His Phe Ser Gly Gln Phe Thr Gly Thr Ala Gly Ala Cys Arg
            35                  40                  45
Tyr Gly Pro Phe Gly Pro Pro Pro Pro Ser Gln Ala Ser Ser Gly Gln
    50                  55                  60
Ala Arg Met Phe Pro Asn Ala Pro Tyr Leu Pro Ser Cys Leu Glu Ser
65                  70                  75                  80
Gln Pro Ala Ile Arg Asn Gln Gly Tyr Ser Thr Val Thr Phe Asp Gly
                85                  90                  95
Thr Pro Ser Tyr Gly His Thr Pro Ser His His Ala Ala Gln Phe Pro
            100                 105                 110
Asn His Ser Phe Lys His Glu Asp Pro Met Gly Gln Gln Gly Ser Leu
        115                 120                 125
Gly Glu Gln Gln Tyr Ser Val Pro Pro Pro Val Tyr Gly Cys His Thr
    130                 135                 140
Pro Thr Asp Ser Cys Thr Gly Ser Gln Ala Leu Leu Leu Arg Thr Pro
145                 150                 155                 160
Tyr Ser Ser Asp Asn Leu Tyr Gln Met Thr Ser Gln Leu Glu Cys Met
                165                 170                 175
Thr Trp Asn Gln Met Asn Leu Gly Ala Thr Leu Lys Gly His Ser Thr
            180                 185                 190
Gly Tyr Glu Ser Asp Asn His Thr Thr Pro Ile Leu Cys Gly Ala Gln
        195                 200                 205
Tyr Arg Ile His Thr His Gly Val Phe Arg Gly Ile Gln Asp Val Arg
    210                 215                 220
Arg Val Pro Gly Val Ala Pro Thr Leu Val Arg Ser Ala Ser Glu Thr
225                 230                 235                 240
Ser Glu Lys Arg Pro Phe Met Cys Ala Tyr Pro Gly Cys Asn Lys Arg
                245                 250                 255
Tyr Phe Lys Leu Ser His Leu Gln Met His Ser Arg Lys His Thr Gly
            260                 265                 270
Glu Lys Pro Tyr Gln Cys Asp Phe Lys Asp Cys Glu Arg Arg Phe Phe
        275                 280                 285
Arg Ser Asp Gln Leu Lys Arg His Gln Arg Arg His Thr Gly Val Lys
    290                 295                 300
Pro Phe Gln Cys Lys Thr Cys Gln Arg Lys Phe Ser Arg Ser Asp His
305                 310                 315                 320
Leu Lys Thr His Thr Arg Thr His Thr Gly Glu Lys Pro Phe Ser Cys
                325                 330                 335
Arg Trp Pro Ser Cys Gln Lys Lys Phe Ala Arg Ser Asp Glu Leu Val
            340                 345                 350
Arg His His Asn Met His Gln Arg Asn Met Thr Lys Leu Gln Leu Ala
        355                 360                 365
Leu
```

<210> 347
<211> 21
<212> DNA
<213> Artificial Sequence

<220>
<223> Primer

<400> 347
ggctccgacg tgcgggacct g 21

<210> 348
<211> 30
<212> DNA
<213> Artificial Sequence

<220>
<223> Primer

<400> 348
gaattctcaa agcgccagct ggagtttggt 30

<210> 349
<211> 21
<212> DNA
<213> Artificial Sequence

<220>
<223> Primer

<400> 349
ggctccgacg tgcgggacct g 21

<210> 350
<211> 30
<212> DNA
<213> Artificial Sequence

<220>
<223> Primer

<400> 350
gaattctcaa agcgccagct ggagtttggt 30

<210> 351
<211> 21
<212> DNA
<213> Artificial Sequence

<220>
<223> Primer

<400> 351
cacagcacag ggtacgagag c 21

<210> 352
<211> 30
<212> DNA
<213> Artificial Sequence

<220>
<223> Primer

<400> 352
gaattctcaa agcgccagct ggagtttggt 30

<210> 353
<211> 29
<212> DNA

<213> Artificial Sequence

<220>
<223> Primer

<400> 353
cacgaagaac agtgcctgag cgcattcac 29

<210> 354
<211> 32
<212> DNA
<213> Artificial Sequence

<220>
<223> Primer

<400> 354
ccggcgaatt catcagtata aattgtcact gc 32

<210> 355
<211> 24
<212> DNA
<213> Artificial Sequence

<220>
<223> Primer

<400> 355
caggctttgc tgctgaggac gccc 24

<210> 356
<211> 34
<212> DNA
<213> Artificial Sequence

<220>
<223> Primer

<400> 356
cacggagaat tcatcactgg tatggtttct cacc 34

<210> 357
<211> 28
<212> DNA
<213> Artificial Sequence

<220>
<223> Primer

<400> 357
cacagcagga agcacactgg tgagaaac 28

<210> 358
<211> 30
<212> DNA
<213> Artificial Sequence

<220>

<223> Primer

<400> 358
ggatatctgc agaattctca aagcgccagc 30

<210> 359
<211> 22
<212> DNA
<213> Artificial Sequence

<220>
<223> Primer

<400> 359
cactccttca tcaaacagga ac 22

<210> 360
<211> 30
<212> DNA
<213> Artificial Sequence

<220>
<223> Primer

<400> 360
ggatatctgc agaattctca aagcgccagc 30

<210> 361
<211> 33
<212> DNA
<213> Artificial Sequence

<220>
<223> Primer

<400> 361
ggttccgacg tgcgggacct gaacgcactg ctg 33

<210> 362
<211> 40
<212> DNA
<213> Artificial Sequence

<220>
<223> Primer

<400> 362
ctgccggcag cagtgcgttc aggtcccgca cgtcggaacc 40

<210> 363
<211> 35
<212> DNA
<213> Artificial Sequence

<220>
<223> Primer

<400> 363

ccggcagttc catccctggg tggcggtgga ggctg 35

<210> 364
<211> 38
<212> DNA
<213> Artificial Sequence

<220>
<223> Primer

<400> 364
cggcagtgcg cagcctccac cgccacccag ggatggaa 38

<210> 365
<211> 35
<212> DNA
<213> Artificial Sequence

<220>
<223> Primer

<400> 365
cgcactgccg gttagcggtg cagcacagtg ggctc 35

<210> 366
<211> 33
<212> DNA
<213> Artificial Sequence

<220>
<223> Primer

<400> 366
cagaactgga gcccactgtg ctgcaccgct aac 33

<210> 367
<211> 38
<212> DNA
<213> Artificial Sequence

<220>
<223> Primer

<400> 367
cagttctgga cttcgcaccg cctggtgcat ccgcatac 38

<210> 368
<211> 39
<212> DNA
<213> Artificial Sequence

<220>
<223> Primer

<400> 368
cagggaaccg tatgcggatg caccaggcgg tgcgaagtc 39

<210> 369

<211> 38
<212> DNA
<213> Artificial Sequence

<220>
<223> Primer

<400> 369
ggttccctgg gtggtccagc acctccgccc gcaacgcc 38

<210> 370
<211> 38
<212> DNA
<213> Artificial Sequence

<220>
<223> Primer

<400> 370
ggcggtgggg gcgttgcggg cggaggtgct ggaccacc 38

<210> 371
<211> 40
<212> DNA
<213> Artificial Sequence

<220>
<223> Primer

<400> 371
cccaccgcct ccaccgcccc cgcactcctt catcaaacag 40

<210> 372
<211> 39
<212> DNA
<213> Artificial Sequence

<220>
<223> Primer

<400> 372
ctaggttcct gtttgatgaa ggagtgcggg ggcggtgga 39

<210> 373
<211> 38
<212> DNA
<213> Artificial Sequence

<220>
<223> Primer

<400> 373
gaacctagct ggggtggtgc agaaccgcac gaagaaca 38

<210> 374
<211> 39
<212> DNA
<213> Artificial Sequence

<220>
<223> Primer

<400> 374
ctcaggcact gttcttcgtg cggttctgca ccaccccag 39

<210> 375
<211> 32
<212> DNA
<213> Artificial Sequence

<220>
<223> Primer

<400> 375
gtgcctgagc gcattctgag aattctgcag at 32

<210> 376
<211> 34
<212> DNA
<213> Artificial Sequence

<220>
<223> Primer

<400> 376
gtgtgatgga tatctgcaga attctcagaa tgcg 34

<210> 377
<211> 1292
<212> DNA
<213> Homo sapiens

<220>
<221> misc_feature
<222> 253,256,517,518,520,521,522,743,753,754, 758
<223> n = A,T,C or G

<400> 377

```
atgggctccg acgttcgtga cctgagcgcg ctgctgccgg cagttccgtc cctgggtgat 60
ggtggtggtt gcgcactgcc ggttagcggt gcagcacagt gggctccggt tctggacttc 120
gcaccgccgg gtgcatccgc acacggtccc ctgggtggtc cggcgccgcc gtcggcaccg 180
ccgccgccgc cgccgccgcc gccgcactcc ttcatcaaac agggaccgag ctggggtggc 240
gcggaactgc ackaakaaca gtacctgagc gcgttcaccg ttcactcctc cggtcaggtt 300
```

cactggcacg gccggggcct gtcgctacgg gcccctcggc ccccctccgc ccagccaggc 360
gtcatccggc caggccagga tgtctcctag cgcgccctgc ctgcccagcc gcctcgagag 420
ccagcccgct acccgcaatc ggggctacag cacggtcacc ttcgacgggg cgtccggcta 480
cggtcacacg ccctcgcacc atgcggcgca gttctcsmar yyactcgtta ggcgtgagga 540
tcccatgggc cagcagggtc cgctgggtga gcagcagtgc tcggcgccgc ccccggcctg 600
tggccgccac acccccgccg acagctgcgc cggcagccag gctttgctgc tgagggcgcc 660
ctgtagcagc gacggtttat accaagtgac gtcccagctt gagtgcatgg cctggagtca 720
gatgagcctc ggggccgcct tamcgggcca cakyacargg tacgagagcg atgatcacac 780
aacgcccggc ctctgcggag cccaatacag aatacacacg cacggtgcct tcaggggcgt 840
tcagggtgtg cgccgtgtgc ctggagtagc cccgactctt gtacggtcgg catctgaggc 900
cagtgaggaa cgccccctca tgtgtgctta cccaggctgc aataggaggt atctgaagct 960
gccccgctta cagatgcacg gtaggaagca cgctggtgag agaccatacc agtgtgactt 1020
caaggactgt ggacggaggt tttctgctc agaccggctc aaaagacacc aggggaggca 1080
tacagatgtg aagccattcc agcgtaagac ctgtcagcga gggttctccc ggcccaacca 1140
cctgaagacc cacgccagga ctcatgcagg tgaaaagccc cccagctgtc ggtggtcaga 1200
ttgtcagaga aagcctgccc ggtcaagtga gttggtccgc catcgcgaca tgcatcagag 1260
gggcatgacc gaactccagc tggcgctttg aa 1292

<210> 378
<211> 1291
<212> DNA
<213> Homo sapiens

<400> 378

atgggctccg acgttcgtga cctaaacgca ctgctgccgg cagttccgtc cccgggtggt 60
ggtggtggtt gcgcactgcc ggttagcggt gcaacacagt gggctccggt tctggacttc 120
gtaccgccgg gtgcgcctgt atgcggttcc ctgggtggcc cggcaccgcc gccagcgccg 180
ccgccgctgc cgccgccgcc gtcgcactcc ttcaccaaac aggaaccgag ttggggtggt 240
acagagccgc acgcaggaca gggccggagc gcactcgtcg ctcactcctc cggccagttc 300
actggcacag ccggagcctg tcgctacggg cccttcggtc ctcctccgcc cagccaggcg 360
tcatccggcc aggccaggat gtttcctaac gcgccctacc tgcccagctg cctcgagagc 420
cagcccgcta ttcgcaatca gggttacagc acggtcacct tcgacgggac gcccagctac 480
ggtcacacgc cctcgcacca tgcggcgcag ttccccaacc actcatccaa gcatgaggac 540
cccatgggcc agcagggctc gccgggtgag cagcagtact cggcgccgcc cccggtctgc 600
ggctgccgca cccccaccgg cagctgcacc ggcagccagg ctttgctgct gagggcgccc 660
tacagcggtg gcgatctaca ccaaacgaca tcccagcttg gacacatggc ctggaatcag 720
acgaacttag gagccacctt aaagggccac ggcacagggt acgagagcga tgaccacaca 780
acgcccatcc tctgcggaac ccagtacagg atacgcgcgc gcggcgtcct ccggggtact 840
caggatgtgc ggtgtgtgcc tggggtggcc ccgactcttg tgcggtcggc atctgagacc 900
agtgagaagc gccccctcat gtgtgcctac ccaggctgca ataagagaca ctttaagccg 960
tcccgcttgc gggtgcgcgg cagggagcgc actggtgaga aaccatacca gcgcgacttc 1020
aaggaccgtg gacgagggct tctccgtcca gaccagctca aaaggcacca gagggggcat 1080
acaggtgtga aacctctcca gtgtgaagct tgacggcgga ggcccccccg acccggccac 1140
ctgaaggtcc acaccaggac ccatacaggt ggagagccct tcagttgtcg gtggccaagt 1200
tgtcaggaga agtctgcccg gccagatgaa tcagcccgcc gtcataacat gcatcagaga 1260
aacatgacca aactccagct ggcgctttga a 1291

<210> 379
<211> 1281
<212> DNA
<213> Homo sapiens

<400> 379

atgggctccg acgttcgtga cctgagtgca ttgctaccga cggccccgtc cctgggtggt 60
ggcggtgact gcacactgcc ggttagcggt acagcacagt gggctccggt cccggcctcc 120
gcaccgccgg gcgcatccgc atacgattcc ctgggtggcc cggcaccgcc gccggcgccg 180

ccgccgccgc cgccgccgcc gccgcactcc tgcggcgaac aggggccgag ctggggtggt 240
gcagaaccgc gcgaggggca atgcctgagt gcgcccgccg tccgcttctc cggccggttc 300
accggcacag tcggagcctg tcgctatggg cccctcggtc ctcctccgcc cagccaggcg 360
ccatccggcc agaccaggat gttgcccagc gcgccctatc tgtcgagttg cctcaggagc 420
cggtccgcta tccgtagtca gggtcgcagc acggcacctt cagcggggcg cccagctatg 480
gcacccaccc tcgcaccacc ggcgcagtcc cactactccc aacatggggt cctacatggg 540
ccagcagggc tcgctgggtg agcagcagta ctcggtgccg cccccggtct atggctgcca 600
cacccccacc gacagctgca ccggcagcca ggctttgctg ctgaggacgc cctacagcag 660
tgacaattta taccaaatga catcccagct tgaatgcatg acctggaatc agatgaactt 720
aggagccacc ttaaagggcc acagcacagg gtacgagagc gataaccaca caacgcccat 780
cctctgcgga gcccaataca gaatacacac gcacggtgtc ttcagaggca ttcaggatgt 840
gcgacgtgtg cctggagtag ccccgactct tgtacggtag cacctgagac cagtgagaac 900
gccccttggt gtgtgttacc ggggctgcag taaggggtat tttaagccgt cccacttacg 960
ggtgcacagc aggaagcgca ttggtgagac gccacgccag tgcgactcca agggccgtgg 1020
acgagggcct ctccgttcgg gaccagccca agggacacca aaggagacat acaggtacgc 1080
aaccactcca gtgtaaggct tgtcagcgaa ggttcccccg gtccgaccac ctgagggccc 1140
acgccagggc ccacacgggt gggaagcccc tcagctgccg gtggccaagc tgccagagag 1200
ggttcgccca gtcagacgaa ttagtccgtc atcacaacat gtatcagcga aacatgacta 1260
aactccagct ggcgctttga a 1281

<210> 380
<211> 3020
<212> DNA
<213> Homo sapiens

<400> 380

gttcaaggca gcgcccacac ccgggggctc tccgcaaccc gaccgcctgt ccgctccccc 60
acttcccgcc ctccctccca cctactcatt cacccaccca cccacccaga gccgggacgg 120
cagcccaggc gcccgggccc cgccgtctcc tcgccgcgat cctggacttc ctcttgctgc 180
aggacccggc ttccacgtgt gtcccggagc cggcgtctca gcacacgctc cgctccgggc 240
ctgggtgcct acagcagcca gagcagcagg gagtccggga cccgggcggc atctgggcca 300
agttaggcgc cgccgaggcc agcgctgaac gtctccaggg ccggaggagc cgcggggcgt 360
ccgggtctga gcctcagcaa atgggctccg acgtgcggga cctgaacgcg ctgctgcccg 420
ccgtcccctc cctgggtggc ggcggcggct gtgccctgcc tgtgagcggc gcggcgcagt 480
gggcgccggt gctggacttt gcgcccccgg gcgcttcggc ttacgggtcg ttgggcggcc 540
ccgcgccgcc accggctccg ccgccacccc cgccgccgcc gcctcactcc ttcatcaaac 600
aggagccgag ctggggcggc gcggagccgc acgaggagca gtgcctgagc gccttcactg 660
tccacttttc cggccagttc actggcacag ccggagcctg tcgctacggg cccttcggtc 720
ctcctccgcc cagccaggcg tcatccggcc aggccaggat gtttcctaac gcgccctacc 780
tgcccagctg cctcgagagc cagcccgcta ttcgcaatca gggttacagc acggtcacct 840
tcgacgggac gcccagctac ggtcacacgc cctcgcacca tgcggcgcag ttccccaacc 900
actcattcaa gcatgaggat cccatgggcc agcagggctc gctgggtgag cagcagtact 960
cggtgccgcc cccggtctat ggctgccaca cccccaccga cagctgcacc ggcagccagg 1020
ctttgctgct gaggacgccc tacagcagtg acaatttata ccaaatgaca tcccagcttg 1080
aatgcatgac ctggaatcag atgaacttag gagccacctt aaagggagtt gctgctggga 1140
gctccagctc agtgaaatgg acagaagggc agagcaacca cagcacaggg tacgagagcg 1200
ataaccacac aacgcccatc ctctgcggag cccaatacag aatacacacg cacggtgtct 1260
tcagaggcat tcaggatgtg cgacgtgtgc ctggagtagc cccgactctt gtacggtcgg 1320
catctgagac cagtgagaaa cgccccttca tgtgtgctta cccaggctgc aataagagat 1380
attttaagct gtcccactta cagatgcaca gcaggaagca cactggtgag aaaccatacc 1440
agtgtgactt caaggactgt gaacgaaggt tttctcgttc agaccagctc aaaagacacc 1500
aaaggagaca tacaggtgtg aaaccattcc agtgtaaaac ttgtcagcga aagttctccc 1560
ggtccgacca cctgaagacc cacaccagga ctcatacagg taaaacaagt gaaaagccct 1620
tcagctgtcg gtggccaagt tgtcagaaaa agtttgcccg gtcagatgaa ttagtccgcc 1680
atcacaacat gcatcagaga aacatgacca aactccagct ggcgctttga ggggtctccc 1740
tcggggaccg ttcagtgtcc caggcagcac agtgtgtgaa ctgctttcaa gtctgactct 1800
ccactcctcc tcactaaaaa ggaaacttca gttgatcttc ttcatccaac ttccaagaca 1860
agataccggt gcttctggaa actaccaggt gtgcctggaa gagttggtct ctgccctgcc 1920

tacttttagt tgactcacag gccctggaga agcagctaac aatgtctggt tagttaaaag 1980
cccattgcca tttggtctgg attttctact gtaagaagag ccatagctga tcatgtcccc 2040
ctgacccttc ccttcttttt ttatgctcgt tttcgctggg gatggaatta ttgtaccatt 2100
ttctatcatg gaatatttat aggccagggc atgtgtatgt gtctgctaat gtaaactttg 2160
tcatggtttc catttactaa cagcaacagc aagaaataaa tcagagagca aggcatcggg 2220
ggtgaatctt gtctaacatt cccgaggtca gccaggctgc taacctggaa agcaggatgt 2280
agttctgcca ggcaactttt aaagctcatg catttcaagc agctgaagaa agaatcagaa 2340
ctaaccagta cctctgtata gaaatctaaa agaattttac cattcagtta attcaatgtg 2400
aacactggca cactgctctt aagaaactat gaagatctga gatttttttg tgtatgtttt 2460
tgactctttt gagtggtaat catatgtgtc tttatagatg tacatacctc cttgcacaaa 2520
tggaggggaa ttcattttca tcactgggac tgtccttagt gtataaaaac catgctggta 2580
tatggcttca agttgtaaaa atgaaagtga ctttaaaaga aaatagggga tggtccagga 2640
tctccactga taagactgtt tttaagtaac ttaaggacct ttgggtctac aagtatatgt 2700
gaaaaaaatg agacttactg ggtgaggaaa tccattgttt aaagatggtc gtgtgtgtgt 2760
gtgtgtgtgt gtgtgtgttg tgttgtgttt tgttttttaa gggagggaat ttattattta 2820
ccgttgcttg aaattactgt gtaaatatat gtctgataat gatttgctct ttgacaacta 2880
aaattaggac tgtataagta ctagatgcat cactgggtgt tgatcttaca agatattgat 2940
gataacactt aaaattgtaa cctgcatttt tcactttgct ctcaattaaa gtctattcaa 3000
aaggaaaaaa aaaaaaaaaa 3020

<210> 381
<211> 1291
<212> DNA
<213> Homo sapiens

<400> 381

```
atgggctccg acgttcgtga cctgaacgca ctgctgccgg cagttccgtc cctgggtggt 60
ggtggtggtt gcgcactgcc ggttagcggt gcagcacagt gggctccggt tctggacttc 120
gcaccgccgg gtgcatccgc atacggttcc ctgggtggtc cggcaccgcc gccggcaccg 180
ccgccgccgc cgccgccgcc gccgcactcc ttcatcaaac aggaaccgag ctggggtggt 240
gcagaaccgc acgaagaaca gtgcctgagc gcattcaccg ttcacttctc cggccagttc 300
actggcacag ccggagcctg tcgctacggg cccttcggtc ctcctccgcc cagccaggcg 360
tcatccggcc aggccaggat gtttcctaac gcgccctacc tgcccagctg cctcgagagc 420
cagcccgcta ttcgcaatca gggttacagc acggtcacct tcgacgggac gcccagctac 480
ggtcacacgc cctcgcacca tgcggcgcag ttccccaacc actcattcaa gcatgaggat 540
cccatgggcc agcagggctc gctgggtgag cagcagtact cggtgccgcc cccggtctat 600
ggctgccaca cccccaccga cagctgcacc ggcagccagg ctttgctgct gaggacgccc 660
tacagcagtg acaatttata ccaaatgaca tcccagcttg aatgcatgac ctggaatcag 720
atgaacttag gagccacctt aaagggccac agcacagggt acgagagcga taaccacaca 780
acgcccatcc tctgcggagc ccaatacaga atacacacgc acggtgtctt cagaggcatt 840
caggatgtgc gacgtgtgcc tggagtagcc ccgactcttg tacggtcggc atctgagacc 900
agtgagaaac gccccttcat gtgtgcttac ccaggctgca ataagagata ttttaagctg 960
tcccacttac agatgcacag caggaagcac actggtgaga aaccatacca gtgtgacttc 1020
aaggactgtg aacgaaggtt ttttcgttca gaccagctca aaagacacca aaggagacat 1080
acaggtgtga aaccattcca gtgtaaaact tgtcagcgaa agttctcccg gtccgaccac 1140
ctgaagaccc acaccaggac tcatacaggt gaaaagccct tcagctgtcg gtggccaagt 1200
tgtcagaaaa agtttgcccg gtcagatgaa ttagtccgcc atcacaacat gcatcagaga 1260
aacatgacca aactccagct ggcgctttga g                                1291
```

<210> 382
<211> 1491
<212> DNA
<213> Homo sapiens

<400> 382

```
atggcggccc ccggcgcccg gcggtcgctg ctcctgctgc tgctggcagg ccttgcacat 60
```

```
ggcgcctcag cactctttga ggatctaatg ggctccgacg ttcgtgacct gaacgcactg 120
ctgccggcag ttccgtccct gggtggtggt ggtggttgcg cactgccggt tagcggtgca 180
gcacagtggg ctccggttct ggacttcgca ccgccgggtg catccgcata cggttccctg 240
ggtggtccgg caccgccgcc ggcaccgccg ccgccgccgc cgccgcactc cttcatcaaa 300
caggaaccga gctggggtgg tgcagaaccg cacgaagaac agtgcctgag cgcattcacc 360
gttcacttct ccggccagtt cactggcaca gccggagcct gtcgctacgg gcccttcggt 420
cctcctccgc ccagccaggc gtcatccggc caggccagga tgtttcctaa cgcgccctac 480
ctgcccagct gcctcgagag ccagcccgct attcgcaatc agggttacag cacggtcacc 540
ttcgacggga cgcccagcta cggtcacacg ccctcgcacc atgcggcgca gttccccaac 600
cactcattca agcatgagga tcccatgggc cagcagggct cgctgggtga gcagcagtac 660
tcggtgccgc ccccggtcta tggctgccac acccccaccg acagctgcac cggcagccag 720
gctttgctgc tgaggacgcc ctacagcagt gacaatttat accaaatgac atcccagctt 780
gaatgcatga cctggaatca gatgaactta ggagccacct taaagggcca cagcacaggg 840
tacgagagcg ataaccacac aacgcccatc ctctgcggag cccaatacag aatacacacg 900
cacggtgtct tcagaggcat tcaggatgtg cgacgtgtgc ctggagtagc ccgactcttt 960
gtacggtcgg catctgagac cagtgagaaa cgccccttca tgtgtgctta cccaggctgc 1020
aataagagat attttaagct gtcccactta cagatgcaca gcaggaagca cactggtgag 1080
aaaccatacc agtgtgactt caaggactgt gaacgaaggt tttttcgttc agaccagctc 1140
aaaagacacc aaaggagaca tacaggtgtg aaaccattcc agtgtaaaac ttgtcagcga 1200
aagttctccc ggtccgacca cctgaagacc cacaccagga ctcatacagg tgaaaagccc 1260
ttcagctgtc ggtggccaag ttgtcagaaa aagtttgccc ggtcagatga attagtccgc 1320
catcacaaca tgcatcagag aaacatgacc aaactccagc tggcgcttct taacaacatg 1380
ttgatcccca ttgctgtggg cggtgccctg gcagggctgg tcctcatcgt cctcattgcc 1440
tacctcattg gcaggaagag gagtcacgcc ggctatcaga ccatctagtg a          1491
```

<210> 383
<211> 1251
<212> DNA
<213> Homo sapiens

<400> 383

```
atggcgcccc gcagcgcccg gcgacccctg ctgctgctac tgcctgttgc tgctgctcgg 60
cctcatgcat tgtcgtcagc agccatgttt atggtgaaaa atggcaacgg gaccgcgtgc 120
ataatggcca acttctctgc tgccttctca gtgaactacg acaccaagag tggccccaag 180
aacatgacct ttgacctgcc atcagatgcc acagtggtgc tcaaccgcag ctcctgtgga 240
aaagagaaca cttctgaccc cagtctcgtg attgcttttg gaagaggaca tacactcact 300
ctcaatttca cgagaaatgc aacacgttac agcgttcagc tcatgagttt tgtttataac 360
ttgtcagaca cacacctttt ccccaatgcg agctccaaag aaatcaagac tgtggaatct 420
ataactgaca tcagggcaga tatagataaa aaatacagat gtgttagtgg cacccaggtc 480
cacatgaaca acgtgaccgt aacgctccat gatgccacca tccaggcgta cctttccaac 540
agcagcttca gcaggggaga gacacgctgt gaacaagaca ggccttcccc aaccacagcg 600
ccccctgcgc cacccagccc ctcgccctca cccgtgccca agagcccctc tgtggacaag 660
tacaacgtga gcggcaccaa cgggacctgc ctgctggcca gcatggggct gcagctgaac 720
ctcacctatg agaggaagga caacacgacg gtgacaaggc ttctcaacat caaccccaac 780
aagacctcgg ccagcgggag ctgcggcgcc cacctggtga ctctggagct gcacagcgag 840
ggcaccaccg tcctgctctt ccagttcggg atgaatgcaa gttctagccg gtttttccta 900
caaggaatcc agttgaatac aattcttcct gacgccagag accctgcctt taaagctgcc 960
aacggctccc tgcgagcgct gcaggccaca gtcggcaatt cctacaagtg caacgcggag 1020
gagcacgtcc gtgtcacgaa ggcgttttca gtcaatatat tcaaagtgtg ggtccaggct 1080
ttcaaggtgg aaggtggcca gtttggctct gtggaggagt gtctgctgga cgagaacagc 1140
acgctgatcc ccatcgctgt gggtggtgcc ctggcggggc tggtcctcat cgtcctcatc 1200
gcctacctcg tcggcaggaa gaggagtcac gcaggctacc agactatcta g          1251
```

<210> 384
<211> 228

<212> DNA
<213> Homo sapiens

<400> 384

```
atgcagatct tcgtgaagac tctgactggt aagaccatca ccctcgaggt ggagcccagt 60
gacaccatcg agaatgtcaa ggcaaagatc caagataagg aaggcattcc tcctgatcag 120
cagaggttga tctttgccgg aaaacagctg gaagatggtc gtaccctgtc tgactacaac 180
atccagaaag agtccacctt gcacctggta ctccgtctca gaggtggg              228
```

<210> 385
<211> 1515
<212> DNA
<213> Homo sapiens

<400> 385

```
atgcagatct tcgtgaagac cctgaccggc aagaccatca ccctggaagt ggagcccagt 60
gacaccatcg aaaatgtgaa ggccaagatc caggataaag aaggcatccc tcccgaccag 120
cagaggctca tctttgcagg caagcagcta gaagatggcc gcactctttc tgactacaac 180
atccagaagg agtcgaccct gcacctggtc cttcgcctga gaggtgccat gggctccgac 240
gttcgtgacc tgaacgcact gctgccggca gttccgtccc tgggtggtgg tggtggttgc 300
gcactgccgg ttagcggtgc agcacagtgg gctccggttc tggacttcgc accgccgggt 360
gcatccgcat acggttccct gggtggtccg gcaccgccgc cggcaccgcc gccgccgccg 420
ccgccgccgc actccttcat caaacaggaa ccgagctggg gtggtgcaga accgcacgaa 480
gaacagtgcc tgagcgcatt caccgttcac ttctccggcc agttcactgg cacagccgga 540
gcctgtcgct acgggccctt cggtcctcct ccgcccagcc aggcgtcatc cggccaggcc 600
aggatgtttc ctaacgcgcc ctatctgccc agctgcctcg agagccagcc cgctattcgc 660
aatcagggtt acagcacggt caccttcgac gggacgccca gctacggtca cacgccctcg 720
caccatgcgg cgcagttccc caaccactca ttcaagcatg aggatcccat gggccagcag 780
ggctcgctgg gtgagcagca gtactcggtg ccgcccccgg tctatggctg ccacaccccc 840
accgacagct gcaccggcag ccaggctttg ctgctgagga cgccctacag cagtgacaat 900
ttataccaaa tgacatccca gcttgaatgc atgacctgga atcagatgaa cttaggagcc 960
accttaaagg gccacagcac agggtacgag agcgataacc acacaacgcc catcctctgc 1020
ggagcccaat acagaataca cacgcacggt gtcttcagag gcattcagga tgtgcgacgt 1080
gtgcctggag tagccccgac tcttgtacgg tcggcatctg agaccagtga gaaacgcccc 1140
ttcatgtgtg cttacccagg ctgcaataag agatatttta agctgtccca cttacagatg 1200
cacagcagga agcacactgg tgagaaacca taccagtgtg acttcaagga ctgtgaacga 1260
aggtttttc gttcagacca gctcaaaaga caccaaagga gacatacagg tgtgaaacca 1320
ttccagtgta aaacttgtca gcgaaagttc tcccggtccg accacctgaa gacccacacc 1380
aggactcata caggtgaaaa gcccttcagc tgtcggtggc caagttgtca gaaaaagttt 1440
gcccggtcag atgaattagt ccgccatcac aacatgcatc agagaaacat gaccaaactc 1500
cagctggcgc tttga                                                  1515
```

<210> 386
<211> 648
<212> DNA
<213> Homo sapiens

<400> 386

atgcactcct tcatcaaaca ggaaccgagc tggggtggtg cagaaccgca cgaagaacag 60
tgcctgagcg cattcaccgt tcacttctcc ggccagttca ctggcacagc cggagcctgt 120
cgctacgggc ccttcggtcc tcctccgccc agccaggcgt catccggcca ggccaggatg 180
tttcctaacg cgccctacct gcccagctgc ctcgagagcc agcccgctat tcgcaatcag 240
ggttacagca cggtcacctt cgacgggacg cccagctacg gtcacacgcc ctcgcaccat 300
gcggcgcagt tccccaacca ctcattcaag catgaggatc ccatgggcca gcagggctcg 360
ctgggtgagc agcagtactc ggtgccgccc ccggtctatg gctgccacac ccccaccgac 420
agctgcaccg gcagccaggc tttgctgctg aggacgccct acagcagtga caatttatac 480

caaatgacat cccagcttga atgcatgacc tggaatcaga tgaacttagg agccacctta 540
aagggccaca gcacagggta cgagagcgat aaccacacaa cgcccatcct ctgcggagcc 600
caatacagaa tacacacgca cggtgtcttc agaggcattc agtgatga 648

<210> 387
<211> 1089
<212> DNA
<213> Homo sapiens

<400> 387

atgcactcct tcatcaaaca ggaaccgagc tggggtggtg cagaaccgca cgaagaacag 60
tgcctgagcg cattcaccgt tcacttctcc ggccagttca ctggcacagc cggagcctgt 120
cgctacgggc ccttcggtcc tcctccgccc agccaggcgt catccggcca ggccaggatg 180
tttcctaacg cgccctacct gcccagctgc ctcgagagcc agcccgctat tcgcaatcag 240
ggttacagca cggtcacctt cgacgggacg cccagctacg gtcacacgcc ctcgcaccat 300
gcggcgcagt tccccaacca ctcattcaag catgaggatc ccatgggcca gcagggctcg 360
ctgggtgagc agcagtactc ggtgccgccc ccggtctatg gctgccacac ccccaccgac 420
agctgcaccg gcagccaggc tttgctgctg aggacgccct acagcagtga caatttatac 480
caaatgacat cccagcttga atgcatgacc tggaatcaga tgaacttagg agccacctta 540
aagggccaca gcacagggta cgagagcgat aaccacacaa cgcccatcct ctgcggagcc 600
caatacagaa tacacacgca cggtgtcttc agaggcattc aggatgtgcg acgtgtgcct 660
ggagtagccc cgactcttgt acggtcggca tctgagacca gtgagaaacg ccccttcatg 720
tgtgcttacc caggctgcaa taagagatat tttaagctgt cccacttaca gatgcacagc 780
aggaagcaca ctggtgagaa accataccag tgtgacttca aggactgtga acgaaggttt 840
tttcgttcag accagctcaa aagacaccaa aggagacata caggtgtgaa accattccag 900
tgtaaaactt gtcagcgaaa gttctcccgg tccgaccacc tgaagaccca caccaggact 960
catacaggtg aaaagccctt cagctgtcgg tggccaagtt gtcagaaaaa gtttgcccgg 1020
tcagatgaat tagtccgcca tcacaacatg catcagagaa acatgaccaa actccagctg 1080
gcgctttga 1089

<210> 388
<211> 1035
<212> DNA
<213> Homo sapiens

<400> 388

```
atgacggccg cgtccgataa cttccagctg tcccagggtg ggcagggatt cgccattccg 60
atcgggcagg cgatggcgat cgcgggccag atcaagcttc ccaccgttca tatcgggcct 120
accgccttcc tcggcttggg tgttgtcgac aacaacggca acggcgcacg agtccaacgc 180
gtggtcggga gcgctccggc ggcaagtctc ggcatctcca ccggcgacgt gatcaccgcg 240
gtcgacggcg ctccgatcaa ctcggccacc gcgatggcgg acgcgcttaa cgggcatcat 300
cccggtgacg tcatctcggt gacctggcaa accaagtcgg gcggcacgcg tacagggaac 360
gtgacattgg ccgagggacc cccggccgaa ttccactcct tcatcaaaca ggaaccgagc 420
tggggtggtg cagaaccgca cgaagaacag tgcctgagcg cattcaccgt tcacttctcc 480
ggccagttca ctggcacagc cggagcctgt cgctacgggc ccttcggtcc tcctccgccc 540
agccaggcgt catccggcca ggccaggatg tttcctaacg cgccctacct gcccagctgc 600
ctcgagagcc agcccgctat tcgcaatcag ggttacagca cggtcacctt cgacgggacg 660
cccagctacg gtcacacgcc ctcgcaccat gcggcgcagt tccccaacca ctcattcaag 720
catgaggatc ccatgggcca gcagggctcg ctgggtgagc agcagtactc ggtgccgccc 780
ccggtctatg gctgccacac ccccaccgac agctgcaccg gcagccaggc tttgctgctg 840
aggacgccct acagcagtga caatttatac caaatgacat cccagcttga atgcatgacc 900
tggaatcaga tgaacttagg agccacctta aagggccaca gcacagggta cgagagcgat 960
aaccacacaa cgcccatcct ctgcggagcc caatacagaa tacacacgca cggtgtcttc 1020
agaggcattc agtga                                                  1035
```

<210> 389
<211> 1263
<212> DNA
<213> Homo sapiens

<400> 389

```
atgacggccg cgtccgataa cttccagctg tcccagggtg ggcagggatt cgccattccg 60
atcgggcagg cgatggcgat cgcgggccag atcaagcttc ccaccgttca tatcgggcct 120
accgccttcc tcggcttggg tgttgtcgac aacaacggca acggcgcacg agtccaacgc 180
gtggtcggga gcgctccggc ggcaagtctc ggcatctcca ccggcgacgt gatcaccgcg 240
gtcgacggcg ctccgatcaa ctcggccacc gcgatggcgg acgcgcttaa cgggcatcat 300
cccggtgacg tcatctcggt gacctggcaa accaagtcgg gcggcacgcg tacagggaac 360
gtgacattgg ccgagggacc cccggccgaa ttcccgctgg tgccgcgcgg cagcccgatg 420
ggctccgacg ttcgggacct gaacgcactg ctgccggcag ttccgtccct gggtggtggt 480
ggtggttgcg cactgccggt tagcggtgca gcacagtggg ctccggttct ggacttcgca 540
ccgccgggtg catccgcata cggttccctg ggtggtccgg caccgccgcc ggcaccgccg 600
ccgccgccgc cgccgccgcc gcactccttc atcaaacagg aaccgagctg ggtggtgca 660
gaaccgcacg aagaacagtg cctgagcgca ttcaccgttc acttctccgg ccagttcact 720
ggcacagccg gagcctgtcg ctacgggccc ttcggtcctc ctccgcccag ccaggcgtca 780
tccggccagg ccaggatgtt tcctaacgcg ccctacctgc ccagctgcct cgagagccag 840
cccgctattc gcaatcaggg ttacagcacg gtcaccttcg acgggacgcc cagctacggt 900
cacacgccct cgcaccatgc ggcgcagttc cccaaccact cattcaagca tgaggatccc 960
atgggccagc agggctcgct gggtgagcag cagtactcgg tgccgccccc ggtctatggc 1020
tgccacaccc ccaccgacag ctgcaccggc agccaggctt tgctgctgag gacgccctac 1080
agcagtgaca atttatacca aatgacatcc cagcttgaat gcatgacctg gaatcagatg 1140
aacttaggag ccaccttaaa gggccacagc acagggtacg agagcgataa ccacacaacg 1200
cccatcctct gcggagccca atacagaata cacacgcacg gtgtcttcag aggcattcag 1260
tga                                                               1263
```

<210> 390
<211> 1707
<212> DNA
<213> Homo sapiens

<400> 390

atgacggccg cgtccgataa cttccagctg tcccagggtg ggcagggatt cgccattccg 60
atcgggcagg cgatggcgat cgcgggccag atcaagcttc ccaccgttca tatcgggcct 120
accgccttcc tcggcttggg tgttgtcgac aacaacggca acggcgcacg agtccaacgc 180
gtggtcggga gcgctccggc ggcaagtctc ggcatctcca ccggcgacgt gatcaccgcg 240
gtcgacggcg ctccgatcaa ctcggccacc gcgatggcgg acgcgcttaa cgggcatcat 300
cccggtgacg tcatctcggt gacctggcaa accaagtcgg gcggcacgcg tacagggaac 360
gtgacattgg ccgagggacc cccggccgaa ttcccgctgg tgccgcgcgg cagcccgatg 420
ggctccgacg ttcgggacct gaacgcactg ctgccggcag ttccgtccct gggtggtggt 480
ggtggttgcg cactgccggt tagcggtgca gcacagtggg ctccggttct ggacttcgca 540
ccgccgggtg catccgcata cggttccctg ggtggtccgg caccgccgcc ggcaccgccg 600
ccgccgccgc cgccgccgcc gcactccttc atcaaacagg aaccgagctg ggtggtgca 660
gaaccgcacg aagaacagtg cctgagcgca ttcaccgttc acttctccgg ccagttcact 720
ggcacagccg gagcctgtcg ctacgggccc ttcggtcctc ctccgcccag ccaggcgtca 780
tccggccagg ccaggatgtt tcctaacgcg ccctacctgc ccagctgcct cgagagccag 840
cccgctattc gcaatcaggg ttacagcacg gtcaccttcg acgggacgcc cagctacggt 900
cacacgccct cgcaccatgc ggcgcagttc cccaaccact cattcaagca tgaggatccc 960
atgggccagc agggctcgct gggtgagcag cagtactcgg tgccgccccc ggtctatggc 1020
tgccacaccc ccaccgacag ctgcaccggc agccaggctt tgctgctgag gacgccctac 1080
agcagtgaca atttatacca aatgacatcc cagcttgaat gcatgacctg gaatcagatg 1140
aacttaggag ccaccttaaa gggccacagc acagggtacg agagcgataa ccacacaacg 1200
cccatcctct gcggagccca atacagaata cacacgcacg gtgtcttcag aggcattcag 1260
gatgtgcgac gtgtgcctgg agtagccccg actcttgtac ggtcggcatc tgagaccagt 1320
gagaaacgcc ccttcatgtg tgcttaccca ggctgcaata agagatattt taagctgtcc 1380
cacttacaga tgcacagcag gaagcacact ggtgagaaac cataccagtg tgacttcaag 1440
gactgtgaac gaaggttttt tcgttcagac cagctcaaaa gacaccaaag gagacataca 1500
ggtgtgaaac cattccagtg taaaacttgt cagcgaaagt tctcccggtc cgaccacctg 1560

aagacccaca ccaggactca tacaggtgaa aagcccttca gctgtcggtg gccaagttgt 1620
cagaaaaagt ttgcccggtc agatgaatta gtccgccatc acaacatgca tcagagaaac 1680
atgaccaaac tccagctggc gctttga 1707

<210> 391
<211> 344
<212> PRT
<213> Homo sapiens

<400> 391

```
Met Thr Ala Ala Ser Asp Asn Phe Gln Leu Ser Gln Gly Gly Gln Gly
                5                  10                  15

Phe Ala Ile Pro Ile Gly Gln Ala Met Ala Ile Ala Gly Gln Ile Lys
            20                  25                  30

Leu Pro Thr Val His Ile Gly Pro Thr Ala Phe Leu Gly Leu Gly Val
        35                  40                  45

Val Asp Asn Asn Gly Asn Gly Ala Arg Val Gln Arg Val Val Gly Ser
    50                  55                  60

Ala Pro Ala Ala Ser Leu Gly Ile Ser Thr Gly Asp Val Ile Thr Ala
65                  70                  75                  80

Val Asp Gly Ala Pro Ile Asn Ser Ala Thr Ala Met Ala Asp Ala Leu
                85                  90                  95

Asn Gly His His Pro Gly Asp Val Ile Ser Val Thr Trp Gln Thr Lys
            100                 105                 110

Ser Gly Gly Thr Arg Thr Gly Asn Val Thr Leu Ala Glu Gly Pro Pro
        115                 120                 125

Ala Glu Phe His Ser Phe Ile Lys Gln Glu Pro Ser Trp Gly Gly Ala
    130                 135                 140

Glu Pro His Glu Glu Gln Cys Leu Ser Ala Phe Thr Val His Phe Ser
145                 150                 155                 160

Gly Gln Phe Thr Gly Thr Ala Gly Ala Cys Arg Tyr Gly Pro Phe Gly
                165                 170                 175

Pro Pro Pro Pro Ser Gln Ala Ser Ser Gly Gln Ala Arg Met Phe Pro
            180                 185                 190

Asn Ala Pro Tyr Leu Pro Ser Cys Leu Glu Ser Gln Pro Ala Ile Arg
        195                 200                 205

Asn Gln Gly Tyr Ser Thr Val Thr Phe Asp Gly Thr Pro Ser Tyr Gly
    210                 215                 220

His Thr Pro Ser His His Ala Ala Gln Phe Pro Asn His Ser Phe Lys
225                 230                 235                 240

His Glu Asp Pro Met Gly Gln Gln Gly Ser Leu Gly Glu Gln Gln Tyr
                245                 250                 255
```

```
Ser Val Pro Pro Pro Val Tyr Gly Cys His Thr Pro Thr Asp Ser Cys
            260                 265                 270

Thr Gly Ser Gln Ala Leu Leu Leu Arg Thr Pro Tyr Ser Ser Asp Asn
        275                 280                 285

Leu Tyr Gln Met Thr Ser Gln Leu Glu Cys Met Thr Trp Asn Gln Met
    290                 295                 300

Asn Leu Gly Ala Thr Leu Lys Gly His Ser Thr Gly Tyr Glu Ser Asp
305                 310                 315                 320

Asn His Thr Thr Pro Ile Leu Cys Gly Ala Gln Tyr Arg Ile His Thr
                325                 330                 335

His Gly Val Phe Arg Gly Ile Gln
            340
```

<210> 392
<211> 568
<212> PRT
<213> Homo sapiens

<400> 392

```
Met Thr Ala Ala Ser Asp Asn Phe Gln Leu Ser Gln Gly Gly Gln Gly
                5                   10                  15

Phe Ala Ile Pro Ile Gly Gln Ala Met Ala Ile Ala Gly Gln Ile Lys
            20                  25                  30

Leu Pro Thr Val His Ile Gly Pro Thr Ala Phe Leu Gly Leu Gly Val
        35                  40                  45

Val Asp Asn Asn Gly Asn Gly Ala Arg Val Gln Arg Val Val Gly Ser
    50                  55                  60

Ala Pro Ala Ala Ser Leu Gly Ile Ser Thr Gly Asp Val Ile Thr Ala
65                  70                  75                  80

Val Asp Gly Ala Pro Ile Asn Ser Ala Thr Ala Met Ala Asp Ala Leu
                85                  90                  95

Asn Gly His His Pro Gly Asp Val Ile Ser Val Thr Trp Gln Thr Lys
            100                 105                 110

Ser Gly Gly Thr Arg Thr Gly Asn Val Thr Leu Ala Glu Gly Pro Pro
        115                 120                 125

Ala Glu Phe Pro Leu Val Pro Arg Gly Ser Pro Met Gly Ser Asp Val
    130                 135                 140

Arg Asp Leu Asn Ala Leu Leu Pro Ala Val Pro Ser Leu Gly Gly Gly
145                 150                 155                 160

Gly Gly Cys Ala Leu Pro Val Ser Gly Ala Ala Gln Trp Ala Pro Val
                165                 170                 175
```

```
Leu Asp Phe Ala Pro Pro Gly Ala Ser Ala Tyr Gly Ser Leu Gly Gly
            180                 185                 190

Pro Ala Pro Pro Pro Ala Pro Pro Pro Pro Pro Pro Pro Pro Pro His
        195                 200                 205

Ser Phe Ile Lys Gln Glu Pro Ser Trp Gly Gly Ala Glu Pro His Glu
    210                 215                 220

Glu Gln Cys Leu Ser Ala Phe Thr Val His Phe Ser Gly Gln Phe Thr
225                 230                 235                 240

Gly Thr Ala Gly Ala Cys Arg Tyr Gly Pro Phe Gly Pro Pro Pro Pro
                245                 250                 255

Ser Gln Ala Ser Ser Gly Gln Ala Arg Met Phe Pro Asn Ala Pro Tyr
            260                 265                 270

Leu Pro Ser Cys Leu Glu Ser Gln Pro Ala Ile Arg Asn Gln Gly Tyr
        275                 280                 285

Ser Thr Val Thr Phe Asp Gly Thr Pro Ser Tyr Gly His Thr Pro Ser
    290                 295                 300

His His Ala Ala Gln Phe Pro Asn His Ser Phe Lys His Glu Asp Pro
305                 310                 315                 320

Met Gly Gln Gln Gly Ser Leu Gly Glu Gln Gln Tyr Ser Val Pro Pro
                325                 330                 335

Pro Val Tyr Gly Cys His Thr Pro Thr Asp Ser Cys Thr Gly Ser Gln
            340                 345                 350

Ala Leu Leu Leu Arg Thr Pro Tyr Ser Ser Asp Asn Leu Tyr Gln Met
        355                 360                 365

Thr Ser Gln Leu Glu Cys Met Thr Trp Asn Gln Met Asn Leu Gly Ala
    370                 375                 380

Thr Leu Lys Gly His Ser Thr Gly Tyr Glu Ser Asp Asn His Thr Thr
385                 390                 395                 400

Pro Ile Leu Cys Gly Ala Gln Tyr Arg Ile His Thr His Gly Val Phe
                405                 410                 415

Arg Gly Ile Gln Asp Val Arg Arg Val Pro Gly Val Ala Pro Thr Leu
            420                 425                 430

Val Arg Ser Ala Ser Glu Thr Ser Glu Lys Arg Pro Phe Met Cys Ala
        435                 440                 445

Tyr Pro Gly Cys Asn Lys Arg Tyr Phe Lys Leu Ser His Leu Gln Met
    450                 455                 460

His Ser Arg Lys His Thr Gly Glu Lys Pro Tyr Gln Cys Asp Phe Lys
465                 470                 475                 480

Asp Cys Glu Arg Arg Phe Phe Arg Ser Asp Gln Leu Lys Arg His Gln
```

485 490 495

Arg Arg His Thr Gly Val Lys Pro Phe Gln Cys Lys Thr Cys Gln Arg
500 505 510

Lys Phe Ser Arg Ser Asp His Leu Lys Thr His Thr Arg Thr His Thr
515 520 525

Gly Glu Lys Pro Phe Ser Cys Arg Trp Pro Ser Cys Gln Lys Lys Phe
530 535 540

Ala Arg Ser Asp Glu Leu Val Arg His His Asn Met His Gln Arg Asn
545 550 555 560

Met Thr Lys Leu Gln Leu Ala Leu
565

<210> 393
<211> 420
<212> PRT
<213> Homo sapiens

<400> 393

```
Met Thr Ala Ala Ser Asp Asn Phe Gln Leu Ser Gln Gly Gly Gln Gly
                5                   10                  15

Phe Ala Ile Pro Ile Gly Gln Ala Met Ala Ile Ala Gly Gln Ile Lys
            20                  25                  30

Leu Pro Thr Val His Ile Gly Pro Thr Ala Phe Leu Gly Leu Gly Val
        35                  40                  45

Val Asp Asn Asn Gly Asn Gly Ala Arg Val Gln Arg Val Val Gly Ser
    50                  55                  60

Ala Pro Ala Ala Ser Leu Gly Ile Ser Thr Gly Asp Val Ile Thr Ala
65                  70                  75                  80

Val Asp Gly Ala Pro Ile Asn Ser Ala Thr Ala Met Ala Asp Ala Leu
                85                  90                  95

Asn Gly His His Pro Gly Asp Val Ile Ser Val Thr Trp Gln Thr Lys
            100                 105                 110

Ser Gly Gly Thr Arg Thr Gly Asn Val Thr Leu Ala Glu Gly Pro Pro
        115                 120                 125

Ala Glu Phe Pro Leu Val Pro Arg Gly Ser Pro Met Gly Ser Asp Val
    130                 135                 140

Arg Asp Leu Asn Ala Leu Leu Pro Ala Val Pro Ser Leu Gly Gly Gly
145                 150                 155                 160

Gly Gly Cys Ala Leu Pro Val Ser Gly Ala Ala Gln Trp Ala Pro Val
                165                 170                 175

Leu Asp Phe Ala Pro Pro Gly Ala Ser Ala Tyr Gly Ser Leu Gly Gly
```

180 185 190

Pro Ala Pro Pro Pro Ala Pro Pro Pro Pro Pro Pro Pro Pro Pro His
195 200 205

Ser Phe Ile Lys Gln Glu Pro Ser Trp Gly Gly Ala Glu Pro His Glu
210 215 220

Glu Gln Cys Leu Ser Ala Phe Thr Val His Phe Ser Gly Gln Phe Thr
225 230 235 240

Gly Thr Ala Gly Ala Cys Arg Tyr Gly Pro Phe Gly Pro Pro Pro Pro
245 250 255

Ser Gln Ala Ser Ser Gly Gln Ala Arg Met Phe Pro Asn Ala Pro Tyr
260 265 270

Leu Pro Ser Cys Leu Glu Ser Gln Pro Ala Ile Arg Asn Gln Gly Tyr
275 280 285

Ser Thr Val Thr Phe Asp Gly Thr Pro Ser Tyr Gly His Thr Pro Ser
290 295 300

His His Ala Ala Gln Phe Pro Asn His Ser Phe Lys His Glu Asp Pro
305 310 315 320

Met Gly Gln Gln Gly Ser Leu Gly Glu Gln Gln Tyr Ser Val Pro Pro
325 330 335

Pro Val Tyr Gly Cys His Thr Pro Thr Asp Ser Cys Thr Gly Ser Gln
340 345 350

Ala Leu Leu Leu Arg Thr Pro Tyr Ser Ser Asp Asn Leu Tyr Gln Met
355 360 365

Thr Ser Gln Leu Glu Cys Met Thr Trp Asn Gln Met Asn Leu Gly Ala
370 375 380

Thr Leu Lys Gly His Ser Thr Gly Tyr Glu Ser Asp Asn His Thr Thr
385 390 395 400

Pro Ile Leu Cys Gly Ala Gln Tyr Arg Ile His Thr His Gly Val Phe
405 410 415

Arg Gly Ile Gln
420

<210> 394
<211> 362
<212> PRT
<213> Homo sapiens

<400> 394

```
Met His Ser Phe Ile Lys Gln Glu Pro Ser Trp Gly Gly Ala Glu Pro
                1                   5                  10                  15
```

```
His Glu Glu Gln Cys Leu Ser Ala Phe Thr Val His Phe Ser Gly Gln
```

20 25 30

Phe Thr Gly Thr Ala Gly Ala Cys Arg Tyr Gly Pro Phe Gly Pro Pro
35 40 45

Pro Pro Ser Gln Ala Ser Ser Gly Gln Ala Arg Met Phe Pro Asn Ala
50 55 60

Pro Tyr Leu Pro Ser Cys Leu Glu Ser Gln Pro Ala Ile Arg Asn Gln
65 70 75 80

Gly Tyr Ser Thr Val Thr Phe Asp Gly Thr Pro Ser Tyr Gly His Thr
85 90 95

Pro Ser His His Ala Ala Gln Phe Pro Asn His Ser Phe Lys His Glu
100 105 110

Asp Pro Met Gly Gln Gln Gly Ser Leu Gly Glu Gln Gln Tyr Ser Val
115 120 125

Pro Pro Pro Val Tyr Gly Cys His Thr Pro Thr Asp Ser Cys Thr Gly
130 135 140

Ser Gln Ala Leu Leu Leu Arg Thr Pro Tyr Ser Ser Asp Asn Leu Tyr
145 150 155 160

Gln Met Thr Ser Gln Leu Glu Cys Met Thr Trp Asn Gln Met Asn Leu
165 170 175

Gly Ala Thr Leu Lys Gly His Ser Thr Gly Tyr Glu Ser Asp Asn His
180 185 190

Thr Thr Pro Ile Leu Cys Gly Ala Gln Tyr Arg Ile His Thr His Gly
195 200 205

Val Phe Arg Gly Ile Gln Asp Val Arg Arg Val Pro Gly Val Ala Pro
210 215 220

Thr Leu Val Arg Ser Ala Ser Glu Thr Ser Glu Lys Arg Pro Phe Met
225 230 235 240

Cys Ala Tyr Pro Gly Cys Asn Lys Arg Tyr Phe Lys Leu Ser His Leu
245 250 255

Gln Met His Ser Arg Lys His Thr Gly Glu Lys Pro Tyr Gln Cys Asp
260 265 270

Phe Lys Asp Cys Glu Arg Arg Phe Phe Arg Ser Asp Gln Leu Lys Arg
275 280 285

His Gln Arg Arg His Thr Gly Val Lys Pro Phe Gln Cys Lys Thr Cys
290 295 300

Gln Arg Lys Phe Ser Arg Ser Asp His Leu Lys Thr His Thr Arg Thr
305 310 315 320

His Thr Gly Glu Lys Pro Phe Ser Cys Arg Trp Pro Ser Cys Gln Lys
325 330 335

Lys Phe Ala Arg Ser Asp Glu Leu Val Arg His His Asn Met His Gln
340 345 350

Arg Asn Met Thr Lys Leu Gln Leu Ala Leu
355 360

<210> 395
<211> 214
<212> PRT
<213> Homo sapiens

<400> 395

Met His Ser Phe Ile Lys Gln Glu Pro Ser Trp Gly Gly Ala Glu Pro
5 10 15

His Glu Glu Gln Cys Leu Ser Ala Phe Thr Val His Phe Ser Gly Gln
20 25 30

Phe Thr Gly Thr Ala Gly Ala Cys Arg Tyr Gly Pro Phe Gly Pro Pro
35 40 45

Pro Pro Ser Gln Ala Ser Ser Gly Gln Ala Arg Met Phe Pro Asn Ala
50 55 60

Pro Tyr Leu Pro Ser Cys Leu Glu Ser Gln Pro Ala Ile Arg Asn Gln
65 70 75 80

Gly Tyr Ser Thr Val Thr Phe Asp Gly Thr Pro Ser Tyr Gly His Thr
85 90 95

Pro Ser His His Ala Ala Gln Phe Pro Asn His Ser Phe Lys His Glu
100 105 110

Asp Pro Met Gly Gln Gln Gly Ser Leu Gly Glu Gln Gln Tyr Ser Val
115 120 125

Pro Pro Pro Val Tyr Gly Cys His Thr Pro Thr Asp Ser Cys Thr Gly
130 135 140

Ser Gln Ala Leu Leu Leu Arg Thr Pro Tyr Ser Ser Asp Asn Leu Tyr
145 150 155 160

Gln Met Thr Ser Gln Leu Glu Cys Met Thr Trp Asn Gln Met Asn Leu
165 170 175

Gly Ala Thr Leu Lys Gly His Ser Thr Gly Tyr Glu Ser Asp Asn His
180 185 190

Thr Thr Pro Ile Leu Cys Gly Ala Gln Tyr Arg Ile His Thr His Gly
195 200 205

Val Phe Arg Gly Ile Gln
210

<210> 396
<211> 30

<212> DNA
<213> Artificial Sequence

<220>
<223> PCR primer

<400> 396
gacgaaagca tatgcactcc ttcatcaaac 30

<210> 397
<211> 31
<212> DNA
<213> Artificial Sequence

<220>
<223> PCR primer

<400> 397
cgcgtgaatt catcactgaa tgcctctgaa g 31

<210> 398
<211> 31
<212> DNA
<213> Artificial Sequence

<220>
<223> PCR primer

<400> 398
cgataagcat atgacggccg cgtccgataa c 31

<210> 399
<211> 31
<212> DNA
<213> Artificial Sequence

<220>
<223> PCR primer

<400> 399
cgcgtgaatt catcactgaa tgcctctgaa g 31

<210> 400
<211> 31
<212> DNA
<213> Artificial Sequence

<220>
<223> PCR primer

<400> 400
cgataagcat atgacggccg cgtccgataa c 31

<210> 401
<211> 28
<212> DNA
<213> Artificial Sequence

<220>
<223> PCR primer

<400> 401
gtctgcagcg gccgctcaaa gcgccagc 28

<210> 402
<211> 30
<212> DNA
<213> Artificial Sequence

<220>
<223> PCR primer

<400> 402
gacgaaagca tatgcactcc ttcatcaaac 30

<210> 403
<211> 28
<212> DNA
<213> Artificial Sequence

<220>
<223> PCR primer

<400> 403
gtctgcagcg gccgctcaaa gcgccagc 28

<210> 404
<211> 449
<212> PRT
<213> Homo sapiens

<400> 404

```
Met Gly Ser Asp Val Arg Asp Leu Asn Ala Leu Leu Pro Ala Val Pro
 1               5                  10                  15
Ser Leu Gly Gly Gly Gly Gly Cys Ala Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Val His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Phe
```

```
                165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Val Ala Ala Gly Ser Ser Ser
                245                 250                 255
Ser Val Lys Trp Thr Glu Gly Gln Ser Asn His Ser Thr Gly Tyr Glu
            260                 265                 270
Ser Asp Asn His Thr Thr Pro Ile Leu Cys Gly Ala Gln Tyr Arg Ile
        275                 280                 285
His Thr His Gly Val Phe Arg Gly Ile Gln Asp Val Arg Arg Val Pro
    290                 295                 300
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
                325                 330                 335
Leu Ser His Leu Gln Met His Ser Arg Lys His Thr Gly Glu Lys Pro
            340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
                405                 410                 415
Arg Trp Pro Ser Cys Gln Lys Lys Phe Ala Arg Ser Asp Glu Leu Val
            420                 425                 430
Arg His His Asn Met His Gln Arg Asn Met Thr Lys Leu Gln Leu Ala
        435                 440                 445
Leu
```

<210> 405
<211> 428
<212> PRT
<213> Homo sapiens

<400> 405

```
Met Gly Ser Asp Val Arg Asp Leu Asn Ala Leu Leu Pro Ala Val Pro
 1               5                  10                  15
Ser Pro Gly Gly Gly Gly Gly Cys Ala Leu Pro Val Ser Gly Ala Thr
            20                  25                  30
Gln Trp Ala Pro Val Leu Asp Phe Val Pro Pro Gly Ala Pro Val Cys
        35                  40                  45
Gly Ser Leu Gly Gly Pro Ala Pro Pro Pro Ala Pro Pro Pro Leu Pro
    50                  55                  60
Pro Pro Pro Ser His Ser Phe Thr Lys Gln Glu Pro Ser Trp Gly Gly
65                  70                  75                  80
Thr Glu Pro His Ala Gly Gln Gly Arg Ser Ala Leu Val Ala His Ser
                85                  90                  95
Ser Gly Gln Phe Thr Gly Thr Ala Gly Ala Cys Arg Tyr Gly Pro Phe
```

```
                100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Ser
                165                 170                 175
Lys His Glu Asp Pro Met Gly Gln Gln Gly Ser Pro Gly Glu Gln Gln
            180                 185                 190
Tyr Ser Ala Pro Pro Pro Val Cys Gly Cys Arg Thr Pro Thr Gly Ser
        195                 200                 205
Cys Thr Gly Ser Gln Ala Leu Leu Leu Arg Ala Pro Tyr Ser Gly Gly
    210                 215                 220
Asp Leu His Gln Thr Thr Ser Gln Leu Gly His Met Ala Trp Asn Gln
225                 230                 235                 240
Thr Asn Leu Gly Ala Thr Leu Lys Gly His Gly Thr Gly Tyr Glu Ser
                245                 250                 255
Asp Asp His Thr Thr Pro Ile Leu Cys Gly Thr Gln Tyr Arg Ile Arg
            260                 265                 270
Ala Arg Gly Val Leu Arg Gly Thr Gln Asp Val Arg Cys Val Pro Gly
        275                 280                 285
Val Ala Pro Thr Leu Val Arg Ser Ala Ser Glu Thr Ser Glu Lys Arg
    290                 295                 300
Pro Leu Met Cys Ala Tyr Pro Gly Cys Asn Lys Arg His Phe Lys Pro
305                 310                 315                 320
Ser Arg Leu Arg Val Arg Gly Arg Glu Arg Thr Gly Glu Lys Pro Tyr
                325                 330                 335
Gln Arg Asp Phe Lys Asp Arg Gly Arg Gly Leu Leu Arg Pro Asp Gln
            340                 345                 350
Leu Lys Arg His Gln Arg Gly His Thr Gly Val Lys Pro Leu Gln Cys
        355                 360                 365
Glu Ala Arg Arg Arg Pro Pro Arg Pro Gly His Leu Lys Val His Thr
    370                 375                 380
Arg Thr His Thr Gly Gly Glu Pro Phe Ser Cys Arg Trp Pro Ser Cys
385                 390                 395                 400
Gln Glu Lys Ser Ala Arg Pro Asp Glu Ser Ala Arg Arg His Asn Met
                405                 410                 415
His Gln Arg Asn Met Thr Lys Leu Gln Leu Ala Leu
            420                 425
```

<210> 406
<211> 414
<212> PRT
<213> Homo sapiens

<220>
<221> VARIANT
<222> 85, 86, 172, 173, 242, 245, 246, 247
<223> Xaa = Any Amino Acid

<400> 406

```
Met Gly Ser Asp Val Arg Asp Leu Ser Ala Leu Leu Pro Ala Val Pro
 1               5                  10                  15
Ser Leu Gly Asp Gly Gly Gly Cys Ala Leu Pro Val Ser Gly Ala Ala
            20                  25                  30

Gln Trp Ala Pro Val Leu Asp Phe Ala Pro Pro Gly Ala Ser Ala His
        35                  40                  45
Gly Pro Leu Gly Gly Pro Ala Pro Pro Ser Ala Pro Pro Pro Pro Pro
    50                  55                  60
Pro Pro Pro Pro His Ser Phe Ile Lys Gln Gly Pro Ser Trp Gly Gly
65                  70                  75                  80
Ala Glu Leu His Xaa Xaa Gln Tyr Leu Ser Ala Phe Thr Val His Ser
                85                  90                  95
Ser Gly Gln Val His Trp His Gly Arg Gly Leu Ser Leu Arg Ala Pro
            100                 105                 110
Arg Pro Pro Ser Ala Gln Pro Gly Val Ile Arg Pro Gly Gln Asp Val
        115                 120                 125
Ser Arg Ala Leu Pro Ala Gln Pro Pro Arg Glu Pro Ala Arg Tyr Pro
    130                 135                 140
Gln Ser Gly Leu Gln His Gly His Leu Arg Arg Gly Val Arg Leu Arg
145                 150                 155                 160
Ser His Ala Leu Ala Pro Cys Gly Ala Val Leu Xaa Xaa Thr Arg Ala
                165                 170                 175
Gly Ser His Gly Pro Ala Gly Ser Ala Gly Ala Ala Val Leu Gly Ala
            180                 185                 190
Ala Pro Gly Leu Trp Pro Pro His Pro Arg Arg Gln Leu Arg Arg Gln
        195                 200                 205
Pro Gly Phe Ala Ala Glu Gly Ala Leu Gln Arg Arg Phe Ile Pro Ser
    210                 215                 220
Asp Val Pro Ala Val His Gly Leu Glu Ser Asp Glu Pro Arg Gly Arg
225                 230                 235                 240
Leu Xaa Gly Pro Xaa Xaa Xaa Val Arg Glu Arg Ser His Asn Ala Arg
                245                 250                 255
Pro Leu Arg Ser Pro Ile Gln Asn Thr His Ala Arg Cys Leu Gln Gly
            260                 265                 270
Arg Ser Gly Cys Ala Pro Cys Ala Trp Ser Ser Pro Asp Ser Cys Thr
        275                 280                 285
Val Gly Ile Gly Gln Gly Thr Pro Pro His Val Cys Leu Pro Arg Leu
    290                 295                 300
Gln Glu Val Ser Glu Ala Ala Pro Leu Thr Asp Ala Arg Glu Ala Arg
305                 310                 315                 320
Trp Glu Thr Ile Pro Val Leu Gln Gly Leu Trp Thr Glu Val Phe Leu
                325                 330                 335
Leu Arg Pro Ala Gln Lys Thr Pro Gly Glu Ala Tyr Arg Cys Glu Ala
            340                 345                 350
Ile Pro Ala Asp Leu Ser Ala Arg Val Leu Pro Ala Gln Pro Pro Glu
        355                 360                 365
Asp Pro Arg Gln Asp Ser Cys Arg Lys Ala Pro Gln Leu Ser Val Val
    370                 375                 380
Arg Leu Ser Glu Lys Ala Cys Pro Val Lys Val Gly Pro Pro Ser Arg
385                 390                 395                 400
His Ala Ser Glu Gly His Asp Arg Thr Pro Ala Gly Ala Leu
                405                 410
```

<210> 407
<211> 417
<212> PRT

<213> Homo sapiens

<400> 407

Met Gly Ser Asp Val Arg Asp Leu Ser Ala Leu Leu Pro Thr Ala Pro
1 5 10 15

```
Ser Leu Gly Gly Gly Gly Asp Cys Thr Leu Pro Val Ser Gly Thr Ala
            20                  25                  30
Gln Trp Ala Pro Val Pro Ala Ser Ala Pro Pro Gly Ala Ser Ala Tyr
        35                  40                  45
Asp Ser Leu Gly Gly Pro Ala Pro Pro Pro Ala Pro Pro Pro Pro Pro
    50                  55                  60
Pro Pro Pro Pro His Ser Cys Gly Glu Gln Gly Pro Ser Trp Gly Gly
65                  70                  75                  80
Ala Glu Pro Arg Glu Gly Gln Cys Leu Ser Ala Pro Ala Val Arg Phe
                85                  90                  95
Ser Gly Arg Phe Thr Gly Thr Val Gly Ala Cys Arg Tyr Gly Pro Leu
            100                 105                 110
Gly Pro Pro Pro Pro Ser Gln Ala Pro Ser Gly Gln Thr Arg Met Leu
        115                 120                 125
Pro Ser Ala Pro Tyr Leu Ser Ser Cys Leu Arg Ser Arg Ser Ala Ile
    130                 135                 140
Arg Ser Gln Gly Arg Ser Thr Ala Pro Ser Ala Gly Arg Pro Ala Met
145                 150                 155                 160
Ala Pro Thr Leu Ala Pro Pro Ala Gln Ser His Tyr Ser Gln His Gly
                165                 170                 175
Val Leu His Gly Pro Ala Gly Leu Ala Gly Ala Ala Val Leu Gly Ala
            180                 185                 190
Ala Pro Gly Leu Trp Leu Pro His Pro His Arg Gln Leu His Arg Gln
        195                 200                 205
Pro Gly Phe Ala Ala Glu Asp Ala Leu Gln Gln Gln Phe Ile Pro Asn
    210                 215                 220
Asp Ile Pro Ala Met His Asp Leu Glu Ser Asp Glu Leu Arg Ser His
225                 230                 235                 240
Leu Lys Gly Pro Gln His Arg Val Arg Glu Arg Pro His Asn Ala His
                245                 250                 255
Pro Leu Arg Ser Pro Ile Gln Asn Thr His Ala Arg Cys Leu Gln Arg
            260                 265                 270
His Ser Gly Cys Ala Thr Cys Ala Trp Ser Ser Pro Asp Ser Cys Thr
        275                 280                 285
Val Ala Pro Glu Thr Ser Glu Asn Ala Pro Trp Cys Val Leu Pro Gly
    290                 295                 300
Leu Gln Gly Val Phe Ala Val Pro Leu Thr Gly Ala Gln Gln Glu Ala
305                 310                 315                 320
His Trp Asp Ala Thr Pro Val Arg Leu Gln Gly Pro Trp Thr Arg Ala
                325                 330                 335
Ser Pro Phe Gly Thr Ser Pro Arg Asp Thr Lys Gly Asp Ile Gln Val
            340                 345                 350
Arg Asn His Ser Ser Val Arg Leu Val Ser Glu Gly Ser Pro Gly Pro
        355                 360                 365
Thr Thr Gly Pro Thr Pro Gly Pro Thr Arg Val Gly Ser Pro Ser Ala
    370                 375                 380
Ala Gly Gly Gln Ala Ala Arg Glu Gly Ser Pro Ser Gln Thr Asn Ser
385                 390                 395                 400
Val Ile Thr Thr Cys Ile Ser Glu Thr Leu Asn Ser Ser Trp Arg Phe
                405                 410                 415
Glu
```

<210> 408
<211> 429
<212> PRT
<213> Homo sapiens

<400> 408

```
Met Gly Ser Asp Val Arg Asp Leu Asn Ala Leu Leu Pro Ala Val Pro
 1               5                  10                  15
Ser Leu Gly Gly Gly Gly Gly Cys Ala Leu Pro Val Ser Gly Ala Ala
            20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Val His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Phe
                165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
        195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly His Ser Thr Gly Tyr Glu Ser
                245                 250                 255
Asp Asn His Thr Thr Pro Ile Leu Cys Gly Ala Gln Tyr Arg Ile His
            260                 265                 270
Thr His Gly Val Phe Arg Gly Ile Gln Asp Val Arg Arg Val Pro Gly
        275                 280                 285
Val Ala Pro Thr Leu Val Arg Ser Ala Ser Glu Thr Ser Glu Lys Arg
    290                 295                 300
Pro Phe Met Cys Ala Tyr Pro Gly Cys Asn Lys Arg Tyr Phe Lys Leu
305                 310                 315                 320
Ser His Leu Gln Met His Ser Arg Lys His Thr Gly Glu Lys Pro Tyr
                325                 330                 335
Gln Cys Asp Phe Lys Asp Cys Glu Arg Arg Phe Phe Arg Ser Asp Gln
            340                 345                 350
Leu Lys Arg His Gln Arg Arg His Thr Gly Val Lys Pro Phe Gln Cys
        355                 360                 365
Lys Thr Cys Gln Arg Lys Phe Ser Arg Ser Asp His Leu Lys Thr His
    370                 375                 380
Thr Arg Thr His Thr Gly Glu Lys Pro Phe Ser Cys Arg Trp Pro Ser
385                 390                 395                 400
Cys Gln Lys Lys Phe Ala Arg Ser Asp Glu Leu Val Arg His His Asn
                405                 410                 415
Met His Gln Arg Asn Met Thr Lys Leu Gln Leu Ala Leu
            420                 425
```

<210> 409
<211> 495
<212> PRT
<213> Homo sapiens

<400> 409

```
Met Ala Ala Pro Gly Ala Arg Arg Ser Leu Leu Leu Leu Leu Leu Ala
1               5                   10                  15
Gly Leu Ala His Gly Ala Ser Ala Leu Phe Glu Asp Leu Met Gly Ser
            20                  25                  30
Asp Val Arg Asp Leu Asn Ala Leu Leu Pro Ala Val Pro Ser Leu Gly
        35                  40                  45
Gly Gly Gly Gly Cys Ala Leu Pro Val Ser Gly Ala Ala Gln Trp Ala
    50                  55                  60
Pro Val Leu Asp Phe Ala Pro Pro Gly Ala Ser Ala Tyr Gly Ser Leu
65                  70                  75                  80
Gly Gly Pro Ala Pro Pro Pro Ala Pro Pro Pro Pro Pro Pro Pro His
                85                  90                  95
Ser Phe Ile Lys Gln Glu Pro Ser Trp Gly Gly Ala Glu Pro His Glu
            100                 105                 110
Glu Gln Cys Leu Ser Ala Phe Thr Val His Phe Ser Gly Gln Phe Thr
        115                 120                 125
Gly Thr Ala Gly Ala Cys Arg Tyr Gly Pro Phe Gly Pro Pro Pro Pro
    130                 135                 140
Ser Gln Ala Ser Ser Gly Gln Ala Arg Met Phe Pro Asn Ala Pro Tyr
145                 150                 155                 160
Leu Pro Ser Cys Leu Glu Ser Gln Pro Ala Ile Arg Asn Gln Gly Tyr
                165                 170                 175
Ser Thr Val Thr Phe Asp Gly Thr Pro Ser Tyr Gly His Thr Pro Ser
            180                 185                 190
His His Ala Ala Gln Phe Pro Asn His Ser Phe Lys His Glu Asp Pro
        195                 200                 205
Met Gly Gln Gln Gly Ser Leu Gly Glu Gln Gln Tyr Ser Val Pro Pro
    210                 215                 220
Pro Val Tyr Gly Cys His Thr Pro Thr Asp Ser Cys Thr Gly Ser Gln
225                 230                 235                 240
Ala Leu Leu Leu Arg Thr Pro Tyr Ser Ser Asp Asn Leu Tyr Gln Met
                245                 250                 255
Thr Ser Gln Leu Glu Cys Met Thr Trp Asn Gln Met Asn Leu Gly Ala
            260                 265                 270
Thr Leu Lys Gly His Ser Thr Gly Tyr Glu Ser Asp Asn His Thr Thr
        275                 280                 285
Pro Ile Leu Cys Gly Ala Gln Tyr Arg Ile His Thr His Gly Val Phe
    290                 295                 300
Arg Gly Ile Gln Asp Val Arg Arg Val Pro Gly Val Ala Pro Thr Leu
305                 310                 315                 320
Val Arg Ser Ala Ser Glu Thr Ser Glu Lys Arg Pro Phe Met Cys Ala
                325                 330                 335
Tyr Pro Gly Cys Asn Lys Arg Tyr Phe Lys Leu Ser His Leu Gln Met
            340                 345                 350
His Ser Arg Lys His Thr Gly Glu Lys Pro Tyr Gln Cys Asp Phe Lys
        355                 360                 365
Asp Cys Glu Arg Arg Phe Phe Arg Ser Asp Gln Leu Lys Arg His Gln
    370                 375                 380
Arg Arg His Thr Gly Val Lys Pro Phe Gln Cys Lys Thr Cys Gln Arg
385                 390                 395                 400
Lys Phe Ser Arg Ser Asp His Leu Lys Thr His Thr Arg Thr His Thr
                405                 410                 415
```

```
Gly Glu Lys Pro Phe Ser Cys Arg Trp Pro Ser Cys Gln Lys Lys Phe
            420                 425                     430
Ala Arg Ser Asp Glu Leu Val Arg His His Asn Met His Gln Arg Asn
        435                 440                 445
Met Thr Lys Leu Gln Leu Ala Leu Leu Asn Asn Met Leu Ile Pro Ile
    450                 455                 460
Ala Val Gly Gly Ala Leu Ala Gly Leu Val Leu Ile Val Leu Ile Ala
465                 470                 475                     480
Tyr Leu Ile Gly Arg Lys Arg Ser His Ala Gly Tyr Gln Thr Ile
                485                 490                     495
```

<210> 410
<211> 504
<212> PRT
<213> Homo sapiens

<400> 410

```
Met Gln Ile Phe Val Lys Thr Leu Thr Gly Lys Thr Ile Thr Leu Glu
 1               5                  10                  15
Val Glu Pro Ser Asp Thr Ile Glu Asn Val Lys Ala Lys Ile Gln Asp
            20                  25                  30
Lys Glu Gly Ile Pro Pro Asp Gln Gln Arg Leu Ile Phe Ala Gly Lys
        35                  40                  45
Gln Leu Glu Asp Gly Arg Thr Leu Ser Asp Tyr Asn Ile Gln Lys Glu
    50                  55                  60
Ser Thr Leu His Leu Val Leu Arg Leu Arg Gly Ala Met Gly Ser Asp
65                  70                  75                  80
Val Arg Asp Leu Asn Ala Leu Leu Pro Ala Val Pro Ser Leu Gly Gly
                85                  90                  95
Gly Gly Gly Cys Ala Leu Pro Val Ser Gly Ala Ala Gln Trp Ala Pro
            100                 105                 110
Val Leu Asp Phe Ala Pro Pro Gly Ala Ser Ala Tyr Gly Ser Leu Gly
        115                 120                 125
Gly Pro Ala Pro Pro Pro Ala Pro Pro Pro Pro Pro Pro Pro Pro His
    130                 135                 140
Ser Phe Ile Lys Gln Glu Pro Ser Trp Gly Gly Ala Glu Pro His Glu
145                 150                 155                 160
Glu Gln Cys Leu Ser Ala Phe Thr Val His Phe Ser Gly Gln Phe Thr
                165                 170                 175
Gly Thr Ala Gly Ala Cys Arg Tyr Gly Pro Phe Gly Pro Pro Pro Pro
            180                 185                 190
Ser Gln Ala Ser Ser Gly Gln Ala Arg Met Phe Pro Asn Ala Pro Tyr
        195                 200                 205
Leu Pro Ser Cys Leu Glu Ser Gln Pro Ala Ile Arg Asn Gln Gly Tyr
    210                 215                 220
Ser Thr Val Thr Phe Asp Gly Thr Pro Ser Tyr Gly His Thr Pro Ser
225                 230                 235                 240
His His Ala Ala Gln Phe Pro Asn His Ser Phe Lys His Glu Asp Pro
                245                 250                 255
Met Gly Gln Gln Gly Ser Leu Gly Glu Gln Gln Tyr Ser Val Pro Pro
            260                 265                 270
Pro Val Tyr Gly Cys His Thr Pro Thr Asp Ser Cys Thr Gly Ser Gln
        275                 280                 285
Ala Leu Leu Leu Arg Thr Pro Tyr Ser Ser Asp Asn Leu Tyr Gln Met
    290                 295                 300
Thr Ser Gln Leu Glu Cys Met Thr Trp Asn Gln Met Asn Leu Gly Ala
305                 310                 315                 320
```

```
Thr Leu Lys Gly His Ser Thr Gly Tyr Glu Ser Asp Asn His Thr Thr
                325                 330                 335
Pro Ile Leu Cys Gly Ala Gln Tyr Arg Ile His Thr His Gly Val Phe
            340                 345                 350
Arg Gly Ile Gln Asp Val Arg Arg Val Pro Gly Val Ala Pro Thr Leu
        355                 360                 365
Val Arg Ser Ala Ser Glu Thr Ser Glu Lys Arg Pro Phe Met Cys Ala
    370                 375                 380
Tyr Pro Gly Cys Asn Lys Arg Tyr Phe Lys Leu Ser His Leu Gln Met
385                 390                 395                 400
His Ser Arg Lys His Thr Gly Glu Lys Pro Tyr Gln Cys Asp Phe Lys
                405                 410                 415
Asp Cys Glu Arg Arg Phe Phe Arg Ser Asp Gln Leu Lys Arg His Gln
            420                 425                 430
Arg Arg His Thr Gly Val Lys Pro Phe Gln Cys Lys Thr Cys Gln Arg
        435                 440                 445
Lys Phe Ser Arg Ser Asp His Leu Lys Thr His Thr Arg Thr His Thr
    450                 455                 460
Gly Glu Lys Pro Phe Ser Cys Arg Trp Pro Ser Cys Gln Lys Lys Phe
465                 470                 475                 480
Ala Arg Ser Asp Glu Leu Val Arg His His Asn Met His Gln Arg Asn
                485                 490                 495
Met Thr Lys Leu Gln Leu Ala Leu
            500
```

<210> 411
<211> 10
<212> PRT
<213> Homo sapiens

<400> 411

```
Val Leu Asp Phe Ala Pro Pro Gly Ala Ser
1               5                   10
```

<210> 412
<211> 15
<212> PRT
<213> Homo sapiens

<400> 412

```
Gln Trp Ala Pro Val Leu Asp Phe Ala Pro Pro Gly Ala Ser Ala
1               5                   10                  15
```

<210> 413
<211> 15
<212> PRT
<213> Homo sapiens

<400> 413

Val Leu Asp Phe Ala Pro Pro Gly Ala Ser Ala Tyr Gly Ser Leu
1 5 10 15

## Description

This invention was made in part with government support under NIH SBIR Phase I grant number IR43 CA81752-01A1. The Government may have certain rights in this invention.

BACKGROUND OF THE INVENTION

Field of the Invention

The present invention relates generally to the immunotherapy of malignant diseases such as leukemia and cancers. The invention is more specifically related to compositions for generating or enhancing an immune response to WT1, and to the use of such compositions for preventing and/or treating malignant diseases.

Description of the Related Art

Cancer and leukemia are significant health problems in the United States and throughout the world. Although advances have been made in detection and treatment of such diseases, no vaccine or other universally successful method for prevention or treatment of cancer and leukemia is currently available. Management of the diseases currently relies on a combination of early diagnosis and aggressive treatment, which may include one or more of a variety of treatments such as surgery, radiotherapy, chemotherapy and hormone therapy. The course of treatment for a particular cancer is often selected based on a variety of prognostic parameters, including an analysis of specific tumor markers. However, the use of established markers often leads to a result that is difficult to interpret, and the high mortality continues to be observed in many cancer patients.

Immunotherapies have the potential to substantially improve cancer and leukemia treatment and survival. Recent data demonstrate that leukemia can be cured by immunotherapy in the context of bone marrow transplantation (e.g., donor lymphocyte infusions). Such therapies may involve the generation or enhancement of an immune response to a tumor-associated antigen (TAA). However, to date relatively few TAAs are known and the generation of an immune response against such antigens has, with rare exception, not been shown to be therapeutically beneficial.

Accordingly, there is a need in the art for improved methods for leukemia and cancer prevention and therapy. The present invention fulfills these needs and further provides other related advantages.

In a first aspect of the present invention there is provided an isolated polypeptide consisting of amino acids 1-281 of the WT1 polypeptide set forth in SEQ ID No. 408 and wherein the ability of said polypeptide to react with WT1-specific T-cell lines or clones is not substantially diminished relative to the WT1 polypeptide set forth in SEQ ID No. 408.

There is also provided an isolated polynucleotide that encodes the polypeptide of the present invention, together with a expression vector comprising the isolated polynucleotide of the present invention.

In another aspect of the present invention there is provided an isolated host cell transformed or transfected with the expression vector of the present invention.

There are also provided pharmaceutical and vaccine compositions including the polypeptide of the present invention.

There is also provided the polypeptide of the present invention for use in therapy, in particular, their use in the treatment of malignant diseases.

Briefly stated, this invention provides compositions and their use in methods for the therapy of diseases such as leukemia and cancer. In one aspect, the present invention provides polypeptides comprising an immunogenic portion of a native WT1, or a variant thereof that differs in one or more substitutions, deletions, additions and/or insertions such that the ability of the variant to react with antigen-specific antisera and/or T-cell lines or clones is not substantially diminished. The immunogenic portion preferably binds to an MHC class I and/or class II molecule.

Within further aspects, the present invention provides polypeptides comprising a variant of an immunogenic portion of a WT1 protein, wherein the variant differs from the immunogenic portion due to substitutions at between 1 and 3 amino acid positions within the immunogenic portion such that the ability of the variant to react with antigen-specific antisera and/or T-cell lines or clones is enhanced relative to a native WT1 protein.

The present invention further provides WT1 polynucleotides that encode a WT1 polypeptide of the invention as described above.

Within other aspects, the present invention provides pharmaceutical compositions and vaccines. The immune response enhancer for use with the vaccines of the present invention may be an adjuvant. Preferably, an immune response enhancer enhances a T cell response.

There is also disclosed methods for enhancing or inducing an immune response in a patient, comprising administering to a patient a pharmaceutical composition or vaccine as described above. In certain embodiments, the patient is a human.

The present disclosure further provides methods for inhibiting the development of a malignant disease in a patient, comprising administering to a patient a pharmaceutical composition or vaccine as described above. Malignant diseases include, but are not limited to leukemias (*e.g.,* acute myeloid, acute lymphocytic and chronic myeloid) and cancers (e.g., breast, lung, thyroid or gastrointestinal cancer or a melanoma). The patient may, but need not, be afflicted with the malignant disease, and the administration of the pharmaceutical composition or vaccine may inhibit the onset of such a disease, or may inhibit progression and/or metastasis of an existing disease.

The present disclosure further provides, within other aspects, methods for removing cells expressing WT1 from bone marrow and/or peripheral blood or fractions thereof, comprising contacting bone marrow, peripheral blood or a fraction of bone marrow or peripheral blood with T cells that specifically react with a WT1 polypeptide, wherein the step of contacting is performed under conditions and for a time sufficient to permit the removal of WT1 positive cells to less than 10%, preferably less than 5% and more preferably less than 1%, of the number of myeloid or lymphatic cells in the bone marrow, peripheral blood or fraction. Bone marrow, peripheral blood and fractions may be obtained from a patient afflicted with a disease associated with WT1 expression, or may be obtained from a human or non-human mammal not afflicted with such a disease.

Within related aspects, the present disclosure provides methods for inhibiting the development of a malignant disease in a patient, comprising administering to a patient bone marrow, peripheral blood or a fraction of bone marrow or peripheral blood prepared as described above. Such bone marrow, peripheral blood or fractions may be autologous, or may be derived from a related or unrelated human or non-human animal (*e.g.*, syngeneic or allogeneic).

In other aspects, the present disclosure provides methods for stimulating (or priming) and/or expanding T cells, comprising contacting T cells with a WT1 polypeptide under conditions and for a time sufficient to permit the stimulation and/or expansion of T cells. Such T cells may be autologous, allogeneic, syngeneic or unrelated WT1-specific T cells, and may be stimulated *in vitro* or *in vivo.* Expanded T cells may, within certain embodiments, be present within bone marrow, peripheral blood or a fraction of bone marrow or peripheral blood, and may (but need not) be clonal. Within certain embodiments, T cells may be present in a mammal during stimulation and/or expansion. WT1-specific T cells may be used, for example, within donor lymphocyte infusions.

Within related disclosed aspects, methods are provided for inhibiting the development of a malignant disease in a patient, comprising administering to a patient T cells prepared as described above. Such T cells may, within certain embodiments, be autologous, syngeneic or allogeneic.

The present disclosure further provides, within other aspects, methods for monitoring the effectiveness of an immunization or therapy for a malignant disease associated with WT1 expression in a patient. Such methods are based on monitoring antibody, CD4+ T cell and/or CD8+ T cell responses in the patient. Within certain such aspects, a method may comprise the steps of: (a) incubating a first biological sample with one or more of: (i) a WT1 polypeptide; (ii) a polynucleotide encoding a WT1 polypeptide; or (iii) an antigen presenting cell that expresses a WT1 polypeptide, wherein the first biological sample is obtained from a patient prior to a therapy or immunization, and wherein the incubation is performed under conditions and for a time sufficient to allow immunocomplexes to form; (b) detecting immunocomplexes formed between the WT1 polypeptide and antibodies in the biological sample that specifically bind to the WT1 polypeptide; (c) repeating steps (a) and (b) using a second biological sample obtained from the same patient following therapy or immunization; and (d) comparing the number of immunocomplexes detected in the first and second biological samples, and therefrom monitoring the effectiveness of the therapy or immunization in the patient. disease in the patient. Within certain embodiments, the step of incubating the T cells may be repeated one or more times.

Within other aspects, the present disclosure provides methods for inhibiting the development of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide, such that the T cells proliferate; (b) cloning one or more cells that proliferated; and (c) administering to the patient an effective amount of the cloned T cells.

Methods are also disclosed for determining the presence or absence of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide; and (b) detecting the presence or absence of specific activation of the T cells, therefrom determining the presence or absence of a malignant disease associated with WT1 expression. Within certain embodiments, the step of detecting comprises detecting the presence or absence of proliferation of the T cells.

Methods for determining the presence or absence of a malignant disease associated with WT1 expression in a patient are also disclosed, comprising the steps of: (a) incubating a biological sample obtained from a patient with one or more of: (i) a WT1 polypeptide; (ii) a polynucleotide encoding a WT1 polypeptide; or (iii) an antigen presenting cell that expresses a WT1 polypeptide, wherein the incubation is performed under conditions and for a time sufficient to allow immunocomplexes to form; and (b) detecting immunocomplexes formed between the WT1 polypeptide and antibodies in the biological sample that specifically bind to the WT1 polypeptide; and therefrom determining the presence or absence of a malignant disease associated with WT1 expression.

Within certain embodiments of the above disclosed methods, the step of detecting comprises (a) incubating the immunocomplexes with a detection reagent that is capable of binding to the immunocomplexes, wherein the detection reagent comprises a reporter group, (b) removing unbound detection reagent, and (c) detecting the presence or absence of the reporter group. The detection reagent may comprise, for example, a second antibody, or antigen-binding fragment thereof, capable of binding to the antibodies that specifically bind to the WT1 polypeptide or a molecule such as Protein A. Within other embodiments, a reporter group is bound to the WT1 polypeptide, and the step of detecting comprises removing unbound WT1 polypeptide and subsequently detecting the presence or absence of the reporter group.

Within further disclosed aspects, methods for monitoring the effectiveness of an immunization or therapy for a malignant disease associated with WT1 expression in a patient may comprise the steps of: (a) incubating a first biological sample with one or more of: (i) a WT1 polypeptide; (ii) a polynucleotide encoding a WT1 polypeptide; or (iii) an antigen presenting cell that expresses a WT1 polypeptide, wherein the biological sample comprises CD4+ and/or CD8+ T cells and is obtained from a patient prior to a therapy or immunization, and wherein the incubation is performed under conditions and for a time sufficient to allow specific activation, proliferation and/or lysis of T cells; (b) detecting an amount of activation, proliferation and/or lysis of the T cells; (c) repeating steps (a) and (b) using a second biological sample comprising CD4+ and/or CD8+ T cells, wherein the second biological sample is obtained from the same patient following therapy or immunization; and (d) comparing the amount of activation, proliferation and/or lysis of T cells in the first and second biological samples, and therefrom monitoring the effectiveness of the therapy or immunization in the patient.

The present disclosure further provides methods for inhibiting the development of a malignant disease associated with WT1 expression in a patient, comprising the steps of: (a) incubating $CD4^+$ and/or CD8+ T cells isolated from a patient with one or more of: (i) a WT1 polypeptide; (ii) a polynucleotide encoding a WT1 polypeptide; or (iii) an antigen presenting cell that expresses a WT1 polypeptide, such that the T cells proliferate; and (b) administering to the patient an effective amount of the proliferated T cells, and therefrom inhibiting the development of a malignant

These and other aspects of the present invention will become apparent upon reference to the following detailed description and attached drawings.

BRIEF DESCRIPTION OF THE DRAWINGS

Figure 1 depicts a comparison of the mouse (MO) and human (HU) WT1 protein sequences (SEQ ID NOS: 320 and 319 respectively).

Figure 2 is a Western blot illustrating the detection of WT1 specific antibodies in patients with hematological malignancy (AML). Lane 1 shows molecular weight markers; lane 2 shows a positive control (WT1 positive human leukemia cell line immunoprecipitated with a WT1 specific antibody); lane 3 shows a negative control (WT1 positive cell line immunoprecipitated with mouse sera); and lane 4 shows a WT1 positive cell line immunoprecipitated with sera of a patient with AML. For lanes 2-4, the immunoprecipitate was separated by gel electrophoresis and probed with a WT1 specific antibody.

Figure 3 is a Western blot illustrating the detection of a WT1 specific antibody response in B6 mice immunized with TRAMP-C, a WT1 positive tumor cell line. Lanes 1, 3 and 5 show molecular weight markers, and lanes 2, 4 and 6 show a WT1 specific positive control (N180, Santa Cruz Biotechnology, polypeptide spanning 180 amino acids of the N-terminal region of the WT1 protein, migrating on the Western blot at 52 kD). The primary antibody used was WT180 in lane 2, sera of non-immunized B6 mice in lane 4 and sera of the immunized B6 mice in lane 6.

Figure 4 is a Western blot illustrating the detection of WT1 specific antibodies in mice immunized with representative WT1 peptides. Lanes 1, 3 and 5 show molecular weight markers and lanes 2, 4 and 6 show a WT1 specific positive control (N180, Santa Cruz Biotechnology, polypeptide spanning 180 amino acids of the N-terminal region of the WT1 protein, migrating on the Western blot at 52 kD). The primary antibody used was WT180 in lane 2, sera of non-immunized B6 mice in lane 4 and sera of the immunized B6 mice in lane 6.

Figures 5A to 5C are graphs illustrating the stimulation of proliferative T cell responses in mice immunized with representative WT1 peptides. Thymidine incorporation assays were performed using one T cell line and two different clones, as indicated, and results were expressed as cpm. Controls indicated on the x axis were no antigen (No Ag) and B6/media; antigens used were p6-22 human (p1), p117-139 (p2) or p244-262 human (p3).

Figure 6A and 6B are histograms illustrating the stimulation of proliferative T cell responses in mice immunized with representative WT1 peptides. Three weeks after the third immunization, spleen cells of mice that had been inoculated with Vaccine A or Vaccine B were cultured with medium alone (medium) or spleen cells and medium (B6/no antigen), B6 spleen cells pulsed with the peptides p6-22 (p6), p117-139 (p117), p244-262 (p244) (Vaccine A; Figure 6A) or p287-301 (p287), p299-313 (p299), p421-435 (p421) (Vaccine B; Figure 6B) and spleen cells pulsed with an irrelevant control peptide (irrelevant peptide) at 25ug/ml and were assayed after 96hr for proliferation by ($^3$H) thymidine incor-

poration. Bars represent the stimulation index (SI), which is calculated as the mean of the experimental wells divided by the mean of the control (B6 spleen cells with no antigen).

Figures 7A-7D are histograms illustrating the generation of proliferative T-cell lines and clones specific for p117-139 and p6-22. Following *in vivo* immunization, the initial three *in vitro* stimulations (IVS) were carried out using all three peptides of Vaccine A or B, respectively. Subsequent IVS were carried out as single peptide stimulations using only the two relevant peptides p117-139 and p6-22. Clones were derived from both the p6-22 and p117-139 specific T cell lines, as indicated. T cells were cultured with medium alone (medium) or spleen cells and medium (B6/no antigen), B6 spleen cells pulsed with the peptides p6-22 (p6), p1 17-139 (p117) or an irrelevant control peptide (irrelevant peptide) at 25ug/ml and were assayed after 96hr for proliferation by ($^3$H) thymidine incorporation. Bars represent the stimulation index (SI), which is calculated as the mean of the experimental wells divided by the mean of the control (B6 spleen cells with no antigen).

Figures 8A and 8B present the results of TSITES Analysis of human WT1 (SEQ ID NO:319) for peptides that have the potential to elicit Th responses. Regions indicated by "A" are AMPHI midpoints of blocks, "R" indicates residues matching the Rothbard/'Taylor motif, "D" indicates residues matching the IAd motif, and 'd' indicates residues matching the IEd motif.

Figures 9A and 9B are graphs illustrating the elicitation of WT1 peptide-specific CTL in mice immunized with WT1 peptides. Figure 9A illustrates the lysis of target cells by allogeneic cell lines and Figure 9B shows the lysis of peptide coated cell lines. In each case, the % lysis (as determined by standard chromium release assays) is shown at three indicated effector:target ratios. Results are provided for lymphoma cells (LSTRA and E10), as well as E10 + p235-243 (E10+P235). E10 cells are also referred to herein as EL-4 cells.

Figures 10A-10D are graphs illustrating the elicitation of WT1 specific CTL, which kill WT1 positive tumor cell lines but do not kill WT1 negative cell lines, following vaccination of B6 mice with WT1 peptide P117. Figure 10A illustrates that T-cells of non-immunized B6 mice do not kill WT1 positive tumor cell lines. Figure 10B illustrates the lysis of the target cells by allogeneic cell lines. Figures 10C and 10D demonstrate the lysis of WT1 positive tumor cell lines, as compared to WT1 negative cell lines in two different experiments. In addition, Figures 10C and 10D show the lysis of peptide-coated cell lines (WT1 negative cell line E10 coated with the relevant WT1 peptide P117) In each case; the % lysis (as determined by standard chromium release assays) is shown at three indicated effector:target ratios. Results are provided for lymphoma cells (E10), prostate cancer cells (TRAMP-C), a transformed fibroblast cell line (BLK-SV40), as well as E10+p117.

Figures 11A and 11B are histograms illustrating the ability of representative peptide P117-139 specific CTL to lyse WT1 positive tumor cells. Three weeks after the third immunization, spleen cells of mice that had been inoculated with the peptides p235-243 or p117-139 were stimulated *in vitro* with the relevant peptide and tested for ability to lyse targets incubated with WT1 peptides as well as WT1 positive and negative tumor cells. The bars represent the mean % specific lysis in chromium release assays performed in triplicate with an E:T ratio of 25:1. Figure 11A shows the cytotoxic activity of the p235-243 specific T cell line against the WT1 negative cell line EL-4 (EL-4, WT1 negative); EL-4 pulsed with the relevant (used for immunization as well as for restimulation) peptide p235-243 (EL-4+p235); EL-4 pulsed with the irrelevant peptides p117-139 (EL-4+p117), p126-134 (EL-4+p126) or p130-138 (EL-4+p130) and the WT1 positive tumor cells BLK-SV40 (BLK-SV40, WT1 positive) and TRAMP-C (TRAMP-C, WT1 positive), as indicated. Figure 11B shows cytotoxic activity of the p117-139 specific T cell line against EL-4; EL-4 pulsed with the relevant peptide P117-139 (EL-4+p117) and EL-4 pulsed with the irrelevant peptides p123-131 (EL-4+p123), or p128-136 (EL-4+p128); BLK-SV40 and TRAMP-C, as indicated.

Figures 12A and 12B are histograms illustrating the specificity of lysis of WT1 positive tumor cells, as demonstrated by cold target inhibition. The bars represent the mean % specific lysis in chromium release assays performed in triplicate with an E:T ratio of 25:1. Figure 12A shows the cytotoxic activity of the p117-139 specific T cell line against the WT1 negative cell line EL-4 (EL-4, WT1 negative); the WT1 positive tumor cell line TRAMP-C (TRAMP-C, WT1 positive); TRAMP-C cells incubated with a ten-fold excess (compared to the hot target) of EL-4 cells pulsed with the relevant peptide p117-139 (TRAMP-C + p117 cold target) without $^{51}$Cr labeling and TRAMP-C cells incubated with EL-4 pulsed with an irrelevant peptide without $^{51}$Cr labeling (TRAMP-C + irrelevant cold target), as indicated. Figure 12B shows the cytotoxic activity of the p117-139 specific T cell line against the WT1 negative cell line EL-4 (EL-4, WT1 negative); the WT1 positive tumor cell line BLK-SV40 (BLK-SV40, WT1 positive); BLK-SV40 cells incubated with the relevant cold target (BLK-SV40 + p117 cold target) and BLK-SV40 cells incubated with the irrelevant cold target (BLK-SV40 + irrelevant cold target), as indicated.

Figures 13A-13C are histograms depicting an evaluation of the 9mer CTL epitope within p117-139. The p117-139 tumor specific CTL line was tested against peptides within aa117-139 containing or lacking an appropriate H-$2^b$ class I binding motif and following restimulation with p126-134 or p130-138. The bars represent the mean % specific lysis in chromium release assays performed in triplicate with an E:T ratio of 25:1. Figure 13A shows the cytotoxic activity of the p117-139 specific T cell line against the WT1 negative cell line EL-4 (EL-4, WT1 negative) and EL-4 cells pulsed with the peptides p117-139 (EL-4 + p117), p119-127 (EL-4 + p119), p120-128 (EL-4 + p120), p123-131 (EL-4 + p123), p126-134 (EL-4 + p126), p128-136 (EL-4 + p128), and p130-138 (EL-4 + p130). Figure 13B shows the cytotoxic activity of the CTL line after restimulation with p126-134 against the WT1 negative cell line EL-4, EL-4 cells pulsed with p117-139 (EL-4 + p117), p126-134 (EL-4 + p126) and the WT1 positive tumor cell line TRAMP-C. Figure 13C shows the cytotoxic activity of the CTL line after restimulation with p130-138 against EL-4, EL-4 cells pulsed with p117-139 (EL-4 + p117), p130-138 (EL-4 + p130) and the WT1 positive tumor cell line TRAMP-C.

Figure 14 depicts serum antibody reactivity to WT1 in 63 patients with AML. Reactivity of serum antibody to WT1/N-terminus protein was evaluated by ELISA in patients with AML. The first and second lanes represent the positive and negative controls, respectively. The first and second lanes represent the ositive and negative controls, respectively. Commercially obtained WT1 specific antibody WT180 was used for the positive control. The next 63 lanes represent results using sera from each individual patient. The OD values depicted were from ELISA using a 1:500 serum dilution. The figure includes cumulative data from 3 separate experiments.

Figure 15 depicts serum antibody reactivity to WT1 proteins and control proteins in 2 patients with AML. Reactivity of serum antibody to WT1/full-length, WT1N-terminus, TRX and Ra12 proteins was evaluated by ELISA in 2 patients with AML. The OD values depicted were from ELISA using a 1:500 serum dilution. AML-1 and AML-2 denote serum from 2 of the individual patients in Figure 1 with demonstrated antibody reactivity to WT1/full-length. The WT1 full-length protein was expressed as a fusion protein with Ra12. The WT1/N-terminus protein was expressed as a fusion protein with TRX. The control Ra12 and TRX proteins were purified in a similar manner. The results confirm that the serum antibody reactivity against the WT1 fusion proteins is directed against the WT1 portions of the protein.

Figure 16 depicts serum antibody reactivity to WT1 in 81 patients with CML. Reactivity of serum antibody to WT1/full-length protein was evaluated by ELISA in patients with AML. The first and second lanes represent the positive and negative controls, respectively. Commercially obtained WT1 specific antibody WT180 was used for the positive control. The next 81 lanes represent results using sera from each individual patient. The OD values depicted were from ELISA using a 1:500 serum dilution. The figure includes cumulative data from 3 separate experiments.

Figure 17 depicts serum antibody reactivity to WT1 proteins and control proteins in 2 patients with CML. Reactivity of serum antibody to WT1/full-length, WT1/N-terminus, TRX and Ra12 proteins was evaluated by ELISA in 2 patients with CML. The OD values depicted were from ELISA using a 1:500 serum dilution. CML-1 and CML-2 denote serum from 2 of the individual patients in Figure 3 with demonstrated antibody reactivity to WT1/full-length. The WT1/full-length protein was expressed as a fusion protein with Ra12. The WT1/N-terminus protein was expressed as a fusion protein with TRX. The control Ra12 and TRX proteins were purified in a similar manner. The results confirm that the serum antibody reactivity against the WT1 fusion proteins is directed against the WT1 portions of the protein.

Figure 18 provides the characteristics of the recombinant WT1 proteins used for serological analysis.

Figure 19A-19E is a bar graph depicting the antibody responses in mice elicited by vaccination with different doses of WT1 protein.

Figure 20A and 20B is a bar graph of the proliferative T-cell responses in mice immunized with WT1 protein.

Figure 21 is a photograph of human DC, examined by fluorescent microscopy, expressing WT1 following adeno WT1 and Vaccinia WT1 infection.

Figure 22 is a photograph that demonstrates that WT1 expression in human DC is reproducible following adeno WT1 infection and is not induced by a control Adeno infection.

Figure 23 is a graph of an IFN-gamma ELISPOT assay showing that WT1 whole gene *in vitro* priming elicits WT1 specific T-cell responses.

## DETAILED DESCRIPTION OF THE INVENTION

As noted above, the present invention is generally directed to compositions and methods for the immunotherapy and diagnosis of malignant diseases. The compositions described herein may include WT1 polypeptides, WT1 polynucleotides, antigen-presenting cells (APC, *e.g.,* dendritic cells) that express a WT1 polypeptide, agents such as antibodies that bind to a WT1 polypeptide and/or immune system cells (e.g., T cells) specific for WT1. WT1 Polypeptides of the present invention generally comprise at least a portion of a Wilms Tumor gene product (WT1) or a variant thereof. Nucleic acid sequences of the subject invention generally comprise a DNA or RNA sequence that encodes all or a portion of such a polypeptide, or that is complementary to such a sequence. Antibodies are generally immune system proteins, or antigen-binding fragments thereof, that are capable of binding to a portion of a WT1 polypeptide. T cells that may be employed within such compositions are generally T cells (*e.g.*, $CD4^+$ and/or $CD8^+$) that are specific for a WT1 polypeptide. Certain methods described herein further employ antigen-presenting cells that express a WT1 polypeptide as provided herein.

The present invention is based on the discovery that an immune response raised against a Wilms Tumor (WT) gene product (e.g., WT1) can provide prophylactic and/or therapeutic benefit for patients afflicted with malignant diseases characterized by increased WT1 gene expression. Such diseases include, but are not limited to, leukemias (e.g., acute myeloid leukemia (AML), chronic myeloid leukemia (CML), acute lymphocytic leukemia (ALL) and childhood ALL), as well as many cancers such as lung, breast, thyroid and gastrointestinal cancers and melanomas. The WT1 gene was originally identified and isolated on the basis of a cytogenetic deletion at chromosome 11p13 in patients with Wilms' tumor *(see* Call et al., U.S. Patent No. 5,350,840). The gene consists of 10 exons and encodes a zinc finger transcription factor, and sequences of mouse and human WT1 proteins are provided in Figure 1 and SEQ ID NOs: 319 and 320.

WT1 Polypeptides

Within the context of the present invention, a WT1 polypeptide is a polypeptide that comprises at least an immunogenic portion of a native WT1 (*i.e.,* a WT1 protein expressed by an organism that is not genetically modified), or a variant thereof, as described herein. A WT1 polypeptide may be of any length, provided that it comprises at least an immunogenic portion of a native protein or a variant thereof. In other words, a WT1 polypeptide may be an oligopeptide (*i.e.,* consisting of a relatively small number of amino acid residues, such as 8-10 residues, joined by peptide bonds), a full length WT1 protein (e.g., present within a human or non-human animal, such as a mouse) or a polypeptide of intermediate size. Within certain embodiments, the use of WT1 polypeptides that contain a small number of consecutive amino acid residues of a native WT1 polypeptide is preferred. Such polypeptides are preferred for certain uses in which the generation of a T cell response is desired. For example, such a WT1 polypeptide may contain less than 23, preferably no more than 18, and more preferably no more than 15 consecutive amino acid residues, of a native WT1 polypeptide. Polypeptides comprising nine consecutive amino acid residues of a native WT1 polypeptide are generally suitable for such purposes. Additional sequences derived from the native protein and/or heterologous sequences may be present within any WT1 polypeptide, and such sequences may (but need not) possess further immunogenic or antigenic properties. Polypeptides as provided herein may further be associated (covalently or noncovalently) with other polypeptide or non-polypeptide compounds.

An "immunogenic portion," as used herein is a portion of a polypeptide that is recognized (*i.e.,* specifically bound) by a B-cell and/or T-cell surface antigen receptor. Certain preferred immunogenic portions bind to an MHC class I or class II molecule. As used herein, an immunogenic portion is said to "bind to" an MHC class I or class II molecule if such binding is detectable using any assay known in the art. For example, the ability of a polypeptide to bind to MHC class I may be evaluated indirectly by monitoring the ability to promote incorporation of $^{125}I$ labeled β2-microglobulin (β2m) into MHC class I/β2m/peptide heterotrimeric complexes *(see* Parker et al., J. Immunol. 152:163, 1994). Alternatively, functional peptide competition assays that are known in the art may be employed. Certain immunogenic portions have one or more of the sequences recited within one or more of Tables II - XIV. Representative immunogenic portions include, but are not limited to, RDLNALLPAVPSLGGGG (human WT1 residues 6-22; SEQ ID NO:1), PSQASSGQARMFPNAPYLPSCLE (human and mouse WT1 residues 117-139; SEQ ID NOs: 2 and 3 respectively), GATLKGVAAGSSSSVKWTE (human WT1 residues 244-262; SEQ ID NO:4), GATLKGVAA (human WT1 residues 244-252; SEQ ID NO: 88), CMTWNQMNL (human and mouse WT1 residues 235-243; SEQ ID NOs: 49 and 258 respectively), SCLESQPTI (mouse WT1 residues 136-144; SEQ ID NO:296), SCLESQPAI (human WT1 residues 136-144; SEQ ID NO:198), NLYQMTSQL (human and mouse WT1 residues 225-233; SEQ ID NOs: 147 and 284 respectively); ALLPAVSSL (mouse WT1 residues 10-18; SEQ ID NO:255); RMFPNAPYL (human and mouse WT1 residues 126-134; SEQ ID NOs: 185 and 293 respectively), VLDFAPPGA (human WT1 residues 37-45; SEQ ID NO:241), or VLDFAPPGAS (human WT1 residues 37-46; SEQ ID NO:411). Further immunogenic portions are provided herein, and others may generally be identified using well known techniques, such as those summarized in Paul, Fundamental Immunology, 3rd ed., 243-247 (Raven Press, 1993) and references cited therein. Representative techniques for identifying immunogenic portions include screening polypeptides for the ability to react with antigen-specific antisera and/or T-cell lines or clones. An immunogenic portion of a native WT1 polypeptide is a portion that reacts with such antisera and/or T-cells at a level that is not substantially less than the reactivity of the full length WT1 (*e.g.,* in an ELISA and/or T-cell reactivity assay). In other words, an immunogenic portion may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988.

Alternatively, immunogenic portions may be identified using computer analysis, such as the Tsites program *(see* Rothbard and Taylor, EMBO J. 7:93-100, 1988; Deavin et al., Mol. Immunol. 33:145-155, 1996), which searches for peptide motifs that have the potential to elicit Th responses. CTL peptides with motifs appropriate for binding to murine and human class I or class II MHC may be identified according to BIMAS (Parker et al., J. Immunol. 152:163, 1994) and other HLA peptide binding prediction analyses. To confirm immunogenicity, a peptide may be tested using an HLA A2 transgenic mouse model and/or an *in vitro* stimulation assay using dendritic cells, fibroblasts or peripheral blood cells.

As noted above, a composition may comprise a variant of a native WT1 protein. A polypeptide "variant," as used herein, is a polypeptide that differs from a native polypeptide in one or more substitutions, deletions, additions and/or insertions, such that the immunogenicity of the polypeptide is retained (*i.e.,* the ability of the variant to react with antigen-specific antisera and/or T-cell lines or clones is not substantially diminished relative to the native polypeptide). In other words, the ability of a variant to react with antigen-specific antisera and/or T-cell lines or clones may be enhanced or unchanged, relative to the native polypeptide, or may be diminished by less than 50%, and preferably less than 20%, relative to the native polypeptide. Such variants may generally be identified by modifying one of the above polypeptide sequences and evaluating the reactivity of the modified polypeptide with antisera and/or T-cells as described herein. It has been found, within the context of the present invention, that a relatively small number of substitutions (e.g., 1 to 3) within an immunogenic portion of a WT1 polypeptide may serve to enhance the ability of the polypeptide to elicit an immune response. Suitable substitutions may generally be identified by using computer programs, as described above, and the effect confirmed based on the reactivity of the modified polypeptide with antisera and/or T-cells as described herein. Accordingly, within certain preferred embodiments, a WT1 polypeptide comprises a variant in which 1 to 3 amino acid resides within an immunogenic portion are substituted such that the ability to react with antigen-specific antisera and/or T-cell lines or clones is statistically greater than that for the unmodified polypeptide. Such substitutions are preferably located within an MHC binding site of the polypeptide, which may be identified as described above. Preferred substitutions allow increased binding to MHC class I or class II molecules.

Certain variants contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. Variants may also (or alternatively) be modified by, for example, the deletion or addition of amino acids that have minimal influence on the immunogenicity, secondary structure and hydropathic nature of the polypeptide.

In a preferred embodiment, a variant polypeptide of the WT1 N-terminus (amino acids 1-249) is constructed, wherein the variant polypeptide is capable of binding to an antibody that recognizes full-length WT1 and/or WT1 N-terminus polypeptide. A non-limiting example of an antibody is anti WT1 antibody WT180 (Santa Cruz Biotechnology, Inc., Santa Cruz, CA).

As noted above, WT1 polypeptides may be conjugated to a signal (or leader) sequence at the N-terminal end of the protein which co-translationally or post-translationally directs transfer of the protein. A polypeptide may also, or alternatively, be conjugated to a linker or other sequence for ease of synthesis, purification or identification of the polypeptide (e.g., poly-His), or to enhance binding of the polypeptide to a solid support. For example, a polypeptide may be conjugated to an immunoglobulin Fc region.

WT1 polypeptides may be prepared using any of a variety of well known techniques. Recombinant polypeptides encoded by a WT1 polynucleotide as described herein may be readily prepared from the polynucleotide. In general, any of a variety of expression vectors known to those of ordinary skill in the art may be employed to express recombinant WT1 polypeptides. Expression may be achieved in any appropriate host cell that has been transformed or transfected with an expression vector containing a DNA molecule that encodes a recombinant polypeptide. Suitable host cells include prokaryotes, yeast and higher eukaryotic cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. The concentrate may then be applied to a suitable purification matrix such as an affinity matrix or an ion exchange resin. Finally, one or more reverse phase HPLC steps can be employed to further purify a recombinant polypeptide. Such techniques may be used to prepare native polypeptides or variants thereof. For example, polynucleotides that encode a variant of a native polypeptide may generally be prepared using standard mutagenesis techniques, such as oligonucleotide-directed site-specific mutagenesis, and sections of the DNA sequence may be removed to permit preparation of truncated polypeptides.

Certain portions and other variants may also be generated by synthetic means, using techniques well known to those of ordinary skill in the art. For example, polypeptides having fewer than about 500 amino acids, preferably fewer than about 100 amino acids, and more preferably fewer than about 50 amino acids, may be synthesized. Polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Applied BioSystems, Inc. (Foster City, CA), and may be operated according to the manufacturer's instructions.

In general, polypeptides and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

Within further aspects, the present invention provides mimetics of WT1 polypeptides. Such mimetics may comprise amino acids linked to one or more amino acid mimetics (*i.e.,* one or more amino acids within the WT1 protein may be replaced by an amino acid mimetic) or may be entirely nonpeptide mimetics. An amino acid mimetic is a compound that is conformationally similar to an amino acid such that it can be substituted for an amino acid within a WT1 polypeptide without substantially diminishing the ability to react with antigen-specific antisera and/or T cell lines or clones. A nonpeptide mimetic is a compound that does not contain amino acids, and that has an overall conformation that is similar to a WT1 polypeptide such that the ability of the mimetic to react with WT1-specific antisera and/or T cell lines or clones is not substantially diminished relative to the ability of a WT1 polypeptide. Such mimetics may be designed based on standard techniques (e.g., nuclear magnetic resonance and computational techniques) that evaluate the three dimensional structure of a peptide sequence. Mimetics may be designed where one or more of the side chain functionalities of the WT1 polypeptide are replaced by groups that do not necessarily have the same size or volume, but have similar chemical and/or physical properties which produce similar biological responses. It should be understood that, within embodiments described herein, a mimetic may be substituted for a WT1 polypeptide.

Within other illustrative embodiments, a polypeptide may be a fusion polypeptide that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the polypeptide or to enable the polypeptide to be targeted to desired intracellular compartments. Still further fusion partners include affinity tags, which facilitate purification of the polypeptide.

Fusion polypeptides may generally be prepared using standard techniques, including chemical conjugation. Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

The fusion polypeptide can comprise a polypeptide as described herein together with an unrelated immunogenic protein, such as an immunogenic protein capable of eliciting a recall response. Examples of such proteins include tetanus, tuberculosis and hepatitis proteins *(see,* for example, Stoute et al. New Engl. J. Med., 336:86-91, 1997).

In one preferred embodiment, the immunological fusion partner is derived from a Mycobacterium sp., such as a Mycobacterium tuberculosis-derived Ra12 fragment. Ra12 compositions and methods for their use in enhancing the expression and/or immunogenicity of heterologous polynucleotide/polypeptide sequences is described in U.S. Patent Application 60/158,585, the disclosure of which is incorporated herein by reference in its entirety. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis.* The nucleotide sequence and amino acid sequence of MTB32A have been described (for example, U.S. Patent Application 60/158,585; see also, Skeiky et al., Infection and Immun. (1999) 67:3998-4007, incorporated herein by reference). C-terminal fragments of the MTB32A coding sequence express at high levels and remain as soluble polypeptides throughout the purification process. Moreover, Ra12 may enhance the immunogenicity of heterologous immunogenic polypeptides with which it is fused. One preferred Ra12 fusion polypeptide comprises a 14 KD C-terminal fragment corresponding to amino acid residues 192 to 323 of MTB32A. Other preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide. Ra12 polynucleotides may comprise a native sequence (*i.e.,* an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Within other preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g.,* the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see* Biotechnology 10:795-798, 1992). Within a preferred embodiment, a repeat portion of LYTA may be incorporated into a fusion polypeptide. A repeat portion is found in the C-terminal region starting at residue 178. A particularly preferred repeat portion incorporates residues 188-305.

Yet another illustrative embodiment involves fusion polypeptides, and the polynucleotides encoding them, wherein the fusion partner comprises a targeting signal capable of directing a polypeptide to the endosomal/lysosomal compartment, as described in U.S. Patent No. 5,633,234. An immunogenic polypeptide of the invention, when fused with this targeting signal, will associate more efficiently with MHC class II molecules and thereby provide enhanced in vivo stimulation of $CD4^+$ T-cells specific for the polypeptide.

The invention provides truncated forms of WT1 polypeptides that can be recombinantly expressed in E. *coli* without the addition of a fusion partner. Examples of these truncated forms are shown in SEQ ID NOs:342-346, and are encoded by polynucleotides shown in SEQ ID NOs:337-341. In variations of these truncations, the first 76 amino acids of WT1 can be fused to the C-terminus of the protein, creating a recombinant protein that is easier to express in E. *coli.* Other hosts in addition to E. *coli* can also be used, such as, for example, *B. megaterium.* The protein can further be prepared without a histidine tag.

In other embodiments, different subunits can be made and fused together in an order which differs from that of native WT1. In addition, fusions can be made with, for example, Ra12. Exemplary fusion proteins are shown in SEQ ID NOs: 332-336 and can be encoded by polynucleotides shown in SEQ ID NOs: 327-331.

WT1 Polynucleotides

Any polynucleotide that encodes a WT1 polypeptide as described herein is a WT1 polynucleotide encompassed by the present invention. Such polynucleotides may be single-stranded (coding or antisense) or double-stranded, and may be DNA (genomic, cDNA or synthetic) or RNA molecules. Additional coding or non-coding sequences may, but need not, be present within a polynucleotide of the present invention, and a polynucleotide may, but need not, be linked to other molecules and/or support materials.

WT1 polynucleotides may encode a native WT1 protein, or may encode a variant of WT1 as described herein. Polynucleotide variants may contain one or more substitutions, additions, deletions and/or insertions such that the immunogenicity of the encoded polypeptide is not diminished, relative to a native WT1 protein. The effect on the immunogenicity of the encoded polypeptide may generally be assessed as described herein. Preferred variants contain nucleotide substitutions, deletions, insertions and/or additions at no more than 20%, preferably at no more than 10%, of the nucleotide positions that encode an immunogenic portion of a native WT1 sequence. Certain variants are substantially homologous to a native gene, or a portion thereof. Such polynucleotide variants are capable of hybridizing under moderately stringent conditions to a naturally occurring DNA sequence encoding a WT1 polypeptide (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS). Such hybridizing DNA sequences are also within the scope of this invention.

It will be appreciated by those of ordinary skill in the art that, as a result of the degeneracy of the genetic code, there are many nucleotide sequences that encode a WT1 polypeptide. Some of these polynucleotides bear minimal homology to the nucleotide sequence of any native gene. Nonetheless, polynucleotides that vary due to differences in codon usage are specifically contemplated by the present invention.

Once an immunogenic portion of WT1 is identified, as described above, a WT1 polynucleotide may be prepared using any of a variety of techniques. For example, a WT1 polynucleotide may be amplified from cDNA prepared from cells that express WT1. Such polynucleotides may be amplified via polymerase chain reaction (PCR). For this approach, sequence-specific primers may be designed based on the sequence of the immunogenic portion and may be purchased or synthesized. For example, suitable primers for PCR amplification of a human WT1 gene include: first step - P118: 1434-1414: 5' GAG AGT CAG ACT TGA AAG CAGT 3' (SEQ ID NO:5) and P135: 5' CTG AGC CTC AGC AAA TGG GC 3' (SEQ ID NO:6); second step - P136: 5' GAG CAT GCA TGG GCT CCG ACG TGC GGG 3' (SEQ ID NO:7) and P137: 5' GGG GTA CCC ACT GAA CGG TCC CCG A 3' (SEQ ID NO:8). Primers for PCR amplification of a mouse WT1 gene include: first step - P138: 5' TCC GAG CCG CAC CTC ATG 3' (SEQ ID NO:9) and P139: 5' GCC TGG GAT GCT GGA CTG 3' (SEQ ID NO:10), second step - P140: 5' GAG CAT GCG ATG GGT TCC GAC GTG CGG 3' (SEQ ID NO:11) and P141: 5' GGG GTA CCT CAA AGC GCC ACG TGG AGT TT 3' (SEQ ID NO:12).

An amplified portion may then be used to isolate a full length gene from a human genomic DNA library or from a suitable cDNA library, using well known techniques. Alternatively, a full length gene can be constructed from multiple PCR fragments. WT1 polynucleotides may also be prepared by synthesizing oligonucleotide components, and ligating components together to generate the complete polynucleotide.

WT1 polynucleotides may also be synthesized by any method known in the art, including chemical synthesis (*e.g.,* solid phase phosphoramidite chemical synthesis). Modifications in a polynucleotide sequence may also be introduced using standard mutagenesis techniques, such as oligonucleotide-directed site-specific mutagenesis (*see* Adelman et al., DNA 2:183, 1983). Alternatively, RNA molecules may be generated by *in vitro* or *in vivo* transcription of DNA sequences encoding a WT1 polypeptide, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo (e.g.,* by transfecting antigen-presenting cells such as dendritic cells with a cDNA construct encoding a WT1 polypeptide, and administering the transfected cells to the patient).

Polynucleotides that encode a WT1 polypeptide may generally be used for production of the polypeptide, *in vitro* or *in vivo.* WT1 polynucleotides that are complementary to a coding sequence (*i.e.,* antisense polynucleotides) may also be used as a probe or to inhibit WT1 expression. cDNA constructs that can be transcribed into antisense RNA may also be introduced into cells of tissues to facilitate the production of antisense RNA.

Any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (e.g., avian pox virus). Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art. cDNA constructs within such a vector may be used, for example, to transfect human or animal cell lines for use in establishing WT1 positive tumor models which may be used to perform tumor protection and adoptive immunotherapy experiments to demonstrate tumor or leukemia-growth inhibition or lysis of such cells.

Other therapeutic formulations for polynucleotides include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle in *vitro* and *in vivo* is a liposome (*i.e.,* an artificial membrane vesicle). The preparation and use of such systems is well known in the art.

<u>Antibodies and Fragments Thereof</u>

The present invention further provides binding agents, such as antibodies and antigen-binding fragments thereof, that specifically bind to a WT1 polypeptide. As used herein, an agent is said to "specifically bind" to a WT1 polypeptide if it reacts at a detectable level (within, for example, an ELISA) with a WT1 polypeptide, and does not react detectably with unrelated proteins under similar conditions. As used herein, "binding" refers to a noncovalent association between two separate molecules such that a "complex" is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about $10^3$ L/mol. The binding constant maybe determined using methods well known in the art.

Any agent that satisfies the above requirements may be a binding agent. In a preferred embodiment, a binding agent is an antibody or an antigen-binding fragment thereof. Certain antibodies are commercially available from, for example, Santa Cruz Biotechnology (Santa Cruz, CA). Alternatively, antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies and fragments thereof may be coupled to one or more therapeutic agents. Suitable agents in this regard include radioactive tracers and chemotherapeutic agents, which may be used, for example, to purge autologous bone marrow *in vitro).* Representative therapeutic agents include radionuclides, differentiation inducers, drugs, toxins, and derivatives thereof. Preferred radionuclides include $^{90}Y$, $^{123}I$, $^{125}I$, $^{131}I$, $^{186}Re$, $^{188}Re$, $^{211}At$, and $^{212}Bi$. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein. For diagnostic purposes, coupling of radioactive agents may be used to facilitate tracing of metastases or to determine the location of WT1-positive tumors.

A therapeutic agent may be coupled (*e.g.,* covalently bonded) to a suitable monoclonal antibody either directly or indirectly (*e.g.,* via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (e.g., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.,* U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (e.g., U.S. Patent No. 4,489,710, to Spitler), by irradiation of a photolabile bond (e.g., U.S. Patent No. 4,625,014, to Senter et al.), by hydrolysis of derivatized amino acid side chains (e.g., U.S. Patent No. 4,638,045, to Kohn et al.), by serum complement-mediated hydrolysis (*e.g.,* U.S. Patent No. 4,671,958, to Rodwell et al.), and acid-catalyzed hydrolysis (*e.g.,* U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used. A carrier may bear the agents in a variety of ways, including covalent bonding either directly or via a linker group. Suitable carriers include proteins such as albumins (e.g., U.S. Patent No. 4,507,234, to Kato et al.), peptides and polysaccharides such as aminodextran (*e.g.,* U.S. Patent No. 4,699,784, to Shih et al.). A carrier may also bear an agent by noncovalent bonding or by encapsulation, such as within a liposome vesicle (e.g., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

Also provided herein are anti-idiotypic antibodies that mimic an immunogenic portion of WT1. Such antibodies may be raised against an antibody, or antigen-binding fragment thereof, that specifically binds to an immunogenic portion of WT1, using well known techniques. Anti-idiotypic antibodies that mimic an immunogenic portion of WT1 are those antibodies that bind to an antibody, or antigen-binding fragment thereof, that specifically binds to an immunogenic portion of WT1, as described herein.

T Cells

Immunotherapeutic compositions may also, or alternatively, comprise T cells specific for WT1. Such cells may generally be prepared *in vitro* or *ex vivo,* using standard procedures. For example, T cells may be present within (or isolated from) bone marrow, peripheral blood or a fraction of bone marrow or peripheral blood of a mammal, such as a patient, using a commercially available cell separation system, such as the CEPRATE™ system, available from CellPro Inc., Bothell WA (see also U.S. Patent No. 5,240,856; U.S. Patent No. 5,215,926; WO 89/06280; WO 91/16116 and WO 92/07243). Alternatively, T cells may be derived from related or unrelated humans, non-human animals, cell lines or cultures.

T cells may be stimulated with WT1 polypeptide, polynucleotide encoding a WT1 polypeptide and/or an antigen presenting cell (APC) that expresses a WT1 polypeptide. Such stimulation is performed under conditions and for a time sufficient to permit the generation of T cells that are specific for the WT1 polypeptide. Preferably, a WT1 polypeptide or polynucleotide is present within a delivery vehicle, such as a microsphere, to facilitate the generation of antigen-specific T cells. Briefly, T cells, which may be isolated from a patient or a related or unrelated donor by routine techniques (such as by Ficoll/Hypaque density gradient centrifugation of peripheral blood lymphocytes), are incubated with WT1 polypeptide. For example, T cells may be incubated *in vitro* for 2-9 days (typically 4 days) at 37°C with WT1 polypeptide (e.g., 5 to 25 μg/ml) or cells synthesizing a comparable amount of WT1 polypeptide. It may be desirable to incubate a separate aliquot of a T cell sample in the absence of WT1 polypeptide to serve as a control.

T cells are considered to be specific for a WT1 polypeptide if the T cells kill target cells coated with a WT1 polypeptide or expressing a gene encoding such a polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (e.g., by pulse-labeling cultures of T cells with tritiated thymidine and measuring the amount of tritiated thymidine incorporated into DNA). Other ways to detect T cell proliferation include measuring increases in interleukin-2 (IL-2) production, $Ca^{2+}$ flux, or dye uptake, such as 3-(4,5-dimethylthiazol-2-yl)-2,5-diphenyl-tetrazolium. Alternatively, synthesis of lymphokines (such as interferon-gamma) can be measured or the relative number of T cells that can respond to a WT1 polypeptide may be quantified. Contact with a WT1 polypeptide (200 ng/ml - 100 μg/ml, preferably 100 ng/ml - 25 μg/ml) for 3 - 7 days should result in at least a two fold increase in proliferation of the T cells and/or contact as described above for 2-3 hours should result in activation of the T cells, as measured using standard cytokine assays in which a two fold increase in the level of cytokine release (*e.g.,* TNF or IFN-γ) is indicative of T cell activation *(see* Coligan et al., Current Protocols in Immunology, vol. 1, Wiley Interscience (Greene 1998). WT1 specific T cells may be expanded using standard techniques. Within preferred embodiments, the T cells are derived from a patient or a related or unrelated donor and are administered to the patient following stimulation and expansion.

T cells that have been activated in response to a WT1 polypeptide, polynucleotide or WT1-expressing APC may be $CD4^+$ and/or $CD8^+$. Specific activation of $CD4^+$ or $CD8^+$ T cells may be detected in a variety of ways. Methods for detecting specific T cell activation include detecting the proliferation of T cells, the production of cytokines (e.g., lymphokines), or the generation of cytolytic activity (*i.e.*, generation of cytotoxic T cells specific for WT1). For $CD4^+$ T cells, a preferred method for detecting specific T cell activation is the detection of the proliferation of T cells. For $CD8^+$ T cells, a preferred method for detecting specific T cell activation is the detection of the generation of cytolytic activity.

For therapeutic purposes, $CD4^+$ or $CD8^+$ T cells that proliferate in response to the WT1 polypeptide, polynucleotide or APC can be expanded in number either *in vitro* or *in vivo.* Proliferation of such T cells in *vitro* may be accomplished in a variety of ways. For example, the T cells can be re-exposed to WT1 polypeptide, with or without the addition of T cell growth factors, such as interleukin-2, and/or stimulator cells that synthesize a WT1 polypeptide. The addition of stimulator cells is preferred where generating $CD8^+$ T cell responses. T cells can be grown to large numbers in *vitro* with retention of specificity in response to intermittent restimulation with WT1 polypeptide. Briefly, for the primary *in vitro* stimulation (IVS), large numbers of lymphocytes (*e.g.,* greater than 4 x $10^7$) may be placed in flasks with media containing human serum. WT1 polypeptide *(e.g.,* peptide at 10 μg/ml) may be added directly, along with tetanus toxoid *(e.g.,* 5 μg/ml). The flasks may then be incubated *(e.g.,* 37°C for 7 days). For a second IVS, T cells are then harvested and placed in new flasks with 2-3 x $10^7$ irradiated peripheral blood mononuclear cells. WT1 polypeptide *(e.g.,* 10 μg/ml) is added directly. The flasks are incubated at 37°C for 7 days. On day 2 and day 4 after the second IVS, 2-5 units of interleukin-2 (IL-2) may be added. For a third IVS, the T cells may be placed in wells and stimulated with the individual's own EBV transformed B cells coated with the peptide. IL-2 may be added on days 2 and 4 of each cycle. As soon as the cells are shown to be specific cytotoxic T cells, they may be expanded using a 10 day stimulation cycle with higher IL-2 (20 units) on days 2, 4 and 6.

Alternatively, one or more T cells that proliferate in the presence of WT1 polypeptide can be expanded in number by cloning. Methods for cloning cells are well known in the art, and include limiting dilution. Responder T cells may be purified from the peripheral blood of sensitized patients by density gradient centrifugation and sheep red cell rosetting and established in culture by stimulating with the nominal antigen in the presence of irradiated autologous filler cells. In order to generate $CD4^+$ T cell lines, WT1 polypeptide is used as the antigenic stimulus and autologous peripheral blood lymphocytes (PBL) or lymphoblastoid cell lines (LCL) immortalized by infection with Epstein Barr virus are used as antigen presenting cells. In order to generate $CD8^+$ T cell lines, autologous antigen-presenting cells transfected with an expression vector which produces WT1 polypeptide may be used as stimulator cells. Established T cell lines may be cloned 2-4 days following antigen stimulation by plating stimulated T cells at a frequency of 0.5 cells per well in 96-well flat-bottom plates with 1 x $10^6$ irradiated PBL or LCL cells and recombinant interleukin-2 (rIL2) (50 U/ml). Wells with established clonal growth may be identified at approximately 2-3 weeks after initial plating and restimulated with appropriate antigen in the presence of autologous antigen-presenting cells, then subsequently expanded by the addition of low doses of rIL2 (10 U/ml) 2-3 days following antigen stimulation. T cell clones may be maintained in 24-well plates by periodic restimulation with antigen and rIL2 approximately every two weeks.

Within certain embodiments, allogeneic T-cells may be primed (*i.e.,* sensitized to WT1) *in vivo* and/or *in vitro.* Such priming may be achieved by contacting T cells with a WT1 polypeptide, a polynucleotide encoding such a polypeptide or a cell producing such a polypeptide under conditions and for a time sufficient to permit the priming of T cells. In general, T cells are considered to be primed if, for example, contact with a WT1 polypeptide results in proliferation and/or activation of the T cells, as measured by standard proliferation, chromium release and/or cytokine release assays as described herein. A stimulation index of more than two fold increase in proliferation or lysis, and more than three fold increase in the level of cytokine, compared to negative controls, indicates T-cell specificity. Cells primed *in vitro* may be employed, for example, within a bone marrow transplantation or as donor lymphocyte infusion.

T cells specific for WT1 can kill cells that express WT1 protein. Introduction of genes encoding T-cell receptor (TCR) chains for WT1 are used as a means to quantitatively and qualitatively improve responses to WT1 bearing leukemia and cancer cells. Vaccines to increase the number of T cells that can react to WT1 positive cells are one method of targeting WT1 bearing cells. T cell therapy with T cells specific for WT1 is another method. An alternative method is to introduce the TCR chains specific for WT1 into T cells or other cells with lytic potential. In a suitable embodiment, the TCR alpha and beta chains are cloned out from a WT1 specific T cell line and used for adoptive T cell therapy, such as described in WO96/30516, incorporated herein by reference.

## Pharmaceutical Compositions and Vaccines

Within certain aspects, polypeptides, polynucleotides, antibodies and/or T cells may be incorporated into pharmaceutical compositions or vaccines. Alternatively, a pharmaceutical composition may comprise an antigen-presenting cell (e.g., a dendritic cell) transfected with a WT1 polynucleotide such that the antigen presenting cell expresses a WT1 polypeptide. Pharmaceutical compositions comprise one or more such compounds or cells and a physiologically acceptable carrier or excipient. Certain vaccines may comprise one or more such compounds or cells and a non-specific immune response enhancer, such as an adjuvant or a liposome (into which the compound is incorporated). Pharmaceutical compositions and vaccines may additionally contain a delivery system, such as biodegradable microspheres which are disclosed, for example, in U.S. Patent Nos. 4,897,268 and 5,075,109. Pharmaceutical compositions and vaccines within the scope of the present invention may also contain other compounds, which may be biologically active or inactive.

Within certain embodiments, pharmaceutical compositions and vaccines are designed to elicit T cell responses specific for a WT1 polypeptide in a patient, such as a human. In general, T cell responses may be favored through the use of relatively short polypeptides (*e.g.*, comprising less than 23 consecutive amino acid residues of a native WT1 polypeptide, preferably 4-16 consecutive residues, more preferably 8-16 consecutive residues and still more preferably 8-10 consecutive residues. Alternatively, or in addition, a vaccine may comprise a non-specific immune response enhancer that preferentially enhances a T cell response. In other words, the immune response enhancer may enhance the level of a T cell response to a WT1 polypeptide by an amount that is proportionally greater than the amount by which an antibody response is enhanced. For example, when compared to a standard oil based adjuvant, such as CFA, an immune response enhancer that preferentially enhances a T cell response may enhance a proliferative T cell response by at least two fold, a lytic response by at least 10%, and/or T cell activation by at least two fold compared to WT1-megative control cell lines, while not detectably enhancing an antibody response. The amount by which a T cell or antibody response to a WT1 polypeptide is enhanced may generally be determined using any representative technique known in the art, such as the techniques provided herein.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacterial and viral expression systems and mammalian expression systems. Appropriate nucleic acid expression systems contain the necessary DNA, cDNA or RNA sequences for expression in the patient (such as a suitable promoter and terminating signal). Bacterial delivery systems involve the administration of a bacterium (such as *Bacillus-Calmette-Guerrin)* that expresses an immunogenic portion of the polypeptide on its cell surface. In a preferred embodiment, the DNA may be introduced using a viral expression system (*e.g.*, vaccinia or other pox virus, retrovirus, or adenovirus), which may involve the use of a non-pathogenic (defective), replication competent virus. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

As noted above, a pharmaceutical composition or vaccine may comprise an antigen-presenting cell that expresses a WT1 polypeptide. For therapeutic purposes, as described herein, the antigen presenting cell is preferably an autologous dendritic cell. Such cells may be prepared and transfected using standard techniques, such as those described by Reeves et al., Cancer Res. 56:5672-5677, 1996; Tuting et al., J. Immunol. 160:1139-1147, 1998; and Nair et al., Nature Biotechnol. 16:364-369, 1998). Expression of a WT1 polypeptide on the surface of an antigen-presenting cell may be confirmed by *in vitro* stimulation and standard proliferation as well as chromium release assays, as described herein.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.,* polylactate polyglycolate) may also be employed as carriers for the pharmaceutical compositions of this invention. For certain topical applications, formulation as a cream or lotion, using well known components, is preferred.

Such compositions may also comprise buffers (e.g., neutral buffered saline or phosphate buffered saline), carbohydrates (*e.g.*, glucose, mannose, sucrose or dextrans), mannitol, proteins, polypeptides or amino acids such as glycine, antioxidants, chelating agents such as EDTA or glutathione, adjuvants (*e.g.,* aluminum hydroxide) and/or preservatives. Alternatively, compositions of the present invention may be formulated as a lyophilizate. Compounds may also be encapsulated within liposomes using well known technology. In one embodiment of the present invention, compositions comprise a buffer comprising one or more sugars including, but not limited to, trehalose, maltose, sucrose, fructose, and glucose, each at a concentration generally between about 1 and 25%, typically between about 7 and 13 %. In a further embodiment, the concentration is between about 8 and about 12%. In yet a further embodiment the concentration is about 10%. In an additional aspect of the present invention, the compositions may comprise ethanolamine; cysteine; or Polysorbate-80, generally at concentrations effective for enhancing the efficacy, stability and/or solubility of the formulation.

Any of a variety of non-specific immune response enhancers, such as adjuvants, may be employed in the vaccines of this invention. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable non-specific immune response enhancers include alum-based adjuvants (e.g., Alhydrogel, Rehydragel, aluminum phosphate, Algammulin, aluminum hydroxide); oil based adjuvants (Freund's adjuvant (FA), Specol, RIBI, TiterMax, Montanide ISA50 or Montanide ISA 720 (Seppic, France); cytokines (e.g., GM-CSF or Flat3-ligand); microspheres; nonionic block copolymer-based adjuvants; dimethyl dioctadecyl ammoniumbromide (DDA) based adjuvants AS-1, AS-2 (Smith Kline Beecham); Ribi Adjuvant system based adjuvants; QS21 (Aquila); saponin based adjuvants (crude saponin, the saponin Quil A ); muramyl dipeptide (MDP) based adjuvants such as SAF (Syntex adjuvant in its microfluidized form (SAF-m)); dimethyl-dioctadecyl ammonium bromide (DDA); human complement based adjuvants *m. vaccae* and derivatives; immune stimulating complex (iscom) based adjuvants; inactivated toxins; and attenuated infectious agents (such as *M. tuberculosis*).

Additional illustrative adjuvants for use in the pharmaceutical compositions of the invention include, SAF (Chiron, California, United States), ISCOMS (CSL), MF-59 (Chiron), the SBAS series of adjuvants (e.g., SBAS-2 or SBAS-4, available from SmithKline Beecham, Rixensart, Belgium), Detox (Enhanzyn®) (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula

$$(I): \quad HO(CH_2CH_2O)_n\text{-}A\text{-}R,$$

wherein, n is 1-50, A is a bond or-C(O)-, R is $C_{1-50}$ alkyl or Phenyl $C_{1-50}$ alkyl.

One embodiment of the present invention consists of a vaccine formulation comprising a polyoxyethylene ether of general formula (I), wherein *n* is between 1 and 50, preferably 4-24, most preferably 9; the R component is $C_{1-50}$,

preferably $C_4$-$C_{20}$ alkyl and most preferably $C_{12}$ alkyl, and A is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index ($12^{th}$ edition: entry 7717). These adjuvant molecules are described in WO 99/52549.

The polyoxyethylene ether according to the general formula (I) above may, if desired, be combined with another adjuvant. For example, a preferred adjuvant combination is preferably with CpG as described in the pending UK patent application GB 9820956.2.

As noted above, within certain embodiments, immune response enhancers are chosen for their ability to preferentially elicit or enhance a T cell response *(e.g.,* $CD4^+$ and/or $CD8^+$) to a WT1 polypeptide. Such immune response enhancers are well known in the art, and include (but are not limited to) Montanide ISA50, Seppic MONTANIDE ISA 720, cytokines *(e.g.,* GM-CSF, Flat3-ligand), microspheres, dimethyl dioctadecyl ammoniumbromide (DDA) based adjuvants, AS-1 (Smith Kline Beecham), AS-2 (Smith Kline Beecham), Ribi Adjuvant system based adjuvants, QS21 (Aquila), saponin based adjuvants (crude saponin, the saponin Quil A), Syntex adjuvant in its microfluidized form (SAF-m), MV, ddMV (Genesis), immune stimulating complex (iscom) based adjuvants and inactivated toxins.

In another aspect of the present invention, compositions may comprise adjuvants for eliciting a predominantly Th1-type response. Certain preferred adjuvants for eliciting a predominantly Th1-type response include, for example, a combination of monophosphoryl lipid A, preferably 3-de-O-acylated monophosphoryl lipid A, together with an aluminum salt. MPL® adjuvants, such as MPL-SE, are available from Corixa Corporation (Seattle, WA; *see,* for example, US Patent Nos. 4,436,727; 4,877,611; 4,866,034 and 4,912,094, incorporated herein in their entirety). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., Science 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins . Other preferred formulations include more than one saponin in the adjuvant combinations of the present invention, for example combinations of at least two of the following group comprising QS21, QS7, Quil A, β-escin, or digitonin.

The compositions and vaccines described herein may be administered as part of a sustained release formulation (*i.e.,* a formulation such as a capsule or sponge that effects a slow release of compound following administration). Such formulations may generally be prepared using well known technology and administered by, for example, oral, rectal or subcutaneous implantation, or by implantation at the desired target site. Sustained-release formulations may contain a polypeptide, polynucleotide, antibody or cell dispersed in a carrier matrix and/or contained within a reservoir surrounded by a rate controlling membrane. Carriers for use within such formulations are biocompatible, and may also be biodegradable; preferably the formulation provides a relatively constant level of active component release. The amount of active compound contained within a sustained release formulation depends upon the site of implantation, the rate and expected duration of release and the nature of the condition to be treated or prevented.

## Therapy of Malignant Diseases

In further aspects of the present invention, the compositions and vaccines described herein may be used to inhibit the development of malignant diseases (*e.g.,* progressive or metastatic diseases or diseases characterized by small tumor burden such as minimal residual disease). In general, such methods may be used to prevent, delay or treat a disease associated with WT1 expression. In other words, therapeutic methods provided herein may be used to treat an existing WT1-associated disease, or may be used to prevent or delay the onset of such a disease in a patient who is free of disease or who is afflicted with a disease that is not yet associated with WT1 expression.

As used herein, a disease is "associated with WT1 expression" if diseased cells (e.g., tumor cells) at some time during the course of the disease generate detectably higher levels of a WT1 polypeptide than normal cells of the same tissue. Association of WT1 expression with a malignant disease does not require that WT1 be present on a tumor. For example, overexpression of WT1 may be involved with initiation of a tumor, but the protein expression may subsequently be lost. Alternatively, a malignant disease that is not characterized by an increase in WT1 expression may, at a later time, progress to a disease that is characterized by increased WT1 expression. Accordingly, any malignant disease in which diseased cells formerly expressed, currently express or are expected to subsequently express increased levels of WT1 is considered to be "associated with WT1 expression."

Immunotherapy may be performed using any of a variety of techniques, in which compounds or cells provided herein function to remove WT1-expressing cells from a patient. Such removal may take place as a result of enhancing or inducing an immune response in a patient specific for WT1 or a cell expressing WT1. Alternatively, WT1-expressing cells may be removed *ex vivo (e.g.,* by treatment of autologous bone marrow, peripheral blood or a fraction of bone marrow or peripheral blood). Fractions of bone marrow or peripheral blood may be obtained using any standard technique in the art.

Within such methods, pharmaceutical compositions and vaccines may be administered to a patient. As used herein, a "patient" refers to any warm-blooded animal, preferably a human. A patient may or may not be afflicted with a malignant disease. Accordingly, the above pharmaceutical compositions and vaccines may be used to prevent the onset of a disease *(i.e.,* prophylactically) or to treat a patient afflicted with a disease (e.g., to prevent or delay progression and/or metastasis of an existing disease). A patient afflicted with a disease may have a minimal residual disease (e.g., a low tumor burden in a leukemia patient in complete or partial remission or a cancer patient following reduction of the tumor burden after surgery radiotherapy and/or chemotherapy). Such a patient may be immunized to inhibit a relapse (*i.e.,* prevent or delay the relapse, or decrease the severity of a relapse). Within certain preferred embodiments, the patient is afflicted with a leukemia (e.g., AML, CML, ALL or childhood ALL), a myelodysplastic syndrome (MDS) or a cancer (e.g., gastrointestinal, lung, thyroid or breast cancer or a melanoma), where the cancer or leukemia is WT1 positive (*i.e.,* reacts detectably with an anti-WT1 antibody, as provided herein or expresses WT1 mRNA at a level detectable by RT-PCR, as described herein) or suffers from an autoimmune disease directed against WT1-expressing cells.

Other diseases associated with WT1 overexpression include kidney cancer (such as renal cell carcinoma, or Wilms tumor), as described in Satoh F., et al., Pathol. Int. 50(6):458-71(2000), and Campbell C. E. et al., Int. J. Cancer 78(2):182-8 (1998); and mesothelioma, as described in Amin, K.M. et al., Am. J. Pathol. 146(2):344-56 (1995). Harada et al. (Mol. Urol. 3(4):357-364 (1999) describe WT1 gene expression in human testicular germ-cell tumors. Nonomura et al. Hinyokika Kiyo 45(8):593-7 (1999) describe molecular staging of testicular cancer using polymerase chain reaction of the testicular cancer-specific genes. Shimizu et al., Int. J. Gynecol. Pathol. 19(2):158-63 (2000) describe the immunohistochemical detection of the Wilms' tumor gene (WT1) in epithelial ovarian tumors.

WT1 overexpression was also described in desmoplastic small round cell tumors, by Barnoud, R. et al., Am. J. Surg. Pathol. 24(6):830-6 (2000); and Pathol. Res. Pract. 194(10):693-700 (1998). WT1 overexpression in glioblastoma and other cancer was described by Menssen, H.D. et al., J. Cancer Res. Clin. Oncol. 126(4):226-32 (2000), "Wilms' tumor gene (WT1) expression in lung cancer, colon cancer and glioblastoma cell lines compared to freshly isolated tumor specimens." Other diseases showing WT1 overexpression include EBV associated diseases, such as Burkitt's lymphoma and nasopharyngeal cancer (Spinsanti P. et al., .Leuk Lymphoma 38(5-6):611-9 (2000), "Wilms' tumor gene expression by normal and malignant human B lymphocytes."

In Leukemia 14(9):1634-4 (2000), Pan et al., describe in vitro IL-12 treatment of peripheral blood mononuclear cells from patients with leukemia or myelodysplastic syndromes, and reported an increase in cytotoxicity and reduction in WT1 gene expression. In Leukemia 13(6):891-900 (1999), Patmasiriwat et al. reported WT1 and GATA1 expression in myelodysplastic syndrome and acute leukemia. In Leukemia 13(3):393-9 (1999), Tamaki et al. reported that the Wilms' tumor gene WT1 is a good marker for diagnosis of disease progression of myelodysplastic syndromes. Expression of the Wilms' tumor gene WT1 in solid tumors, and its involvement in tumor cell growth, was discussed in relation to gastric cancer, colon cancer, lung cancer, breast cancer cell lines, germ cell tumor cell line, ovarian cancer, the uterine cancer, thyroid cancer cell line, hepatocellular carcinoma, in Oji et al., Jpn. J Cancer Res. 90(2):194-204 (1999).

The compositions provided herein may be used alone or in combination with conventional therapeutic regimens such as surgery, irradiation, chemotherapy and/or bone marrow transplantation (autologous, syngeneic, allogeneic or unrelated). As discussed in greater detail below, binding agents and T cells as provided herein may be used for purging of autologous stem cells. Such purging may be beneficial prior to, for example, bone marrow transplantation or transfusion of blood or components thereof. Binding agents, T cells, antigen presenting cells (APC) and compositions provided herein may further be used for expanding and stimulating (or priming) autologous, allogeneic, syngeneic or unrelated WT1-specific T-cells *in vitro* and/or *in vivo*. Such WT1-specific T cells may be used, for example, within donor lymphocyte infusions.

Routes and frequency of administration, as well as dosage, will vary from individual to individual, and may be readily established using standard techniques. In general, the pharmaceutical compositions and vaccines may be administered by injection (e.g., intracutaneous, intramuscular, intravenous or subcutaneous), intranasally (*e.g.,* by aspiration) or orally. In some tumors, pharmaceutical compositions or vaccines may be administered locally (by, for example, rectocoloscopy, gastroscopy, videoendoscopy, angiography or other methods known in the art). Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response that is at least 10-50% above the basal (*i.e.,* untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (e.g., more frequent complete or partial remissions, or longer disease-free and/or overall survival) in vaccinated patients as compared to non-vaccinated patients. In general, for pharmaceutical compositions and vaccines comprising one or more polypeptides, the amount of each polypeptide present in a dose ranges from about 100 μg to 5 mg. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent complete or partial remissions, or longer disease-free and/or overall survival) in treated patients as compared to non-treated patients. Increases in preexisting immune responses to WT1 generally correlate with an improved clinical outcome. Such immune responses may generally be evaluated using standard proliferation, cytotoxicity or cytokine assays, which may be performed using samples obtained from a patient before and after treatment.

Within further aspects, methods for inhibiting the development of a malignant disease associated with WT1 expression involve the administration of autologous T cells that have been activated in response to a WT1 polypeptide or WT1-expressing APC, as described above. Such T cells may be $CD4^+$ and/or $CD8^+$, and may be proliferated as described above. The T cells may be administered to the individual in an amount effective to inhibit the development of a malignant disease. Typically, about 1 x $10^9$ to 1 x $10^{11}$ T cells/$M^2$ are administered intravenously, intracavitary or in the bed of a resected tumor. It will be evident to those skilled in the art that the number of cells and the frequency of administration will be dependent upon the response of the patient.

Within certain embodiments, T cells may be stimulated prior to an autologous bone marrow transplantation. Such stimulation may take place *in vivo* or *in vitro*. For *in vitro* stimulation, bone marrow and/or peripheral blood (or a fraction of bone marrow or peripheral blood) obtained from a patient may be contacted with a WT1 polypeptide, a polynucleotide encoding a WT1 polypeptide and/or an APC that expresses a WT1 polypeptide under conditions and for a time sufficient to permit the stimulation of T cells as described above. Bone marrow, peripheral blood stem cells and/or WT1-specific T cells may then be administered to a patient using standard techniques.

Within related embodiments, T cells of a related or unrelated donor may be stimulated prior to a syngeneic or allogeneic (related or unrelated) bone marrow transplantation. Such stimulation may take place *in vivo* or *in vitro.* For *in vitro* stimulation, bone marrow and/or peripheral blood (or a fraction of bone marrow or peripheral blood) obtained from a related or unrelated donor may be contacted with a WT1 polypeptide, WT1 polynucleotide and/or APC that expresses a WT1 polypeptide under conditions and for a time sufficient to permit the stimulation of T cells as described above. Bone marrow, peripheral blood stem cells and/or WT1-specific T cells may then be administered to a patient using standard techniques.

Within other embodiments, WT1-specific T cells as described herein may be used to remove cells expressing WT1 from autologous bone marrow, peripheral blood or a fraction of bone marrow or peripheral blood (e.g., $CD34^+$ enriched peripheral blood (PB) prior to administration to a patient). Such methods may be performed by contacting bone marrow or PB with such T cells under conditions and for a time sufficient to permit the reduction of WT1 expressing cells to less than 10%, preferably less than 5% and more preferably less than 1%, of the total number of myeloid or lymphatic cells in the bone marrow or peripheral blood. The extent to which such cells have been removed may be readily determined by standard methods such as, for example, qualitative and quantitative PCR analysis, morphology, immunohistochemistry and FACS analysis. Bone marrow or PB (or a fraction thereof) may then be administered to a patient using standard techniques.

## Diagnostic Methods

The present invention further provides methods for detecting a malignant disease associated with WT1 expression, and for monitoring the effectiveness of an immunization or therapy for such a disease. Such methods are based on the discovery, within the present invention, that an immune response specific for WT1 protein can be detected in patients afflicted with such diseases, and that methods which enhance such immune responses may provide a preventive or therapeutic benefit.

To determine the presence or absence of a malignant disease associated with WT1 expression, a patient may be tested for the level of T cells specific for WT1. Within certain methods, a biological sample comprising $CD4^+$ and/or $CD8^+$ T cells isolated from a patient is incubated with a WT1 polypeptide, a polynucleotide encoding a WT1 polypeptide and/or an APC that expresses a WT1 polypeptide, and the presence or absence of specific activation of the T cells is detected, as described herein. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated in *vitro* for 2-9 days (typically 4 days) at 37°C with WT1 polypeptide (e.g., 5 - 25 μg/ml). It may be desirable to incubate another aliquot of a T cell sample in the absence of WT1 polypeptide to serve as a control. For $CD4^+$ T cells, activation is preferably detected by evaluating proliferation of the T cells. For $CD8^+$ T cells, activation is preferably detected by evaluating cytolytic activity. A level of proliferation that is at least two fold greater and/or a level of cytolytic activity that is at least 20% greater than in disease-free patients indicates the presence of a malignant disease associated with WT1 expression. Further correlation may be made, using methods well known in the art, between the level of proliferation and/or cytolytic activity and the predicted response to therapy. In particular, patients that display a higher antibody, proliferative and/or lytic response may be expected to show a greater response to therapy.

Within other methods, a biological sample obtained from a patient is tested for the level of antibody specific for WT1. The biological sample is incubated with a WT1 polypeptide, a polynucleotide encoding a WT1 polypeptide and/or an APC that expresses a WT1 polypeptide under conditions and for a time sufficient to allow immunocomplexes to form. Immunocomplexes formed between the WT1 polypeptide and antibodies in the biological sample that specifically bind to the WT1 polypeptide are then detected. A biological sample for use within such methods may be any sample obtained from a patient that would be expected to contain antibodies. Suitable biological samples include blood, sera, ascites, bone marrow, pleural effusion, and cerebrospinal fluid.

The biological sample is incubated with the WT1 polypeptide in a reaction mixture under conditions and for a time sufficient to permit immunocomplexes to form between the polypeptide and antibodies specific for WT1. For example, a biological sample and WT1 polypeptide may be incubated at 4°C for 24-48 hours.

Following the incubation, the reaction mixture is tested for the presence of immunocomplexes. Detection of immunocomplexes formed between the WT1 polypeptide and antibodies present in the biological sample may be accomplished by a variety of known techniques, such as radioimmunoassays (RIA) and enzyme linked immunosorbent assays (ELISA). Suitable assays are well known in the art and are amply described in the scientific and patent literature (e.g., Harlow and Lane, Antibodies: A Laboratory Manual, Cold Spring Harbor Laboratory, 1988). Assays that may be used include, but are not limited to, the double monoclonal antibody sandwich immunoassay technique of David et al. (U.S. Patent 4,376,110); monoclonal-polyclonal antibody sandwich assays (Wide et al., in Kirkham and Hunter, eds., Radioimmunoassay Methods, E. and S. Livingstone, Edinburgh, 1970); the "western blot" method of Gordon et al. (U.S. Patent 4,452,901); immunoprecipitation of labeled ligand (Brown et al., J. Biol. Chem. 255:4980-4983, 1980); enzyme-linked immunosorbent assays as described by, for example, Raines and Ross (J. Biol. Chem. 257:5154-5160, 1982); immunocytochemical techniques, including the use of fluorochromes (Brooks et al., Clin. Exp. Immunol. 39: 477, 1980); and neutralization of activity (Bowen-Pope et al., Proc. Natl. Acad Sci. USA 81:2396-2400, 1984). Other immunoassays include, but are not limited to, those described in U.S. Patent Nos.: 3,817,827; 3,850,752; 3,901,654; 3,935,074; 3,984,533; 3,996,345; 4,034,074; and 4,098,876.

For detection purposes, WT1 polypeptide may either be labeled or unlabeled. Unlabeled WT1 polypeptide may be used in agglutination assays or in combination with labeled detection reagents that bind to the immunocomplexes (*e.g.,* anti-immunoglobulin, protein G, protein A or a lectin and secondary antibodies, or antigen-binding fragments thereof, capable of binding to the antibodies that specifically bind to the WT1 polypeptide). If the WT1 polypeptide is labeled, the reporter group may be any suitable reporter group known in the art, including radioisotopes, fluorescent groups, luminescent groups, enzymes, biotin and dye particles.

Within certain assays, unlabeled WT1 polypeptide is immobilized on a solid support. The solid support may be any material known to those of ordinary skill in the art to which the polypeptide may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The polypeptide may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the antigen and functional groups on the support or may be a linkage by way of a cross-linking agent). Immobilization by adsorption to a well in a microtiter plate or to a membrane is preferred. In such cases, adsorption may be achieved by contacting the WT1 polypeptide, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of polypeptide ranging from about 10 ng to about 10 μg, and preferably about 100 ng to about 1 μg, is sufficient to immobilize an adequate amount of polypeptide.

Following immobilization, the remaining protein binding sites on the support are typically blocked. Any suitable blocking agent known to those of ordinary skill in the art, such as bovine serum albumin, Tween 20™ (Sigma Chemical Co., St. Louis, MO), heat-inactivated normal goat serum (NGS), or BLOTTO (buffered solution of nonfat dry milk which also contains a preservative, salts, and an antifoaming agent). The support is then incubated with a biological sample suspected of containing specific antibody. The sample can be applied neat, or, more often, it can be diluted, usually in a buffered solution which contains a small amount (0.1 %-5.0% by weight) of protein, such as BSA, NGS, or BLOTTO. In general, an appropriate contact time *(i.e.,* incubation time) is a period of time that is sufficient to detect the presence of antibody that specifically binds WT1 within a sample containing such an antibody. Preferably, the contact time is sufficient to achieve a level of binding that is at least about 95% of that achieved at equilibrium between bound and unbound antibody. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. A detection reagent that binds to the immunocomplexes and that comprises a reporter group may then be added. The detection reagent is incubated with the immunocomplex for an amount of time sufficient to detect the bound antibody. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups (e.g., horseradish peroxidase, beta-galactosidase, alkaline phosphatase and glucose oxidase) may generally be detected by the addition of substrate (generally for a specific period of time), followed by spectroscopic or other analysis of the reaction products. Regardless of the specific method employed, a level of bound detection reagent that is at least two fold greater than background (*i.e.,* the level observed for a biological sample obtained from a disease-free individual) indicates the presence of a malignant disease associated with WT1 expression.

In general, methods for monitoring the effectiveness of an immunization or therapy involve monitoring changes in the level of antibodies or T cells specific for WT1 in the patient. Methods in which antibody levels are monitored may comprise the steps of: (a) incubating a first biological sample, obtained from a patient prior to a therapy or immunization, with a WT1 polypeptide, wherein the incubation is performed under conditions and for a time sufficient to allow immunocomplexes to form; (b) detecting immunocomplexes formed between the WT1 polypeptide and antibodies in the biological sample that specifically bind to the WT1 polypeptide; (c) repeating steps (a) and (b) using a second biological sample taken from the patient following therapy or immunization; and (d) comparing the number of immunocomplexes detected in the first and second biological samples. Alternatively, a polynucleotide encoding a WT1 polypeptide, or an APC expressing a WT1 polypeptide may be employed in place of the WT1 polypeptide. Within such methods, immunocomplexes between the WT1 polypeptide encoded by the polynucleotide, or expressed by the APC, and antibodies in the biological sample are detected.

Methods in which T cell activation and/or the number of WT1 specific precursors are monitored may comprise the steps of: (a) incubating a first biological sample comprising CD4+ and/or CD8+ cells (*e.g.,* bone marrow, peripheral blood or a fraction thereof), obtained from a patient prior to a therapy or immunization, with a WT1 polypeptide, wherein the incubation is performed under conditions and for a time sufficient to allow specific activation, proliferation and/or lysis of T cells; (b) detecting an amount of activation, proliferation and/or lysis of the T cells; (c) repeating steps (a) and (b) using a second biological sample comprising CD4+ and/or CD8+ T cells, and taken from the same patient following therapy or immunization; and (d) comparing the amount of activation, proliferation and/or lysis of T cells in the first and second biological samples. Alternatively, a polynucleotide encoding a WT1 polypeptide, or an APC expressing a WT1 polypeptide may be employed in place of the WT1 polypeptide.

A biological sample for use within such methods may be any sample obtained from a patient that would be expected to contain antibodies, CD4+ T cells and/or CD8+ T cells. Suitable biological samples include blood, sera, ascites, bone marrow, pleural effusion and cerebrospinal fluid. A first biological sample may be obtained prior to initiation of therapy or immunization or part way through a therapy or vaccination regime. The second biological sample should be obtained in a similar manner, but at a time following additional therapy or immunization. The second biological sample may be obtained at the completion of, or part way through, therapy or immunization, provided that at least a portion of therapy or immunization takes place between the isolation of the first and second biological samples.

Incubation and detection steps for both samples may generally be performed as described above. A statistically significant increase in the number of immunocomplexes in the second sample relative to the first sample reflects successful therapy or immunization.

The following Examples are offered by way of illustration and not by way of limitation.

## EXAMPLES

### EXAMPLE 1

### IDENTIFICATION OF AN IMMUNE RESPONSE TO WT1 IN PATIENTS WITH HEMATOLOGICAL MALIGNANCIES

This Example illustrates the identification of an existent immune response in patients with a hematological malignancy.

To evaluate the presence of preexisting WT1 specific antibody responses in patients, sera of patients with acute myelogenous leukemia (AML), acute lymphocytic leukemia (ALL), chronic myelogenous leukemia (CML) and severe aplastic anemia were analyzed using Western blot analysis. Sera were tested for the ability to immunoprecipitate WT1 from the human leukemic cell line K562 (American Type Culture Collection, Manassas, VA). In each case, immunoprecipitates were separated by gel electrophoresis, transferred to membrane and probed with the anti WT1 antibody WT180 (Santa Cruz Biotechnology, Inc., Santa Cruz, CA). This Western blot analysis identified potential WT1 specific antibodies in patients with hematological malignancy. A representative Western blot showing the results for a patient with AML is shown in Figure 2. A 52 kD protein in the immunoprecipitate generated using the patient sera was recognized by the WT1 specific antibody. The 52 kD protein migrated at the same size as the positive control.

Additional studies analyzed the sera of patients with AML and CML for the presence of antibodies to full-length and truncated WT1 proteins. CDNA constructs representing the human WT1/full-length (aa 1-449), the N-terminus (aa 1-249) (WT1/N-terminus) and C-terminus (aa 267-449) (WT1/C-terminus) region were subcloned into modified pET28 vectors. The WT1/full-length and WT1/N-terminus proteins were expressed as Ra12 fusion proteins. Ra12 is the C-terminal fragment of a secreted Mycobacterium tuberculosis protein, denoted as MTB32B. (Skeiky et al., Infect Immun. 67;3998, 1999). The Ra12-WT1/full-length fusion region was cloned 3' to a histidine-tag in a histidine-tag modified pET28 vector. The WT1/N-terminus region was subcloned into a modified pET28 vector that has a 5' histidine-tag followed by the thioredoxin (TRX)-WT1/N-terminus fusion region followed by a 3' histidine-tag. The WT1/C-terminus coding region was subcloned into a modified pET28 vector without a fusion partner containing only the 5' and 3' histidine-tag, followed by a Thrombin and EK site.

BL21 pLysS E. *coli* (Stratagene, La Jolla, CA) were transformed with the three WT1 expression constructs, grown overnight and induced with isopropyl-β-D-thiogalactoside (IPTG). WT1 proteins were purified as follows: Cells were harvested and lysed by incubation in 10mM Tris, pH 8.0 with Complete Protease Inhibitor Tablets (Boehringer Mannheim Biochemicals, Indianapolis, IN) at 37°C followed by repeated rounds of sonication. Inclusion bodies were washed twice with 10mM Tris, pH 8.0. Proteins were then purified by metal chelate affinity chromatography over nickel-nitrilotriacetic acid resin (QIAGEN Inc., Valencia, CA; Hochuli et al., Biologically Active Molecules :217, 1989) followed by chromatography on a Source Q anion exchange resin (Amersham Pharmacia Biotech, Upsala, Sweden). The identity of the WT1 proteins was confirmed by N-terminal sequencing.

Sera from adult patients with *de nova* AML or CML were studied for the presence of WT1 specific Ab. Recombinant proteins were adsorbed to TC microwell plates (Nunc, Roskilde, Denmark). Plates were washed with PBS/0.5%Tween 20 and blocked with 1% BSA/PBS/0.1%Tween 20. After washing, serum dilutions were added and incubated overnight at 4°C. Plates were washed and Donkey anti-human IgG-HRP secondary antibody was added (Jackson-Immunochem, West Grove, PA) and incubated for 2h at room temperature. Plates were washed, incubated with TMB Peroxidase substrate solution (Kirkegaard and Perry Laboratories, MA), quenched with 1N $H_2SO_4$, and immediately read (Cyto-Fluor 2350; Millipore, Bedford, MA).

For the serological survey, human sera were tested by ELISA over a range of serial dilutions from 1:50 to 1:20,000. A positive reaction was defined as an OD value of a 1:500 diluted serum that exceeded the mean OD value of sera from normal donors (n=96) by three (WT1/full-length, WT1C-terminus) standard deviations. Due to a higher background in normal donors to the WT1/N-terminus protein a positive reaction to WT1/N-terminus was defined as an OD value of 1:500 diluted serum that exceeded the mean OD value of sera from normal donors by four standard deviations. To verify that the patient Ab response was directed against WT1 and not to the Ra12 or TRX fusion part of the protein or possible E. *coli* contaminant proteins, controls included the Ra12 and TRX protein alone purified in a similar manner. Samples that showed reactivity against the Ra12 and/or TRX proteins were excluded from the analysis.

To evaluate for the presence of immunity to WT1, Ab to recombinant full-length and truncated WT1 proteins in the sera of normal individuals and patients with leukemia were determined. Antibody reactivity was analyzed by ELISA reactivity to WT1/full-length protein, WT1/N-terminus protein and WT1/C-terminus protein.

Only 2 of 96 normal donors had serum antibodies reactive with WT1/full-length protein (Figure 18). One of those individuals had antibody to WT1/N-terminus protein and one had antibody to WT1/C-terminus protein. In contrast, 16 of 63 patients (25%) with AML had serum antibodies reactive with WT1/full-length protein. By marked contrast, only 2 of 63 patients (3%) had reactivity to WT1/C-terminus protein. Fifteen of 81 patients (19%) with CML had serum antibodies reactive with WT1/full-length protein and 12 of 81 patients (15%) had serum antibodies reactive with WT1/N-terminus. Only 3 of 81 patients (3%) had reactivity to WT1/C-terminus protein. (Figures 16 and 17.)

These data demonstrate that Ab responses to WT1 are detectable in some patients with AML and CML. The greater incidence of antibody in leukemia patients provides strong evidence that immunization to the WT1 protein occurred as a result of patients bearing malignancy that expresses or at some time expressed WT1. Without being limited to a specific theory, it is believed that the observed antibody responses to WT1 most probably result from patients becoming immune to WT1 on their own leukemia cells and provide direct evidence that WT1 can be immunogenic despite being a "self" protein.

The presence of antibody to WT1 strongly implies that concurrent helper T cell responses are also present in the same patients. WT1 is an internal protein. Thus, CTL responses are likely to be the most effective in terms of leukemia therapy and the most toxic arm of immunity. Thus, these data provide evidence that therapeutic vaccines directed against WT1 will be able to elicit an immune response to WT1.

The majority of the antibodies detected were reactive with epitopes within the N-terminus while only a small subgroup of patients showed a weak antibody response to the C-terminus. This is consistent with observations in the animal model, where immunization with peptides derived from the N-terminus elicited antibody, helper T cell and CTL responses, whereas none of the peptides tested from the C-terminus elicited antibody or T cell responses (Gaiger et al., Blood 96:1334, 2000).

EXAMPLE 2

INDUCTION OF ANTIBODIES TO WT1 IN MICE IMMUNIZED WITH CELL LINES EXPRESSING WT1

This Example illustrates the use of cells expressing WT1 to induce a WT1 specific antibody response *in vivo.*

Detection of existent antibodies to WT1 in patients with leukemia strongly implied that it is possible to immunize to WT1 protein to elicit immunity to WT1. To test whether immunity to WT1 can be generated by vaccination, mice were injected with TRAMP-C, a WT1 positive tumor cell line of B6 origin. Briefly, male B6 mice were immunized with 5 x $10^6$ TRAMP-C cells subcutaneously and boosted twice with 5 x $10^6$ cells at three week intervals. Three weeks after the final immunization, sera were obtained and single cell suspensions of spleens were prepared in RPMI 1640 medium (GIBCO) with 25$\mu$M $\beta$-2-mercaptoethanol, 200 units of penicillin per ml, 10mM L-glutamine, and 10% fetal bovine serum.

Following immunization to TRAMP-C, a WT1 specific antibody response in the immunized animals was detectable. A representative Western blot is shown in Figure 3. These results show that immunization to WT1 protein can elicit an immune response to WT1 protein.

EXAMPLE 3

INDUCTION OF TH AND ANTIBODY RESPONSES IN MICE IMMUNIZED WITH WT1 PEPTIDES

This Example illustrates the ability of immunization with WT1 peptides to elicit an immune response specific for WT1.

Peptides suitable for eliciting Ab and proliferative T cell responses were identified according to the Tsites program (Rothbard and Taylor, EMBO J. 7:93-100, 1988; Deavin et al., Mol. Immarnol. 33:145-155, 1996), which searches for peptide motifs that have the potential to elicit Th responses. Peptides shown in Table I were synthesized and sequenced.

Table I

WT1 Peptides

| Peptide | Sequence | Comments |
|---|---|---|
| Mouse: p6-22 | RDLNALLPAVSSLGGGG (SEQ ID NO: 13) | 1 mismatch relative to human WT1 sequence |
| Human: p6-22 | RDLNALLPAVPSLGGGG (SEQ ID NO: 1) | |
| Human/mouse: p117-139 | PSQASSGQARMFPNAPYLPSCLE (SEQ ID NOs: 2 and 3) | |
| Mouse: p244-262 | GATLKGMAAGSSSSVKWTE (SEQ ID NO:14) | 1 mismatch relative to human WT1 sequence |
| Human: p244-262 | GATLKGVAAGSSSSVKWTE (SEQ ID NO:4) | |
| Human/mouse: p287-301 | RIHTHGVFRGIQDVR (SEQ ID NOs: 15 and 16) | |
| Mouse: p299-313 | VRRVSGVAPTLVRS (SEQ ID NO:17) | 1 mismatch relative to human WT1 sequence |
| Human/mouse: p421-435 | CQKKFARSDELVRHH (SEQ ID NOs: 19 and 20) | |

For immunization, peptides were grouped as follows:

Group A: p6-22 human: 10.9mg in 1ml (10μl = 100μg)
p117-139 human/mouse: 7.6mg in 1ml (14μl = 100μg)
p244-262 human: 4.6.mg in 1ml (22μl = 100μg)

Group B: p287-301 human/mouse: 7.2mg in 1ml (14μl = 100μg) mouse p299-313: 6.6.mg in 1ml (15μl = 100μg)
p421-435 human/mouse: 3.3mg in 1ml (30μl = 100μg)

Control: (FBL peptide 100μg) + CFA/IFA

Control: (CD45 peptide 100μg) + CFA/IFA

Group A contained peptides present within the amino terminus portion of WT1 (exon 1) and Group B contained peptides present within the carboxy terminus, which contains a four zinc finger region with sequence homology to other DNA-binding proteins. Within group B, p287-301 and p299-313 were derived from exon 7, zinc finger 1, and p421-435 was derived from exon 10, zinc finger IV.

B6 mice were immunized with a group of WT1 peptides or with a control peptide. Peptides were dissolved in 1ml sterile water for injection, and B6 mice were immunized 3 times at time intervals of three weeks. Adjuvants used were CFA/IFA, GM-CSF, and Montinide. The presence of antibodies specific for WT1 was then determined as described in Examples 1 and 2, and proliferative T cell responses were evaluated using a standard thymidine incorporation assay, in which cells were cultured in the presence of antigen and proliferation was evaluated by measuring incorporated radioactivity (Chen et al., Cancer Res. 54:1065-1070, 1994). In particular, lymphocytes were cultured in 96-well plates at $2x10^5$ cells per well with $4x10^5$ irradiated (3000 rads) syngeneic spleen cells and the designated peptide.

Immunization of mice with the group of peptides designated as Group A elicited an antibody response to WT1 (Figure 4). No antibodies were detected following immunization to Vaccine B, which is consistent with a lack of helper T cell response from immunization with Vaccine B. P117-139 elicited proliferative T cell responses (Figures 5A-5C). The stimulation indices (SI) varied between 8 and 72. Other peptides (P6-22 and P299-313) also were shown to elicit proliferative T cell responses. Immunization with P6-22 resulted in a stimulation index (SI) of 2.3 and immunization with P299-313 resulted in a SI of 3.3. Positive controls included ConA stimulated T cells, as well as T cells stimulated with known antigens, such as CD45 and FBL, and allogeneic T cell lines (DeBruijn et al., Eur. J. Immunol. 21:2963-2970, 1991).

Figures 6A and 6B show the proliferative response observed for each of the three peptides within vaccine A (Figure 6A) and vaccine B (Figure 6B). Vaccine A elicited proliferative T cell responses to the immunizing peptides p6-22 and p117-139, with stimulation indices (SI) varying between 3 and 8 (bulk lines). No proliferative response to p244-262 was detected (Figure 6A).

Subsequent *in vitro* stimulations were carried out as single peptide stimulations using only p6-22 and p117-139. Stimulation of the Vaccine A specific T cell line with p117-139 resulted in proliferation to p117-139 with no response to p6-22 (Figure7A). Clones derived from the line were specific for p117-139 (Figure 7B). By contrast, stimulation of the Vaccine A specific T cell line with p6-22 resulted in proliferation to p6-22 with no response to p117-139 (Figure 7C). Clones derived from the line were specific for p6-22 (Figure 7D).

These results show that vaccination with WT1 peptides can elicit antibody responses to WT1 protein and proliferative T cell responses to the immunizing peptides.

EXAMPLE 4

INDUCTION OF CTL RESPONSES IN MICE IMMUNIZED WITH WT1 PEPTIDES

This Example illustrates the ability of WT1 peptides to elicit CTL immunity.

Peptides (9-mers) with motifs appropriate for binding to class I MHC were identified using a BIMAS HLA peptide binding prediction analysis (Parker et al., J. Immunol. 152:163, 1994). Peptides identified within such analyses are shown in Tables II - XLIV. In each of these tables, the score reflects the theoretical binding affinity (half-time of dissociation) of the peptide to the MHC molecule indicated.

Peptides identified using the Tsites program (Rothbard and Taylor, EMBO J. 7:93-100, 1988; Deavin et al., Mol. Immunol. 33:145-155, 1996), which searches for peptide motifs that have the potential to elicit Th responses are further shown in Figures 8A and 8B, and Table XLV.

Table II

| Results of BIMAS HLA Peptide Binding Prediction Analysis for Binding of Human WT1 Peptides to Human HLA A1 | | | |
|---|---|---|---|
| Rank | Start Position | Subsequence Residue Listing | Score (Estimate of Half Time of Disassociation of a Molecule Containing This Subsequence) |
| 1 | 137 | CLESQPAIR (SEQ ID NO:47) | 18.000 |
| 2 | 80 | GAEPHEEQC (SEQ ID NO:87) | 9.000 |
| 3 | 40 | FAPPGASAY (SEQ ID NO:74) | 5.000 |
| 4 | 354 | QCDFKDCER (SEQ ID NO: 162) | 5.000 |
| 5 | 2 | GSDVRDLNA (SEQ ID NO: 101) | 3.750 |
| 6 | 152 | VTFDGTPSY (SEQ ID NO:244) | 2.500 |
| 7 | 260 | WTEGQSNHS (SEQ ID NO: 247) | 2.250 |
| 8 | 409 | TSEKPFSCR (SEQ ID NO:232) | 1.350 |
| 9 | 73 | KQEPSWGGA (SEQ ID NO: 125) | 1.350 |
| 10 | 386 | KTCQRKFSR (SEQ ID NO: 128) | 1.250 |
| 11 | 37 | VLDFAPPGA (SEQ ID NO:241) | 1.000 |
| 12 | 325 | CAYPGCNKR (SEQ ID NO:44) | 1.000 |
| 13 | 232 | QLECMTWNQ (SEQ ID NO: 167) | 0.900 |
| 14 | 272 | ESDNHTTPI (SEQ ID NO:71) | 0.750 |
| 15 | 366 | RSDQLKRHQ (SEQ ID NO: 193) | 0.750 |
| 16 | 222 | SSDNLYQMT (SEQ ID NO: 217) | 0.750 |
| 17 | 427 | RSDELVRHH (SEQ ID NO: 191) | 0.750 |
| 18 | 394 | RSDHLKTHT (SEQ ID NO:192) | 0.750 |
| 19 | 317 | TSEKRPFMC (SEQ ID NO: 233) | 0.675 |
| 20 | 213 | QALLLRTPY (SEQ ID NO:160) | 0.500 |

Table III

| Results of BIMAS HLA Peptide Binding Prediction Analysis for Binding of Human WT1 Peptides to Human HLA A 0201 | | | |
|---|---|---|---|
| Rank | Start Position | Subsequence Residue Listing | Score (Estimate of Half Time of Disassociation of a Molecule Containing This Subsequence) |
| 1 | 126 | RMFPNAPYL (SEQ ID NO: 185) | 313.968 |

(continued)

| Results of BIMAS HLA Peptide Binding Prediction Analysis for Binding of Human WT1 Peptides to Human HLA A 0201 | | | |
|---|---|---|---|
| Rank | Start Position | Subsequence Residue Listing | Score (Estimate of Half Time of Disassociation of a Molecule Containing This Subsequence) |
| 2 | 187 | SLGEQQYSV (SEQ ID NO: 214) | 285.163 |
| 3 | 10 | ALLPAVPSL (SEQ ID NO:34) | 181.794 |
| 4 | 242 | NLGATLKGV (SEQ ID NO: 146) | 159.970 |
| 5 | 225 | NLYQMTSQL (SEQ ID NO: 147) | 68.360 |
| 6 | 292 | GVFRGIQDV (SEQ ID NO: 103) | 51.790 |
| 7 | 191 | QQYSVPPPV (SEQ ID NO: 171) | 22.566 |
| 8 | 280 | ILCGAQYRI (SEQ ID NO:116) | 17.736 |
| 9 | 235 | CMTWNQMNL (SEQ ID NO: 49) | 15.428 |
| 10 | 441 | NMTKLQLAL (SEQ ID NO:149) | 15.428 |
| 11 | 7 | DLNALLPAV (SEQ ID NO:58) | 11.998 |
| 12 | 227 | YQMTSQLEC (SEQ ID NO: 251) | 8.573 |
| 13 | 239 | NQMNLGATL (SEQ ID NO: 151) | 8.014 |
| 14 | 309 | TLVRSASET (SEQ ID NO:226) | 7.452 |
| 15 | 408 | KTSEKPFSC (SEQ ID NO:129) | 5.743 |
| 16 | 340 | LQMHSRKHT (SEQ ID NO: 139) | 4.752 |
| 17 | 228 | QMTSQLECM (SEQ ID NO: 169) | 4.044 |
| 18 | 93 | TVHFSGQFT (SEQ ID NO: 235) | 3.586 |
| 19 | 37 | VLDFAPPGA (SEQ ID NO: 241) | 3.378 |
| 20 | 86 | EQCLSAFTV (SEQ ID NO:69) | 3.068 |

Table IV

| Results of BIMAS HLA Peptide Binding Prediction Analysis for Binding of Human WT1 Peptides to Human HLA A 0205 | | | |
|---|---|---|---|
| Rank | Start Position | Subsequence Residue Listing | Score (Estimate of Half Time of Disassociation of a Molecule Containing This Subsequence) |
| 1 | 10 | ALLPAVPSL (SEQ ID NO:34) | 42.000 |

(continued)

| Results of BIMAS HLA Peptide Binding Prediction Analysis for Binding of Human WT1 Peptides to Human HLA A 0205 | | | |
|---|---|---|---|
| Rank | Start Position | Subsequence Residue Listing | Score (Estimate of Half Time of Disassociation of a Molecule Containing This Subsequence) |
| 2 | 292 | GVFRGIQDV (SEQ ID NO: 103) | 24.000 |
| 3 | 126 | RMFPNAPYL (SEQ ID NO: 185) | 21.000 |
| 4 | 225 | NLYQMTSQL (SEQ ID NO: 147) | 21.000 |
| 5 | 239 | NQMNLGATL (SEQ ID NO: 151) | 16.800 |
| 6 | 302 | RVPGVAPTL (SEQ ID NO: 195) | 14.000 |
| 7 | 441 | NMTKLQLAL (SEQ ID NO:149) | 7.000 |
| 8 | 235 | CMTWNQMNL (SEQ ID NO: 49) | 7.000 |
| 9 | 187 | SLGEQQYSV (SEQ ID NO: 214) | 6.000 |
| 10 | 191 | QQYSVPPPV (SEQ ID NO: 171) | 4.800 |
| 11 | 340 | LQMHSRKHT(SEQ ID NO: 139) | 4.080 |
| 12 | 242 | NLGATLKGV(SEQ ID NO:146) | 4.000 |
| 13 | 227 | YQMTSQLEC (SEQ ID NO: 251) | 3.600 |
| 14 | 194 | SVPPPVYGC(SEQ ID NO: 218) | 2.000 |
| 15 | 93 | TVHFSGQFT(SEQ ID NO:235) | 2.000 |
| 16 | 280 | ILCGAQYRI (SEQ ID NO:116) | 1.700 |
| 17 | 98 | GQFTGTAGA (SEQ ID NO:99) | 1.200 |
| 18 | 309 | TLVRSASET (SEQ ID NO:226) | 1.000 |
| 19 | 81 | AEPHEEQCL (SEQ ID NO:30) | 0.980 |
| 20 | 73 | KQEPSWGGA (SEQ ID NO: 125) | 0.960 |

Table V

| Results of BIMAS HLA Peptide Binding Prediction Analysis for Binding of Human WT1 Peptides to Human HLA A24 | | | |
|---|---|---|---|
| Rank | Start Position | Subsequence Residue Listing | Score (Estimate of Half Time of Disassociation of a Molecule Containing This Subsequence) |
| 1 | 302 | RVPGVAPTL (SEQ ID NO:195) | 16.800 |
| 2 | 218 | RTPYSSDNL (SEQ ID NO:194) | 12.000 |

(continued)

| Results of BIMAS HLA Peptide Binding Prediction Analysis for Binding of Human WT1 Peptides to Human HLA A24 | | | |
|---|---|---|---|
| Rank | Start Position | Subsequence Residue Listing | Score (Estimate of Half Time of Disassociation of a Molecule Containing This Subsequence) |
| 3 | 356 | DFKDCERRF (SEQ ID NO:55) | 12.000 |
| 4 | 126 | RMFPNAPYL (SEQ ID NO:185) | 9.600 |
| 5 | 326 | AYPGCNKRY (SEQ ID NO:42) | 7.500 |
| 6 | 270 | GYESDNHT (SEQ ID NO:106)T | 7.500 |
| 7 | 239 | NQMNLGATL (SEQ ID NO:151) | 7.200 |
| 8 | 10 | ALLPAVPSL (SEQ ID NO:34) | 7.200 |
| 9 | 130 | NAPYLPSCL (SEQ ID NO:144) | 7.200 |
| 10 | 329 | GCNKRYFKL (SEQ ID NO:90) | 6.600 |
| 11 | 417 | RWPSCQKKF (SEQ ID NO: 196) | 6.600 |
| 12 | 47 | AYGSLGGPA (SEQ ID NO:41) | 6.000 |
| 13 | 180 | DPMGQQGSL (SEQ ID NO:59) | 6.000 |
| 14 | 4 | DVRDLNALL (SEQ ID NO:62) | 5.760 |
| 15 | 285 | QYRIHTHGV (SEQ ID NO:175) | 5.000 |
| 16 | 192 | QYSVPPPVY (SEQ ID NO:176) | 5.000 |
| 17 | 207 | DSCTGSQAL (SEQ ID NO:61) | 4.800 |
| 18 | 441 | NMTKLQLAL (SEQ ID NO:149) | 4.800 |
| 19 | 225 | NLYQMTSQL (SEQ ID NO:147) | 4.000 |
| 20 | 235 | CMTWNQMNL (SEQ ID NO:49) | 4.000 |

Table VI

| Results of BIMAS HLA Peptide Binding Prediction Analysis for Binding of Human WT1 Peptides to Human HLA A3 | | | |
|---|---|---|---|
| Rank | Start Position | Subsequence Residue Listing | Score (Estimate of Half Time of Disassociation of a Molecule Containing This Subsequence) |
| 1 | 436 | NMHQRNMTK (SEQ ID NO: 148) | 40.000 |
| 2 | 240 | QMNLGATLK (SEQ ID NO: 168) | 20.000 |
| 3 | 88 | CLSAFTVHF (SEQ ID NO:48) | 6.000 |
| 4 | 126 | RMFPNAPYL (SEQ ID NO:185) | 4.500 |
| 5 | 169 | AQFPNHSFK (SEQ ID NO:36) | 4.500 |
| 6 | 10 | ALLPAVPSL (SEQ ID NO:34) | 4.050 |
| 7 | 137 | CLESQPAIR (SEQ ID NO:47) | 4.000 |
| 8 | 225 | NLYQMTSQL (SEQ ID NO: 147) | 3.000 |
| 9 | 32 | AQWAPVLDF (SEQ ID NO:37) | 2.700 |

(continued)

| Results of BIMAS HLA Peptide Binding Prediction Analysis for Binding of Human WT1 Peptides to Human HLA A3 | | | |
|---|---|---|---|
| Rank | Start Position | Subsequence Residue Listing | Score (Estimate of Half Time of Disassociation of a Molecule Containing This Subsequence) |
| 10 | 280 | ILCGAQYRI (SEQ ID NO:116) | 2.700 |
| 11 | 386 | KTCQRKFSR (SEQ ID NO: 128) | 1.800 |
| 12 | 235 | CMTWNQMNL (SEQ ID NO: 49) | 1.200 |
| 13 | 441 | NMTKLQLAL (SEQ ID NO:149) | 1.200 |
| 14 | 152 | VTFDGTPSY (SEQ ID NO:244) | 1.000 |
| 15 | 187 | SLGEQQYSV (SEQ ID NO: 214) | 0.900 |
| 16 | 383 | FQCKTCQRK (SEQ ID NO:80) | 0.600 |
| 17 | 292 | GVFRGIQDV (SEQ ID NO:103) | 0.450 |
| 18 | 194 | SVPPPVYGC (SEQ ID NO: 218) | 0.405 |
| 19 | 287 | RIHTHGVFR (SEQ ID NO:182) | 0.400 |
| 20 | 263 | GQSNHSTGY (SEQ ID NO: 100) | 0.360 |

Table VII

| Results of BIMAS HLA Peptide Binding Prediction Analysis for Binding of Human WT1 Peptides to Human HLA A68.1 | | | |
|---|---|---|---|
| Rank | Start Position | Subsequence Residue Listing | Score (Estimate of Half Time of Disassociation of a Molecule Containing This Subsequence) |
| 1 | 100 | FTGTAGACR (SEQ ID NO:84) | 100.000 |
| 2 | 386 | KTCQRKFSR (SEQ ID NO: 128) | 50.000 |
| 3 | 368 | DQLKRHQRR (SEQ ID NO:60) | 30.000 |
| 4 | 312 | RSASETSEK (SEQ ID NO:190) | 18.000 |
| 5 | 337 | LSHLQMHSR (SEQ ID NO: 141) | 15.000 |
| 6 | 364 | FSRSDQLKR (SEQ ID NO:83) | 15.000 |
| 7 | 409 | TSEKPFSCR (SEQ ID NO:232) | 15.000 |
| 8 | 299 | DVRRVPGVA (SEQ ID NO:63) | 12.000 |
| 9 | 4 | DVRDLNALL (SEQ ID NO:62) | 12.000 |
| 10 | 118 | SQASSGQAR (SEQ ID NO: 216) | 10.000 |
| 11 | 343 | HSRKHTGEK (SEQ ID NO: 111) | 9.000 |
| 12 | 169 | AQFPNHSFK (SEQ ID NO:36) | 9.000 |

(continued)

| Results of BIMAS HLA Peptide Binding Prediction Analysis for Binding of Human WT1 Peptides to Human HLA A68.1 | | | |
|---|---|---|---|
| Rank | Start Position | Subsequence Residue Listing | Score (Estimate of Half Time of Disassociation of a Molecule Containing This Subsequence) |
| 13 | 292 | GVFRGIQDV (SEQ ID NO:103) | 8.000 |
| 14 | 325 | CAYPGCNKR (SEQ ID NO:44) | 7.500 |
| 15 | 425 | FARSDELVR (SEQ ID NO:75) | 7.500 |
| 16 | 354 | QCDFKDCER (SEQ ID NO: 162) | 7.500 |
| 17 | 324 | MCAYPGCNK (SEQ ID NO: 142) | 6.000 |
| 18 | 251 | AAGSSSSVK (SEQ ID NO:28) | 6.000 |
| 19 | 379 | GVKPFQCKT (SEQ ID NO: 104) | 6.000 |
| 20 | 137 | CLESQPAIR (SEQ ID NO:47) | 5.000 |

Table VIII

| Results of BIMAS HLA Peptide Binding Prediction Analysis for Binding of Human WT1 Peptides to Human HLA A 1101 | | | |
|---|---|---|---|
| Rank | Start Position | Subsequence Residue Listing | Score (Estimate of Half Time of Disassociation of a Molecule Containing This Subsequence) |
| 1 | 386 | KTCQRKFSR (SEQ ID NO: 128) | 1.800 |
| 2 | 169 | AQFPNHSFK (SEQ ID NO:36) | 1.200 |
| 3 | 436 | NMHQRNMTK (SEQ ID NO: 148) | 0.800 |
| 4 | 391 | KFSRSDHLK (SEQ ID NO: 120) | 0.600 |
| 5 | 373 | HQRRHTGVK (SEQ ID NO: 109) | 0.600 |
| 6 | 383 | FQCKTCQRK (SEQ ID NO: 80) | 0.600 |
| 7 | 363 | RFSRSDQLK (SEQ ID NO: 178) | 0.600 |
| 8 | 240 | QMNLGATLK (SEQ ID NO: 168) | 0.400 |
| 9 | 287 | RIHTHGVFR (SEQ ID NO: 182) | 0.240 |
| 10 | 100 | FTGTAGACR (SEQ ID NO: 84) | 0.200 |
| 11 | 324 | MCAYPGCNK (SEQ ID NO: 142) | 0.200 |
| 12 | 251 | AAGSSSSVK (SEQ ID NO:28) | 0.200 |

(continued)

| Results of BIMAS HLA Peptide Binding Prediction Analysis for Binding of Human WT1 Peptides to Human HLA A 1101 | | | |
|---|---|---|---|
| Rank | Start Position | Subsequence Residue Listing | Score (Estimate of Half Time of Disassociation of a Molecule Containing This Subsequence) |
| 13 | 415 | SCRWPSCQK (SEQ ID NO: 201) | 0.200 |
| 14 | 118 | SQASSGQAR (SEQ ID NO: 216) | 0.120 |
| 15 | 292 | GVFRGIQDV (SEQ ID NO: 103) | 0.120 |
| 16 | 137 | CLESQPAIR (SEQ ID NO:47) | 0.080 |
| 17 | 425 | FARSDELVR (SEQ ID NO:75) | 0.080 |
| 18 | 325 | CAYPGCNKR (SEQ ID NO: 44) | 0.0817 |
| 19 | 312 | RSASETSEK (SEQ ID NO: 190) | 0.060 |
| 20 | 65 | PPPPHSFI (SEQ ID NO:156) K | 0.060 |

Table IX

| Results of BIMAS HLA Peptide Binding Prediction Analysis for Binding of Human WT1 Peptides to Human HLA A 3101 | | | |
|---|---|---|---|
| Rank | Start Position | Subsequence Residue Listing | Score (Estimate of Half Time of Disassociation of a Molecule Containing This Subsequence) |
| 1 | 386 | KTCQRKFSR (SEQ ID NO: 128) | 9.000 |
| 2 | 287 | RIHTHGVFR (SEQ ID NO: 182) | 6.000 |
| 3 | 137 | CLESQPAIR (SEQ ID NO:47) | 2.000 |
| 4 | 118 | SQASSGQAR (SEQ ID NO: 216) | 2.000 |
| 5 | 368 | DQLKRHQRR (SEQ ID NO: 60) | 1.200 |
| 6 | 100 | FTGTAGACR (SEQ ID NO: 84) | 1.000 |
| 7 | 293 | VFRGIQDVR (SEQ ID NO: 238) | 0.600 |
| 8 | 325 | CAYPGCNKR (SEQ ID NO: 44) | 0.600 |
| 9 | 169 | AQFPNHSFK (SEQ ID NO:36) | 0.600 |
| 10 | 279 | PILCGAQYR (SEQ ID NO: 155) | 0.400 |
| 11 | 436 | NMHQRNMTK (SEQ ID NO: 148) | 0.400 |

(continued)

| Results of BIMAS HLA Peptide Binding Prediction Analysis for Binding of Human WT1 Peptides to Human HLA A 3101 | | | |
|---|---|---|---|
| Rank | Start Position | Subsequence Residue Listing | Score (Estimate of Half Time of Disassociation of a Molecule Containing This Subsequence) |
| 12 | 425 | FARSDELVR (SEQ ID NO:75) | 0.400 |
| 13 | 32 | AQWAPVLDF (SEQ ID NO: 37) | 0.240 |
| 14 | 240 | QMNLGATLK (SEQ ID NO: 168) | 0.200 |
| 15 | 354 | QCDFKDCER (SEQ ID NO: 162) | 0.200 |
| 16 | 373 | HQRRHTGVK (SEQ ID NO: 109) | 0.200 |
| 17 | 383 | FQCKTCQRK (SEQ ID NO: 80) | 0.200 |
| 18 | 313 | SASETSEKR (SEQ ID NO: 197) | 0.200 |
| 19 | 358 | KDCERRFSR (SEQ ID NO: 118) | 0.180 |
| 20 | 391 | KFSRSDHLK (SEQ ID NO: 120) | 0.180 |

Table X

| Results of BIMAS HLA Peptide Binding Prediction Analysis for Binding of Human WT1 Peptides to Human HLA A 3302 | | | |
|---|---|---|---|
| Rank | Start Position | Subsequence Residue Listing | Score (Estimate of Half Time of Disassociation of a Molecule Containing This Subsequence) |
| 1 | 337 | LSHLQMHSR (SEQ ID NO: 141) | 15.000 |
| 2 | 409 | TSEKPFSCR (SEQ ID NO: 232) | 15.000 |
| 3 | 364 | FSRSDQLKR (SEQ ID NO:83) | 15.000 |
| 4 | 137 | CLESQPAIR (SEQ ID NO:47) | 9.000 |
| 5 | 368 | DQLKRHQRR (SEQ ID NO: 60) | 9.000 |
| 6 | 287 | RIHTHGVFR (SEQ ID NO: 182) | 4.500 |
| 7 | 210 | TGSQALLLR (SEQ ID NO: 223) | 3.000 |
| 8 | 425 | FARSDELVR (SEQ ID NO:75) | 3.000 |
| 9 | 313 | SASETSEKR (SEQ ID NO: 197) | 3.000 |
| 10 | 293 | VFRGIQDVR (SEQ ID NO: 238) | 3.000 |

(continued)

| Results of BIMAS HLA Peptide Binding Prediction Analysis for Binding of Human WT1 Peptides to Human HLA A 3302 | | | |
|---|---|---|---|
| Rank | Start Position | Subsequence Residue Listing | Score (Estimate of Half Time of Disassociation of a Molecule Containing This Subsequence) |
| 11 | 354 | QCDFKDCER (SEQ ID NO: 162) | 3.000 |
| 12 | 100 | FTGTAGACR (SEQ ID NO:84) | 3.000 |
| 13 | 118 | SQASSGQAR (SEQ ID NO: 216) | 3.000 |
| 14 | 325 | CAYPGCNKR (SEQ ID NO:44) | 3.000 |
| 15 | 207 | DSCTGSQAL (SEQ ID NO:61) | 1.500 |
| 16 | 139 | ESQPAIRNQ (SEQ ID NO:72) | 1.500 |
| 17 | 299 | DVRRVPGVA (SEQ ID NO:63) | 1.500 |
| 18 | 419 | PSCQKKFAR (SEQ ID NO: 159) | 1.500 |
| 19 | 272 | ESDNHTTPI (SEQ ID NO:71) | 1.500 |
| 20 | 4 | DVRDLNALL (SEQ ID NO:62) | 1.500 |

Table XI

| Results of BIMAS HLA Peptide Binding Prediction Analysis for Binding of Human WT1 Peptides to Human HLA B14 | | | |
|---|---|---|---|
| Rank | Start Position | Subsequence Residue Listing | Score (Estimate of Half Time of Disassociation of a Molecule Containing This Subsequence) |
| 1 | 362 | RRFSRSDQL (SEQ ID NO: 187) | 1000.000 |
| 2 | 332 | KRYFKLSHL (SEQ ID NO:127) | 300.000 |
| 3 | 423 | KKFARSDEL (SEQ ID NO:122) | 150.000 |
| 4 | 390 | RKFSRSDHL (SEQ ID NO:183) | 150.000 |
| 5 | 439 | QRNMTKLQL (SEQ ID NO: 173) | 20.000 |
| 6 | 329 | GCNKRYFKL (SEQ ID NO:90) | 10.000 |
| 7 | 10 | ALLPAVPSL (SEQ ID NO:34) | 10.000 |
| 8 | 180 | DPMGQQGSL (SEQ ID NO:59) | 9.000 |
| 9 | 301 | RRVPGVAPT (SEQ ID NO: 189) | 6.000 |
| 10 | 126 | RMFPNAPYL (SEQ ID NO: 185) | 5.000 |
| 11 | 371 | KRHQRRHTG (SEQ ID NO: 126) | 5.000 |
| 12 | 225 | NLYQMTSQL (SEQ ID NO: 147) | 5.000 |
| 13 | 144 | IRNQGYSTV (SEQ ID NO:117) | 4.000 |

(continued)

| Results of BIMAS HLA Peptide Binding Prediction Analysis for Binding of Human WT1 Peptides to Human HLA B14 | | | |
|---|---|---|---|
| Rank | Start Position | Subsequence Residue Listing | Score (Estimate of Half Time of Disassociation of a Molecule Containing This Subsequence) |
| 14 | 429 | DELVRHHNM (SEQ ID NO:53) | 3.000 |
| 15 | 437 | MHQRNMTKL (SEQ ID NO: 143) | 3.000 |
| 16 | 125 | ARMFPNAPY (SEQ ID NO:38) | 3.000 |
| 17 | 239 | NQMNLGATL (SEQ ID NO: 151) | 3.000 |
| 18 | 286 | YRIHTHGVF (SEQ ID NO:252) | 3.000 |
| 19 | 174 | HSFKHEDPM (SEQ ID NO: 110) | 3.000 |
| 20 | 372 | RHQRRHTGV (SEQ ID NO: 181) | 3.000 |

Table XII

| Results of BIMAS HLA Peptide Binding Prediction Analysis for Binding of Human WT1 Peptides to Human HLA B40 | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO:30) | 40.000 |
| 2 | 429 | DELVRHHNM (SEQ ID NO:53) | 24.000 |
| 3 | 410 | SEKPFSCRW (SEQ ID NO: 207) | 20.000 |
| 4 | 318 | SEKRPFMCA (SEQ ID NO: 208) | 15.000 |
| 5 | 233 | LECMTWNQM (SEQ ID NO: 131) | 12.000 |
| 6 | 3 | SDVRDLNAL (SEQ ID NO:206) | 10.000 |
| 7 | 349 | GEKPYQCDF (SEQ ID NO:91) | 8.000 |
| 8 | 6 | RDLNALLPA (SEQ ID NO:177) | 5.000 |
| 9 | 85 | EEQCLSAFT (SEQ ID NO:65) | 4.000 |
| 10 | 315 | SETSEKRPF (SEQ ID NO:209) | 4.000 |
| 11 | 261 | TEGQSNHST (SEQ ID NO: 221) | 4.000 |
| 12 | 23 | GCALPVSGA (SEQ ID NO:89) | 3.000 |
| 13 | 38 | LDFAPPGAS (SEQ ID NO:130) | 3.000 |
| 14 | 273 | SDNHTTPIL (SEQ ID NO:204) | 2.500 |
| 15 | 206 | TDSCTGSQA (SEQ ID NO: 220) | 2.500 |
| 16 | 24 | CALPVSGAA (SEQ ID NO:43) | 2.000 |
| 17 | 98 | GQFTGTAGA (SEQ ID NO:99) | 2.000 |

(continued)

| Results of BIMAS HLA Peptide Binding Prediction Analysis for Binding of Human WT1 Peptides to Human HLA B40 | | | |
|---|---|---|---|
| Rank | Start Position | Subsequence Residue Listing | Score (Estimate of Half Time of Disassociation of a Molecule Containing This Subsequence) |
| 18 | 30 | GAAQWAPVL (SEQ ID NO:86) | 2.000 |
| 19 | 84 | HEEQCLSAF (SEQ ID NO:107) | 2.000 |
| 20 | 26 | LPVSGAAQW (SEQ ID NO: 138) | 2.000 |

Table XIII

| Results of BIMAS HLA Peptide Binding Prediction Analysis for Binding of Human WT1 Peptides to Human HLA B60 | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO:30) | 160.000 |
| 2 | 3 | SDVRDLNAL (SEQ ID NO:206) | 40.000 |
| 3 | 429 | DELVRHHNM (SEQ ID NO:53) | 40.000 |
| 4 | 233 | LECMTWNQM (SEQ ID NO: 131) | 22.000 |
| 5 | 273 | SDNHTTPIL (SEQ ID NO:204) | 20.000 |
| 6 | 209 | CTGSQALLL (SEQ ID NO:52) | 8.000 |
| 7 | 30 | GAAQWAPVL (SEQ ID NO:86) | 8.000 |
| 8 | 318 | SEKRPFMCA (SEQ ID NO: 208) | 8.000 |
| 9 | 180 | DPMGQQGSL (SEQ ID NO:59) | 8.000 |
| 10 | 138 | LESQPAIRN (SEQ ID NO:132) | 5.280 |
| 11 | 239 | NQMNLGATL (SEQ ID NO: 151) | 4.400 |
| 12 | 329 | GCNKRYFKL (SEQ ID NO:90) | 4.400 |
| 13 | 130 | NAPYLPSCL (SEQ ID NO:144) | 4.400 |
| 14 | 85 | EEQCLSAFT (SEQ ID NO:65) | 4.400 |
| 15 | 208 | SCTGSQALL (SEQ ID NO:202) | 4.000 |
| 16 | 207 | DSCTGSQAL (SEQ ID NO:61) | 4.000 |
| 17 | 218 | RTPYSSDNL (SEQ ID NO:194) | 4.000 |
| 18 | 261 | TEGQSNHST (SEQ ID NO: 221) | 4.000 |
| 19 | 18 | LGGGGGCAL (SEQ ID NO: 134) | 4.000 |
| 20 | 221 | YSSDNLYQM (SEQ ID NO: 253) | 2.200 |

Table XIV

| Results of BIMAS HLA Peptide Binding Prediction Analysis for Binding of Human WT1 Peptides to Human HLA B61 | | | |
|---|---|---|---|
| Rank | Start Position | Subsequence Residue Listing | Score (Estimate of Half Time of Disassociation of a Molecule Containing This Subsequence) |
| 1 | 318 | SEKRPFMCA (SEQ ID NO: 208) | 20.000 |
| 2 | 429 | DELVRHHNM (SEQ ID NO:53) | 16.000 |
| 3 | 298 | QDVRRVPGV (SEQ ID NO: 164) | 10.000 |
| 4 | 81 | AEPHEEQCL (SEQ ID NO:30) | 8.000 |
| 5 | 233 | LECMTWNQM (SEQ ID NO: 131) | 8.000 |
| 6 | 6 | RDLNALLPA (SEQ ID NO:177) | 5.500 |
| 7 | 85 | EEQCLSAFT (SEQ ID NO:65) | 4.000 |
| 8 | 261 | TEGQSNHST (SEQ ID NO: 221) | 4.000 |
| 9 | 206 | TDSCTGSQA (SEQ ID NO: 220) | 2.500 |
| 10 | 295 | RGIQDVRRV (SEQ ID NO:179) | 2.200 |
| 11 | 3 | SDVRDLNAL (SEQ ID NO:206) | 2.000 |
| 12 | 250 | VAAGSSSSV (SEQ ID NO:236) | 2.000 |
| 13 | 29 | SGAAQWAPV (SEQ ID NO: 211) | 2.000 |
| 14 | 315 | SETSEKRPF (SEQ ID NO:209) | 1.600 |
| 15 | 138 | LESQPAIRN (SEQ ID NO:132) | 1.200 |
| 16 | 244 | GATLKGVAA (SEQ ID NO:88) | 1.100 |
| 17 | 20 | GGGGCALPV (SEQ ID NO:92) | 1.100 |
| 18 | 440 | RNMTKLQLA (SEQ ID NO:186) | 1.100 |
| 19 | 23 | GCALPVSGA (SEQ ID NO:89) | 1.100 |
| 20 | 191 | QQYSVPPPV (SEQ ID NO: 171) | 1.000 |

Table XV

| Results of BIMAS HLA Peptide Binding Prediction Analysis for Binding of Human WT1 Peptides to Human HLA B62 | | | |
|---|---|---|---|
| Rank | Start Position | Subsequence Residue Listing | Score (Estimate of Half Time of Disassociation of a Molecule Containing This Subsequence) |
| 1 | 146 | NQGYSTVTF (SEQ ID NO: 150) | 211.200 |
| 2 | 32 | AQWAPVLDF (SEQ ID NO:37) | 96.000 |
| 3 | 263 | GQSNHSTGY (SEQ ID NO: 100) | 96.000 |
| 4 | 88 | CLSAFTVHF (SEQ ID NO:48) | 96.000 |

(continued)

| Results of BIMAS HLA Peptide Binding Prediction Analysis for Binding of Human WT1 Peptides to Human HLA B62 | | | |
|---|---|---|---|
| Rank | Start Position | Subsequence Residue Listing | Score (Estimate of Half Time of Disassociation of a Molecule Containing This Subsequence) |
| 5 | 17 | SLGGGGGCA (SEQ ID NO: 215) | 9.600 |
| 6 | 239 | NQMNLGATL (SEQ ID NO: 151) | 8.800 |
| 7 | 191 | QQYSVPPPV (SEQ ID NO: 171) | 8.000 |
| 8 | 98 | GQFTGTAGA (SEQ ID NO:99) | 8.000 |
| 9 | 384 | QCKTCQRKF (SEQ ID NO: 163) | 6.000 |
| 10 | 40 | FAPPGASAY (SEQ ID NO:74) | 4.800 |
| 11 | 227 | YQMTSQLEC (SEQ ID NO: 251) | 4.800 |
| 12 | 187 | SLGEQQYSV (SEQ ID NO: 214) | 4.400 |
| 13 | 86 | EQCLSAFTV (SEQ ID NO:69) | 4.400 |
| 14 | 152 | VTFDGTPSY (SEQ ID NO:244) | 4.400 |
| 15 | 101 | TGTAGACRY (SEQ ID NO: 224) | 4.000 |
| 16 | 242 | NLGATLKGV (SEQ ID NO:146) | 4.000 |
| 17 | 92 | FTVHFSGQF (SEQ ID NO:85) | 4.000 |
| 18 | 7 | DLNALLPAV (SEQ ID NO:58) | 4.000 |
| 19 | 123 | GQARMFPNA (SEQ ID NO:98) | 4.000 |
| 20 | 280 | ILCGAQYRI (SEQ ID NO:116) | 3.120 |

Table XVI

| Results of BIMAS HLA Peptide Binding Prediction Analysis for Binding of Human WT1 Peptides to Human HLA B7 | | | |
|---|---|---|---|
| Rank | Start Position | Subsequence Residue Listing | Score (Estimate of Half Time of Disassociation of a Molecule Containing This Subsequence) |
| 1 | 180 | DPMGQQGSL (SEQ ID NO:59) | 240.000 |
| 2 | 4 | DVRDLNALL (SEQ ID NO:62) | 200.000 |
| 3 | 302 | RVPGVAPTL (SEQ ID NO:195) | 20.000 |
| 4 | 30 | GAAQWAPVL (SEQ ID NO:86) | 12.000 |
| 5 | 239 | NQMNLGATL (SEQ ID NO: 151) | 12.000 |
| 6 | 130 | NAPYLPSCL (SEQ ID NO:144) | 12.000 |
| 7 | 10 | ALLPAVPSL (SEQ ID NO:34) | 12.000 |
| 8 | 299 | DVRRVPGVA (SEQ ID NO:63) | 5.000 |

(continued)

| Results of BIMAS HLA Peptide Binding Prediction Analysis for Binding of Human WT1 Peptides to Human HLA B7 | | | |
|---|---|---|---|
| Rank | Start Position | Subsequence Residue Listing | Score (Estimate of Half Time of Disassociation of a Molecule Containing This Subsequence) |
| 9 | 208 | SCTGSQALL (SEQ ID NO:202) | 4.000 |
| 10 | 303 | VPGVAPTLV (SEQ ID NO:242) | 4.000 |
| 11 | 18 | LGGGGGCAL (SEQ ID NO: 134) | 4.000 |
| 12 | 218 | RTPYSSDNL (SEQ ID NO:194) | 4.000 |
| 13 | 207 | DSCTGSQAL (SEQ ID NO:61) | 4.000 |
| 14 | 209 | CTGSQALLL (SEQ ID NO:52) | 4.000 |
| 15 | 329 | GCNKRYFKL (SEQ ID NO:90) | 4.000 |
| 16 | 235 | CMTWNQMNL (SEQ ID NO: 49) | 4.000 |
| 17 | 441 | NMTKLQLAL (SEQ ID NO:149) | 4.000 |
| 18 | 126 | RMFPNAPYL (SEQ ID NO:185) | 4.000 |
| 19 | 225 | NLYQMTSQL (SEQ ID NO: 147) | 4.000 |
| 20 | 143 | AIRNQGYST (SEQ ID NO:33) | 3.000 |

Table XVII

| Results of BIMAS HLA Peptide Binding Prediction Analysis for Binding of Human WT1 Peptides to Human HLA B 8 | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO:90) | 16.000 |
| 2 | 4 | DVRDLNALL (SEQ ID NO:62) | 12.000 |
| 3 | 316 | ETSEKRPFM (SEQ ID NO:73) | 3.000 |
| 4 | 180 | DPMGQQGSL (SEQ ID NO:59) | 1.600 |
| 5 | 208 | SCTGSQALL (SEQ ID NO:202) | 0.800 |
| 6 | 130 | NAPYLPSCL (SEQ ID NO:144) | 0.800 |
| 7 | 244 | GATLKGVAA (SEQ ID NO:88) | 0.800 |
| 8 | 30 | GAAQWAPVL (SEQ ID NO:86) | 0.800 |
| 9 | 299 | DVRRVPGVA (SEQ ID NO:63) | 0.400 |
| 10 | 420 | SCQKKFARS (SEQ ID NO: 200) | 0.400 |
| 11 | 387 | TCQRKFSRS (SEQ ID NO: 219) | 0.400 |
| 12 | 225 | NLYQMTSQL (SEQ ID NO: 147) | 0.400 |
| 13 | 141 | QPAIRNQGY (SEQ ID NO:170) | 0.400 |
| 14 | 10 | ALLPAVPSL (SEQ ID NO:34) | 0.400 |

(continued)

| Results of BIMAS HLA Peptide Binding Prediction Analysis for Binding of Human WT1 Peptides to Human HLA B 8 | | | |
|---|---|---|---|
| Rank | Start Position | Subsequence Residue Listing | Score (Estimate of Half Time of Disassociation of a Molecule Containing This Subsequence) |
| 15 | 207 | DSCTGSQAL (SEQ ID NO:61) | 0.400 |
| 16 | 384 | QCKTCQRKF (SEQ ID NO: 163) | 0.400 |
| 17 | 136 | SCLESQPAI (SEQ ID NO:198) | 0.300 |
| 18 | 347 | HTGEKPYQC (SEQ ID NO: 112) | 0.300 |
| 19 | 401 | HTRTHTGKT (SEQ ID NO:114) | 0.200 |
| 20 | 332 | KRYFKLSHL (SEQ ID NO:127) | 0.200 |

Table XVIII

| Results of BIMAS HLA Peptide Binding Prediction Analysis for Binding of Human WT1 Peptides to Human HLA B 2702 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 900.000 |
| 2 | 362 | RRFSRSDQL (SEQ ID NO: 187) | 900.000 |
| 3 | 286 | YRIHTHGVF (SEQ ID NO: 252) | 200.000 |
| 4 | 125 | ARNIFPNAPY (SEQ ID NO: 38) | 200.000 |
| 5 | 375 | RRHTGVKPF (SEQ ID NO: 188) | 180.000 |
| 6 | 32 | AQWAPVLDF (SEQ ID NO: 37) | 100.000 |
| 7 | 301 | RRVPGVAPT (SEQ ID NO: 189) | 60.000 |
| 8 | 439 | QRNMTKLQL (SEQ ID NO: 173) | 60.000 |
| 9 | 126 | RMFPNAPYL (SEQ ID NO: 185) | 22.500 |
| 10 | 426 | ARSDELVRH (SEQ ID NO:39) | 20.000 |
| 11 | 146 | NQGYSTVTF (SEQ ID NO: 150) | 20.000 |
| 12 | 144 | IRNQGYSTV (SEQ ID NO: 117) | 20.000 |
| 13 | 389 | QRKFSRSDH (SEQ ID NO: 172) | 20.000 |

(continued)

| Results of BIMAS HLA Peptide Binding Prediction Analysis for Binding of Human WT1 Peptides to Human HLA B 2702 | | | |
|---|---|---|---|
| Rank | Start Position | Subsequence Residue Listing | Score (Estimate of Half Time of Disassociation of a Molecule Containing This Subsequence) |
| 14 | 263 | GQSNHSTGY (SEQ ID NO: 100) | 20.000 |
| 15 | 416 | CRWPSCQKK (SEQ ID NO: 50) | 20.000 |
| 16 | 191 | QQYSVPPPV (SEQ ID NO: 171) | 10.000 |
| 17 | 217 | LRTPYSSDN (SEQ ID NO: 140) | 10.000 |
| 18 | 107 | CRYGPFGPP (SEQ ID NO: 51) | 10.000 |
| 19 | 98 | GQFTGTAGA (SEQ ID NO: 99) | 10.000 |
| 20 | 239 | NQMNLGATL (SEQ ID NO: 151) | 6.000 |

Table XIX

| Results of BIMAS HLA Peptide Binding Prediction Analysis for Binding of Human WT1 Peptides to Human HLA B 2705 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 30000.000 |
| 2 | 362 | RRFSRSDQL (SEQ ID NO: 187) | 30000.000 |
| 3 | 416 | CRWPSCQKK (SEQ ID NO: 50) | 10000.000 |
| 4 | 439 | QRNMTKLQL (SEQ ID NO: 173) | 2000.000 |
| 5 | 286 | YRIHTHGVF (SEQ TD NO: 252) | 1000.000 |
| 6 | 125 | ARMFPNAPY (SEQ ID NO: 38) | 1000.000 |
| 7 | 294 | FRGIQDVRR (SEQ ID NO:81) | 1000.000 |
| 8 | 432 | VRHHNMHQR (SEQ ID NO: 243) | 1000.000 |
| 9 | 169 | AQFPNHSFK (SEQ ID NO:36) | 1000.000 |
| 10 | 375 | RRHTGVKPF (SEQ ID NO: 188) | 900.000 |
| 11 | 126 | RMFPNAPYL (SEQ ID NO: 185) | 750.000 |

(continued)

| Results of BIMAS HLA Peptide Binding Prediction Analysis for Binding of Human WT1 Peptides to Human HLA B 2705 | | | |
|---|---|---|---|
| Rank | Start Position | Subsequence Residue Listing | Score (Estimate of Half Time of Disassociation of a Molecule Containing This Subsequence) |
| 12 | 144 | IRNQGYSTV (SEQ ID NO: 117) | 600.000 |
| 13 | 301 | RRVPGVAPT (SEQ ID NO: 189) | 600.000 |
| 14 | 32 | AQWAPVLDF (SEQ ID NO: 37) | 500.000 |
| 15 | 191 | QQYSVPPPV (SEQ ID NO: 171) | 300.000 |
| 16 | 373 | HQRRHTGVK (SEQ ID NO: 109) | 200.000 |
| 17 | 426 | ARSDELVRH (SEQ ID NO:39) | 200.000 |
| 18 | 383 | FQCKTCQRK (SEQ ID NO: 80) | 200.000 |
| 19 | 239 | NQMNLGATL (SEQ ID NO: 151) | 200.000 |
| 20 | 389 | QRKFSRSDH (SEQ ID NO: 172) | 200.000 |

Table XX

| Results of BIMAS HLA Peptide Binding Prediction Analysis for Binding of Human WT1 Peptides to Human HLA B 3501 | | | |
|---|---|---|---|
| Rank | Start Position | Subsequence Residue Listing | Score (Estimate of Half Time of Disassociation of a Molecule Containing This Subsequence) |
| 1 | 278 | TPILCGAQY (SEQ ID NO: 227) | 40.000 |
| 2 | 141 | QPAIRNQGY (SEQ ID NO: 170) | 40.000 |
| 3 | 219 | TPYSSDNLY (SEQ ID NO: 231) | 40.000 |
| 4 | 327 | YPGCNKRYF (SEQ ID NO: 250) | 20.000 |
| 5 | 163 | TPSHHAAQF (SEQ ID NO: 228) | 20.000 |
| 6 | 180 | DPMGQQGSL (SEQ ID NO: 59) | 20.000 |
| 7 | 221 | YSSDNLYQM (SEQ ID NO: 253) | 20.000 |
| 8 | 26 | LPVSGAAQW (SEQ ID NO: 138) | 10.000 |
| 9 | 174 | HSFKHEDPM (SEQ ID NO: 110) | 10.000 |

(continued)

| Results of BIMAS HLA Peptide Binding Prediction Analysis for Binding of Human WT1 Peptides to Human HLA B 3501 | | | |
|---|---|---|---|
| Rank | Start Position | Subsequence Residue Listing | Score (Estimate of Half Time of Disassociation of a Molecule Containing This Subsequence) |
| 10 | 82 | EPHEEQCLS (SEQ ID NO:68) | 6.000 |
| 11 | 213 | QALLLRTPY (SEQ ID NO: 160) | 6.000 |
| 12 | 119 | QASSGQARM (SEQ ID NO: 161) | 6.000 |
| 13 | 4 | DVRDLNALL (SEQ ID NO:62) | 6.000 |
| 14 | 40 | FAPPGASAY (SEQ ID NO:74) | 6.000 |
| 15 | 120 | ASSGQARMF (SEQ ID NO: 40) | 5.000 |
| 16 | 207 | DSCTGSQAL (SEQ ID NO: 61) | 5.000 |
| 17 | 303 | VPGVAPTLV (SEQ ID NO: 242) | 4.000 |
| 18 | 316 | ETSEKRPFM (SEQ ID NO:73) | 4.000 |
| 19 | 152 | VTFDGTPSY (SEQ ID NO: 244) | 4.000 |
| 20 | 412 | KPFSCRWPS (SEQ ID NO: 123) | 4.000 |

Table XXI

| Results of BIMAS HLA Peptide Binding Prediction Analysis for Binding of Human WT1 Peptides to Human HLA B 3701 | | | |
|---|---|---|---|
| Rank | Start Position | Subsequence Residue Listing | Score (Estimate of Half Time of Disassociation of a Molecule Containing This Subsequence) |
| 1 | 3 | SDVRDLNAL (SEQ ID NO: 206) | 40.000 |
| 2 | 273 | SDNHTTPIL (SEQ ID NO:204) | 40.000 |
| 3 | 81 | AEPHEEQCL (SEQ ID NO:30) | 10.000 |
| 4 | 298 | QDVRRVPGV (SEQ ID NO: 164) | 8.000 |
| 5 | 428 | SDELVRHHN (SEQ ID NO: 203) | 6.000 |
| 6 | 85 | EEQCLSAFT (SEQ ID NO:65) | 5.000 |
| 7 | 208 | SCTGSQALL (SEQ ID NO: 202) | 5.000 |
| 8 | 4 | DVRDLNALL (SEQ ID NO:62) | 5.000 |
| 9 | 209 | CTGSQALLL (SEQ ID NO:52) | 5.000 |
| 10 | 38 | LDFAPPGAS (SEQ ID NO: 130) | 4.000 |

(continued)

| Results of BIMAS HLA Peptide Binding Prediction Analysis for Binding of Human WT1 Peptides to Human HLA B 3701 | | | |
|---|---|---|---|
| Rank | Start Position | Subsequence Residue Listing | Score (Estimate of Half Time of Disassociation of a Molecule Containing This Subsequence) |
| 11 | 223 | SDNLYQMTS (SEQ ID NO: 205) | 4.000 |
| 12 | 179 | EDPMGQQGS (SEQ ID NO: 64) | 4.000 |
| 13 | 206 | TDSCTGSQA (SEQ ID NO: 220) | 4.000 |
| 14 | 6 | RDLNALLPA (SEQ ID NO:177) | 4.000 |
| 15 | 84 | HEEQCLSAF (SEQ ID NO: 107) | 2.000 |
| 16 | 233 | LECMTWNQM (SEQ ID NO: 131) | 2.000 |
| 17 | 429 | DELVRHHNM (SEQ ID NO:53) | 2.000 |
| 18 | 315 | SETSEKRPF (SEQ ID NO: 209) | 2.000 |
| 19 | 349 | GEKPYQCDF (SEQ ID NO:91) | 2.000 |
| 20 | 302 | RVPGVAPTL (SEQ ID NO: 195) | 1.500 |

Table XXII

| Results of BIMAS HLA Peptide Binding Prediction Analysis for Binding of Human WT1 Peptides to Human HLA B 3801 | | | |
|---|---|---|---|
| Rank | Start Position | Subsequence Residue Listing | Score (Estimate of Half Time of Disassociation of a Molecule Containing This Subsequence) |
| 1 | 437 | MHQRNMTKL (SEQ ID NO: 143) | 36.000 |
| 2 | 434 | HHNMHQRNM (SEQ ID NO: 108) | 6.000 |
| 3 | 372 | RHQRRHTGV (SEQ ID NO: 181) | 6.000 |
| 4 | 180 | DPMGQQGSL (SEQ ID NO:59) | 4.000 |
| 5 | 433 | RHHNMHQRN (SEQ ID NO: 180) | 3.900 |
| 6 | 165 | SHHAAQFPN (SEQ ID NO: 213) | 3.900 |
| 7 | 202 | CHTPTDSCT (SEQ ID NO:45) | 3.000 |
| 8 | 396 | DHLKTHTRT (SEQ ID NO:57) | 3.000 |
| 9 | 161 | GHTPSHHAA (SEQ ID NO:94) | 3.000 |
| 10 | 302 | RVPGVAPTL (SEQ ID NO:195) | 2.600 |

(continued)

| Results of BIMAS HLA Peptide Binding Prediction Analysis for Binding of Human WT1 Peptides to Human HLA B 3801 | | | |
|---|---|---|---|
| Rank | Start Position | Subsequence Residue Listing | Score (Estimate of Half Time of Disassociation of a Molecule Containing This Subsequence) |
| 11 | 417 | RWPSCQKKF (SEQ ID NO: 196) | 2.400 |
| 12 | 327 | YPGCNKRYF (SEQ ID NO: 250) | 2.400 |
| 13 | 208 | SCTGSQALL (SEQ ID NO:202) | 2.000 |
| 14 | 163 | TPSHHAAQF (SEQ ID NO: 228) | 2.000 |
| 15 | 120 | ASSGQARMF (SEQ ID NO:40) | 2.000 |
| 16 | 18 | LGGGGGCAL (SEQ ID NO: 134) | 2.000 |
| 17 | 177 | KHEDPMGQQ (SEQ ID NO: 121) | 1.800 |
| 18 | 83 | PHEEQCLSA (SEQ ID NO: 154) | 1.800 |
| 19 | 10 | ALLPAVPSL (SEQ ID NO:34) | 1.300 |
| 20 | 225 | NLYQMTSQL (SEQ ID NO: 147) | 1.300 |

Table XXIII

| Results of BIMAS HLA Peptide Binding Prediction Analysis for Binding of Human WT1 Peptides to Human HLA B 3901 | | | |
|---|---|---|---|
| Rank | Start Position | Subsequence Residue Listing | Score (Estimate of Half Time of Disassociation of a Molecule Containing This Subsequence) |
| 1 | 437 | MHQRNMTKL (SEQ ID NO: 143) | 135.000 |
| 2 | 332 | KRYFKLSHL (SEQ ID NO:127) | 45.000 |
| 3 | 434 | HHNMHQRNM (SEQ ID NO: 108) | 30.000 |
| 4 | 362 | RRFSRSDQL (SEQ ID NO: 187) | 30.000 |
| 5 | 372 | RHQRRHTGV (SEQ ID NO: 181) | 30.000 |
| 6 | 10 | ALLPAVPSL (SEQ ID NO:34) | 9.000 |
| 7 | 439 | QRNMTKLQL (SEQ ID NO: 173) | 7.500 |
| 8 | 390 | RKFSRSDHL (SEQ ID NO: 183) | 6.000 |
| 9 | 396 | DHLKTHTRT (SEQ ID NO:57) | 6.000 |

(continued)

| Results of BIMAS HLA Peptide Binding Prediction Analysis for Binding of Human WT1 Peptides to Human HLA B 3901 | | | |
|---|---|---|---|
| Rank | Start Position | Subsequence Residue Listing | Score (Estimate of Half Time of Disassociation of a Molecule Containing This Subsequence) |
| 10 | 239 | NQMNLGATL (SEQ ID NO: 151) | 6.000 |
| 11 | 423 | KKFARSDEL (SEQ ID NO:122) | 6.000 |
| 12 | 126 | RMFPNAPYL (SEQ ID NO: 185) | 6.000 |
| 13 | 225 | NLYQMTSQL (SEQ ID NO: 147) | 6.000 |
| 14 | 180 | DPMGQQGSL (SEQ ID NO:59) | 6.000 |
| 15 | 144 | IRNQGYSTV (SEQ ID NO:117) | 5.000 |
| 16 | 136 | SCLESQPAI (SEQ ID NO:198) | 4.000 |
| 17 | 292 | GVFRGIQDV (SEQ ID NO:103) | 3.000 |
| 18 | 302 | RVPGVAPTL (SEQ ID NO:195) | 3.000 |
| 19 | 208 | SCTGSQALL (SEQ ID NO:202) | 3.000 |
| 20 | 207 | DSCTGSQAL (SEQ ID NO:61) | 3.000 |

Table XXIV

| Results of BIMAS HLA Peptide Binding Prediction Analysis for Binding of Human WT1 Peptides to Human HLA B 3902 | | | |
|---|---|---|---|
| Rank | Start Position | Subsequence Residue Listing | Score (Estimate of Half Time of Disassociation of a Molecule Containing This Subsequence) |
| 1 | 239 | NQMNLGATL (SEQ ID NO: 151) | 24.000 |
| 2 | 390 | RKFSRSDHL (SEQ ID NO: 183) | 20.000 |
| 3 | 423 | KKFARSDEL (SEQ ID NO: 122) | 20.000 |
| 4 | 32 | AQWAPVLDF (SEQ ID NO: 37) | 5.000 |
| 5 | 146 | NQGYSTVTF (SEQ ID NO: 150) | 5.000 |
| 6 | 130 | NAPYLPSCL (SEQ ID NO: 144) | 2.400 |
| 7 | 225 | NLYQMTSQL (SEQ ID NO: 147) | 2.400 |
| 8 | 30 | GAAQWAPVL (SEQ ID NO: 86) | 2.400 |
| 9 | 441 | NMTKLQLAL (SEQ ID NO: 149) | 2.400 |

(continued)

| Results of BIMAS HLA Peptide Binding Prediction Analysis for Binding of Human WT1 Peptides to Human HLA B 3902 | | | |
|---|---|---|---|
| Rank | Start Position | Subsequence Residue Listing | Score (Estimate of Half Time of Disassociation of a Molecule Containing This Subsequence) |
| 10 | 302 | RVPGVAPTL (SEQ ID NO: 195) | 2.400 |
| 11 | 126 | RMFPNAPYL (SEQ ID NO: 185) | 2.000 |
| 12 | 218 | RTPYSSDNL (SEQ ID NO: 194) | 2.000 |
| 13 | 209 | CTGSQALLL (SEQ ID NO:52) | 2.000 |
| 14 | 332 | KRYFKLSHL (SEQ ID NO: 127) | 2.000 |
| 15 | 180 | DPMGQQGSL (SEQ ID NO: 59) | 2.000 |
| 16 | 437 | MHQRNMTKL (SEQ ID NO: 143) | 2.000 |
| 17 | 207 | DSCTGSQAL (SEQ ID NO: 61) | 2.000 |
| 18 | 208 | SCTGSQALL (SEQ ID NO: 202) | 2.000 |
| 19 | 329 | GCNKRYFKL (SEQ ID NO:90) | 2.000 |
| 20 | 10 | ALLPAVPSL (SEQ ID NO:34) | 2.000 |

Table XXV

| Results of BIMAS HLA Peptide Binding Prediction Analysis for Binding of Human WT1 Peptides to Human HLA B 4403 | | | |
|---|---|---|---|
| Rank | Start Position | Subsequence Residue Listing | Score (Estimate of Half Time of Disassociation of a Molecule Containing This Subsequence) |
| 1 | 315 | SETSEKRPF (SEQ ID NO: 209) | 80.000 |
| 2 | 349 | GEKPYQCDF (SEQ ID NO:91) | 80.000 |
| 3 | 84 | HEEQCLSAF (SEQ ID NO: 107) | 60.000 |
| 4 | 410 | SEKPFSCRW (SEQ ID NO: 207) | 48.000 |
| 5 | 429 | DELVRHHNM (SEQ ID NO:53) | 24.000 |
| 6 | 278 | TPILCGAQY (SEQ ID NO:227) | 15.000 |
| 7 | 141 | QPAIRNQGY (SEQ ID NO: 170) | 9.000 |
| 8 | 40 | FAPPGASAY (SEQ ID NO:74) | 9.000 |
| 9 | 213 | QALLLRTPY (SEQ ID NO:160) | 9.000 |
| 10 | 318 | SEKRPFMCA (SEQ ID NO: 208) | 8.000 |

(continued)

| Results of BIMAS HLA Peptide Binding Prediction Analysis for Binding of Human WT1 Peptides to Human HLA B 4403 | | | |
|---|---|---|---|
| Rank | Start Position | Subsequence Residue Listing | Score (Estimate of Half Time of Disassociation of a Molecule Containing This Subsequence) |
| 11 | 81 | AEPHEEQCL (SEQ ID NO:30) | 8.000 |
| 12 | 152 | VTFDGTPSY (SEQ ID NO: 244) | 4.500 |
| 13 | 101 | TGTAGACRY (SEQ ID NO: 224) | 4.500 |
| 14 | 120 | ASSGQARMF (SEQ ID NO:40) | 4.500 |
| 15 | 261 | TEGQSNHST (SEQ ID NO: 221) | 4.000 |
| 16 | 85 | EEQCLSAFT (SEQ ID NO:65) | 4.000 |
| 17 | 233 | LECMTWNQM (SEQ ID NO: 131) | 4.000 |
| 18 | 104 | AGACRYGPF (SEQ ID NO:31) | 4.000 |
| 19 | 3 | SDVRDLNAL (SEQ ID NO: 206) | 3.000 |
| 20 | 185 | QGSLGEQQY (SEQ ID NO: 166) | 3.000 |

Table XXVI

| Results of BIMAS HLA Peptide Binding Prediction Analysis for Binding of Human WT1 Peptides to Human HLA B 5101 | | | |
|---|---|---|---|
| Rank | Start Position | Subsequence Residue Listing | Score (Estimate of Half Time of Disassociation of a Molecule Containing This Subsequence) |
| 1 | 303 | VPGVAPTLV (SEQ ID NO: 242) | 314.600 |
| 2 | 180 | DPMGQQGSL (SEQ ID NO: 59) | 242.000 |
| 3 | 250 | VAAGSSSSV (SEQ ID NO: 236) | 157.300 |
| 4 | 130 | NAPYLPSCL (SEQ ID NO: 144) | 50.000 |
| 5 | 30 | GAAQWAPVL (SEQ ID NO: 86) | 50.000 |
| 6 | 20 | GGGGCALPV (SEQ ID NO: 92) | 44.000 |
| 7 | 64 | PPPPPHSFI (SEQ ID NO: 157) | 40.000 |
| 8 | 29 | SGAAQWAPV (SEQ ID NO: 211) | 40.000 |
| 9 | 18 | LGGGGGCAL (SEQ ID NO: 134) | 31.460 |

(continued)

| Results of BIMAS HLA Peptide Binding Prediction Analysis for Binding of Human WT1 Peptides to Human HLA B 5101 | | | |
|---|---|---|---|
| Rank | Start Position | Subsequence Residue Listing | Score (Estimate of Half Time of Disassociation of a Molecule Containing This Subsequence) |
| 10 | 295 | RGIQDVRRV (SEQ ID NO: 179) | 22.000 |
| 11 | 119 | QASSGQARM (SEQ ID NO: 161) | 18.150 |
| 12 | 418 | WPSCQKKFA (SEQ ID NO: 246) | 12.100 |
| 13 | 82 | EPHEEQCLS (SEQ ID NO:68) | 12.100 |
| 14 | 110 | GPFGPPPPS (SEQ ID NO: 96) | 11.000 |
| 15 | 272 | ESDNHTTPI (SEQ ID NO:71) | 8.000 |
| 16 | 306 | VAPTLVRSA (SEQ ID NO: 237) | 7.150 |
| 17 | 280 | ILCGAQYRI (SEQ ID NO:116) | 6.921 |
| 18 | 219 | TPYSSDNLY (SEQ ID NO: 231) | 6.600 |
| 19 | 128 | FPNAPYLPS (SEQ ID NO:79) | 6.500 |
| 20 | 204 | TPTDSCTGS (SEQ ID NO: 230) | 6.050 |

Table XXVII

| Results of BIMAS HLA Peptide Binding Prediction Analysis for Binding of Human WT1 Peptides to Human HLA B 5102 | | | |
|---|---|---|---|
| Rank | Start Position | Subsequence Residue Listing | Score (Estimate of Half Time of Disassociation of a Molecule Containing This Subsequence) |
| 1 | 295 | RGIQDVRRV (SEQ ID NO: 179) | 290.400 |
| 2 | 303 | VPGVAPTLV (SEQ ID NO: 242) | 200.000 |
| 3 | 180 | DPMGQQGSL (SEQ ID NO: 59) | 133.100 |
| 4 | 250 | VAAGSSSSV (SEQ ID NO: 236) | 110.000 |
| 5 | 30 | GAAQWAPVL (SEQ ID NO: 86) | 55.000 |
| 6 | 130 | NAPYLPSCL (SEQ ID NO: 144) | 50.000 |
| 7 | 20 | GGGGCALPV (SEQ ID NO: 92) | 44.000 |
| 8 | 29 | SGAAQWAPV (SEQ ID NO: 211) | 44.000 |

(continued)

| Results of BIMAS HLA Peptide Binding Prediction Analysis for Binding of Human WT1 Peptides to Human HLA B 5102 | | | |
|---|---|---|---|
| Rank | Start Position | Subsequence Residue Listing | Score (Estimate of Half Time of Disassociation of a Molecule Containing This Subsequence) |
| 9 | 64 | PPPPPHSFI (SEQ ID NO: 157) | 40.000 |
| 10 | 119 | QASSGQARM (SEQ ID NO: 161) | 36.300 |
| 11 | 110 | GPFGPPPPS (SEQ ID NO: 96) | 27.500 |
| 12 | 412 | KPFSCRWPS (SEQ ID NO: 123) | 25.000 |
| 13 | 18 | LGGGGGCAL (SEQ ID NO: 134) | 24.200 |
| 14 | 24 | CALPVSGAA (SEQ ID NO:43) | 16.500 |
| 15 | 219 | TPYSSDNLY (SEQ ID NO: 231) | 15.000 |
| 16 | 292 | GVFRGIQDV (SEQ ID NO: 103) | 14.641 |
| 17 | 136 | SCLESQPAI (SEQ ID NO: 198) | 14.520 |
| 18 | 418 | WPSCQKKFA (SEQ ID NO: 246) | 12.100 |
| 19 | 269 | TGYESDNHT (SEQ ID NO: 225) | 11.000 |
| 20 | 351 | KPYQCDFKD (SEQ ID NO: 124) | 11.000 |

Table XXVIII

| Results of BIMAS HLA Peptide Binding Prediction Analysis for Binding of Human WT1 Peptides to Human HLA B 5201 | | | |
|---|---|---|---|
| Rank | Start Position | Subsequence Residue Listing | Score (Estimate of Half Time of Disassociation of a Molecule Containing This Subsequence) |
| 1 | 191 | QQYSVPPPV (SEQ ID NO: 171) | 100.000 |
| 2 | 32 | AQWAPVLDF (SEQ ID NO: 37) | 30.000 |
| 3 | 243 | LGATLKGVA (SEQ ID NO: 133) | 16.500 |
| 4 | 303 | VPGVAPTLV (SEQ ID NO: 242) | 13.500 |
| 5 | 86 | EQCLSAFTV (SEQ ID NO:69) | 12.000 |
| 6 | 295 | RGIQDVRRV (SEQ ID NO: 179) | 10.000 |

(continued)

| Results of BIMAS HLA Peptide Binding Prediction Analysis for Binding of Human WT1 Peptides to Human HLA B 5201 | | | |
|---|---|---|---|
| Rank | Start Position | Subsequence Residue Listing | Score (Estimate of Half Time of Disassociation of a Molecule Containing This Subsequence) |
| 7 | 98 | GQFTGTAGA (SEQ ID NO: 99) | 8.250 |
| 8 | 292 | GVFRGIQDV (SEQ ID NO: 103) | 8.250 |
| 9 | 29 | SGAAQWAPV (SEQ ID NO: 211) | 6.000 |
| 10 | 146 | NQGYSTVTF (SEQ ID NO: 150) | 5.500 |
| 11 | 20 | GGGGCALPV (SEQ ID NO: 92) | 5.000 |
| 12 | 239 | NQMNLGATL (SEQ ID NO: 151) | 4.000 |
| 13 | 64 | PPPPPHSFI (SEQ ID NO: 157) | 3.600 |
| 14 | 273 | SDNHTTPIL (SEQ ID NO:204) | 3.300 |
| 15 | 286 | YRIHTHGVF (SEQ ID NO: 252) | 3.000 |
| 16 | 269 | TGYESDNHT (SEQ ID NO: 225) | 3.000 |
| 17 | 406 | TGKTSEKPF (SEQ ID NO: 222) | 2.750 |
| 18 | 327 | YPGCNKRYF (SEQ ID NO: 250) | 2.750 |
| 19 | 7 | DLNALLPAV (SEQ ID NO:58) | 2.640 |
| 20 | 104 | AGACRYGPF (SEQ ID NO: 31) | 2.500 |

Table XXIX

| Results of BIMAS HLA Peptide Binding Prediction Analysis for Binding of Human WT1 Peptides to Human HLA B 5801 | | | |
|---|---|---|---|
| Rank | Start Position | Subsequence Residue Listing | Score (Estimate of Half Time of Disassociation of a Molecule Containing This Subsequence) |
| 1 | 230 | TSQLECMTW (SEQ ID NO: 234) | 96.800 |
| 2 | 92 | FTVHFSGQF (SEQ ID NO:85) | 60.000 |
| 3 | 120 | ASSGQARMF (SEQ ID NO: 40) | 40.000 |
| 4 | 168 | AAQFPNHSF (SEQ ID NO:29) | 20.000 |
| 5 | 408 | KTSEKPFSC (SEQ ID NO: 129) | 12.000 |

(continued)

| Results of BIMAS HLA Peptide Binding Prediction Analysis for Binding of Human WT1 Peptides to Human HLA B 5801 | | | |
|---|---|---|---|
| Rank | Start Position | Subsequence Residue Listing | Score (Estimate of Half Time of Disassociation of a Molecule Containing This Subsequence) |
| 6 | 394 | RSDHLKTHT (SEQ ID NO: 192) | 9.900 |
| 7 | 276 | HTTPILCGA (SEQ ID NO:115) | 7.200 |
| 8 | 218 | RTPYSSDNL (SEQ ID NO: 194) | 6.600 |
| 9 | 152 | VTFDGTPSY (SEQ ID NO: 244) | 6.000 |
| 10 | 40 | FAPPGASAY (SEQ ID NO:74) | 6.000 |
| 11 | 213 | QALLLRTPY (SEQ ID NO:160) | 4.500 |
| 12 | 347 | HTGEKPYQC (SEQ ID NO: 112) | 4.400 |
| 13 | 252 | AGSSSSVKW (SEQ ID NO: 32) | 4.400 |
| 14 | 211 | GSQALLLRT (SEQ ID NO: 102) | 4.356 |
| 15 | 174 | HSFKHEDPM (SEQ ID NO: 110) | 4.000 |
| 16 | 317 | TSEKRPFMC (SEQ ID NO: 233) | 4.000 |
| 17 | 26 | LPVSGAAQW (SEQ ID NO: 138) | 4.000 |
| 18 | 289 | HTHGVFRGI (SEQ ID NO: 113) | 3.600 |
| 19 | 222 | SSDNLYQMT (SEQ ID NO: 217) | 3.300 |
| 20 | 96 | FSGQFTGTA (SEQ ID NO:82) | 3.300 |

Table XXX

| Results of BIMAS HLA Peptide Binding Prediction Analysis for Binding of Human WT1 Peptides to Human HLA CW0301 | | | |
|---|---|---|---|
| Rank | Start Position | Subsequence Residue Listing | Score (Estimate of Half Time of Disassociation of a Molecule Containing This Subsequence) |
| 1 | 10 | ALLPAVPSL (SEQ ID NO:34) | 100.000 |
| 2 | 332 | KRYFKLSHL (SEQ ID NO: 127) | 48.000 |
| 3 | 126 | RMFPNAPYL (SEQ ID NO: 185) | 36.000 |

(continued)

| Results of BIMAS HLA Peptide Binding Prediction Analysis for Binding of Human WT1 Peptides to Human HLA CW0301 | | | |
|---|---|---|---|
| Rank | Start Position | Subsequence Residue Listing | Score (Estimate of Half Time of Disassociation of a Molecule Containing This Subsequence) |
| 4 | 3 | SDVRDLNAL (SEQ ID NO: 206) | 30.000 |
| 5 | 239 | NQMNLGATL (SEQ ID NO: 151) | 24.000 |
| 6 | 225 | NLYQMTSQL (SEQ ID NO: 147) | 24.000 |
| 7 | 180 | DPMGQQGSL (SEQ ID NO: 59) | 20.000 |
| 8 | 362 | RRFSRSDQL (SEQ ID NO: 187) | 12.000 |
| 9 | 329 | GCNKRYFKL (SEQ ID NO: 90) | 10.000 |
| 10 | 286 | YRIHTHGVF (SEQ ID NO: 252) | 10.000 |
| 11 | 301 | RRVPGVAPT (SEQ ID NO: 189) | 10.000 |
| 12 | 24 | CALPVSGAA (SEQ ID NO:43) | 10.000 |
| 13 | 136 | SCLESQPAI (SEQ ID NO: 198) | 7.500 |
| 14 | 437 | MHQRNMTKL (SEQ ID NO: 143) | 7.200 |
| 15 | 390 | RKFSRSDHL (SEQ ID NO: 183) | 6.000 |
| 16 | 423 | KKFARSDEL (SEQ ID NO: 122) | 6.000 |
| 17 | 92 | FTVHFSGQF (SEQ ID NO:85) | 5.000 |
| 18 | 429 | DELVRHHNM (SEQ ID NO: 53) | 5.000 |
| 19 | 130 | NAPYLPSCL (SEQ ID NO: 144) | 4.800 |
| 20 | 30 | GAAQWAPVL (SEQ ID NO: 86) | 4.000 |

Table XXXI

| Results of BIMAS HLA Peptide Binding Prediction Analysis for Binding of Human WT1 Peptides to Human HLA CW0401 | | | |
|---|---|---|---|
| Rank | Start Position | Subsequence Residue Listing | Score (Estimate of Half Time of Disassociation of a Molecule Containing This Subsequence) |
| 1 | 356 | DFKDCERRF (SEQ ID NO: 55) | 120.000 |
| 2 | 334 | YFKLSHLQM (SEQ ID NO: 248) | 100.000 |
| 3 | 180 | DPMGQQGSL (SEQ ID NO: 59) | 88.000 |
| 4 | 163 | TPSHHAAQF (SEQ ID NO: 228) | 52.800 |
| 5 | 327 | YPGCNKRYF (SEQ ID NO: 250) | 40.000 |
| 6 | 285 | QYRIHTHGV (SEQ ID NO: 175) | 27.500 |
| 7 | 424 | KFARSDELV (SEQ ID NO: 119) | 25.000 |
| 8 | 326 | AYPGCNKRY (SEQ ID NO: 42) | 25.000 |
| 9 | 192 | QYSVPPPVY (SEQ ID NO: 176) | 25.000 |
| 10 | 417 | RWPSCQKKF (SEQ ID NO: 196) | 22.000 |
| 11 | 278 | TPILCGAQY (SEQ ID NO: 227) | 12.000 |
| 12 | 10 | ALLPAVPSL (SEQ ID NO:34) | 11.616 |
| 13 | 141 | QPAIRNQGY (SEQ ID NO: 170) | 11.000 |
| 14 | 303 | VPGVAPTLV (SEQ ID NO: 242) | 11.000 |
| 15 | 219 | TPYSSDNLY (SEQ ID NO: 231) | 10.000 |
| 16 | 39 | DFAPPGASA (SEQ ID NO: 54) | 7.920 |
| 17 | 99 | QFTGTAGAC (SEQ ID NO: 165) | 6.000 |
| 18 | 4 | DVRDLNALL (SEQ ID NO:62) | 5.760 |
| 19 | 70 | SFIKQEPSW (SEQ ID NO: 210) | 5.500 |
| 20 | 63 | PPPPPPHSF (SEQ ID NO: 158) | 5.280 |

Table XXXII

| Results of BIMAS HLA Peptide Binding Prediction Analysis for Binding of Human WT1 Peptides to Human HLA CW0602 | | | |
|---|---|---|---|
| Rank | Start Position | Subsequence Residue Listing | Score (Estimate of Half Time of Disassociation of a Molecule Containing This Subsequence) |
| 1 | 332 | KRYFKLSHL (SEQ ID NO: 127) | 9.680 |
| 2 | 239 | NQMNLGATL (SEQ ID NO: 151) | 6.600 |
| 3 | 130 | NAPYLPSCL (SEQ ID NO: 144) | 6.600 |
| 4 | 7 | DLNALLPAV (SEQ ID NO:58) | 6.000 |
| 5 | 441 | NMTKLQLAL (SEQ ID NO: 149 | 6.000 |
| 6 | 225 | NLYQMTSQL (SEQ ID NO: 147) | 6.000 |
| 7 | 4 | DVRDLNALL (SEQ ID NO:62) | 6.000 |
| 8 | 3 | SDVRDLNAL (SEQ ID NO: 206) | 4.400 |
| 9 | 10 | ALLPAVPSL (SEQ ID NO:34) | 4.000 |
| 10 | 213 | QALLLRTPY (SEQ ID NO: 160) | 3.300 |
| 11 | 319 | EKRPFMCAY (SEQ ID NO: 67) | 3.000 |
| 12 | 30 | GAAQWAPVL (SEQ ID NO: 86) | 2.200 |
| 13 | 242 | NLGATLKGV (SEQ ID NO: 146) | 2.200 |
| 14 | 292 | GVFRGIQDV (SEQ ID NO: 103) | 2.200 |
| 15 | 207 | DSCTGSQAL (SEQ ID NO: 61) | 2.200 |
| 16 | 362 | RRFSRSDQL (SEQ ID NO: 187) | 2.200 |
| 17 | 439 | QRNMTKLQL (SEQ ID NO: 173) | 2.200 |
| 18 | 295 | RGIQDVRRV (SEQ ID NO: 179) | 2.200 |
| 19 | 423 | KKFARSDEL (SEQ ID NO: 122) | 2.200 |
| 20 | 180 | DPMGQQGSL (SEQ ID NO: 59) | 2.200 |

Table XXXIII

| Results of BIMAS HLA Peptide Binding Prediction Analysis for Binding of Human WT1 Peptides to Human HLA CW0702 | | | |
|---|---|---|---|
| Rank | Start Position | Subsequence Residue Listing | Score (Estimate of Half Time of Disassociation of a Molecule Containing This Subsequence) |
| 1 | 319 | EKRPFMCAY (SEQ ID NO:67) | 26.880 |
| 2 | 326 | AYPGCNKRY (SEQ ID NO:42) | 24.000 |
| 3 | 40 | FAPPGASAY (SEQ ID NO:74) | 14.784 |
| 4 | 192 | QYSVPPPVY (SEQ ID NO: 176) | 12.000 |
| 5 | 278 | TPILCGAQY (SEQ ID NO:227) | 12.000 |
| 6 | 219 | TPYSSDNLY(SEQ ID NO: 231) | 12.000 |
| 7 | 213 | QALLLRTPY (SEQ ID NO: 160) | 8.800 |
| 8 | 125 | ARMFPNAPY (SEQ ID NO:38) | 8.000 |
| 9 | 327 | YPGCNKRYF(SEQ ID NO: 250) | 6.600 |
| 10 | 152 | VTFDGTPSY (SEQ ID NO: 244) | 5.600 |
| 11 | 141 | QPAIRNQGY (SEQ ID NO: 170) | 4.800 |
| 12 | 345 | RKHTGEKPY (SEQ ID NO: 184) | 4.000 |
| 13 | 185 | QGSLGEQQY(SEQ ID NO: 166) | 4.000 |
| 14 | 101 | TGTAGACRY (SEQ ID NO: 224) | 4.000 |
| 15 | 375 | RRHTGVKPF (SEQ ID NO: 188) | 4.000 |
| 16 | 263 | GQSNHSTGY (SEQ ID NO: 100) | 4.000 |
| 17 | 163 | TPSHHAAQF (SEQ ID NO: 228) | 3.000 |
| 18 | 33 | QWAPVLDFA (SEQ ID NO: 174) | 2.688 |
| 19 | 130 | NAPYLPSCL (SEQ ID NO: 144) | 2.640 |
| 20 | 84 | HEEQCLSAF (SEQ ID NO: 107) | 2.400 |

Table XXXIV

| Results of BIMAS HLA Peptide Binding Prediction Analysis for Binding of Human WT1 Peptides to Mouse MHC Class I Db | | | |
|---|---|---|---|
| Rank | Start Position | Subsequence Residue Listing | Score (Estimate of Half Time of Disassociation of a Molecule Containing This Subsequence) |
| 1 | 235 | CMTWNQMNL (SEQ ID NO: 49) | 5255.712 |
| 2 | 126 | RMFPNAPYL (SEQ ID NO: 185) | 1990.800 |
| 3 | 221 | YSSDNLYQM (SEQ ID NO: 253) | 930.000 |
| 4 | 228 | QMTSQLECM (SEQ ID NO: 169) | 33.701 |
| 5 | 239 | NQMNLGATL (SEQ ID NO: 151) | 21.470 |
| 6 | 441 | NMTKLQLAL (SEQ ID NO: 149) | 19.908 |
| 7 | 437 | MHQRNMTKL (SEQ ID NO: 143) | 19.837 |
| 8 | 136 | SCLESQPAI (SEQ ID NO: 198) | 11.177 |
| 9 | 174 | HSFKHEDPM (SEQ ID NO: 110) | 10.800 |
| 10 | 302 | RVPGVAPTL (SEQ ID NO: 195) | 10.088 |
| 11 | 130 | NAPYLPSCL (SEQ ID NO: 144) | 8.400 |
| 12 | 10 | ALLPAVPSL (SEQ ID NO:34) | 5.988 |
| 13 | 208 | SCTGSQALL (SEQ ID NO: 202) | 4.435 |
| 14 | 209 | CTGSQALLL (SEQ ID NO: 52) | 3.548 |
| 15 | 238 | WNQMNLGAT (SEQ ID NO: 245) | 3.300 |
| 16 | 218 | RTPYSSDNL (SEQ ID NO: 194) | 3.185 |
| 17 | 24 | CALPVSGAA (SEQ ID NO: 43) | 2.851 |
| 18 | 18 | LGGGGGCAL (SEQ ID NO: 134) | 2.177 |
| 19 | 142 | PAIRNQGYS (SEQ ID NO: 152) | 2.160 |
| 20 | 30 | GAAQWAPVL (SEQ ID NO: 86) | 1.680 |

Table XXXV

| Results of BIMAS HLA Peptide Binding Prediction Analysis for Binding of Human WT1 Peptides to Mouse MHC Class I Dd | | | |
|---|---|---|---|
| Rank | Start Position | Subsequence Residue Listing | Score (Estimate of Half Time of Disassociation of a Molecule Containing This Subsequence) |
| 1 | 112 | FGPPPPSQA (SEQ ID NO: 76) | 48.000 |
| 2 | 122 | SGQARMFPN (SEQ ID NO: 212) | 36.000 |
| 3 | 104 | AGACRYGPF (SEQ ID NO: 31) | 30.000 |
| 4 | 218 | RTPYSSDNL (SEQ ID NO: 194) | 28.800 |
| 5 | 130 | NAPYLPSCL (SEQ ID NO: 144) | 20.000 |
| 6 | 302 | RVPGVAPTL (SEQ ID NO: 195) | 20.000 |
| 7 | 18 | LGGGGGCAL (SEQ ID NO: 134) | 20.000 |
| 8 | 81 | AEPHEEQCL (SEQ ID NO: 30) | 10.000 |
| 9 | 29 | SGAAQWAPV (SEQ ID NO: 211) | 7.200 |
| 10 | 423 | KKFARSDEL (SEQ ID NO: 122) | 7.200 |
| 11 | 295 | RGIQDVRRV (SEQ ID NO: 179) | 7.200 |
| 12 | 390 | RKFSRSDHL (SEQ ID NO: 183) | 6.000 |
| 13 | 332 | KRYFKLSHL (SEQ ID NO: 127) | 6.000 |
| 14 | 362 | RRFSRSDQL (SEQ ID NO: 187) | 6.000 |
| 15 | 417 | RWPSCQKKF (SEQ ID NO: 196) | 6.000 |
| 16 | 160 | YGHTPSHHA (SEQ ID NO: 249) | 6.000 |
| 17 | 20 | GGGGCALPV (SEQ ID NO: 92) | 6.000 |
| 18 | 329 | GCNKRYFKL (SEQ ID NO: 90) | 5.000 |
| 19 | 372 | RHQRRHTGV (SEQ ID NO: 181) | 4.500 |
| 20 | 52 | GGPAPPPAP (SEQ ID NO: 93) | 4.000 |

Table XXXVI

| Results of BIMAS HLA Peptide Binding Prediction Analysis for Binding of Human WT1 Peptides to Mouse MHC Class I Kb | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO: 90) | 24.000 |
| 2 | 225 | NLYQMTSQL (SEQ ID NO: 147) | 10.000 |
| 3 | 420 | SCQKKFARS (SEQ ID NO: 200) | 3.960 |
| 4 | 218 | RTPYSSDNL (SEQ ID NO: 194) | 3.630 |
| 5 | 437 | MHQRNMTKL (SEQ ID NO: 143) | 3.600 |
| 6 | 387 | TCQRKFSRS (SEQ ID NO: 219) | 3.600 |
| 7 | 302 | RVPGVAPTL(SEQ ID NO: 195) | 3.300 |
| 8 | 130 | NAPYLPSCL (SEQ ID NO: 144) | 3.000 |
| 9 | 289 | HTHGVFRGI (SEQ ID NO: 113) | 3.000 |
| 10 | 43 | PGASAYGSL (SEQ ID NO: 153) | 2.400 |
| 11 | 155 | DGTPSYGHT (SEQ ID NO: 56) | 2.400 |
| 12 | 273 | SDNHTTPIL(SEQ ID NO: 204) | 2.200 |
| 13 | 126 | RMFPNAPYL(SEQ ID.NO: 185) | 2.200 |
| 14 | 128 | FPNAPYLPS (SEQ ID NO: 79) | 2.000 |
| 15 | 3 | SDVRDLNAL(SEQ ID NO: 206) | 1.584 |
| 16 | 207 | DSCTGSQAL (SEQ ID NO: 61) | 1.584 |
| 17 | 332 | KRYFKLSHL(SEQ ID NO: 127) | 1.500 |
| 18 | 18 | LGGGGGCAL(SEQ ID NO: 134) | 1.320 |
| 19 | 233 | LECMTWNQM(SEQ ID NO: 131) | 1.320 |
| 20 | 441 | NMTKLQLAL (SEQ ID NO: 149) | 1.200 |

Table XXXVII

| Results of BIMAS HLA Peptide Binding Prediction Analysis for Binding of Human WT1 Peptides to Mouse MHC Class I Kd | | | |
|---|---|---|---|
| Rank | Start Position | Subsequence Residue Listing | Score (Estimate of Half Time of Disassociation of a Molecule Containing This Subsequence) |
| 1 | 285 | QYRIHTHGV (SEQ ID NO: 175) | 600.000 |
| 2 | 424 | KFARSDELV (SEQ ID NO: 119) | 288.000 |
| 3 | 334 | YFKLSHLQM (SEQ ID NO: 248) | 120.000 |
| 4 | 136 | SCLESQPTI (SEQ ID NO: 199) | 115.200 |
| 5 | 239 | NQMNLGATL (SEQ ID NO: 151) | 115.200 |
| 6 | 10 | ALLPAVSSL (SEQ ID NO:35) | 115.200 |
| 7 | 47 | AYGSLGGPA (SEQ ID NO: 41) | 86.400 |
| 8 | 180 | DPMGQQGSL (SEQ ID NO: 59) | 80.000 |
| 9 | 270 | GYESDNHTA (SEQ ID NO: 105) | 72.000 |
| 10 | 326 | AYPGCNKRY (SEQ ID NO: 42) | 60.000 |
| 11 | 192 | QYSVPPPVY (SEQ ID NO: 176) | 60.000 |
| 12 | 272 | ESDNHTAPI (SEQ ID NO:70) | 57.600 |
| 13 | 289 | HTHGVFRGI (SEQ ID NO: 113) | 57.600 |
| 14 | 126 | DVRDLNALL (SEQ ID NO: 62) | 57.600 |
| 15 | 4 | CTGSQALLL (SEQ ID NO: 52) | 57.600 |
| 16 | 208 | SCTGSQALL (SEQ ID NO: 202) | 48.000 |
| 17 | 441 | NMTKLQLAL (SEQ ID NO: 149) | 48.000 |
| 18 | 207 | DSCTGSQAL (SEQ ID NO: 61) | 48.000 |
| 19 | 130 | NAPYLPSCL (SEQ ID NO: 144) | 48.000 |
| 20 | 235 | CMTWNQMNL (SEQ ID NO: 49) | 48.000 |

Table XXXVIII

| Results of BIMAS HLA Peptide Binding Prediction Analysis for Binding of Human WT1 Peptides to Mouse MHC Class I Kk | | | |
|---|---|---|---|
| Rank | Start Position | Subsequence Residue Listing | Score (Estimate of Half Time of Disassociation of a Molecule Containing This Subsequence) |
| 1 | 81 | AEPHEEQCL (SEQ ID NO: 30) | 40.000 |
| 2 | 85 | EEQCLSAFT (SEQ ID NO: 65) | 40.000 |
| 3 | 429 | DELVRHHNM (SEQ ID NO: 53) | 20.000 |
| 4 | 315 | SETSEKRPF (SEQ ID NO: 209) | 20.000 |
| 5 | 261 | TEGQSNHST (SEQ ID NO: 221) | 20.000 |
| 6 | 410 | SEKPFSCRW (SEQ ID NO: 207) | 10.000 |
| 7 | 272 | ESDNHTTPI (SEQ ID NO:71) | 10.000 |
| 8 | 318 | SEKRPFMCA (SEQ ID NO: 208) | 10.000 |
| 9 | 138 | LESQPAIRN (SEQ ID NO: 132) | 10.000 |
| 10 | 233 | LECMTWNQM (SEQ ID NO: 131) | 10.000 |
| 11 | 298 | QDVRRVPGV (SEQ ID NO: 164) | 10.000 |
| 12 | 84 | HEEQCLSAF (SEQ ID NO: 107) | 10.000 |
| 13 | 349 | GEKPYQCDF (SEQ ID NO: 91) | 10.000 |
| 14 | 289 | HTHGVFRGI (SEQ ID NO: 113) | 10.000 |
| 15 | 179 | EDPMGQQGS (SEQ ID NO: 64) | 8.000 |
| 16 | 136 | SCLESQPAI (SEQ ID NO: 198) | 5.000 |
| 17 | 280 | ILCGAQYRI (SEQ ID NO: 116) | 5.000 |
| 18 | 273 | SDNHTTPIL (SEQ ID NO: 204) | 4.000 |
| 19 | 428 | SDELVRHHN (SEQ ID NO: 203) | 4.000 |
| 20 | 3 | SDVRDLNAL (SEQ ID NO: 206) | 4.000 |

Table XXXIX

| Results of BIMAS HLA Peptide Binding Prediction Analysis for Binding of Human WT1 Peptides to Mouse MHC Class I Ld | | | |
|---|---|---|---|
| Rank | Start Position | Subsequence Residue Listing | Score (Estimate of Half Time of Disassociation of a Molecule Containing This Subsequence) |
| 1 | 163 | TPSHHAAQF (SEQ ID NO: 228) | 360.000 |
| 2 | 327 | YPGCNKRYF (SEQ ID NO: 250) | 300.000 |
| 3 | 180 | DPMGQQGSL (SEQ ID NO: 59) | 150.000 |
| 4 | 26 | LPVSGAAQW (SEQ ID NO: 138) | 93.600 |
| 5 | 278 | TPILCGAQY (SEQ ID NO: 227) | 72.000 |
| 6 | 141 | QPAIRNQGY (SEQ ID NO: 170) | 60.000 |
| 7 | 219 | TPYSSDNLY (SEQ ID NO: 231) | 60.000 |
| 8 | 303 | VPGVAPTLV (SEQ ID NO: 242) | 60.000 |
| 9 | 120 | ASSGQARMF (SEQ ID NO: 40) | 50.000 |
| 10 | 63 | PPPPPPHSF (SEQ ID NO: 158) | 45.000 |
| 11 | 113 | GPPPPSQAS (SEQ ID NO: 97) | 45.000 |
| 12 | 157 | TPSYGHTPS (SEQ ID NO: 229) | 39.000 |
| 13 | 207 | DSCTGSQAL (SEQ ID NO: 61) | 32.500 |
| 14 | 110 | GPFGPPPPS (SEQ ID NO: 96) | 30.000 |
| 15 | 82 | EPHEEQCLS (SEQ ID NO: 68) | 30.000 |
| 16 | 412 | KPFSCRWPS (SEQ ID NO: 123) | 30.000 |
| 17 | 418 | WPSCQKKFA (SEQ ID NO: 246) | 30.000 |
| 18 | 221 | YSSDNLYQM (SEQ ID NO: 253) | 30.000 |
| 19 | 204 | TPTDSCTGS (SEQ ID NO: 230) | 30.000 |
| 20 | 128 | FPNAPYLPS (SEQ ID NO: 79) | 30.000 |

Table XL

| Results of BIMAS HLA Peptide Binding Prediction Analysis for Binding of Human WT1 Peptides to Cattle HLA A20 | | | |
|---|---|---|---|
| Rank | Start Position | Subsequence Residue Listing | Score (Estimate of Half Time of Disassociation of a Molecule Containing This Subsequence) |
| 1 | 350 | EKPYQCDFK (SEQ ID NO:66) | 1000.00 |
| 2 | 319 | EKRPFMCAY (SEQ ID NO:67) | 500.000 |
| 3 | 423 | KKFARSDEL (SEQ ID NO:122) | 500.000 |
| 4 | 345 | RKHTGEKPY (SEQ ID NO: 184) | 500.000 |
| 5 | 390 | RKFSRSDHL (SEQ ID NO:183) | 500:000 |
| 6 | 137 | CLESQPAIR (SEQ ID NO:47) | 120.000 |
| 7 | 380 | VKPFQCKTC (SEQ ID NO:239) | 100.000 |
| 8 | 407 | GKTSEKPFS (SEQ ID NO:95) | 100.000 |
| 9 | 335 | FKLSHLQMH (SEQ ID NO:78) | 100.000 |
| 10 | 247 | LKGVAAGSS (SEQ ID NO:135) | 100.000 |
| 11 | 370 | LKRHQRRHT (SEQ ID NO: 136) | 100.000 |
| 12 | 258 | VKWTEGQSN (SEQ ID NO: 240) | 100.000 |
| 13 | 398 | LKTHTRTHT (SEQ ID NO:137) | 100.000 |
| 14 | 331 | NKRYFKLSH (SEQ ID NO:145) | 100.000 |
| 15 | 357 | FKDCERRFS (SEQ ID NO:77) | 100.000 |
| 16 | 385 | CKTCQRKFS (SEQ ID NO:46) | 100.000 |
| 17 | 294 | FRGIQDVRR (SEQ ID NO:81) | 80.000 |
| 18 | 368 | DQLKRHQRR (SEQ ID NO:60) | 80.000 |
| 19 | 432 | VRHHNMHQR (SEQ ID NO: 243) | 80.000 |
| 20 | 118 | SQASSGQAR (SEQ ID NO: 216) | 80.000 |

Table XLI

| Results of BIMAS HLA Peptide Binding Prediction Analysis for Binding of Mouse WT1 Peptides to Mouse MHC Class I A 0201 | | | |
|---|---|---|---|
| Rank | Start Position | Subsequence Residue Listing | Score (Estimate of Half Time of Disassociation of a Molecule Containing This Subsequence) |
| 1 | 126 | RMFPNAPYL (SEQ ID NO: 293) | 313.968 |
| 2 | 187 | SLGEQQYSV (SEQ ID NO: 299) | 285.163 |

(continued)

| Results of BIMAS HLA Peptide Binding Prediction Analysis for Binding of Mouse WT1 Peptides to Mouse MHC Class I A 0201 | | | |
|---|---|---|---|
| Rank | Start Position | Subsequence Residue Listing | Score (Estimate of Half Time of Disassociation of a Molecule Containing This Subsequence) |
| 3 | 10 | ALLPAVSSL (SEQ ID NO: 255) | 181.794 |
| 4 | 225 | NLYQMTSQL (SEQ ID NO: 284) | 68.360 |
| 5 | 292 | GVFRGIQDV (SEQ ID NO: 270) | 51.790 |
| 6 | 93 | TLHFSGQFT (SEQ ID NO: 302) | 40.986 |
| 7 | 191 | QQYSVPPPV (SEQ ID NO: 290) | 22.566 |
| 8 | 280 | ILCGAQYRI (SEQ ID NO: 274) | 17.736 |
| 9 | 441 | NMTKLHVAL (SEQ ID NO: 285) | 15.428 |
| 10 | 235 | CMTWNQMNL (SEQ ID NO: 258) | 15.428 |
| 11 | 7 | DLNALLPAV (SEQ ID NO: 261) | 11.998 |
| 12 | 242 | NLGATLKGM (SEQ ID NO: 283) | 11.426 |
| 13 | 227 | YQMTSQLEC (SEQ ID NO: 307) | 8.573 |
| 14 | 239 | NQMNLGATL (SEQ ID NO: 286) | 8.014 |
| 15 | 309 | TLVRSASET (SEQ ID NO: 303) | 7.452 |
| 16 | 408 | KTSEKPFSC (SEQ ID NO: 277) | 5.743 |
| 17 | 340 | LQMHSRKHT (SEQ ID NO: 280) | 4.752 |
| 18 | 228 | QMTSQLECM (SEQ ID NO: 289) | 4.044 |
| 19 | 37 | VLDFAPPGA (SEQ ID NO: 304) | 3.378 |
| 20 | 302 | RVSGVAPTL (SEQ ID NO: 295) | 1.869 |

Table XLII

| Results of BIMAS HLA Peptide Binding Prediction Analysis for Binding of Mouse WT1 Peptides to Mouse MHC Class I Db | | | |
|---|---|---|---|
| Rank | Start Position | Subsequence Residue Listing | Score (Estimate of Half Time of Disassociation of a Molecule Containing This Subsequence) |
| 1 | 221 | YSSDNLYQM (SEQ ID NO: 308) | 312.000 |
| 2 | 126 | RMFPNAPYL (SEQ ID NO: 293) | 260.000 |
| 3 | 235 | CMTWNQMNL (SEQ ID NO: 258) | 260.000 |
| 4 | 437 | MHQRNMTKL (SEQ ID NO: 281) | 200.000 |
| 5 | 238 | WNQMNLGAT (SEQ ID NO: 305) | 12.000 |
| 6 | 130 | NAPYLPSCL (SEQ ID NO: 282) | 8.580 |
| 7 | 3 | SDVRDLNAL (SEQ ID NO: 298) | 7.920 |
| 8 | 136 | SCLESQPTI (SEQ ID NO: 296) | 7.920 |
| 9 | 81 | AEPHEEQCL (SEQ ID NO: 254) | 6.600 |
| 10 | 10 | ALLPAVSSL (SEQ ID NO: 255) | 6.600 |
| 11 | 218 | RTPYSSDNL (SEQ ID NO: 294) | 6.000 |
| 12 | 441 | NMTKLHVAL (SEQ ID NO: 285) | 3.432 |
| 13 | 228 | QMTSQLECM (SEQ ID NO: 289) | 3.120 |
| 14 | 174 | HSFKHEDPM (SEQ ID NO: 272) | 3.120 |
| 15 | 242 | NLGATLKGM (SEQ ID NO: 283) | 2.640 |
| 16 | 261 | TEGQSNHGI (SEQ ID NO: 301) | 2.640 |
| 17 | 225 | NLYQMTSQL (SEQ ID NO: 284) | 2.640 |
| 18 | 207 | DSCTGSQAL (SEQ ID NO: 263) | 2.600 |
| 19 | 119 | QASSGQARM (SEQ ID NO: 288) | 2.600 |
| 20 | 18 | LGGGGGCGL (SEQ ID NO: 279) | 2.600 |

Table XLIII

| Results of BIMAS HLA Peptide Binding Prediction Analysis for Binding of Mouse WT1 Peptides to Mouse MHC Class I Kb | | | |
|---|---|---|---|
| Rank | Start Position | Subsequence Residue Listing | Score (Estimate of Half Time of Disassociation of a Molecule Containing This Subsequence) |
| 1 | 329 | GCNKRYFKL (SEQ ID NO: 268) | 24.000 |
| 2 | 225 | NLYQMTSQL (SEQ ID NO: 284) | 10.000 |
| 3 | 420 | SCQKKFARS (SEQ ID NO: 297) | 3.960 |
| 4 | 218 | RTPYSSDNL (SEQ ID NO: 294) | 3.630 |
| 5 | 437 | MHQRNMTKL (SEQ ID NO: 281) | 3.600 |
| 6 | 387 | TCQRKFSRS (SEQ ID NO: 300) | 3.600 |
| 7 | 289 | HTHGVFRGI (SEQ ID NO: 273) | 3.000 |
| 8 | 130 | NAPYLPSCL (SEQ ID NO: 282) | 3.000 |
| 9 | 43 | PGASAYGSL (SEQ ID NO: 287) | 2.400 |
| 10 | 155 | DGAPSYGHT (SEQ ID NO: 260) | 2.400 |
| 11 | 126 | RMFPNAPYL (SEQ ID NO: 293) | 2.200 |
| 12 | 128 | FPNAPYLPS (SEQ ID NO: 267) | 2.000 |
| 13 | 207 | DSCTGSQAL (SEQ ID NO: 263) | 1.584 |
| 14 | 3 | SDVRDLNAL (SEQ ID NO: 298) | 1.584 |
| 15 | 332 | KRYFKLSHL (SEQ ID NO: 276) | 1.500 |
| 16 | 233 | LECMTWNQM (SEQ ID NO: 278) | 1.320 |
| 17 | 18 | LGGGGGCGL (SEQ ID NO: 279) | 1.320 |
| 18 | 242 | NLGATLKGM (SEQ ID NO: 283) | 1.200 |
| 19 | 123 | GQARMFPN (SEQ ID NO: 269)A | 1.200 |
| 20 | 441 | NMTKLHVAL (SEQ ID NO: 285) | 1.200 |

Table XLIV

| Results of BIMAS HLA Peptide Binding Prediction Analysis for Binding of Mouse WT1 Peptides to Mouse MHC Class I Kd | | | |
|---|---|---|---|
| Rank | Start Position | Subsequence Residue Listing | Score (Estimate of Half Time of Disassociation of a Molecule Containing This Subsequence) |
| 1 | 285 | QYRIHTHGV (SEQ ID NO: 291) | 600.000 |
| 2 | 424 | KFARSDELV (SEQ ID NO: 275) | 288.000 |
| 3 | 334 | YFKLSHLQM (SEQ ID NO: 306) | 120.000 |
| 4 | 136 | SCLESQPTI (SEQ ID NO: 296) | 115.200 |
| 5 | 239 | NQMNLGATL (SEQ ID NO: 286) | 115.200 |
| 6 | 10 | ALLPAVSSL (SEQ ID NO: 255) | 115.200 |
| 7 | 47 | AYGSLGGPA (SEQ ID NO: 256) | 86.400 |
| 8 | 180 | DPMGQQGSL (SEQ ID NO: 262) | 80.000 |
| 9 | 270 | GYESDNHTA (SEQ ID NO: 271) | 72.000 |
| 10 | 192 | QYSVPPPVY (SEQ ID NO: 292) | 60.000 |
| 11 | 326 | AYPGCNKRY (SEQ ID NO: 257) | 60.000 |
| 12 | 289 | HTHGVFRGI (SEQ ID NO: 273) | 57.600 |
| 13 | 4 | DVRDLNALL (SEQ ID NO: 264) | 57.600 |
| 14 | 126 | RMFPNAPYL (SEQ ID NO: 293) | 57.600 |
| 15 | 209 | CTGSQALLL (SEQ ID NO: 259) | 48.000 |
| 16 | 86 | EQCLSAFTL (SEQ ID NO: 265) | 48.000 |
| 17 | 302 | RVSGVAPTL (SEQ ID NO: 295) | 48.000 |
| 18 | 218 | RTPYSSDNL (SEQ ID NO: 294) | 48.000 |
| 19 | 272 | ESDNHTAPI (SEQ ID NO: 266) | 48.000 |
| 20 | 225 | NLYQMTSQL (SEQ ID NO: 284) | 48.000 |

Table XLV

| Results of TSites Peptide Binding Prediction Analysis for Human WT1 Peptides Capable of Eliciting a Helper T cell Response | |
|---|---|
| Peptide | Sequence |
| p6-23 | RDLNALLPAVPSLGGGG (SEQ ID NO:1) |
| p30-35 | GAAQWA (SEQ ID NO:309) |
| p45-56 | ASAYGSLGGPAP (SEQ ID NO:310) |
| p91-105 | AFTVHFSGQFTGTAG (SEQ ID NO:311) |
| p117-139 | PSQASSGQARMFPNAPYLPSCLE (SEQ ID NO:2) |
| p167-171 | HAAQF (SEQ ID NO:312) |
| p202-233 | CHTPTDSCTGSQALLLRTPYSSDNLYQMTSQL (SEQ ID NO:313) |
| p244-262 | GATLKGVAAGSSSSVKWTE (SEQ ID NO:4) |
| p287-318 | RIHTHGVFRGIQDVRRVPGVAPTLVRSASETS (SEQ ID NO:314) |
| p333-336 | RYFK (SEQ ID NO:315) |
| p361-374 | ERRFSRSDQLKRHQ (SEQ ID NO:316) |
| p389-410 | QRKFSRSDHLKTHTRTHTGKTS (SEQ ID NO:317) |
| p421-441 | CQKKFARSDELVRHHNMHQRN (SEQ ID NO:318) |

Certain CTL peptides (shown in Table XLVI) were selected for further study. For each peptide in Table XLVI, scores obtained using BIMAS HLA peptide binding prediction analysis are provided.

Table XLVI

| WT1 Peptide Sequences and HLA Peptide Binding Predictions | | |
|---|---|---|
| Peptide | Sequence | Comments |
| p329-337 | GCNKRYFKL (SEQ ID NOs: 90 and 268) | Score 24,000 |
| p225-233 | NLYQMTSQL (SEQ ID NOs: 147 and 284) | binds also to class II and HLA A2, Kd, score 10,000 |
| p235-243 | CMTWNQMNL (SEQ ID NOs: 49 and 258) | binds also to HLA A2, score 5,255,712 |
| p126-134 | RMFPNAPYL (SEQ ID NOs: 185 and 293) | binds also to Kd, class II and HLA A2, score 1,990,800 |
| p221-229 | YSSDNLYQM (SEQ ID NOs: 253 and 308) | binds also to Ld, score 312,000 |
| p228-236 | QMTSQLECM (SEQ ID NOs: 169 and 289) | score 3,120 |
| p239-247 | NQMNLGATL (SEQ ID NOs: 151 and 286) | binds also to HLA A 0201, Kd, score 8,015 |
| mouse p136-144 | SCLESQPTI (SEQ ID NO:296) | binds also to Kd, 1 mismatch to human |
| human p136-144 | SCLESQPAI (SEQ ID NO:198) | score 7,920 |
| mouse p10-18 | ALLPAVSSL (SEQ ID NO:255) | binds also to Kd, HLA A2, 1 mismatch to human |
| human p10-18 | ALLPAVPSL (SEQ ID NO:34) | score 6,600 |

Peptide binding to C57Bl/6 murine MHC was confirmed using the leukemia cell line RMA-S, as described by Ljunggren et al., Nature 346:476-480, 1990. In brief, RMA-S cells were cultured for 7 hours at 26°C in complete medium supplemented with 1% FCS. A total of $10^6$ RMA-S cells were added into each well of a 24-well plate and incubated either alone or with the designated peptide (25ug/ml) for 16 hours at 26°C and additional 3 hours at 37°C in complete medium. Cells were then washed three times and stained with fluorescein isothiocyanate-conjugated anti $D^b$ or anti-$K^b$ antibody (PharMingen, San Diego, CA). Labeled cells were washed twice, resuspended and fixed in 500ul of PBS with 1% paraformaldehyde and analyzed for fluorescence intensity in a flow cytometer (Becton-Dickinson FACSCalibur®). The percentage of increase of $D^b$ or $K^b$ molecules on the surface of the RMA-S cells was measured by increased mean fluorescent intensity of cells incubated with peptide compared with that of cells incubated in medium alone.

Mice were immunized with the peptides capable of binding to murine class I MHC. Following immunization, spleen cells were stimulated *in vitro* and tested for the ability to lyse targets incubated with WT1 peptides. CTL were evaluated with a standard chromium release assay (Chen et al., Cancer Res. 54:1065-1070, 1994). $10^6$ target cells were incubated at 37°C with 150μCi of sodium $^{51}Cr$ for 90 minutes, in the presence or absence of specific peptides. Cells were washed three times and resuspended in RPMI with 5% fetal bovine serum. For the assay, $10^4$ $^{51}Cr$-labeled target cells were incubated with different concentrations of effector cells in a final volume of 200μl in U-bottomed 96-well plates. Supernatants were removed after 4 to 7 hours at 37°C, and the percentage specific lysis was determined by the formula:

% specific lysis = 100 x (experimental release - spontaneous release)/(maximum release-spontaneous release).

The results, presented in Table XLVII, show that some WT1 peptides can bind to class I MHC molecules, which is essential for generating CTL. Moreover, several of the peptides were able to elicit peptide specific CTL (Figures 9A and 9B), as determined using chromium release assays. Following immunization to CTL peptides p10-18 human, p136-144 human, p136-144 mouse and p235-243, peptide specific CTL lines were generated and clones were established. These results indicate that peptide specific CTL can kill malignant cells expressing WT1.

Table XLVII

| Binding of WT1 CTL Peptides to mouse B6 class I antigens | |
|---|---|
| Peptide | Binding Affinity to Mouse MHC Class I |
| Positive control | 91 % |
| negative control | 0.5.-1.3% |
| p235-243 | 33.6% |
| p136-144 mouse | 27.9% |
| p136-144 human | 52% |
| p10-18: human | 2.2% |
| p225-233 | 5.8% |
| p329-337 | 1.2% |
| p126-134 | 0.9% |
| p221-229 | 0.8% |
| p228-236 | 1.2% |
| p239-247 | 1% |

EXAMPLE 5

USE OF A WT1 POLYPEPTIDE TO ELICIT WT1 SPECIFIC CTL IN MICE

This Example illustrates the ability of a representative WT1 polypeptide to elicit CTL immunity capable of killing WT1 positive tumor cell lines.

P117-139, a peptide with motifs appropriate for binding to class I and class II MHC, was identified as described above using TSITES and BIMAS HLA peptide binding prediction analyses. Mice were immunized as described in Example 3. Following immunization, spleen cells were stimulated *in vitro* and tested for the ability to lyse targets incubated with WT1 peptides, as well as WT1 positive and negative tumor cells. CTL were evaluated with a standard chromium release assay. The results, presented in Figures 10A-10D, show that P117 can elicit WT1 specific CTL capable of killing WT1 positive tumor cells, whereas no killing of WT1 negative cells was observed. These results demonstrate that peptide specific CTL in fact kill malignant cells expressing WT1 and that vaccine and T cell therapy are effective against malignancies that express WT1.

Similar immunizations were performed using the 9-mer class I MHC binding peptides p136-144, p225-233, p235-243 as well as the 23-mer peptide p117-139. Following immunization, spleen cells were stimulated *in vitro* with each of the 4 peptides and tested for ability to lyse targets incubated with WT1 peptides. CTL were generated specific for p136-144, p235-243 and p117-139, but not for p225-233. CTL data for p235-243 and p117-139 are presented in Figures 11A and 11B. Data for peptides p136-144 and p225-233 are not depicted.

CTL lysis demands that the target WT1 peptides are endogenously processed and presented in association with tumor cell class I MHC molecules. The above WT1 peptide specific CTL were tested for ability to lyse WT1 positive versus negative tumor cell lines. CTL specific for p235-243 lysed targets incubated with the p235-243 peptides, but failed to lyse cell lines that expressed WT1 proteins (Figure 11A). By marked contrast, CTL specific for p117-139 lysed targets incubated with p117-139 peptides and also lysed malignant cells expressing WT1 (Figure 11B). As a negative control, CTL specific for p117-139 did not lyse WT1 negative EL-4 (also referred to herein as E10).

Specificity of WT1 specific lysis was confirmed by cold target inhibition (Figures 12A-12B). Effector cells were plated for various effector: target ratios in 96-well U-bottom plates. A ten-fold excess (compared to hot target) of the indicated peptide-coated target without $^{51}$Cr labeling was added. Finally, $10^4$ $^{51}$Cr-labeled target cells per well were added and the plates incubated at 37°C for 4 hours. The total volume per well was 200μl.

Lysis of TRAMP-C by p117-139 specific CTL was blocked from 58% to 36% by EL-4 incubated with the relevant peptide p117-139, but not with EL-4 incubated with an irrelevant peptide (Figure 12A). Similarly, lysis of BLK-SV40 was blocked from 18% to 0% by EL-4 incubated with the relevant peptide p117-139 (Figure 12B). Results validate that WT1 peptide specific CTL specifically kill malignant cells by recognition of processed WT1.

Several segments with putative CTL motifs are contained within p117-139. To determine the precise sequence of the CTL epitope all potential 9-mer peptides within p117-139 were synthesized (Table XLVIII). Two of these peptides (p126-134 and p130-138) were shown to bind to H-$2^b$ class I molecules (Table XLVIII). CTL generated by immunization with p117-139 lysed targets incubated with p126-134 and p130-138, but not the other 9-mer peptides within p117-139 (Figure 13A).

The p117-139 specific CTL line was restimulated with either p126-134 or p130-138. Following restimulation with p126-134 or p130-138, both T cell lines demonstrated peptide specific lysis, but only p130-138 specific CTL showed lysis of a WT1 positive tumor cell line (Figures 13B and 13C). Thus, p130-138 appears to be the naturally processed epitope.

Table XLVIII

| Binding of WT1 CTL 9mer Peptides within p117-139 to mouse B6 class I antigens | |
|---|---|
| Peptide | Binding Affinity to Mouse MHC Class I |
| P117-125 PSQASSGQA (SEQ ID NO:221) | 2% |
| P118-126 SQASSGQAR (SEQ ID NO:216) | 2% |
| P119-127 QASSGQARM (SEQ ID Nos: 161 and 288) | 2% |
| P120-128 ASSGQARMF (SEQ ID NO:40 | 1% |
| P121-129 SSGQARMFP (SEQ ID NO:222) | 1% |
| P122-130 SGQARMFPN (SEQ ID NO:212) | 1% |
| P123-131 GQARMFPNA (SEQ ID Nos: 98 and 269) | 1% |
| P124-132 QARMFPNAP (SEQ ID NO:223) | 1% |
| P125-133 ARMFPNAPY (SEQ ID NO:38) | 1% |
| P126-134 RMFPNAPYL (SEQ ID NOs: 185 and 293) | 79% |
| P127-135 MFPNAPYLP (SEQ ID NO:224) | 2% |
| P128-136 FPNAPYLPS (SEQ ID NOs: 79 and 267) | 1% |
| P129-137 PNAPYLPSC (SEQ ID NO:225) | 1% |
| P130-138 NAPYLPSCL (SEQ ID NOs: 144 and 282) | 79% |
| P131-139 APYLPSCLE (SEQ ID NO:226) | 1% |

EXAMPLE 6

IDENTIFICATION OF WT1 SPECIFIC MRNA IN MOUSE TUMOR CELL LINES

This Example illustrates the use of RT-PCR to detect WT1 specific mRNA in cells and cell lines.

Mononuclear cells were isolated by density gradient centrifugation, and were immediately frozen and stored at -80°C until analyzed by RT-PCR for the presence of WT1 specific mRNA. RT-PCR was generally performed as described by Fraizer et al., Blood 86:4704-4706, 1995. Total RNA was extracted from $10^7$ cells according to standard procedures. RNA pellets were resuspended in 25 μL diethylpyrocarbonate treated water and used directly for reverse transcription. The zinc-finger region (exons 7 to 10) was amplified by PCR as a 330 bp mouse cDNA. Amplification was performed in a thermocycler during one or, when necessary, two sequential rounds of PCR. AmpliTaq DNA Polymerase (Perkin Elmer Cetus, Norwalk, CT), 2.5 mM $MgCl_2$ and 20 pmol of each primer in a total reaction volume of 50μl were used. Twenty μL aliquots of the PCR products were electrophoresed on 2% agarose gels stained with ethidium bromide. The gels were photographed with Polaroid film (Polaroid 667, Polaroid Ltd., Hertfordshire, England). Precautions against cross contamination were taken following the recommendations of Kwok and Higuchi, Nature 339:237-238, 1989. Negative controls included the cDNA- and PCR-reagent mixes with water instead of cDNA in each experiment. To avoid false negatives, the presence of intact RNA and adequate cDNA generation was evaluated for each sample by a control PCR using β-actin primers. Samples that did not amplify with these primers were excluded from analysis.

Primers for amplification of WT1 in mouse cell lines were: P115: 1458-1478: 5' CCC AGG CTG CAA TAA GAG ATA 3' (forward primer; SEQ ID NO:21); and P116: 1767-1787: 5' ATG TTG TGA TGG CGG ACC AAT 3' (reverse primer; SEQ ID NO:22) *(see* Inoue et al, Blood 88:2267-2278, 1996; Fraizer et al., Blood 86:4704-4706,1995).

Beta Actin primers used in the control reactions were: 5' GTG GGG CGC CCC AGG CAC CA 3' (sense primer; SEQ ID NO:23); and 5' GTC CTT AAT GTC ACG CAC GAT TTC 3' (antisense primer; SEQ ID NO:24)

Primers for use in amplifying human WT1 include: P117: 954-974: 5' GGC ATC TGA GAC CAG TGA GAA 3' (SEQ ID NO:25); and P118: 1434-1414: 5' GAG AGT CAG ACT TGA AAG CAGT 3' (SEQ ID NO:5). For nested RT-PCR, primers may be: P119: 1023-1043: 5' GCT GTC CCA CTT ACA GAT GCA 3' (SEQ ID NO:26); and P120: 1345-1365: 5' TCA AAG CGC CAG CTG GAG TTT 3' (SEQ ID NO:27).

Table XLVIII shows the results of WT1 PCR analysis of mouse tumor cell lines. Within Table IV, (+++) indicates a strong WT1 PCR amplification product in the first step RT PCR, (++) indicates a WT1 amplification product that is detectable by first step WT1 RT PCR, (+) indicates a product that is detectable only in the second step of WT1 RT PCR, and (-) indicates WT1 PCR negative.

Table XLIX

| Detection of WT1 mRNA in Mouse Tumor Cell Lines | |
|---|---|
| Cell Line | WT1 mRNA |
| K562 (human leukemia; ATCC): Positive control; (Lozzio and Lozzio, Blood 45:321-334, 1975) | +++ |
| TRAMPC (SV40 transformed prostate, B6); Foster et al., Cancer Res. 57:3325-3330, 1997 | +++ |
| BLK-SV40 HD2 (SV40-transf. fibroblast, B6; ATCC); Nature 276:510-511, 1978 | ++ |
| CTLL (T-cell, B6; ATCC); Gillis, Nature 268:154-156, 1977) | + |
| FM (FBL-3 subline, leukemia, B6); Glynn and Fefer, Cancer Res. 28:434-439, 1968 | + |
| BALB 3T3 (ATCC); Aaroston and Todaro, J Cell. Physiol. 72:141-148, 1968 | + |
| S49.1 (Lymphoma, T-cell like, B/C; ATCC); Horibata and Harris, Exp. Cell. Res. 60:61, 1970 | + |
| BNL CL.2 (embryonic liver, B/C; ATCC); Nature 276:510-511, 1978 | + |
| MethA (sarcoma, B/C); Old et al., Ann. NY Acad. Sci. 101:80- 106, 1962 | - |
| P3.6.2.8.1 (myeloma, B/C; ATCC); Proc. Natl. Acad. Sci. USA 66:344, 1970 | - |
| P2N (leukemia, DBA/2; ATCC); Melling et al., J. Immunol. 117:1267-1274,1976 | - |
| BCL1 (lymphoma, B/C; ATCC); Slavin and Strober, Nature 272:624-626, 1977 | - |
| LSTRA (lymphoma, B/C); Glynn et al., Cancer Res. 28:434-439, 1968 | - |
| E10/EL-4 (lymphoma, B6); Glynn et al., Cancer Res. 28:434-439, 1968 | - |

EXAMPLE 7

EXPRESSION IN E. COLI OF WT1 TRX FUSION CONSTRUCT

The truncated open reading frame of WT1 (WT1B) was PCR amplified with the following primers:

Forward Primer starting at amino acid 2
P-37 (SEQ ID NO. 342) 5' ggctccgacgtgcgggacctg 3' Tm 64°C
Reverse Primer creating EcoRI site after stop codon
P-23 (SEQ ID NO. 343) 63°C 5' gaattctcaaagcgccagctggagtttggt 3' Tm

The PCR was performed under the following conditions:

10μl 10X Pfu buffer
1μl 10mM dNTPs
2μl 10μM each oligo
83μL sterile water
1.5μl Pfu DNA polymerase (Stratagene, La Jolla, CA)
50 ng DNA (pPDM FL WT1)

96°C 2 minutes
96°C 20 seconds 63°C 15 seconds 72°C 3 minutes x 40 cycles
72°C 4 minutes

The PCR product was digested with EcoRI restriction enzyme, gel purified and then cloned into pTrx 2H vector (a modified pET28 vector with a Trx fusion on the N-terminal and two His tags surrounding the Trx fusion. After the Trx fusion there exists protease cleavage sites for thrombin and enterokinase). The pTrx2H construct was digested with StuI and EcoRI restriction enzymes. The correct constructs were confirmed by DNA sequence analysis and then transformed into BL21 (DE3) pLys S and BL21 (DE3) CodonPlus expression host cells.

EXAMPLE 8

EXPRESSION IN E. COLI OF WT1 A HIS TAG FUSION CONSTRUCTS

The N-terminal open reading frame of WT1 (WT1A) was PCR amplified with the following primers:

Forward Primer starting at amino acid 2
P-37 (SEQ ID NO. 344) 5'ggctccgacgtgcgggacctg 3' Tm 64°C
Reverse Primer creating EcoRI site after an artificial stop codon put after amino acid 249.
PDM-335 (SEQ ID NO. 345) 5'gaattctcaaagcgccagctggagtttggt 3' Tm 64°C

The PCR was performed under the following conditions:

10μl 10X Pfu buffer
1μl 10mM dNTPs
2μl 10μM each oligo
83μL sterile water
1.5μl Pfu DNA polymerase (Stratagene, La Jolla, CA)
50 ng DNA (pPDM FL WT1)

96°C 2 minutes
96°C 20 seconds 63°C 15 seconds 72°C 1 minute 20 seconds x 40 cycles
72°C 4 minutes

The PCR product was digested with EcoRI restriction enzyme, gel purified and then cloned into pPDM, a modified pET28 vector with a His tag in frame, which had been digested with Eco72I and EcoRI restriction enzymes. The PCR product was also transformed into pTrx 2H vector. The pTrx2H construct was digested with StuI and EcoRI restriction enzymes. The correct constructs were confirmed by DNA sequence analysis and then transformed into BL21 (DE3) pLys S and BL21 (DE3) CodonPlus expression host cells.

EXAMPLE 9

EXPRESSION IN E. COLI OF WT1 B HIS TAG FUSION CONSTRUCTS

The truncated open reading frame of WT1 (WT1A) was PCR amplified with the following primers:

Forward Primer starting at amino acid 250
PDM-346 (SEQ ID NO. 346) 5' cacagcacagggtacgagagc 3' Tm 58°C
Reverse Primer creating EcoRI site after stop codon
P-23 (SEQ ID NO. 347) 5'gaattctcaaagcgccagctggagtttggt 3' Tm 63°C

The PCR was performed under the following conditions:

10μl 10X Pfu buffer
1μl 10mM dNTPs
2μl 10μM each oligo
83μL sterile water
1.5μl Pfu DNA polymerase (Stratagene, La Jolla, CA)
50 ng DNA (pPDM FL WT1)

| | | |
|---|---|---|
| 96°C 2 minutes | | |
| 96°C 20 seconds | 63°C 15 seconds | 72°C 1 minute 30 seconds x 40 cycles |
| 72°C 4 minutes | | |

The PCR product was digested with EcoRI restriction enzyme, gel purified and then cloned into pPDM, a modified pET28 vector with a His tag in frame, which had been digested with Eco72I and EcoRI restriction enzymes. The PCR product was also transformed into pTrx 2H vector. The pTrx 2H construct was digested with StuI and EcoRI restriction enzymes. The correct constructs were confirmed by DNA sequence analysis and then transformed into BL21 (DE3) pLys S and BL21 (DE3) CodonPlus expression host cells.

For Examples 7-9, the following SEQ ID NOs. are disclosed:

SEQ ID NO. 327 is the determined cDNA sequence for Trx_WT1_B
SEQ ID NO. 328 is the determined cDNA sequence for Trx_WT1_A
SEQ ID NO. 329 is the determined cDNA sequence for Trx_WT1
SEQ ID NO. 330 is the determined cDNA sequence for WT1_A
SEQ ID NO. 331 is the determined cDNA sequence for WT1_B
SEQ ID NO. 332 is the predicted amino acid sequence encoded by SEQ ID No. 327
SEQ ID NO. 333 is the predicted amino acid sequence encoded by SEQ ID No. 328
SEQ ID NO. 334 is the predicted amino acid sequence encoded by SEQ ID No. 329
SEQ ID NO. 335 is the predicted amino acid sequence encoded by SEQ ID No. 330
SEQ ID NO. 336 is the predicted amino acid sequence encoded by SEQ ID No. 331

EXAMPLE 10

TRUNCATED FORMS OF WT1 EXPRESSED IN E. COLI

Three reading frames of WT1 were amplified by PCR using the following primers:

For WT1 Tr2:

PDM-441 (SEQ ID NO. 348) 5' cacgaagaacagtgcctgagcgcattcac 3' Tm 63°C

PDM-442 (SEQ ID NO. 349) 5' ccggcgaattcatcagtataaattgtcactgc 3' TM 62°C

For WT1 Tr3:

PDM-443 (SEQ ID NO. 350) 5' caggctttgctgctgaggacgccc 3' Tm 64°C

PDM-444 (SEQ ID NO. 351) 5' cacggagaattcatcactggtatggtttctcacc Tm 64°C

For WT1 Tr4:

PDM-445 (SEQ ID NO. 352) 5' cacagcaggaagcacactggtgagaaac 3' Tm 63°C

PDM-446 (SEQ ID NO. 353) 5' ggatatctgcagaattctcaaagcgccagc 3' TM 63°C

The PCR was performed under the following conditions:

10μl 10X Pfu buffer
1μl 10mM dNTPs
2μl 10μM each oligo
83μL sterile water
1.5μl Pfu DNA polymerase (Stratagene, La Jolla, CA)
50 ng DNA (pPDM FL WT1)

96°C 2 minutes
96°C 20 seconds 63°C 15 seconds 72°C 30 seconds x 40 cycles
72°C 4 minutes

The PCR products were digested with EcoRI and cloned into pPDM His (a modified pET28 vector with a His tag in frame on the 5' end) which has been digested with Eco72I and EcoRI. The constructs were confirmed to be correct through sequence analysis and transformed into BL21 pLys S and BL21 CodonPlus cells or BLR pLys S and BLR CodonPlus cells.

EXAMPLE 11

WT1 C (amino acids 76-437) AND WT1 D (amino acids 91-437) EXPRESSION IN E. COLI

The WT1 C reading frame was amplified by PCR using the following primers:

PDM-504 (SEQ ID NO. 354) 5' cactccttcatcaaacaggaac 3' Tm 61°C
PDM-446 (SEQ ID NO. 355) 5' ggatatctgcagaattctcaaagcgccagc 3' Tm 63°C

The PCR was performed under the following conditions:

10μl 10X Pfu buffer
1μl 10mM dNTPs
2μl 10μM each oligo
83 μL sterile water
1.5μl Pfu DNA polymerase (Stratagene, La Jolla, CA)
50 ng DNA (pPDM FL WT1)

96°C 2 minutes

(continued)

96°C 20 seconds 63°C 15 seconds 72°C 2 minutes x 40 cycles
72°C 4 minutes

The PCR product was digested with EcoRI and cloned into pPDM His which had been digested with Eco72I and EcoRI. The sequence was confirmed through sequence analysis and then transformed into BLR pLys S and BLR which is co-transformed with CodonPlus RP.

EXAMPLE 12

SYNTHETIC PRODUCTION OF WT1 TR-1 BY ANNEALING OVERLAPPING OLIGOS

This example was performed to determine the effect of changing proline codon usage on expression.
The following pairs of oligos were annealed:

1. PDM-505 (SEQ ID NO. 356) 5'
ggttccgacgtgcgggacctgaacgcactgctg 3'
PDM-506 (SEQ ID NO. 357) 5'
ctgccggcagcagtgcgttcaggtcccgcacgtcggaacc 3'

2. PDM-507 (SEQ ID NO. 358) 5'
ccggcagttccatccctgggtggcggtggaggctg 3'
PDM-508 (SEQ ID NO. 359) 5'
cggcagtgcgcagcctccaccgccacccagggatggaa 3'

3. PDM-509 (SEQ ID NO. 360) 5'
cgcactgccggttagcggtgcagcacagtgggctc 3'
PDM-510 (SEQ ID NO. 361) 5'
cagaactggagcccactgtgctgcaccgctaac 3'

4. PDM-511 (SEQ ID NO. 362) 5'
cagttctggacttcgcaccgcctggtgcatccgcatac 3'
PDM-512 (SEQ ID NO. 363) 5'
cagggaaccgtatgcggatgcaccaggcggtgcgaagtc 3'

5. PDM-513 (SEQ ID NO. 364) 5'
ggttccctgggtggtccagcacctccgcccgcaacgcc 3'
PDM-514 (SEQ ID NO. 365) 5'
ggcggtgggggcgttgcgggcggaggtgctggaccacc 3'

6. PDM-515 (SEQ ID NO. 366) 5'
cccaccgcctccaccgcccccgcactccttcatcaaacag 3'
PDM-516 (SEQ ID NO. 367) 5'
ctaggttcctgtttgatgaaggagtgcgggggcggtgga 3'

7. PDM-517 (SEQ ID NO. 368) 5'
gaacctagctggggtggtgcagaaccgcacgaagaaca 3'
PDM-518 (SEQ ID NO. 369) 5'
ctcaggcactgttcttcgtgcggttctgcaccaccccag 3'

8. PDM-519 (SEQ ID NO. 370) 5' gtgcctgagcgcattctgagaattctgcagat 3'
PDM-520 (SEQ ID NO. 371) 5'
gtgtgatggatatctgcagaattctcagaatgcg 3'

Each oligo pair was separately combined then annealed. The pairs were then ligated together and one μl of ligation mix was used for PCR conditions below:

10μl 10X Pfu buffer
1μl 10mM dNTPs
2μl 10μM each oligo
83μL sterile water
1.5μl Pfu DNA polymerase (Stratagene, La Jolla, CA)

96°C 2 minutes
96°C 20 seconds 63°C 15 seconds 72°C 30 seconds x 40 cycles
72°C 4 minutes

The PCR product was digested with EcoRI and cloned into pPDM His which had been digested with Eco72I and EcoRI. The sequence was confirmed and then transformed into BLR pLys S and BLR which is co-transformed with CodonPlus RP.

For examples 10-12, the following SEQ ID NOs. are disclosed:

SEQ ID NO:337 is the determined cDNA sequence for WT1_Tr1
SEQ ID NO:338 is the determined cDNA sequence for WT1_Tr2
SEQ ID NO:339 is the determined cDNA sequence for WT1_Tr3
SEQ ID NO:340 is the determined cDNA sequence for WT1_Tr4
SEQ ID NO:341 is the determined cDNA sequence for WT1_C
SEQ ID NO:342 is the predicted amino acid sequence encoded by SEQ ID NO:337
SEQ ID NO:343 is the predicted amino acid sequence encoded by SEQ ID NO:338
SEQ ID NO:344 is the predicted amino acid sequence encoded by SEQ ID NO:339
SEQ ID NO:345 is the predicted amino acid sequence encoded by SEQ ID NO:340
SEQ ID NO:346 is the predicted amino acid sequence encoded by SEQ ID NO:341

The WT1 C sequence represents a polynucleotide having the coding regions of TR2, TR3 and TR4.

The WT1 TR-1 synthetic sequence represents a polynucleotide in which alternative codons for proline were substituted for the native codons, producing a polynucleotide capable of expressing WT1 TR-1 in E. coli.

EXAMPLE 13

EVALUATION OF THE SYSTEMIC HISTOPATHOLOGICAL AND TOXICOLOGICAL EFFECTS OF WT1 IMMUNIZATION IN MICE

The purpose of this example is to analyze the immunogenicity and potential systemic histopathological and toxicological effects of WT1 protein immunization in a multiple dose titration in mice.

The experimental design for immunization of mice with WT1 protein is outlined in Table L.

Table L

| Experimental Design of WT1 Immunization in Mice | | | | |
|---|---|---|---|---|
| **Histology Group** | **Corixa Group** | **Treatment Description** | **Dose Level** | **Total No. (Females)** |
| 1 | 0 | No treatment | 0 | 4 |
| 2 | 1.1 | MPL-SE (adjuvants alone), 6x, 1 week apart | 10ug | 4 |
| 3 | 1.2 | MPL-SE, 3x, 2 weeks apart | 10ug | 4 |
| 4 | 2.1 | Ra12-WT1+ MPL-SE, 6x | 25ug | 4 |
| 5 | 2.2 | Ra12-WT1 + MPL-SE, 3x | 25ug | 4 |
| 6 | 3.1 | Ra12-WT1 + MPL-SE, 6x | 100ug | 4 |
| 7 | 3.2 | Ra12-WT1 + MPL-SE, 3x | 100ug | 4 |

(continued)

| Experimental Design of WT1 Immunization in Mice | | | | |
|---|---|---|---|---|
| **Histology Group** | **Corixa Group** | **Treatment Description** | **Dose Level** | **Total No. (Females)** |
| 8 | 4.1 | Ra12-WT1 + MPL-SE, 6x | 1000ug | 4 |
| 9 | 4.2 | Ra12-WT1 + MPL-SE, 3x | 1000ug | 4 |

Vaccination to WT1 protein using MPL-SE as adjuvant, in a multiple dose titration study (doses ranging from 25μg, 100μg to 1000μg WT1 protein) in female C57/B6 mice elicited a strong WT1-specific antibody response (Figure 19) and cellular T-cell responses (Figure 20).

No systemic histopathological or toxicological effects of immunization with WT1 protein was observed. No histological evidence for toxicity was seen in the following tissues: adrenal gland, brain, cecum, colon, duodenum, eye, femur and marrow, gall bladder, heart, ileum, jejunum, kidney, larynx, lacrimal gland, liver, lung, lymph node, muscle, esophagus, ovary, pancreas, parathyroid, salivary gland, sternum and marrow, spleen, stomach, thymus, trachea, thyroid, urinary bladder and uterus.

Special emphasis was put on evaluation of potential hematopoietic toxicity. The myeloid/erythroid ratio in sternum and femur marrow was normal. All evaluable blood cell counts and blood chemistry (BUN, creatinine, bilirubin, albumin, globulin) were within the normal range (Table LI).

Given that existent immunity to WT1 is present in some patients with leukemia and that vaccination to WT1 protein can elicit WT1 specific Ab and cellular T-cell responses in mice without toxicity to normal tissues, these experiments validate WT1 as a tumor/leukemia vaccine.

Table LI

Clinical Chemistry and Hematology Analysis

**Table LI: WT1 Dose Titration Study**
**Clinical Chemistry and Hematology Analysis**

| Animal # | K/uL WBC | M/uL RBC | g/dl Hg. | % HCT | fL MCV | pg MCH | % MCHC |
|---|---|---|---|---|---|---|---|
| **Normal** | **5.4-16.0** | **6.7-12.5** | **10.2-16.6** | **32-54** | **31-62** | **9.2-20.8** | **22.0-35.5** |
| | | | | | | | |
| **Group 1** | | | | | | | |
| **1 (0)** | 5.6 | 8.41 | 12.8 | 43.5 | 53 | 15.2 | 29.4 |
| **2 (0)** | 5.5 | 9.12 | 13.4 | 47.5 | 53 | 14.7 | 28.2 |
| **3 (0)** | 7.5 | 9.22 | 13.5 | 48 | 54 | 14.7 | 28.1 |
| **4 (0)** | 3.9 | 9.27 | 13.6 | 46 | 52 | 14.7 | 29.6 |
| **Mean** | 5.6 | 9.0 | 13.3 | 46.3 | 53.0 | 14.8 | 28.8 |
| **STD** | 1.5 | 0.4 | 0.4 | 2.0 | 0.8 | 0.3 | 0.8 |
| | | | | | | | |
| **Group 2** | | | | | | | |
| **5 (1.5)** | 6.6 | 9 | 13.1 | 46 | 54 | 14.5 | 28.5 |
| **6 (1.6)** | 5.2 | 8.58 | 12.6 | 44 | 53 | 14.7 | 28.6 |
| **7 (1.7)** | 7.8 | 9.21 | 13.6 | 46 | 53 | 14.7 | 29.6 |
| **8 (1.8)** | 6.3 | **NA** | **NA** | 41 | **NA** | **NA** | **NA** |
| **Mean** | 6.5 | 8.9 | 13.1 | 44.3 | 53.3 | 14.6 | 28.9 |
| **STD** | 1.1 | 0.3 | 0.5 | 2.4 | 0.6 | 0.1 | 0.6 |
| | | | | | | | |
| **Group 3** | | | | | | | |
| **9 (2.5)** | 8.3 | 9.16 | 13.6 | 50.3 | 55 | 14.9 | 27.1 |
| **10 (2.6)** | 5 | 8.78 | 13 | 44.2 | 50 | 14.8 | 29.3 |

**Table LI: WT1 Dose Titration Study**
**Clinical Chemistry and Hematology Analysis**

| Animal # | K/uL WBC | M/uL RBC | g/dl Hg. | % HCT | fL MCV | pg MCH | % MCHC |
|---|---|---|---|---|---|---|---|
| Normal | 5.4-16.0 | 6.7-12.5 | 10.2-16.6 | 32-54 | 31-62 | 9.2-20.8 | 22.0-35.5 |
| 11 (2.7) | 4 | 8.94 | 13.2 | 48.3 | 54 | 14.7 | 27.3 |
| 12 (2.8) | 8.2 | NA | NA | 41 | NA | NA | NA |
| Mean | 6.4 | 9.0 | 13.3 | 46.0 | 53.0 | 14.8 | 27.9 |
| STD | 2.2 | 0.2 | 0.3 | 4.2 | 2.6 | 0.1 | 1.2 |
| | | | | | | | |
| Group 4 | | | | | | | |
| 13 (3.5) | 6.1 | 8.82 | 13.1 | 46 | 54 | 14.9 | 28.5 |
| 14 (3.6) | 6.1 | 8.64 | 12.9 | 46 | 54 | 15 | 28 |
| 15 (3.7) | 9.3 | 8.93 | 13.2 | 48 | 55 | 14.8 | 27.5 |
| 16 (3.8) | 4.8 | 8.19 | 12.6 | 44 | 55 | 15.3 | 28.6 |
| Mean | 6.6 | 8.6 | 13.0 | 46.0 | 54.5 | 15.0 | 28.2 |
| STD | 1.9 | 0.3 | 0.3 | 1.6 | 0.6 | 0.2 | 0.5 |
| | | | | | | | |
| Group 5 | | | | | | | |
| 17 (4.5) | 3.1 | 8.48 | 12.6 | 46 | 54 | 14.9 | 27.5 |
| 18 (4.6) | 5.7 | 9.12 | 13.7 | 48 | 54 | 15 | 28.5 |
| 19 (4.7) | 5.3 | 8.58 | 13 | 44.5 | 55 | 15.2 | 29.2 |
| 20 (4.8) | 5.3 | NA | NA | 40 | NA | NA | NA |
| Mean | 4.9 | 8.7 | 13.1 | 44.6 | 54.3 | 15.0 | 28.4 |
| STD | 1.2 | 0.3 | 0.6 | 3.4 | 0.6 | 0.2 | 0.9 |
| | | | | | | | |
| Group 6 | | | | | | | |
| 21 (1.1) | 3.5 | 9.36 | 13.5 | 37.6 | 40 | 14.4 | 35.9 |
| 22 (1.2) | 6.9 | 8.93 | 13.6 | 37.3 | 42 | 15.3 | 36.6 |
| 23 (1.3) | 3.6 | 8.3 | 12.5 | 35.3 | 43 | 15.1 | 35.5 |
| 24 (1.4) | NA | NA | NA | NA | NA | NA | NA |
| Mean | 4.7 | 8.9 | 13.2 | 36.7 | 41.7 | 14.9 | 36.0 |
| STD | 1.9 | 0.5 | 0.6 | 1.3 | 1.5 | 0.5 | 0.6 |
| | | | | | | | |
| Group 7 | | | | | | | |
| 25 (2.1) | 4 | NA | NA | 40 | NA | NA | NA |
| 26 (2.2) | 7.4 | 9.12 | 13.2 | 38.5 | 42 | 14.5 | 34.3 |
| 27 (2.3) | 4.5 | 8.19 | 12.1 | 34.5 | 42 | 14.8 | 35.1 |
| 28 (2.4) | 5.8 | 8.25 | 12.3 | 34.1 | 41 | 14.9 | 36.1 |
| Mean | 5.4 | 8.5 | 12.5 | 36.8 | 41.7 | 14.7 | 35.2 |
| STD | 1.5 | 0.5 | 0.6 | 2.9 | 0.6 | 0.2 | 0.9 |
| | | | | | | | |
| Group 8 | | | | | | | |
| 29 (3.1) | 5.1 | 8.53 | 12.6 | 34.9 | 41 | 14.7 | 36 |
| 30 (3.2) | 7.6 | 8.42 | 13 | 36.1 | 43 | 15.4 | 35.9 |
| 31 (3.3) | 3.4 | 8.45 | 12.6 | 34.9 | 41 | 14.9 | 36.1 |
| 32 (3.4) | 6.1 | 8.11 | 12.3 | 34.8 | 43 | 15.2 | 35.5 |
| Mean | 5.6 | 8.4 | 12.6 | 35.2 | 42.0 | 15.1 | 35.9 |
| STD | 1.8 | 0.2 | 0.3 | 0.6 | 1.2 | 0.3 | 0.3 |

**Table LI: WT1 Dose Titration Study**
**Clinical Chemistry and Hematology Analysis**

| Animal # | K/uL WBC | M/uL RBC | g/dl Hg. | % HCT | fL MCV | pg MCH | % MCHC |
|---|---|---|---|---|---|---|---|
| Normal | 5.4-16.0 | 6.7-12.5 | 10.2-16.6 | 32-54 | 31-62 | 9.2-20.8 | 22.0-35.5 |
| Group 9 | | | | | | | |
| 33 (4.1) | NA | NA | NA | NA | NA | NA | NA |
| 34 (4.2) | 4.5 | 8.63 | 12.8 | 36.2 | 42 | 14.8 | 35.2 |
| 35 (4.3) | 3.9 | 8.85 | 13 | 36.6 | 41 | 14.7 | 35.6 |
| 36 (4.4) | 4.7 | 8.14 | 12.3 | 33.8 | 42 | 15.1 | 36.3 |
| Mean | 4.4 | 8.5 | 12.7 | 35.5 | 41.7 | 14.9 | 35.7 |
| STD | 0.4 | 0.4 | 0.4 | 1.5 | 0.6 | 0.2 | 0.6 |

**Table LI (cont'd): WT1 Dose Titration Study**
**Clinical Chemistry and Hematology Analysis**

| Animal # | yes/no Plt. clump | K/uL Platelets | Abs. Baso | Abs. Eos | Abs. Bands | Abs. Polys | Abs. Lymph | Abs. Mono |
|---|---|---|---|---|---|---|---|---|
| Normal | no | 150-1500 | 0.0-0.15 | 0.0-0.51 | 0.0-0.32 | 8.0-42.9 | 8.0-18.0 | 0.0-1.5 |
| Group 1 | | | K/uL | K/uL | K/uL | K/uL | K/uL | K/uL |
| 1 (0) | yes | 726 | 0 | 56 | 0 | 336 | 5208 | 0 |
| 2 (0) | no | 860 | 0 | 0 | 0 | 55 | 5445 | 0 |
| 3 (0) | no | 875 | 0 | 375 | 0 | 525 | 6525 | 75 |
| 4 (0) | yes | 902 | 0 | 0 | 0 | 156 | 3744 | 0 |
| Mean | | 840.8 | 0.0 | 107.8 | 0.0 | 268.0 | 5230.5 | 18.8 |
| STD | | 78.4 | 0.0 | 180.1 | 0.0 | 207.0 | 1144.8 | 37.5 |
| Group 2 | | | | | | | | |
| 5 (1.5) | no | 1193 | 0 | 132 | 0 | 792 | 5214 | 462 |
| 6 (1.6) | no | 1166 | 0 | 52 | 0 | 624 | 4472 | 52 |
| 7 (1.7) | no | 1087 | 0 | 234 | 0 | 1170 | 6396 | 0 |
| 8 (1.8) | yes | NA | 0 | 126 | 0 | 126 | 5922 | 126 |
| Mean | | 1148.7 | 0.0 | 136.0 | 0.0 | 678.0 | 5501.0 | 160.0 |
| STD | | 55.1 | 0.0 | 74.8 | 0.0 | 433.1 | 840.5 | 207.9 |
| Group 3 | | | | | | | | |
| 9 (2.5) | no | 705 | 0 | 166 | 0 | 664 | 7387 | 83 |
| 10 (2.6) | no | 1140 | 0 | 150 | 0 | 500 | 4350 | 0 |
| 11 (2.7) | no | 952 | 0 | 120 | 0 | 680 | 3200 | 0 |
| 12 (2.8) | yes | NA | 0 | 164 | 0 | 656 | 7216 | 164 |
| Mean | | 932.3 | 0.0 | 150.0 | 0.0 | 625.0 | 5538.3 | 61.8 |
| STD | | 218.2 | 0.0 | 21.2 | 0.0 | 83.9 | 2090.6 | 78.6 |
| Group 4 | | | | | | | | |
| 13 (3.5) | no | 785 | 0 | 488 | 0 | 732 | 4636 | 244 |
| 14 (3.6) | yes | 973 | 0 | 0 | 0 | 488 | 5307 | 305 |

| | | | | | | | | |
|---|---|---|---|---|---|---|---|---|
| 15 (3.7) | yes | 939 | 0 | 465 | 0 | 558 | 7812 | 465 |
| 16 (3.8) | yes | 1622 | 0 | 192 | 0 | 480 | 4080 | 48 |
| Mean | | 1079.8 | 0.0 | 286.3 | 0.0 | 564.5 | 5458.8 | 265.5 |
| STD | | 370.6 | 0.0 | 233.4 | 0.0 | 117.0 | 1647.1 | 172.4 |
| Group 5 | | | | | | | | |
| 17 (4.5) | no | 892 | 0 | 31 | 0 | 620 | 2449 | 0 |
| 18 (4.6) | yes | 966 | 57 | 114 | 0 | 855 | 4674 | 0 |
| 19 (4.7) | yes | 883 | 0 | 53 | 0 | 742 | 4452 | 53 |
| 20 (4.8) | yes | NA | 0 | 106 | 0 | 53 | 5141 | 0 |
| Mean | | 913.7 | 14.3 | 76.0 | 0.0 | 567.5 | 4179.0 | 13.3 |
| STD | | 45.5 | 28.5 | 40.4 | 0.0 | 356.2 | 1188.5 | 26.5 |

**Table LI (cont'd): WT1 Dose Titration Study Clinical Chemistry and Hematology Analysis**

| Animal # | yes/no Plt. clump | K/uL Platelets | Abs. Baso | Abs. Eos | Abs. Bands | Abs. Polys | Abs. Lymph | Abs. Mono |
|---|---|---|---|---|---|---|---|---|
| Normal | no | 150-1500 | 0.0-0.15 | 0.0-0.51 | 0.0-0.32 | 8.0-42.9 | 8.0-18.0 | 0.0-1.5 |
| Group 6 | | | | | | | | |
| 21 (1.1) | yes | 784 | 0 | 35 | 0 | 385 | 2870 | 210 |
| 22 (1.2) | yes | 806 | 0 | 69 | 0 | 207 | 6486 | 138 |
| 23 (1.3) | yes | 790 | 0 | 180 | 0 | 396 | 2988 | 36 |
| 24 (1.4) | NA | NA | NA | NA | NA | NA | NA | NA |
| Mean | | 793.3 | 0.0 | 94.7 | 0.0 | 329.3 | 4114.7 | 128.0 |
| STD | | 11.4 | 0.0 | 75.8 | 0.0 | 106.1 | 2054.5 | 87.4 |
| Group 7 | | | | | | | | |
| 25 (2.1) | yes | NA | 0 | 80 | 0 | 200 | 3720 | 0 |
| 26 (2.2) | yes | 753 | 0 | 0 | 0 | 518 | 6734 | 148 |
| 27 (2.3) | yes | 725 | 0 | 90 | 0 | 225 | 4140 | 45 |
| 28 (2.4) | yes | 792 | 0 | 232 | 0 | 754 | 4814 | 0 |
| Mean | | 756.7 | 0.0 | 100.5 | 0.0 | 424.3 | 4852.0 | 48.3 |
| STD | | 33.7 | 0.0 | 96.5 | 0.0 | 263.0 | 1333.1 | 69.8 |
| Group 8 | | | | | | | | |
| 29 (3.1) | yes | 784 | 0 | 153 | 0 | 561 | 4233 | 153 |
| 30 (3.2) | yes | 512 | 0 | 152 | 0 | 304 | 6992 | 152 |
| 31 (3.3) | yes | 701 | 0 | 0 | 0 | 238 | 3094 | 68 |
| 32 (3.4) | yes | 631 | 0 | 305 | 0 | 305 | 5368 | 122 |
| Mean | | 657.0 | 0.0 | 152.5 | 0.0 | 352.0 | 4921.8 | 123.8 |
| STD | | 115.1 | 0.0 | 124.5 | 0.0 | 142.8 | 1663.3 | 39.9 |
| Group 9 | | | | | | | | |
| 33 (4.1) | NA | NA | NA | NA | NA | NA | NA | NA |
| 34 (4.2) | yes | 724 | 0 | 125 | 0 | 540 | 3780 | 45 |
| 35 (4.3) | yes | 758 | 0 | 117 | 0 | 429 | 3315 | 39 |
| 36 (4.4) | yes | 808 | 0 | 47 | 0 | 329 | 4089 | 235 |
| Mean | | 763.3 | 0.0 | 96.3 | 0.0 | 432.7 | 3728.0 | 106.3 |
| STD | | 42.3 | 0.0 | 42.9 | 0.0 | 105.5 | 389.6 | 111.5 |

**Table LI (cont'd): WT1 Dose Titration Study**
**Clinical Chemistry and Hematology Analysis**

| Animal # | mg/dl BUN | mg/dl Creatinine | g/dl T. protein | g/dl Albumin | g/dl Globulin | mg/dl T. Bilirubin |
|---|---|---|---|---|---|---|
| Normal | 13.9-28.3 | 0.3-1.0 | 4.0-8.6 | 2.5-4.8 | 1.5-3.8 | 0.10-0.90 |
| Group 1 | | | | | | |
| 1 (0) | NA | NA | NA | NA | NA | NA |
| 2 (0) | 28 | 0.5 | 4.9 | 3.7 | 1.2 | 0.3 |
| 3 (0) | 25 | 0.5 | 4.9 | 3.8 | 1.1 | 0.2 |
| 4 (0) | 27 | 0.5 | 4.7 | 3.7 | 1 | 0.2 |
| Mean | 26.7 | 0.5 | 4.8 | 3.7 | 1.1 | 0.2 |
| STD | 1.5 | 0.0 | 0.1 | 0.1 | 0.1 | 0.1 |
| Group 2 | | | | | | |
| 5 (1.5) | 34 | 0.5 | 4.6 | 3.6 | 1 | 0.2 |
| 6 (1.6) | 31 | 0.4 | 4.6 | 3.3 | 1.3 | 0.2 |
| 7 (1.7) | 34 | 0.6 | 4.9 | 4 | 0.9 | 0.3 |
| 8 (1.8) | NA | NA | NA | NA | NA | NA |
| Mean | 33.0 | 0.5 | 4.7 | 3.6 | 1.1 | 0.2 |
| STD | 1.7 | 0.1 | 0.2 | 0.4 | 0.2 | 0.1 |
| Group 3 | | | | | | |
| 9 (2.5) | NA | NA | NA | NA | NA | NA |
| 10 (2.6) | 33 | 0.5 | 4.6 | 3.6 | 1 | 0.3 |
| 11 (2.7) | NA | NA | NA | NA | NA | NA |
| 12 (2.8) | 31 | 0.5 | 4.8 | 3.7 | 1.1 | 0.2 |
| Mean | 32.0 | 0.5 | 4.7 | 3.7 | 1.1 | 0.3 |
| STD | 1.4 | 0.0 | 0.1 | 0.1 | 0.1 | 0.1 |
| Group 4 | | | | | | |
| 13 (3.5) | 32 | 0.7 | 4.6 | 3.4 | 1.2 | 0.2 |
| 14 (3.6) | 34 | 0.4 | 4.8 | 3.8 | 1 | 0.2 |
| 15 (3.7) | 30 | 0.4 | 4.7 | 3.4 | 1.3 | 0.2 |
| 16 (3.8) | 24 | 0.3 | 5.1 | 3.8 | 1.3 | 0.2 |
| Mean | 30.0 | 0.5 | 4.8 | 3.6 | 1.2 | 0.2 |
| STD | 4.3 | 0.2 | 0.2 | 0.2 | 0.1 | 0.0 |
| Group 5 | | | | | | |
| 17 (4.5) | 22 | 0.4 | 4.6 | 3.3 | 1.3 | 0.2 |
| 18 (4.6) | 31 | 0.5 | 4.9 | 3.7 | 1.2 | 0.2 |
| 19 (4.7) | 23 | 0.6 | 4.8 | 3.6 | 1.2 | 0.2 |
| 20 (4.8) | 28 | 0.5 | 4.5 | 3.4 | 1.1 | 0.2 |
| Mean | 26.0 | 0.5 | 4.7 | 3.5 | 1.2 | 0.2 |
| STD | 4.2 | 0.1 | 0.2 | 0.2 | 0.1 | 0.0 |

**Table LI (cont'd): WT1 Dose Titration Study**
**Clinical Chemistry and Hematology Analysis**

| Animal # | mg/dl BUN | mg/dl Creatinine | g/dl T. protein | g/dl Albumin | g/dl Globulin | mg/dl T. Bilirubin |
|---|---|---|---|---|---|---|
| Normal | 13.9-28.3 | 0.3-1.0 | 4.0-8.6 | 2.5-4.8 | 1.5-3.8 | 0.10-0.90 |
| Group 6 | | | | | | |
| 21 (1.1) | 28 | 0.3 | 5.1 | 3.4 | 1.7 | 0.2 |
| 22 (1.2) | 36 | 0.3 | 5.1 | 3.8 | 1.3 | 0.2 |
| 23 (1.3) | 32 | 0.4 | 4.9 | 3.5 | 1.4 | 0.1 |
| 24 (1.4) | NA | NA | NA | NA | NA | NA |
| Mean | 32.0 | 0.3 | 5.0 | 3.6 | 1.5 | 0.2 |
| STD | 4.0 | 0.1 | 0.1 | 0.2 | 0.2 | 0.1 |
| Group 7 | | | | | | |
| 25 (2.1) | 32 | 0.2 | 5 | 3.4 | 1.6 | 0.2 |
| 26 (2.2) | 24 | 0.3 | 4.2 | 2.8 | 1.4 | 0.1 |
| 27 (2.3) | 28 | 0.3 | 4.8 | 3.2 | 1.6 | 0.2 |
| 28 (2.4) | 27 | 0.3 | 5 | 3.4 | 1.6 | 0.1 |
| Mean | 27.8 | 0.3 | 4.8 | 3.2 | 1.6 | 0.2 |
| STD | 3.3 | 0.0 | 0.4 | 0.3 | 0.1 | 0.1 |
| Group 8 | | | | | | |
| 29 (3.1) | 32 | 0.3 | 4.9 | 3.3 | 1.6 | 0.2 |
| 30 (3.2) | NA | NA | NA | NA | NA | NA |
| 31 (3.3) | 18 | 0.3 | 4.8 | 3.1 | 1.7 | 0.2 |
| 32 (3.4) | 26 | 0.2 | 4.2 | 2.9 | 1.3 | 0 |
| Mean | 25.3 | 0.3 | 4.6 | 3.1 | 1.5 | 0.1 |
| STD | 7.0 | 0.1 | 0.4 | 0.2 | 0.2 | 0.1 |
| Group 9 | | | | | | |
| 33 (4.1) | 25 | 0.2 | 4.1 | 2.7 | 1.4 | 0.3 |
| 34 (4.2) | NA | NA | NA | NA | NA | NA |
| 35 (4.3) | 23 | 0.2 | 4.7 | 3.1 | 1.6 | 0.2 |
| 36 (4.4) | 29 | 0.3 | 4.7 | 3.2 | 1.5 | 0.3 |
| Mean | 25.7 | 0.2 | 4.5 | 3.0 | 1.5 | 0.3 |
| STD | 3.1 | 0.1 | 0.3 | 0.3 | 0.1 | 0.1 |

Abbreviations: WBC: white blood cells; RBC: red blood cells; Hg.: hemoglobin; HCT: hematocrit ; MCV: Mean corpuscular volume; MCH: mean corpuscular hemoglobin; MCHC: mean corpuscular hemoglobin concentration; Plt.: platelets; Abs.: Absolute; Baso: basophils; Eos: eosinophils; Abs. Bands: immature neutrophils ; Polys: polymorphonuclear cells; Lymph: lymphocytes; Mono: monocytes; BUN: blood urea nitrogen

EXAMPLE 14

ELICITATION OF HUMAN WT1-SPECIFIC T-CELL RESPONSES BY WHOLE GENE IN VITRO PRIMING

This example demonstrates that WT1 specific T-cell responses can be generated from the blood of normal individuals.

Dendritic cells (DC) were differentiated from monocyte cultures derived from PBMC of normal donors by growth for 4-10 days in RPMI medium containing 10% human serum, 50 ng/ml GMCSF and 30 ng/ml IL-4. Following culture, DC were infected 16 hours with recombinant WT1-expressing vaccinia virus at an M.O.I. of 5, or for 3 days with recombinant WT1-expressing adenovirus at an M.O.I. of 10 (Figures 21 and 22). Vaccinia virus was inactivated by U.V. irradiation. CD8+ T-cells were isolated by positive selection using magnetic beads, and priming cultures were initiated in 96-well plates. Cultures were restimulated every 7-10 days using autologous dendritic cells adeno or vaccinia infected to express WT1. Following 3-6 stimulation cycles, CD8+ lines could be identified that specifically produced interferon-gamma when stimulated with autologous-WT1-expressing dendritic cells or fibroblasts. The WT1-specific activity of these lines could be maintained following additional stimulation cycles. These lines were demonstrated to specifically recognize adeno or vaccinia WT1 infected autologous dendritic cells but not adeno or vaccinia EGFP-infected autologous dendritic cells by Elispot assays (Figure 23).

EXAMPLE 15

FORMULATION OF RA12-WT1 FOR INJECTION: USE OF EXCIPIENTS TO STABILIZE LYOPHILIZED PRODUCT

This example describes the formulation that allows the complete solubilization of lyophilized Ra12-WT1.

The following formulation allowed for the recombinant protein Ra12-WT1 to be dissolved into an aqueous medium after being lyophilized to dryness:

Recombinant Ra12-WT1 concentration: 0.5 - 1.0 mg/ml; Buffer: 10-20 mM Ethanolamine, pH 10.0; 1.0 - 5.0 mM Cysteine; 0.05 % Tween-80 (Polysorbate-80); Sugar: 10% Trehalose (T5251, Sigma, MO) 10% Maltose (M9171, Sigma, MO) 10% Sucrose (S7903, Sigma, MO) 10% Fructose (F2543, Sigma, MO) 10% Glucose (G7528, Sigma, MO).

The lyophilized protein with the sugar excipient was found to dissolve significantly more than without the sugar excipient. Analysis by coomassie stained SDS-PAGE showed no signs of remaining solids in the dissolved material.

EXAMPLE 16

FORMULATION OF A WT1 PROTEIN VACCINE

This example describes the induction of WT1-specific immune responses following immunization with WT1 protein and 2 different adjuvant formulations.

According to this example, WT1 protein in combination with MPL-SE induces a strong Ab and Interferon-y (IFN-γ) response to WT1. Described in detail below are the methods used to induce WT1 specific immune responses following WT1 protein immunization using MPL-SE or Enhanzyn as adjuvant in C57/B6 mice.

C57BL/6 mice were immunized with 20 μg rRa12-WT1 combined with either MPL-SE or Enhanzyn adjuvants. One group of control mice was immunized with rRa12-WT1 without adjuvant and one group was immunized with saline alone. Three intramuscular (IM) immunizations were given, three weeks apart. Spleens and sera were harvested 2 weeks post-final immunization. Sera were analyzed for antibody responses by ELISA on plates coated with Ra12-WT1 fusion, Ra12 or WT1TRX. Similar levels of IgG2a and IgG1 antibody titers were observed in mice immunized with Ra12-WT1+MPL-SE and Ra12-WT1+Enhanzyn. Mice immunized with rRa12-WT1 without adjuvant showed lower levels of IgG2a antibodies.

CD4 responses were assessed by measuring Interferon-γ production following stimulation of splenocytes *in vitro* with rRa12-WT1, rRa12 or with WT1 peptides p6, p117 and p287. Both adjuvants improved the CD4 responses over mice immunized with rRA12-WT1 alone. Additionally, the results indicate that rRA12-WT1+MPL-SE induced a stronger CD4 response than did rRA12-WT1+Enhanzyn. IFN-γ OD readings ranged from 1.4-1.6 in the mice immunized with rRA12-WT1+MPL-SE as compared to 1-1.2 in the mice immunized with rRA12-WT1+Enhanzyn. Peptide responses were only observed against p117, and then only in mice immunized with rRa12-WT1+MPL-SE. Strong IFN-γ responses to the positive control, ConA, were observed in all mice. Only responses to ConA were observed in the negative control mice immunized with saline indicating that the responses were specific to rRA12-WT1.

EXAMPLE 17

CONSTRUCTION OF A RANDOMLY MUTATED WT1 LIBRARY

The nucleic acid sequence of human WT1 was randomly mutated using a polymerase chain reaction method in the presence of 8-oxo dGTP and dPTP (journal of Molecular Biology 1996; 255:589-603). The complete unspliced human WT1 gene is disclosed in SEQ ID NO:380 and the corresponding protein sequence is set forth in SEQ ID NO:

404. A splice variant of WT1 was used as a template for the PCR reactions and is disclosed in SEQ ID NOs:381 (DNA) and 408 (protein). Conditions were selected so that the frequency of nucleic acid alterations led to a targeted change in the amino acid sequence, usually 5-30% of the PCR product. The mutated PCR product was then amplified in the absence of the nucleotide analogues using the four normal dNTPs. This PCR product was subcloned into mammalian expression vectors and viral vectors for immunization. This library, therefore, contains a mixed population of randomly mutated WT1 clones. Several clones were selected and sequenced. The mutated WT1 variant DNA sequences are disclosed in SEQ ID NOs:377-379 and the predicted amino acid sequences of the variants are set forth in SEQ ID NOs: 405-407. These altered sequences, and others from the library, can be used as immunogens to induce stronger T cell responses against WT1 protein in cancer cells.

EXAMPLE 18

CONSTRUCTION OF WT1-LAMP FUSIONS

A tripartite fusion was constructed using the polymerase chain reaction and synthetic oligonucleotides containing the desired junctions of human lysosomal associated membrane protein-1 (LAMP-1) and a splice variant of the human WT1 sequence. The splice variant of WT1 and the LAMP-1 sequence used for these fusions are disclosed in SEQ ID NOs:381 and 383. Specifically, the signal peptide of LAMP-1 (base pairs 1-87 of LAMP) was fused to the 5-prime end of the human WT1 open reading frame (1,290 base pairs in length), then the transmembrane and cytoplasmic domain of LAMP-1 (base pairs 1161 to 1281 of LAMP) was fused to the 3-prime end of the WT1 sequence. The sequence of the resulting WT1-LAMP construct is set forth in SEQ ID NO:382 (DNA) and SEQ ID NO:409 (protein). The construct was designed so that when it is expressed in eukaryotic cells, the signal peptide directs the protein to the endoplasmic reticulum (ER) where the localization signals in the transmembrane and cytoplasmic domain of LAMP-1 direct transport of the fusion protein to the lysosomal location where peptides are loaded on to Class II MHC molecules.

EXAMPLE 19

CONSTRUCTION OF WT1-UBIQUITIN FUSIONS FOR ENHANCED MHC CLASS I PRESENTATION

The human ubiquitin open reading frame (SEQ ID NO:384) was mutated such that the nucleotides encoding the last amino acid encode an alanine instead of a glycine. This mutated open reading frame was cloned in frame just upstream of the first codon of a splice variant of human WT1 (SEQ ID NOs:381 and 408, DNA and protein, respectively). The G->A mutation prevents co-translational cleavage of the nacent protein by the proteases that normally process poly-ubiquitin during translation. The DNA and predicted amino acid sequence for the resulting contruct are set forth in SEQ ID NOs:385 and 410, respectively. The resulting protein demonstrated decreased cellular cytotoxicity when it was expressed in human cells. Whereas it was not possible to generate stable lines expressing native WT1, cell lines expressing the fusion protein were readily obtained. The resulting protein is predicted to be targeted to the proteosome by virtue of the added ubiquitin molecule. This should result in more efficient recognition of the protein by WT1 specific CD8+ T cells.

EXAMPLE 20

CONSTRUCTION OF AN ADENOVIRUS VECTOR EXPRESSING HUMAN WT1

A splice variant of human WT1 (SEQ ID NO:381) was cloned into an E1 and E3 deleted adenovirus serotype 5 vector. The expression of the WT1 gene is controlled by the CMV promoter mediating high levels of WT1 protein expression. Infection of human cells with this reagent leads to a high level of expression of the WT1 protein. The antigenic nature of the adenoviral proteins introduced into the host cell during and produced at low levels subsequent to infection can act to increase immune surveillance and immune recognition of WT1 as an immunological target. This vector can be also used to generate immune responses against the WT1 protein when innoculated into human subjects. If these subjects are positive for WT1 expressing tumor cells the immune response could have a theraputic or curative effect on the course of the disease.

EXAMPLE 21

CONSTRUCTION OF A VACCINIA VIRUS VECTOR EXPRESSING HUMAN WT1

A splice variant of the full length human WT1 gene (SEQ ID NO:381) was cloned into the thymidine kinase locus of the Western Reserve strain of the vaccinia virus using the pSCl I shuttle vector. The WT1 gene is under the control of a hybrid vaccinia virus promoter that mediates gene expression throughout the course of vaccinia virus infection. This reagent can be used to express the WT1 protein in human cells in vivo or in vitro. WT1 is a self protein that is overexpressed on some human tumor cells. Thus, immunological responses to WT1 delivered as a protein are unlikely to lead to Major Histocompatibility Class I (MHC class I)-mediated recognition of WT1. However, expression of the protein in the intracellular compartment by the vaccinia virus vector will allow high level MHC class I presentation and recognition of the WT1 protein by CD8+ T cells. Expression of the WT1 protein by the vaccinia virus vector will also lead to presentation of WT1 peptides in the context of MHC class II and thus to recognition by CD4+ T cells.

The uses of this invention include its use as a cancer vaccine. Immunization of human subjects bearing WT1 positive tumors could lead to a theraputic or curative response. The expression of WT1 within the cell will lead to recognition of the protein by both CD4 and CD8 positive T cells.

EXAMPLE 22

GENERATION OF WT1-SPECIFIC CD8+ T-CELL CLONES USING WHOLE GENE PRIMING

Dendritic cells (DC) were differentiated from monocyte cultures derived from PBMC of normal donors by growth for 4-6 days in RPMI medium containing 10% human serum, 50 ng/ml GM-CSF and 30 ng/ml IL-4. Following culture, DC were infected 16 hours with recombinant WT1-expressing vaccinia virus (described in Example 21) at a multiplicity of infection (MOI) of 5 or for 3 days with recombinat WT1-expressing adenovirus at an MOI of 10. Vaccinia virus was inactivated by U.V. irradiation. CD8+ T-cells were isolated by negative depletion using magnetic beads, and priming cultures were initiated in 96-well plates. Cultures were restimulated every 7-10 days using autologous dendritic cells infected with adeno or vaccinia virus engineered to express WT1. Following 4-5 stimulation cycles, CD8+ T-cell lines could be identified that specifically produced interferon-gamma when stimulated with autologous-WT1 expressing dendritic cells or fibroblasts. These lines were cloned and demonstrated to specifically recognize WT1 transduced autologous fibroblasts but not EGFP transduced fibroblasts by Elispot assays.

The Wilms' tumor (WT1) gene participates in leukemogenesis and is overexpressed in most human leukemias as well as in several solid tumors. Previous studies in humans have demonstrated the presence of WT1 specific antibody (Ab) responses in 16/63 (25%) of AML and in 15/81 (19%) of CML patients studied. Previous studies in mice have shown that WT1 peptide based vaccines elicit WT1 specific Ab, Th and CTL responses. The use of peptides as vaccines in humans is limited by their HLA restriction and the tendency to elicit peptide specific responses and only in a minority of patients tumor specific CTL. The advantages of whole gene immunization are that several helper and CTL epitopes can be included in a single vaccine, thus not restricting the vaccine to specific HLA types. The data disclosed herein demonstrate the induction of WT1 specific immune responses using whole gene in vitro priming. and that WT1 specific CD8+ T-cell clones can be generated. Given that existent immunity to WT1 is present in some patients with leukemia and that murine and human WT1 are 96% identical at the amino acid level and vaccination to WT1 protein, DNA or peptides can elicit WT1 specific Ab, and cellular T-cell responses in mice without toxicity to normal tissues in mice, these human in vitro priming experiments provide further validation of WT1 as a tumor/leukemia vaccine. Furthermore, the ability to generate WT1 specific CD8+ T-cell clones may lead to the treatment of malignancies associated with WT1 overexpression using genetically engineered T-cells.

EXAMPLE 23

RECOMBINANT CONSTRUCTS FOR CLINICAL MANUFACTURING OF WT1

Five constructs were made as described in detail below, for the production of clinical grade WT1.

**Design of Ra12/WT-E (SEQ ID NOs:388 (cDNA) and 391 (protein)) and WT-1 E (SEQ ID NOs:386 (cDNA) and 395 (protein)) with No His tag:**

The WT-1 E reading frame was PCR amplified with the following primers for the non-His non fusion construct:

PDM-780 (SEQ ID NO:396) 5' gacgaaagcatatgcactccttcatcaaac 3' Tm 60°C
PDM-779 (SEQ ID NO:397) 5' cgcgtgaattcatcactgaatgcctctgaag 3' Tm 63°C

The following PCR cycling conditions were used: 10μl 10X Pfu buffer, 1μl 10mM dNTPs, 2μl 10μM each oligo, 83μl sterile water 1.5μl Pfu DNA polymerase (Stratagene, La Jolla, CA), 50 ηg DNA (pPDMRa12 WT-1 No His). The reaction was denatured initially at 96°C for 2 minutes, followed by 40 cylces of 96°C for 20 seconds, 62°C for 15 seconds, and 72°C for 1 minute and 40 seconds. This was followed by a final extension of 72°C for 4 minutes. The PCR product was digested with NdeI and EcoRI and cloned into pPDM His (a modified pET28 vector) that had been digested with NdeI and EcoRI. The construct was confirmed through sequence analysis and then transformed into BLR (DE3) pLys S and HMS 174 (DE3) pLys S cells. This construct - pPDM WT-1 E was then digested with NcoI and XbaI and used as the vector backbone for the NcoI and XbaI insert from pPDM Ra12 WT-1 F (see below). The construct was confirmed through sequence analysis and then tranformed into BLR (DE3) pLys S and HMS 174 (DE3) pLys S cells. Protein expression was confirmed by Coomassie stained SDS-PAGE and N-terminal protein sequence analysis.

**Design of Ra12-WT-1-F (a.a. 1-281) with No His tag (SEQ ID NOs:389 (cDNA) and 393 (protein)):**

The Ra12 WT-1 reading frame was PCR amplified with the following primers:

PDM-777 (SEQ ID NO:398) 5' cgataagcatatgacggccgcgtccgataac 3' Tm 66°C

PDM-779 (SEQ ID NO:399) 5' cgcgtgaattcatcactgaatgcctctgaag 3' Tm 63°C

The following PCR cycling conditions were used: 10μl 10X Pfu buffer, 1μl 10mM dNTPs, 2μl 10μM each oligo, 83μl sterile water 1.5μl Pfu DNA polymerase (Stratagene, La Jolla, CA), 50 ηg DNA (pPDMRa12 WT-1 No His). The reaction was denatured initially at 96°C for 2 minutes, followed by 40 cylces of 96°C for 20 seconds, 58°C for 15 seconds, and 72°C for 3 minutes. This was followed by a final extension of 72°C for 4 minutes. The PCR product was digested with NdeI and cloned into pPDM His that had been digested with NdeI and Eco72I. The sequence was confirmed through sequence analysis and then transformed into BLR (DE3) pLys S and HMS 174 (DE3) pLysS cells. Protein expression was confirmed by Coomassie stained SDS-PAGE and N-terminal protein sequence analysis.

**Design of Ra12-WT-1 with No His tag (SEQ ID NOs:390 (cDNA) and 392 (protein)):**

The Ra12 WT-1 reading frame was PCR amplified with the following primers:

PDM-777 (SEQ ID NO:400) 5' cgataagcatatgacggccgcgtccgataac 3' Tm 66°C

PDM-778 (SEQ ID NO:401) 5' gtctgcagcggccgctcaaagcgccagc 3' Tm 70°C

The following PCR cycling conditions were used: 10μl 10X Pfu buffer, 1μl 10mM dNTPs, 2μl 10μM each oligo, 83μl sterile water 1.5μl Pfu DNA polymerase (Stratagene, La Jolla, CA), 50 ηg DNA (pPDMRa12 WT-1 No His). The reaction was denatured initially at 96°C for 2 minutes, followed by 40 cylces of 96°C for 20 seconds, 68°C for 15 seconds, and 72°C for 2 minutes and 30 seconds. This was followed by a final extension of 72°C for 4 minutes. The PCR product was digested with NotI and NdeI and cloned into pPDM His that had been digested with NdeI and NotI. The sequence was confirmed through sequence anaysis and then transformed into BLR (DE3) pLys S and HMS 174 (DE3) pLysS cells. Protein expression was confirmed by Coomassie stained SDS-PAGE and N-terminal protein sequence analysis.

**Design of WT-1 C (a.a. 69-430) in *E. coli* without His tag (SEQ ID NOs:387 (cDNA) and 394 (protein)):**

The WT-1 C reading frame was PCR amplified with the following primers:

PDM-780 (SEQ ID NO:402) 5' gacgaaagcatatgcactccttcatcaaac 3' Tm 60°C

PDM-778 (SEQ ID NO:403) 5' gtctgcagcggccgctcaaagcgccagc 3' Tm 70°C

The following PCR cycling conditions were used: 10μl 10X Pfu buffer, 1μl 10mM dNTPs, 2μl 10μM each oligo, 83μl sterile water 1.5μl Pfu DNA polymerase (Stratagene, La Jolla, CA), 50 ηg DNA (pPDMRa12 WT-1 No His). The reaction was denatured initially at 96°C for 2 minutes, followed by 40 cylces of 96°C for 20 seconds, 62°C for 15 seconds, and 72°C for 2 minutes. This was followed by a final extension of 72°C for 4 minutes. The PCR product was digested with NdeI and cloned into pPDM His that had been digested with NdeI and Eco72I. The sequence was confirmed through sequence analysis and then transformed into BLR (DE3) pLys S and HMS 174 (DE3) pLys S cells. Protein expression was confirmed by Coomassie stained SDS-PAGE and N-terminal protein sequence analysis.

EXAMPLE 24

GENERATION OF WT1-SPECIFIC CD8+ T CELL CLONES USING WHOLE GENE PRIMING AND IDENTIFICATION OF AN HLA-A2-RESTRICTED WT1 EPITOPE

In this example, Adeno and Vaccinia virus delivery vehicles were used to generate WT1-specific T cell lines. A T cell clone from the line was shown to be specific for WT1 and further, the epitope recognized by this clone was identified.

Dendritic cells (DC) were differentiated from monocyte cultures derived from PBMC of normal donors by growth for 4-6 days in RPMI medium containing 10% human serum, 50 ng/ml GM-CSF and 30 ng/ml IL-4. Following culture, DC were infected 16 hours with recombinant WT1-expressing vaccinia virus at a multiplicity of infection (MO1) of 5 or for 2-3 days with recombinant WT1-expressing adeno virus at an MOI of 3-10. Vaccinia virus was inactivated by U.V. irradiation. CD8+ T-cells were isolated by negative depletion using antibodies to CD4, CD14, CD16, CD19 and CD56+ cells, followed by magnetic beads specific for the Fc portion of these Abs.

Priming cultures were initiated in 96-well plates. Cultures were restimulated every 7-14 days using autologous dendritic cells infected with adeno or vaccinia virus engineered to express WT1. Following 4-5 stimulation cycles, CD8+ T cell lines could be identified that specifically produced interferon-γ (IFN-γ) when stimulated with autologous-WT1 expressing dendritic cells or fibroblasts. These lines were cloned and demonstrated to specifically recognize WT1 transduced autologous fibroblasts but not control transduced fibroblasts by Elispot assays.

To further analyze HLA restriction of these WT1 specific CD8+ T-cell clones, fibroblasts derived from an additional donor (D475), sharing only the HLA-A2 allele with the donor (D349) from which the T-cell clone was established, were transduced with WT1. ELISPOT analysis demonstrated recognition of these D475 target cells by the T-cell clone. To further demonstrate HLA A2 restriction and demonstrate that this epitope is expressed by tumor cells "naturally" overxpressing WT1 (as part of their malignant transformation), the leukemia cell line K562 was tested. K562 was transduced with the HLA A2 molecule, and HLA-A2 negative K562 cells were used as controls for nonspecific IFN-γ release. ELISPOT analysis demonstrated that the T cells recognized the A2 positive K562 cell line, but not the A2 negative K562 cells. Further proof of specificity and HLA-A2 restriction of the recognition was documented by HLA-A2 antibody blocking experiments.

To further define the WT1 epitope, 4 truncated WT1 retroviral constructs were generated. Donor 475 fibroblasts were then transduced with these constructs. ELISPOT assays demonstrated recognition of D475 fibroblasts transduced with the WT1 Tr1 construct (aa2-aa92), thus demonstrating that the WT1 epitope is localized within the first 91 N-terminal amino acids of the WT1 protein. To fine map the epitope, 15mer peptides of the WT1 protein, overlapping by 11 amino acids, were synthesized. The WT1 specific T-cell clone recognized two overlapping 15mer peptides, peptide 9 (QWAPVLDFAPPGASA) (SEQ ID NO: 412) and peptide 10 (VLDFAPPGASAYGSL) (SEQ ID NO: 413). To further characterize the minimal epitope recognized, shared 9mer and 10mer peptides of the 15mers (5 total) were used to analyse the specificity of the clone. The clone specifically recognized the 9mer, VLDFAPPGA (SEQ ID NO:241), and the 10mer, VLDFAPPGAS (SEQ ID NO:411).

EXAMPLE 25

CLONING AND SEQUENCING OF TCR ALPHA AND BETA CHAINS DERIVED FROM A CD8 T CELL SPECIFIC FOR WT1

T cell receptor (TCR) alpha and beta chains from CD8+ T cell clones specific for WT1 are cloned. Sequence analysis is carried to demonstrate the family origin of the the alpha and beta chains of the TCR. Additionally, unique diversity and joining segments (contributing to the specificity of the response) are identified.

Total mRNA from 2 x $10^6$ cells from a WT1 specific CD8+ T cell clone is isolated using Trizol reagent and cDNA is synthesized using Ready-to-go kits (Pharmacia). To determine Vα and Vβ sequences in a clone, a panel of Vα and Vβ subtype specific primers are synthesized (based on primer sequences generated by Clontech, Palo Alto, CA) and used in RT-PCR reactions with cDNA generated from each clone. The RT-PCR reactions demonstrate which Vβ and Vα sequence is expressed by each clone.

To clone the full-length TCR alpha and beta chains from a clone, primers are designed that span the initiator and terminator-coding TCR nucleotides. Standard 35 cycle RT-PCR reactions are established using cDNA synthesized from the CTL clone and the above primers using the proofreading thermostable polymerase PWO (Roche, Basel, Switzerland). The resultant specific bands (~850 bp for alpha and ~950 for beta) are ligated into the PCR blunt vector (Invitrogen, Carlsbad, CA) and transformed into *E. coli. E. coli* transformed with plasmids containing full-length alpha and beta chains are identified, and large scale preparations of the corresponding plasmids are generated. Plasmids containing full-length TCR alpha and beta chains are then sequenced using standard methods. The diversity-joining (DJ) region that contributes to the specificity of the TCR is thus determined.

From the foregoing it will be appreciated that, although specific embodiments of the invention have been described herein for purposes of illustration, various modifications may be made without deviating from the scope of the invention. Accordingly, the invention is not limited except as by the appended claims.

**Claims**

1. An isolated polypeptide consisting of amino acids 1-281 of the WT1 polypeptide set forth in SEQ ID No. 408 and wherein the ability of said polypeptide to react with WT1-specific T-cell lines or clones is not substantially diminished relative to the WT1 polypeptide set forth in SEQ ID No. 408.

2. A polypeptide comprising a fusion of the isolated polypeptide of claim 1 with a fusion partner.

3. The polypeptide of claim 2 wherein said fusion partner is selected from the group consisting of Ra12, protein D, LYTA, a HIS tag, and a targeting signal capable of directing a polypeptide to the endosomal/lysosomal compartment.

4. The polypeptide of claim 3 wherein the polypeptide comprises the amino acid sequence set forth in SEQ ID NO:393.

5. An isolated polynucleotide encoding the polypeptide of any one of claims 1-4.

6. An expression vector comprising the isolated polynucleotide of claim 5 operably linked to a promoter.

7. An isolated host cell transformed or transfected with the expression vector according to claim 6.

8. A composition comprising the polypeptide of any one of claims 1-4 in combination with a pharmaceutically acceptable carrier or excipient.

9. A vaccine comprising the polypeptide of any one of claims 1-4 in combination with an immunostimulant.

10. A composition comprising the polynucleotide of claim 5 in combination with a pharmaceutically acceptable carrier or excipient.

11. A vaccine comprising the isolated polynucleotide according to claim 5 in combination with an immunostimulant.

12. The vaccine according to any one of claims 9 or 11 wherein the immunostimulant enhances a T cell response in a patient.

13. The vaccine according to claim 12 wherein the immunostimulant is selected from the group consisting of MPL, RC-529, AGPs, Seppic Montanide ISA 720, a cytokine, a microsphere, AS-2, QS21, Ribi Adjuvant system based adjuvants, saponin based adjuvants, and immune stimulating complex (iscom) based adjuvants.

14. The polypeptide of any one of claims 1 to 4 for use in therapy.

15. The polypeptide of any one of claims 1 to 4 for use in the prevention or treatment ofa malignant disease.

16. The use of the polypeptide of any one of claims 1 to 4 for the manufacture of a medicament for use in the prevention or therapy of a malignant disease.

17. The polypeptide for or use of any one of claims 15 or 16, wherein the malignant disease is selected from the group consisting of leukemia including acute myeloid, acute lymphocytic and chronic myeloid and cancer including breast, lung, thyroid, gastro intestinal cancer or melanoma.

**Patentansprüche**

1. Ein isoliertes Polypeptid, das aus den Aminosäuren 1-231 des WT1 Polypeptids, das in SEQ ID NO: 408 dargelegt ist, besteht und wobei die Fähigkeit des Polypeptids mit WT1 spezifischen T-Zell-Linien oder Klonen zu reagieren nicht wesentlich verringert ist relativ zu dem WT1 Polypeptid, das in SEQ ID NO: 408 dargelegt ist.

2. Ein Polypeptid, das eine Fusion des isolierten Polypeptids nach Anspruch 1 mit einem Fusionspartner umfasst.

3. Das Polypeptid nach Anspruch 2, wobei der Fusionspartner aus der Gruppe, die aus Ra12, Protein D, LYTA, einem HIStag und einem Target-Signal, das ein Polypeptid zu dem endosomalen/lyosomalen Kompartiment leiten kann, besteht, ausgewählt wird.

4. Das Polypeptid nach Anspruch 3, wobei das Polypeptid die Aminsäuresequenz umfasst, die in SEQ ID NO: 393 dargelegt ist, umfasst.

5. Ein isoliertes Polynukleotid, das für das Polypeptid nach einem der Ansprüche 1-4 kodiert.

6. Ein Expressionsvektor, der das isolierte Polynukleotid nach Anspruch 5 umfasst, der in funktioneller Verbindung mit einem Promoter ist.

7. Eine isolierte Wirtszelle, die mit dem Expressionsvektor nach Anspruch 6 transformiert oder transfiziert wurde.

8. Eine Zusammensetzung, die das Polypeptid nach einem der Ansprüche 1-4 zusammen mit einem pharmazeutisch geeigneten Träger oder Hilfsstoff umfasst.

9. Ein Impfstoff, der das Polypeptid nach einem der Ansprüche 1-4 zusammen mit einem Immunostimulanz umfasst.

10. Eine Zusammensetzung, die das Polynukleotid nach Anspruch 5 zusammen mit einem pharmazeutisch geeigneten Träger oder Hilfsstoff umfasst.

11. Ein Impfstoff, der das isolierte Polynukleotid gemäß Anspruch 5 zusammen mit einem Immunostimulanz umfasst.

12. Der Impfstoff gemäß einem der Ansprüche 9 oder 11, wobei das Immunostimulanz eine T-Zellenantwort bei einem Patienten verstärkt.

13. Der Impfstoff gemäß Anspruch 12, wobei das Immunostimulanz aus der Gruppe, die aus MPL, RC-529, AGPs, Seppic Montanide ISA 720, einem Cytokin, einem Mikrokügelchen, AS-2, QS21, einem Hilfsmittel basierend auf dem Ribi-Hilfsmittel-System, einem Hilfsmittel basierend auf Saponin und einem Hilfsmittel basierend auf einem immunostimulierenden Komplex (Iscom) besteht, ausgewählt wird.

14. Das Polypeptid nach einem der' Ansprüche 1 bis 4 für die Verwendung in der Therapie.

15. Das Polypeptid nach einem der Ansprüche 1 bis 4 für die Verwendung in der Prävention oder der Behandlung einer bösartigen Erkrankung.

16. Die Verwendung des Polypeptids nach einem der Ansprüche 1 bis 4 für die Herstellung eines Medikaments für die Verwendung in der Prävention oder der Therapie einer bösartigen Erkrankung.

17. Das Polypeptid für oder die Verwendung nach einem der Ansprüche 15 oder 16, wobei die bösartige Erkrankung aus der Gruppe, die aus Leukämie, einschließlich akuter myeloischer, akuter lymphozytischer und chronisch myeloischer, und Krebs, einschließlich Brust-, Lungen-, Schilddrüsen-, Magen-Darm-Krebs oder Melanom, besteht, ausgewählt wird.

**Revendications**

1. Un peptide isolé constitué de 1 à 281 acides aminés du polypeptide WT1 présenté dans la SEQ ID No. 408 et dans lequel la capacité dudit peptide à réagir spécifiques du WT1 avec les lignées cellulaires T, ou avec des clones, n'est pas diminuée substantiellement par rapport au polypeptide WT1 présenté dans la SEQ ID No. 408.

2. Un polypeptide comportant une fusion du polypeptide isolé selon la revendication 1 avec une fusion partenaire.

3. Le polypeptide selon la revendication 2 dans lequel ladite fusion partenaire est choisie parmi le groupe constitué de Ra12, de la protéine D, du LYTA, du tag HIS, et d'un signal cible capable de diriger un polypeptide vers un compartiment endosomial / lysosomial.

4. Le polypeptide selon la revendication 3 dans lequel le polypeptide comporte la séquence d'acides aminés présentée dans la SEQ ID NO : 393.

5. Un polynucléotide isolé encodant le polypeptide de l'une quelconque des revendications 1 - 4.

6. Un vecteur d'expression comportant le polynucléotide de la revendication 5 lié à un promoteur de manière opérationnelle.

7. Une cellule hôte isolée transformée ou transfectée par un vecteur d'expression en accord avec la revendication 6.

8. Une composition comportant le polypeptide de l'une quelconque des revendications 1 - 4 en combinaison avec un transporteur ou un excipient acceptable sur le plan pharmaceutique.

9. Un vaccin comportant le polypeptide de l'une quelconque des revendications de 1 - 4 en combinaison avec un immunostimulant.

10. Une composition comportant le polynucléotide selon la revendication 5 en combinaison avec un transporteur ou un excipient acceptable sur le plan pharmaceutique.

11. Un vaccin comportant le polynucléotide selon la revendication 5 en combinaison avec un immunostimulant.

12. Un vaccin en accord avec l'une quelconque des revendications 9 ou 11 dans lequel l'immunostimulant favorise la réponse cellulaire T chez un patient.

13. Le vaccin en accord avec la revendication 12 dans lequel l'immunostimulant est choisi parmi le groupe constitué du MPL, du RC-529, de l'AGPs, du Seppic Montanide ISA 720, d'une cytokine, d'une microsphère, de l'AS-2, du QS21, des adjuvants basés sur le système d'adjuvant Ribi, des adjuvants basés sur la saponine, et des adjuvants basés sur le complexe de stimulation immune (iscom).

14. Le polypeptide de l'une quelconque des revendications de 1 à 4 pour une utilisation en thérapeutique.

15. Le polypeptide de l'une quelconque des revendications de 1 à 4 pour une utilisation dans la prévention ou dans le traitement d'une maladie maligne.

16. L'utilisation du polypeptide de l'une quelconque des revendications de 1 à 4 pour la fabrication d'un médicament pour une utilisation dans la prévention ou dans le traitement d'une maladie maligne.

17. Le polypeptide pour ou l'utilisation de l'une quelconque des revendications 15 ou 16, dans lesquels la maladie maligne est choisie parmi le groupe constitué de la leucémie incluant la leucémie myéloïde aigue, la leucémie lymphocytaire aigue et la leucémie myéloïde chronique et du cancer incluant le cancer du sein, le cancer du poumon, le cancer de la thyroide, le cancer gastro-intestinal et le mélanome.

HU:MGSDVRDLNALLPAVPSLGGGGGCALPVSGAAQWAPVLDFAPPGASAYGSL
MO:MGSDVRDLNALLPAVSSLGGGGGCGLPVSGAAQWAPVLDFAPPGASAYGSL

HU:GGPAPPPAPPPPPPPPPHSFIKQEPSWGGAEPHEEQCLSAFTVHFSGQFTGTAG
MO:GGPAPPPAPPPPPPPPPHSFIKQEPSWGGAEPHEEQCLSAFTLHFSGQFTGTAG

HU:ACRYGPFGPPPPSQASSGQARMFPNAPYLPSCLESQPAIRNQGYSTVTFDGTPS
MO:ACRYGPFGPPPPSQASSGQARMFPNAPYLPSCLESQPTIRNQGYSTVTFDGAPS

HU:YGHTPSHHAAQFPNHSFKHEDPMGQQGSLGEQQYSVPPPVYGCHTPTDSCTG
MO:YGHTPSHHAAQFPNHSFKHEDPMGQQGSLGEQQYSVPPPVYGCHTPTDSCTG

HU:SQALLLRTPYSSDNLYQMTSQLECMTWNQMNLGATLKGVAAGSSSSVKWTE
MO:SQALLLRTPYSSDNLYQMTSQLECMTWNQMNLGATLKGMAAGSSSSVKWTE

HU:GQSNHSTGYESDNHTTPILCGAQYRIHTHGVFRGIQDVRRVPGVAPTLVRSAS
MO:GQSNHGIGYESDNHTAPILCGAQYRIHTHGVFRGIQDVRRVSGVAPTLVRSAS

HU:ETSEKRPFMCAYPGCNKRYFKLSHLQMHSRKHTGEKPYQCDFKDCERRFSR
MO:ETSEKRPFMCAYPGCNKRYFKLSHLQMHSRKHTGEKPYQCDFKDCERRFSR

HU:SDQLKRHQRRHTGVKPFQCKTCQRKFSRSDHLKTHTRTHTGKTSEKPFSCR
MO:SDQLKRHQRRHTGVKPFQCKTCQRKFSRSDHLKTHTRTHTGKTSEKPFSCR

HU:WPSCQKKFARSDELVRHHNMHQRNMTKLQLAL
MO:WHSCQKKFARSDELVRHHNMHQRNMTKLHVAL

*FIG. 1*

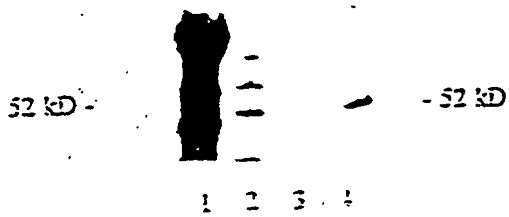


FIG. 2

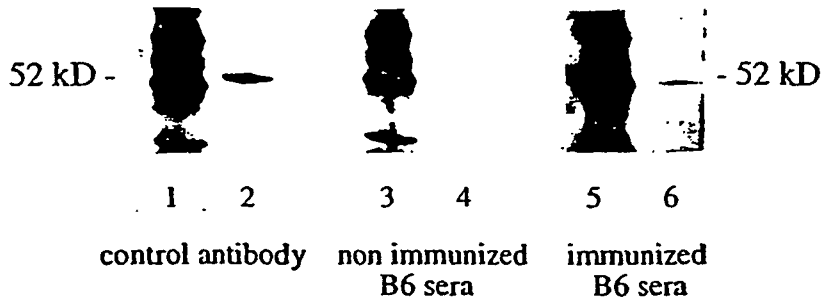
52 kD -
- 52 kD
1
2
3
4
5
6
control antibody
non immunized
B6 sera
immunized
B6 sera

*FIG. 3*

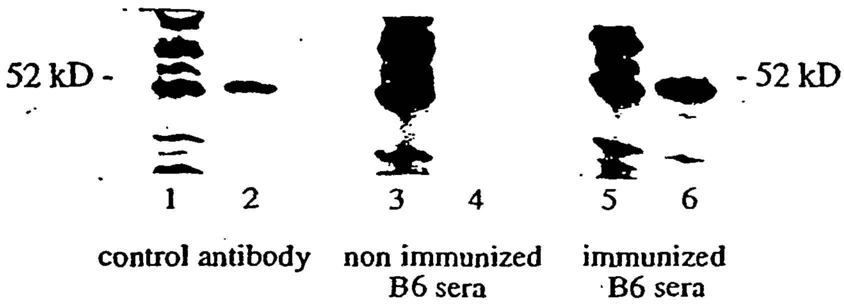
52 kD -
- 52 kD
1
2
3
4
5
6
control antibody
non immunized B6 sera
immunized B6 sera

*FIG. 4*

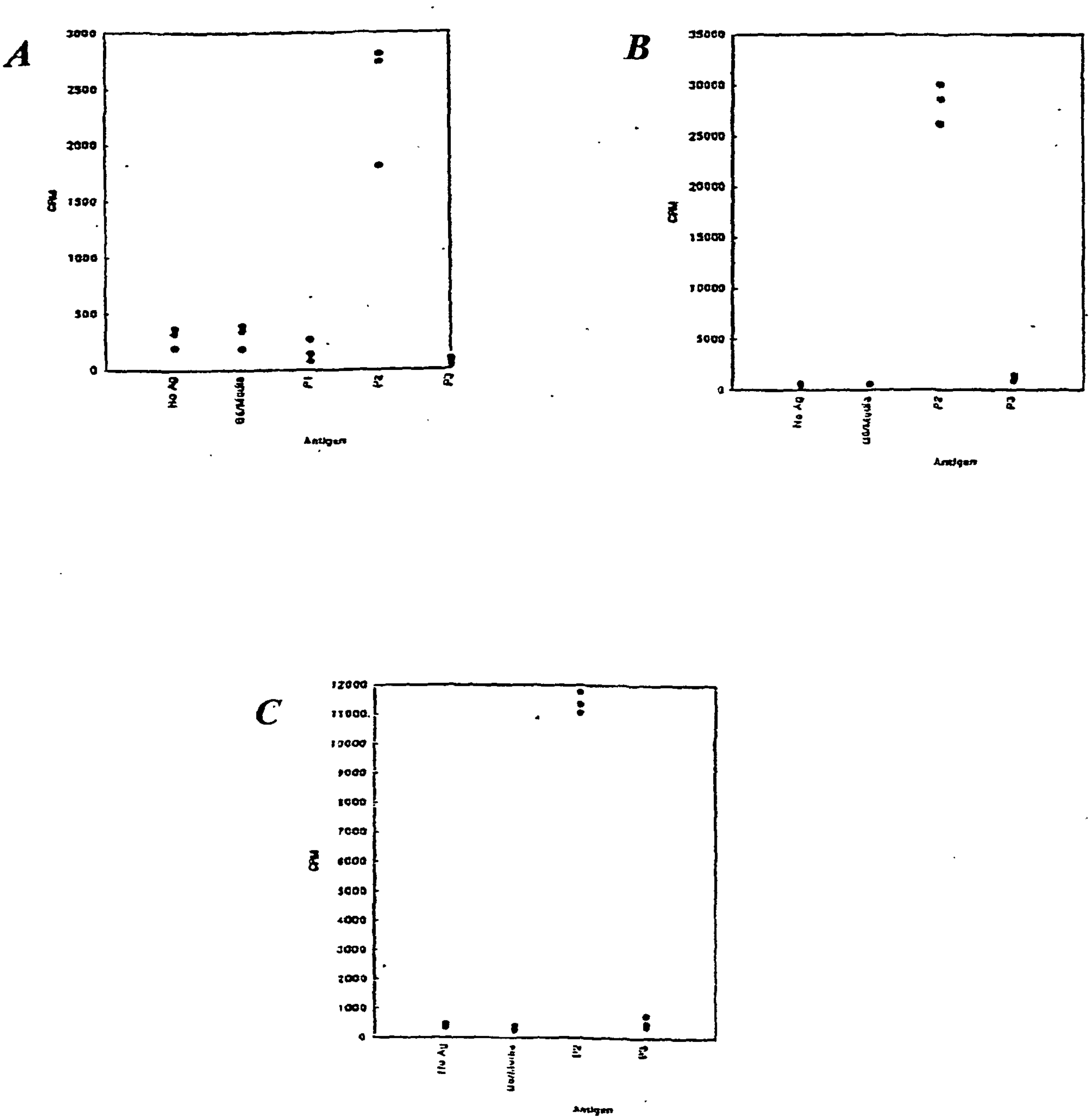
A
B
C
CPM
Antigen
No Ag
P1
P2
P3
0
500
1000
1500
2000
2500
3000
5000
10000
15000
20000
25000
30000
35000
1000
2000
3000
4000
5000
6000
7000
8000
9000
10000
11000
12000

*FIG. 5A-5C*

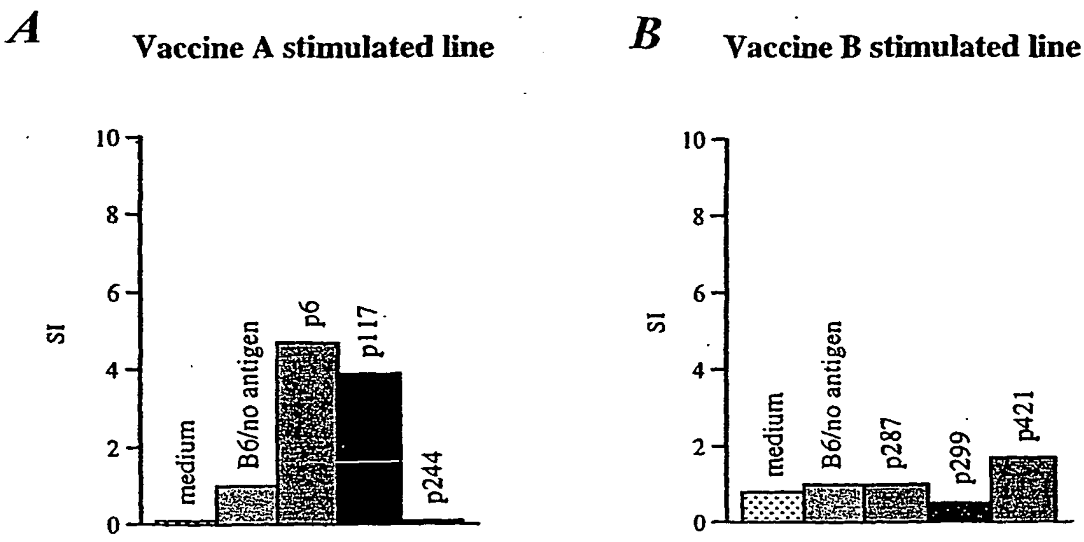
A
Vaccine A stimulated line
SI
10
8
6
4
2
0
medium
B6/no antigen
p6
p117
p244
B
Vaccine B stimulated line
SI
10
8
6
4
2
0
medium
B6/no antigen
p287
p299
p421

*FIG. 6A and 6B*

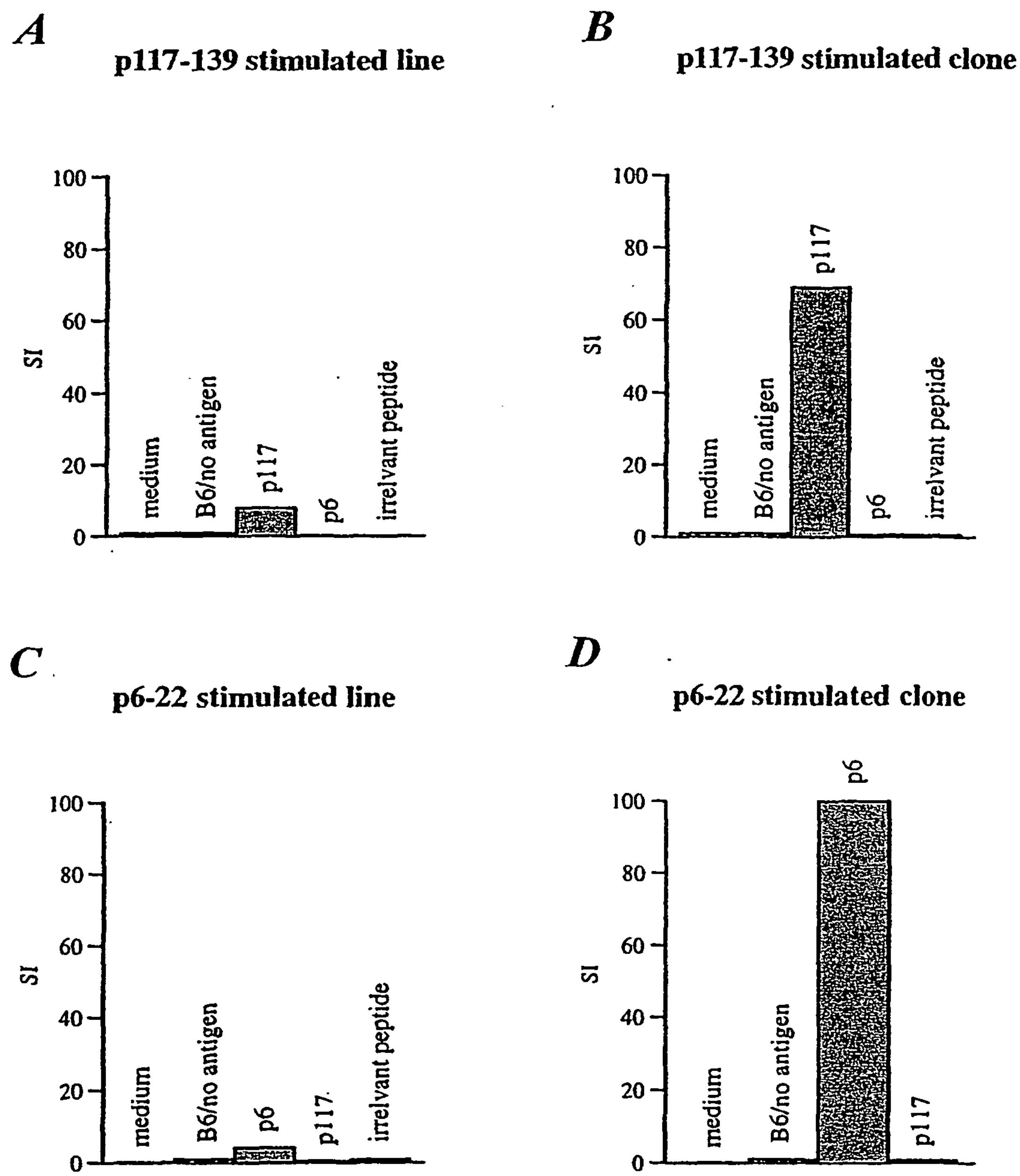
A
p117-139 stimulated line
SI
100
80
60
40
20
0
medium
B6/no antigen
p117
p6
irrelvant peptide
B
p117-139 stimulated clone
SI
100
80
60
40
20
0
medium
B6/no antigen
p117
p6
irrelvant peptide
C
p6-22 stimulated line
SI
100
80
60
40
20
0
medium
B6/no antigen
p6
p117
irrelvant peptide
D
p6-22 stimulated clone
SI
100
80
60
40
20
0
medium
B6/no antigen
p6
p117

*FIG. 7A-7D*

```
    5    10   15   20   25   30   35   40   45   50   55   60   65   70   75
MGSDVRDLNALLPAVPSLGGGGGCALPVSGAAQWAPVLDFAPPGASAYGSLGGPAPPPAPPPPPPPPPHSFIKQE
.....AAAAAAAAAAAAAAAAA.......AAAAAA.........AAAAAAAAAAAA...................
.............................RRRR..........................................
...........................................................................
...........................................................................

   80   85   90   95  100  105  110  115  120  125  130  135  140  145  150
PSWGGAEPHEEQCLSAFTVHFSGQFTGTAGACRYGPFGPPPPSQASSGQARMFPNAPYLPSCLESQPAIRNQGYS
...............AAA......AAAA.....................AAA.....AAAAAA............
..........................RRRR....................RRRRR....................
.........................................DDDDDDDD..........................
...........................................................................

  155  160  165  170  175  180  185  190  195  200  205  210  215  220  225
TVTFDGTPSYGHTPSHHAAQFPNHSFKHEDPMGQCGSLGEQQYSVPPPVYGCHTPTDSCTGSQALLLRTPYSSDN
................AAAAA..............................AAAAAA................AA
................RRRR.......................................................
..........................................................DDDDDDDDDDDDDD...
...........................................................................

  230  235  240  245  250  255  260  265  270  275  280  285  290  295  300
LYQMTSQLECMTWNQMNLGATLKGVAAGSSSSVKWTEGQSNHSTGYESDNHTTPILCGAQYRIHTHGVFRGIQDV
AAAAAAAA............AAA.AAA.....................................AAAAAAAAAAA
..................RRRRRRRRR....RRRR...............................RRRR.....
DDDDDD...............DDDDDDDDDDD...........................................
.............................................................ddddd.........

  305  310  315  320  325  330  335  340  345  350  355  360  365  370  375
RRVPGVAPTLVRSASETSEKRPFMCAYPGCNKRYFKLSHLQMHSRKHTGEKPYQCDFKDCERRFSRSDQLKRHQR
AAAAA..AAAAAAAAAAA..........................................AAAA.AAAAAAAAA.
....RRRRR.......................RRRR.......................................
.......DDDDDD..............................................................
...........................................................................

  380  385  390  395  400  405  410  415  420  425  430  435  440  445  450
RHTGVKPFQCKTCQRKFSRSDHLKTHTRTHTGKTSEKPFSCRWPSCQKKFARSDELVRHHNMHQRNMTKLQLAL
.............AAAA.AAAA..AA....AAAA...........AAA...AAAAAAAA...AAA.........
................................................RRRR..RRRR................
..........................................................................
..................ddddddddddd.............................................
```

*FIG. 8A*

```
    5    10   15   20   25   30   35   40   45   50   55   60   65   70   75
MGSDVRDLNALLPAVSSLGGGGGCGLPVSGAACWAPVLDFAPPGASAYGSLGGPAPPPAPPPPPPPPPHSFIKQE
.....AAAAAAAAAAAAAAAAA.......AAAAAA.........AAAAAAAAAAAA...................
.............................RRRR..........................................
...........................................................................
...........................................................................

   80   85   90   95  100  105  110  115  120  125  130  135  140  145  150
PSWGGAEPHEEQCLSAFTLHFSGQFTGTAGACRYGPFGPPPPSQASSGQARMFPNAPYLPSCLESQPTIRNQGYS
........................AAAA.....................AAA......AAAAAA...........
..........................RRRR....................RRRRR....................
.........................................DDDDDDDD..........................
...........................................................................

  155  160  165  170  175  180  185  190  195  200  205  210  215  220  225
TVTFDGAPSYGHTPSHHAAQFPNHSFKHEDPMGQQGSLGEQQYSVPPPVYGCHTPTDSCTGSQALLLRTPYSSDN
................AAAAA..............................AAAAAA................AA
................RRRR.......................................................
..........................................................DDDDDDDDDDDDDD...
...........................................................................

  230  235  240  245  250  255  260  265  270  275  280  285  290  295  300
LYQMTSQLECMTWNQMNLGATLKGMAAGSSSSVKNTEGQSNHGIGYESDNHTAPILCGAQYRIHTHGVFRGIQDV
AAAAAAAA............AAA.AAA.....................................AAAAAAAAAAA
..................RRRRRRRRRR.....RRRR.............................RRRR.....
DDDDDD...............DDDDDDDDDDD...........................................
.............................................................ddddd.........

  305  310  315  320  325  330  335  340  345  350  355  360  365  370  375
RRVSGVAPTLVRSASETSEKRPFMCAYPGCNKRYFKLSHLQMHSRKHTGEKPYQCDFKDCERRFSRSDQLKRHQR
AAAAA..AAAAAAAAAAA..........................................AAAA.AAAAAAAAA.
....RRRRR.......................RRRR.......................................
..DDDDDDDDDD...............................................................
...........................................................................

  380  385  390  395  400  405  410  415  420  425  430  435  440  445  450
RHTGVKPFQCKTCQRKFSRSDHLKTHTRTHTGKTSEKPFSCRWHSCQKKFARSDELVRHHNMHQRNMTKLHVAL
............AAAA.AAAA..AA....AAAA...........AA...AAAAAAAA...AAAA.......
................................................RRRR..RRRR................
..........................................................................
..................dddddddddddd............................................
```

*FIG. 8B*

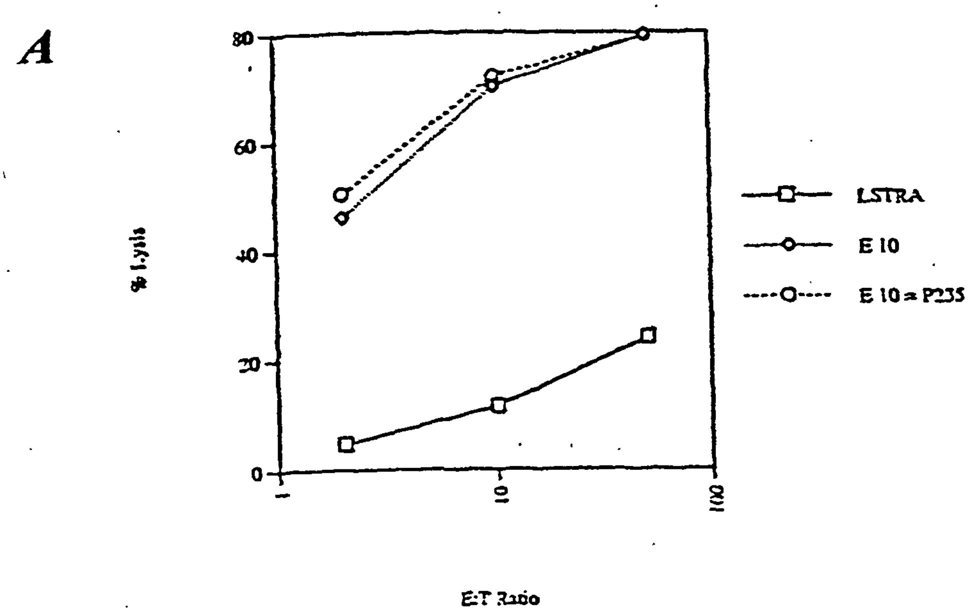
A
80
60
40
20
0
% Lysis
1
10
100
E:T Ratio
LSTRA
E 10
E 10 + P235

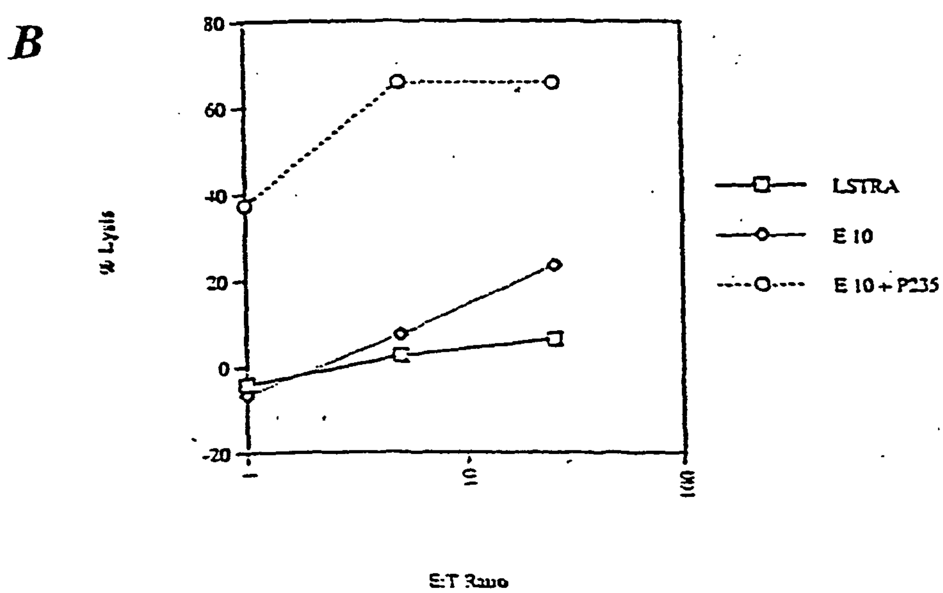
B
80
60
40
20
0
-20
% Lysis
1
10
100
E:T Ratio
LSTRA
E 10
E 10 + P235

*FIG. 9A and 9B*

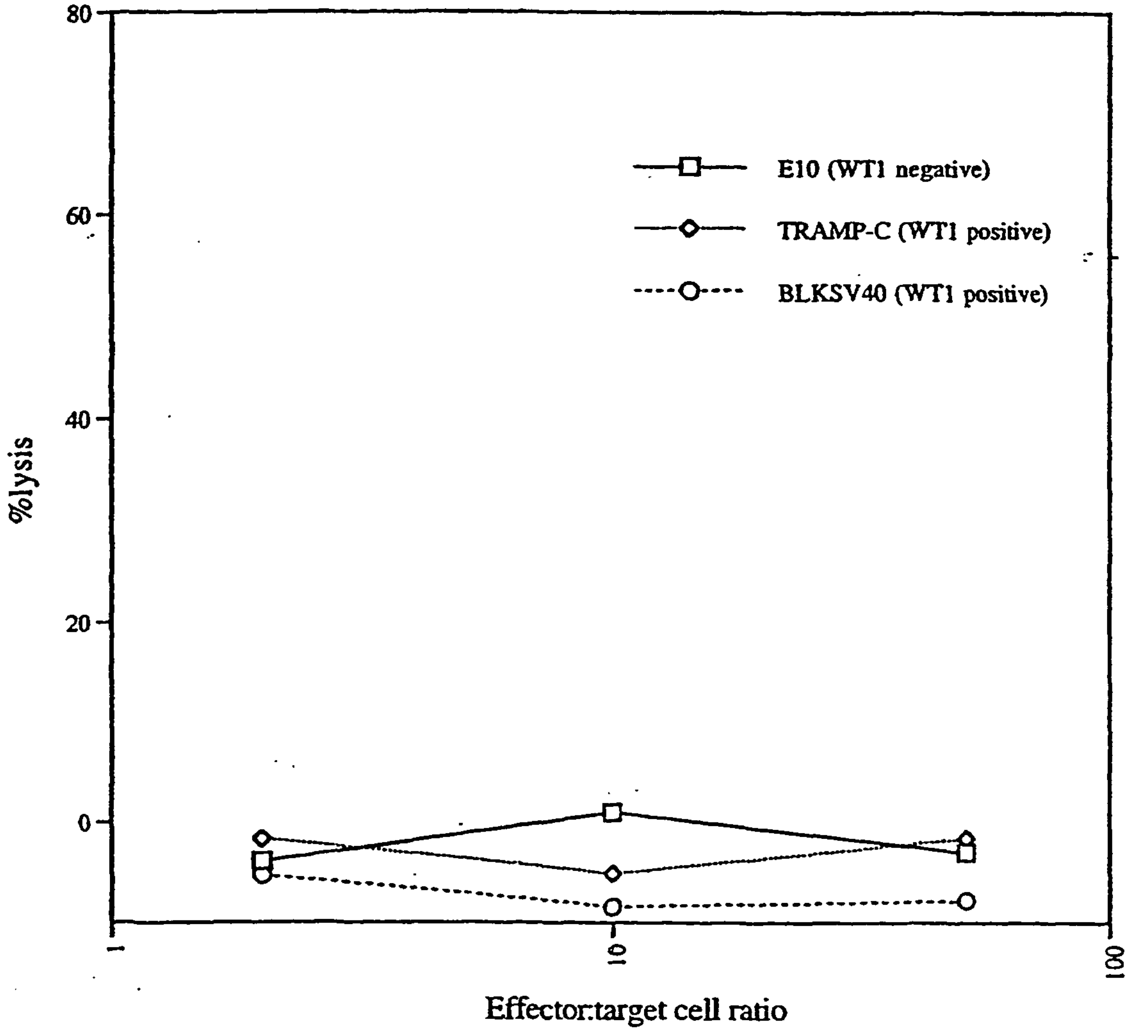
80
60
40
20
0
%lysis
E10 (WT1 negative)
TRAMP-C (WT1 positive)
BLKSV40 (WT1 positive)
1
10
100
Effector:target cell ratio

*FIG. 10A*

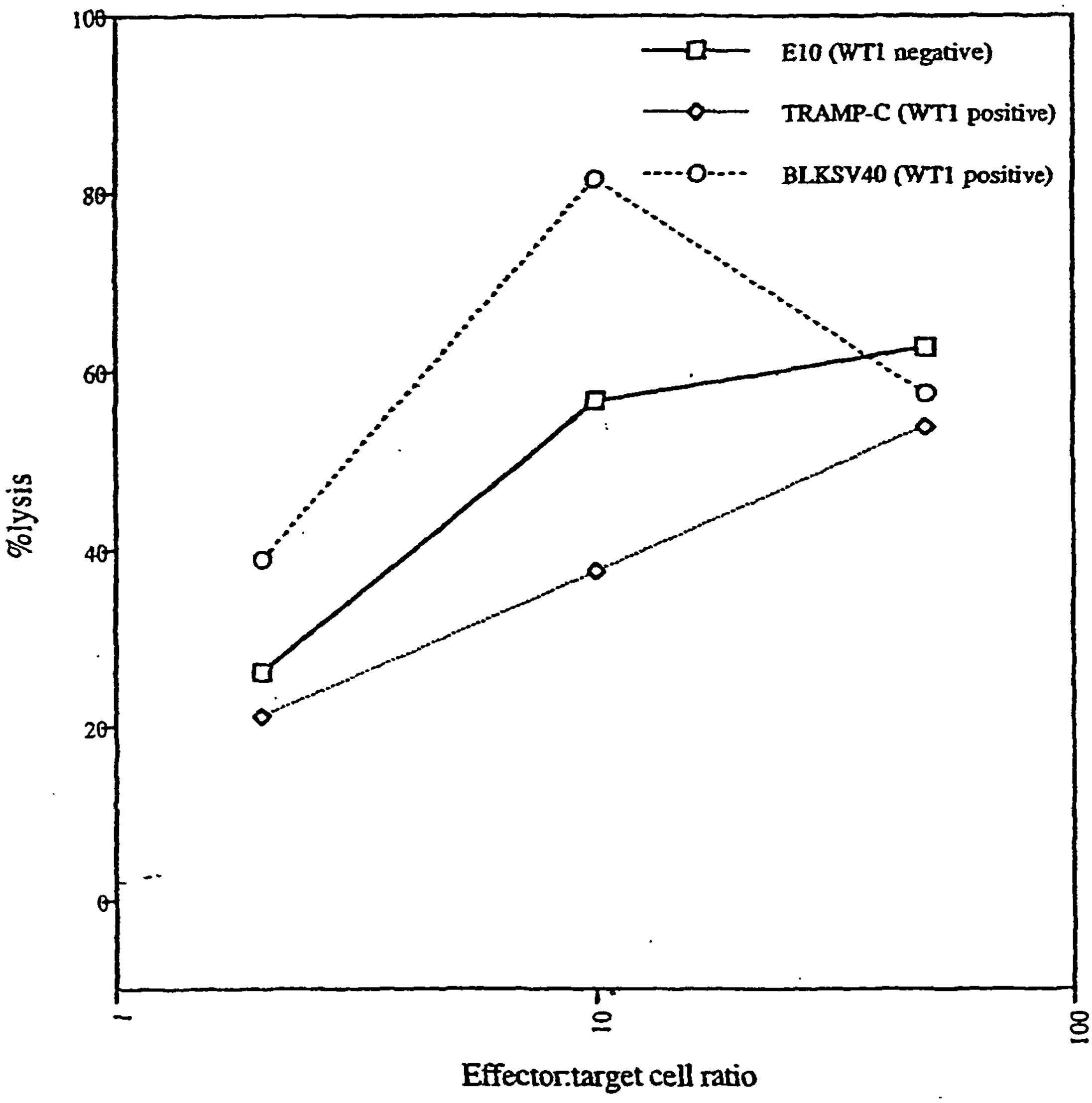
100
80
60
40
20
0
%lysis
E10 (WT1 negative)
TRAMP-C (WT1 positive)
BLKSV40 (WT1 positive)
1
10
100
Effector:target cell ratio

*FIG. 10B*

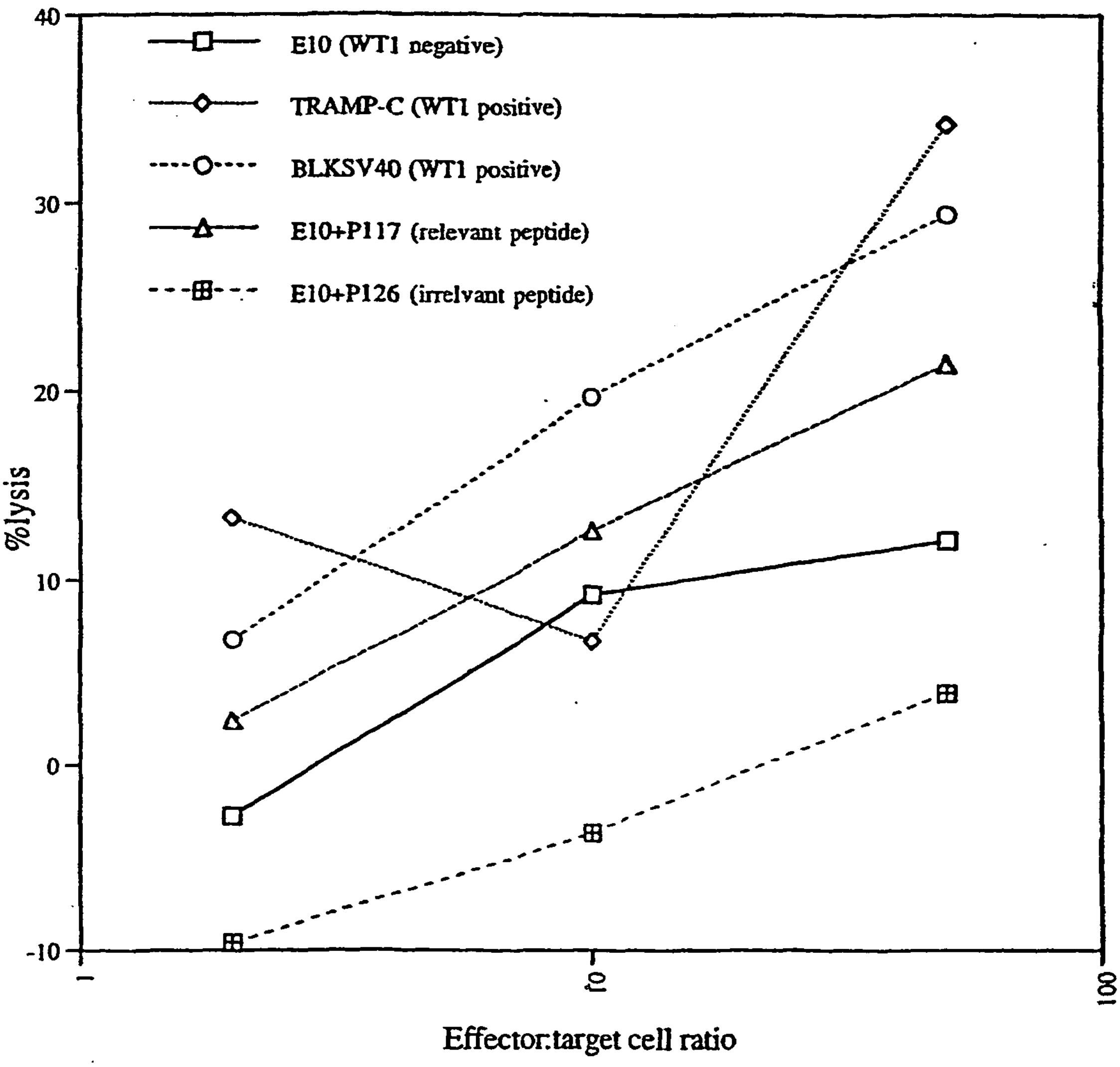
40
30
20
10
0
-10
%lysis
E10 (WT1 negative)
TRAMP-C (WT1 positive)
BLKSV40 (WT1 positive)
E10+P117 (relevant peptide)
E10+P126 (irrelvant peptide)
1
10
100
Effector:target cell ratio

*FIG. 10C*

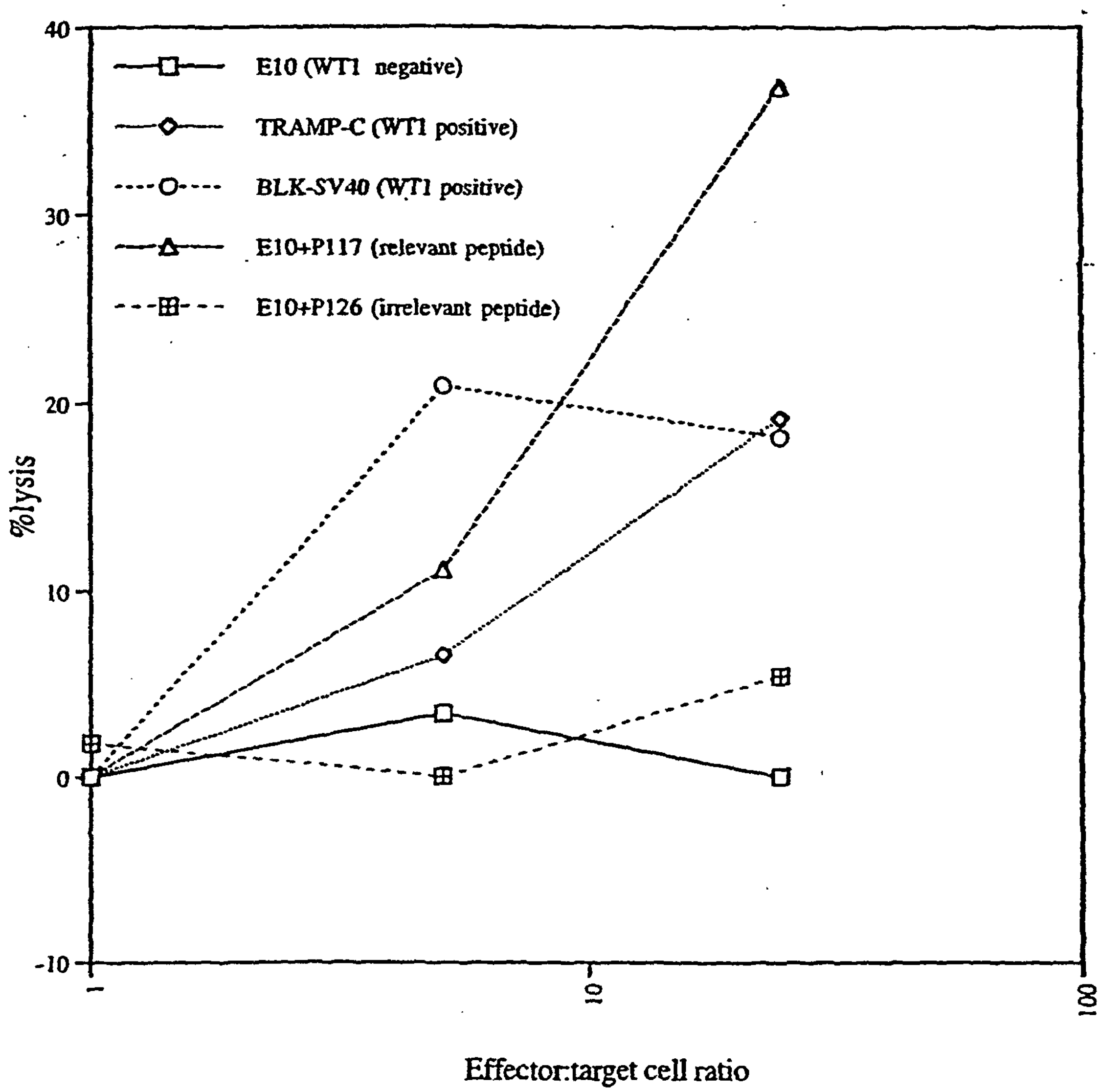
40
30
20
10
0
-10
%lysis
E10 (WT1 negative)
TRAMP-C (WT1 positive)
BLK-SV40 (WT1 positive)
E10+P117 (relevant peptide)
E10+P126 (irrelevant peptide)
1
10
100
Effector:target cell ratio

*FIG. 10D*

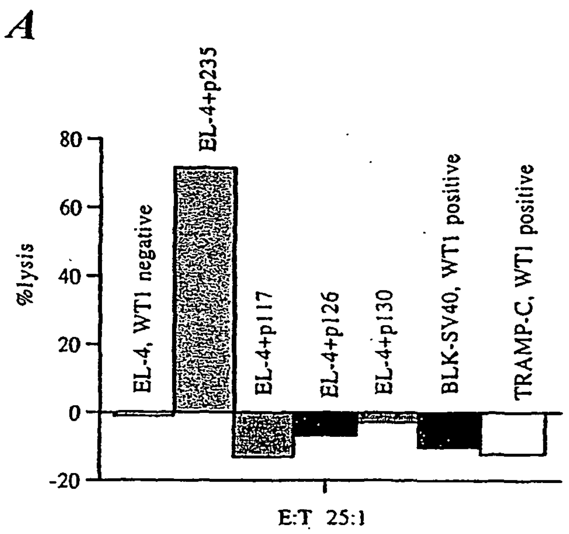


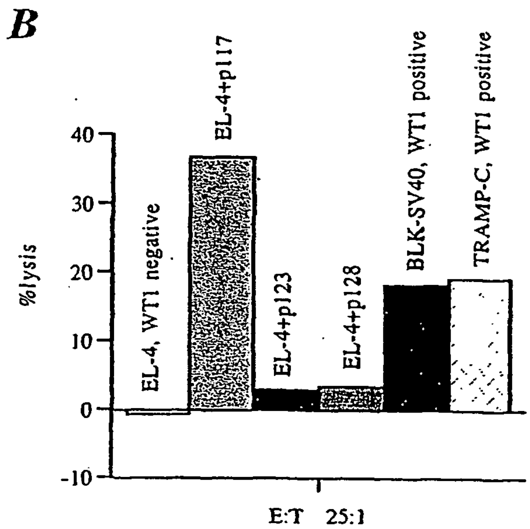


*FIG. 11A and 11B*

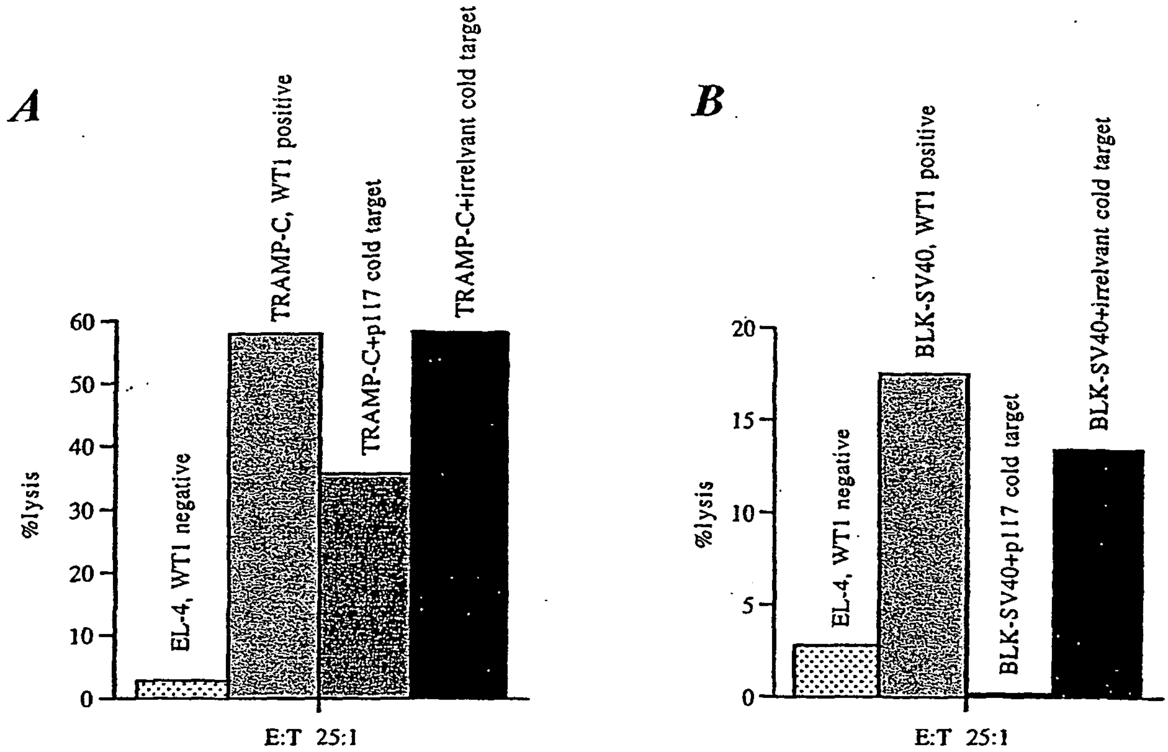


*FIG. 12A and 12B*

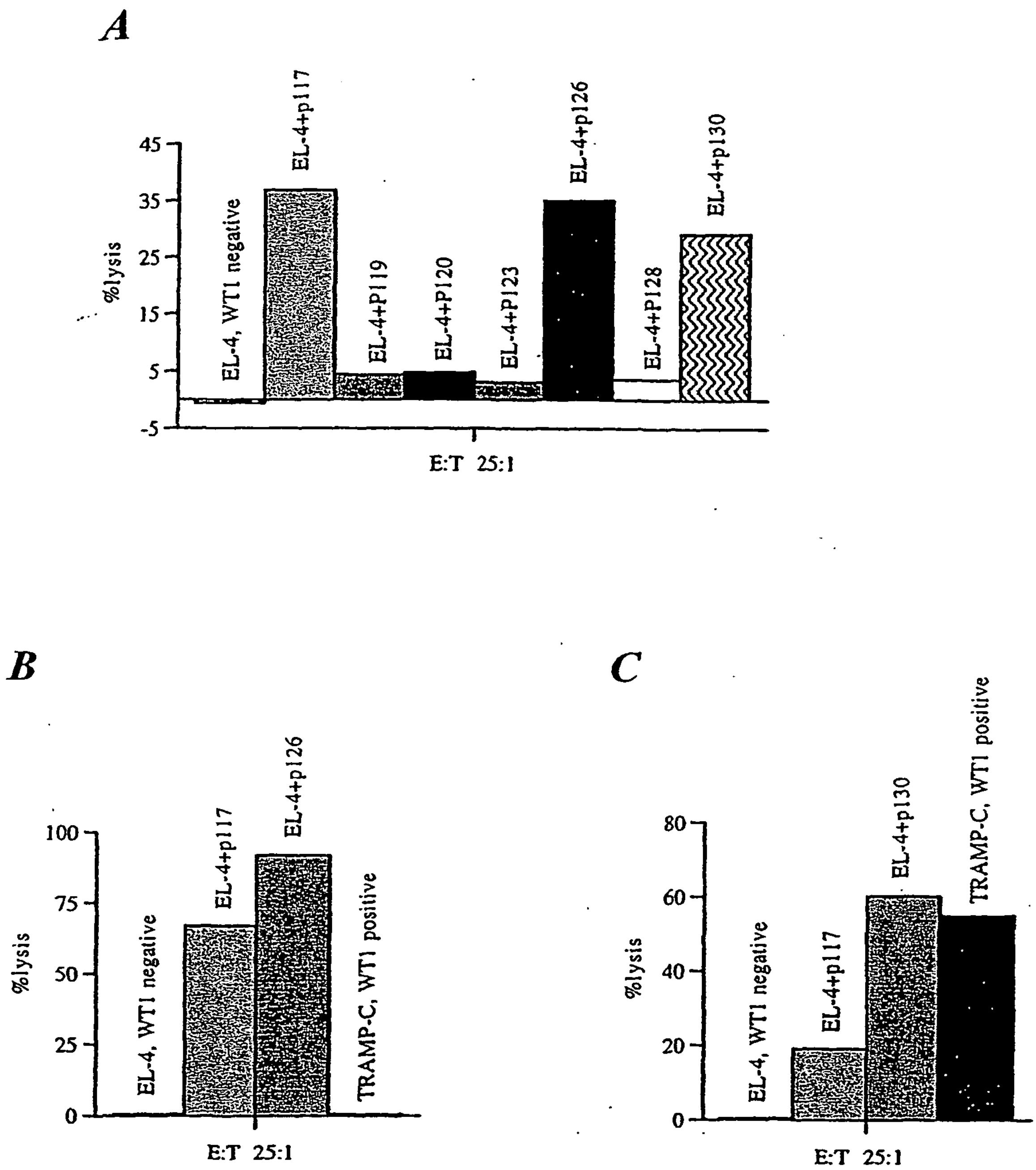
A
%lysis
45
35
25
15
5
-5
EL-4, WT1 negative
EL-4+p117
EL-4+P119
EL-4+P120
EL-4+P123
EL-4+p126
EL-4+P128
EL-4+p130
E:T 25:1
B
%lysis
100
75
50
25
0
EL-4, WT1 negative
EL-4+p117
EL-4+p126
TRAMP-C, WT1 positive
E:T 25:1
C
%lysis
80
60
40
20
0
EL-4, WT1 negative
EL-4+p117
EL-4+p130
TRAMP-C, WT1 positive
E:T 25:1

*FIG. 13A-13C*

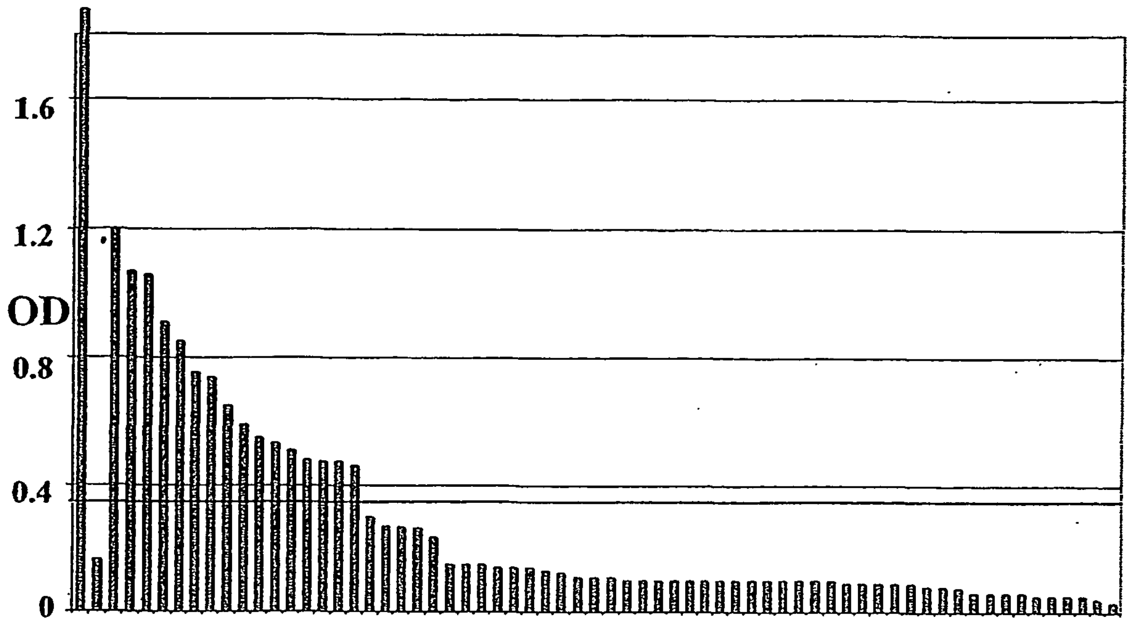


Fig. 14

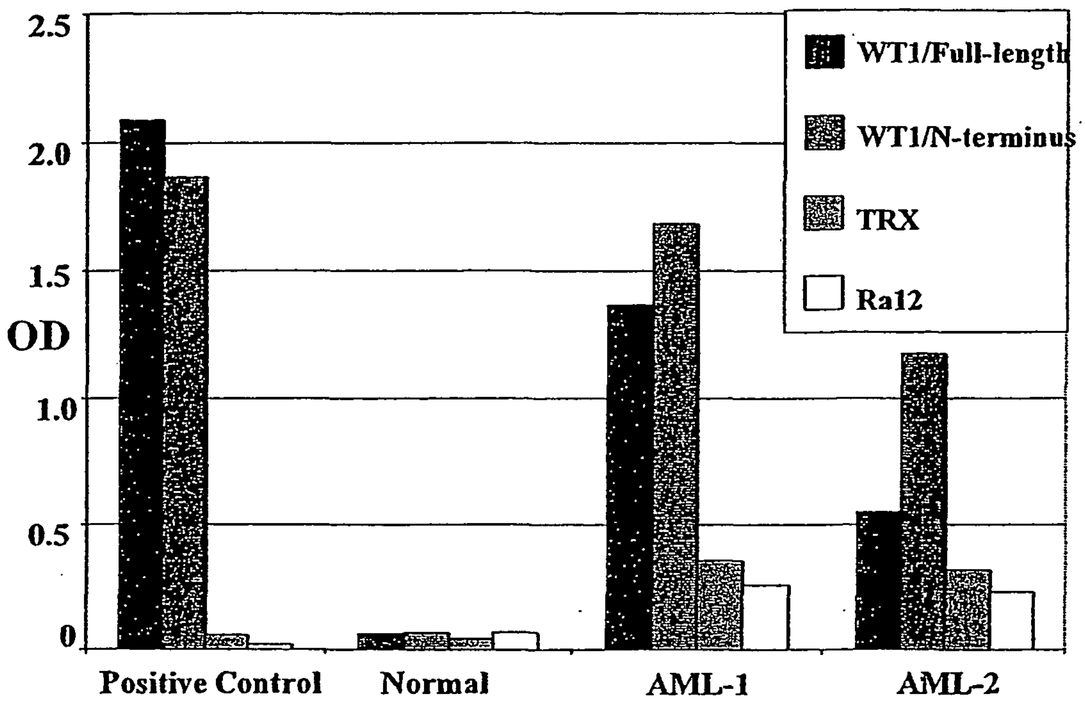
2.5
2.0
1.5
OD
1.0
0.5
0
WT1/Full-length
WT1/N-terminus
TRX
Ra12
Positive Control
Normal
AML-1
AML-2

Fig. 15

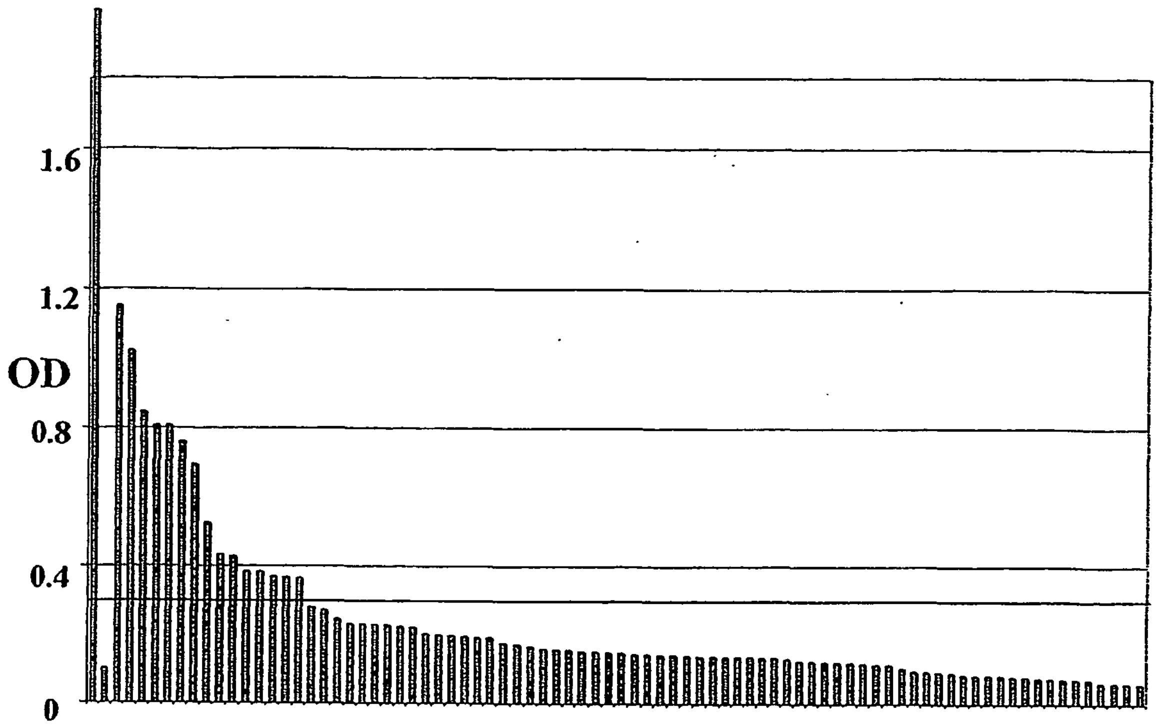


Fig. 16

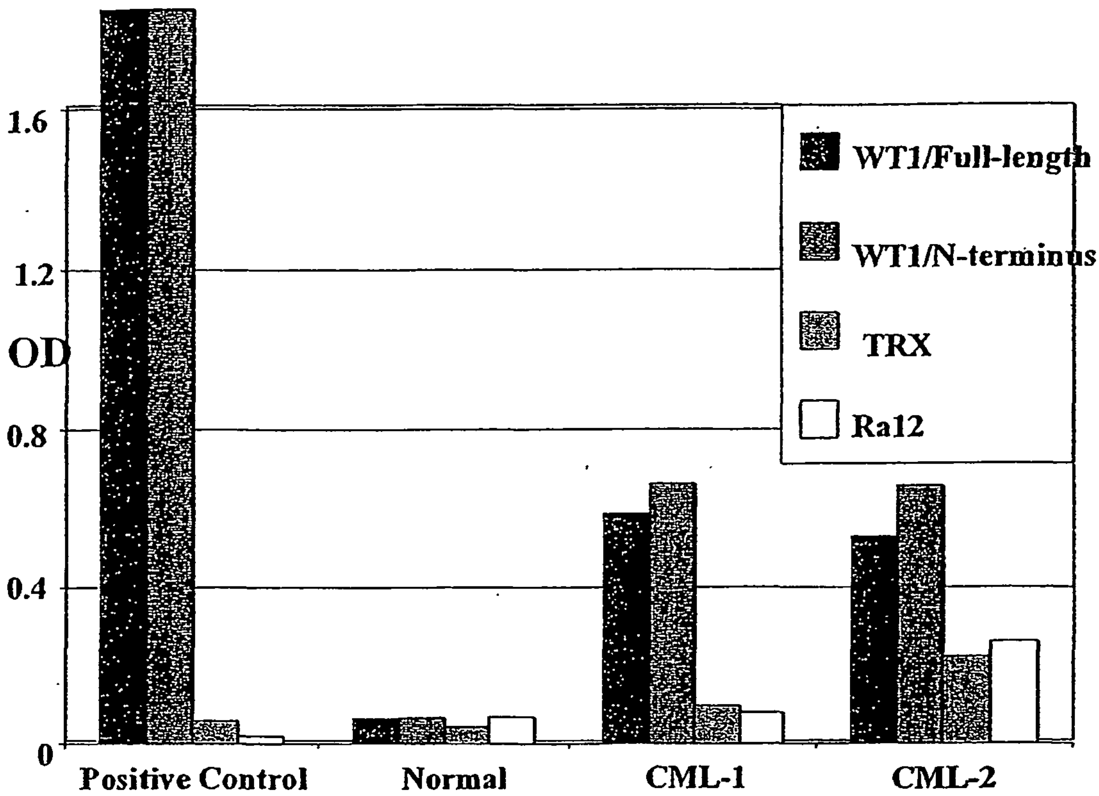
1.6
1.2
OD
0.8
0.4
0
WT1/Full-length
WT1/N-terminus
TRX
Ra12
Positive Control
Normal
CML-1
CML-2

Fig. 17

**TABLE 1: Characteristics of Recombinant WT1 Proteins Used for Serological Analysis**

| Name | Recombinant Protein | WT1 Amino Acid Position | Molecular Weight |
|---|---|---|---|
| WT1/full-length | Ra12-WT1 full length fusion protein | aa 1-449 | 85kDa |
| WT1/N-terminus | TRX-WT1 N-terminus fusion protein | aa 1-249 | 60kDa |
| WT1/C-terminus | WT1 C-terminus protein | aa 267-449 | 50kDa |

Fig. 18

CID000622 Figure 1a Ab responses in group 0 and 1 (controls)

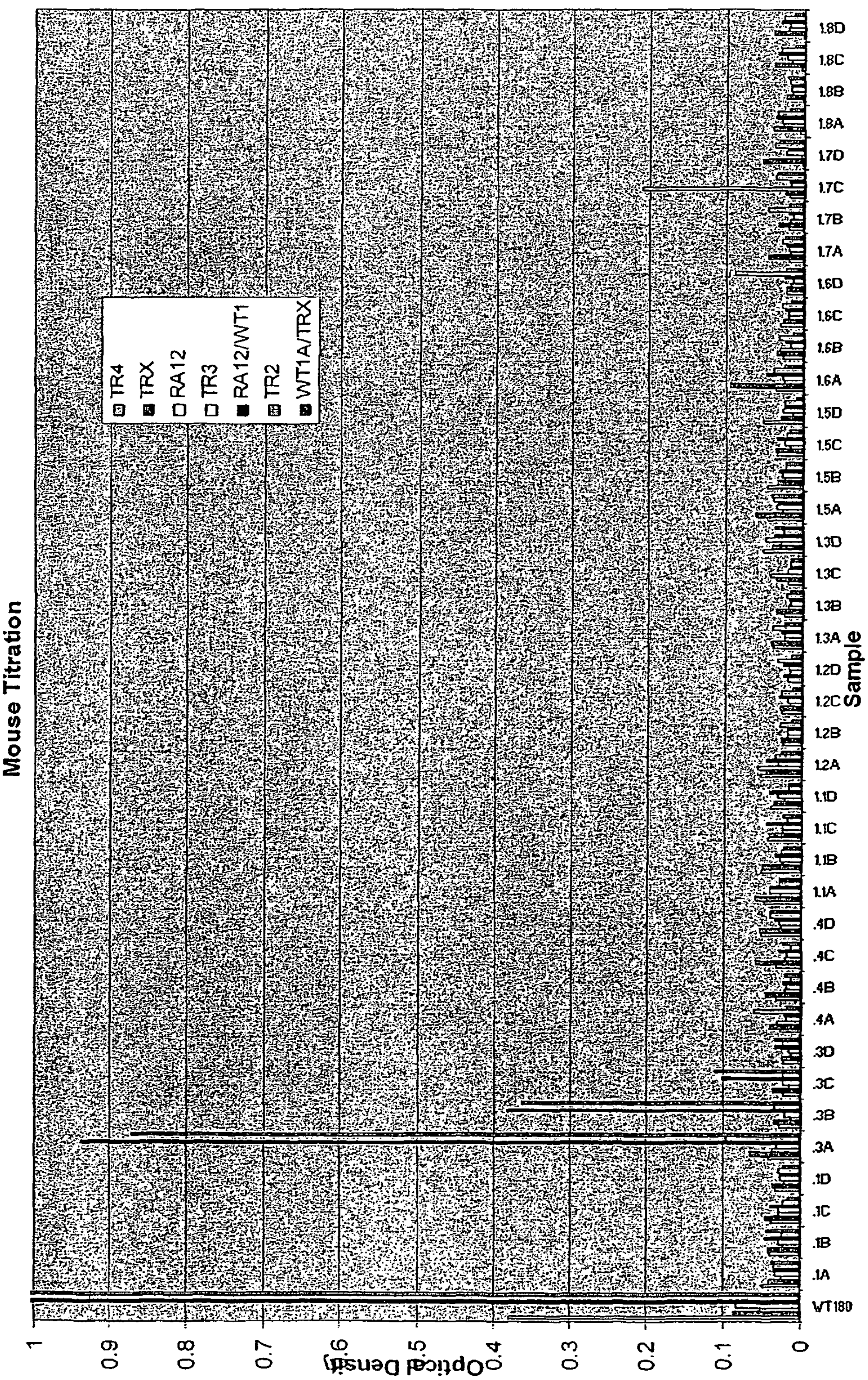


Control groups. A: 1;500 Dilution, B: 1:2000, C: 1: 8000, D: 1:16000

FIG. 19A

CID000622 Figure 1b. Ab responses in group 2 (25ug Ra12/WT1)

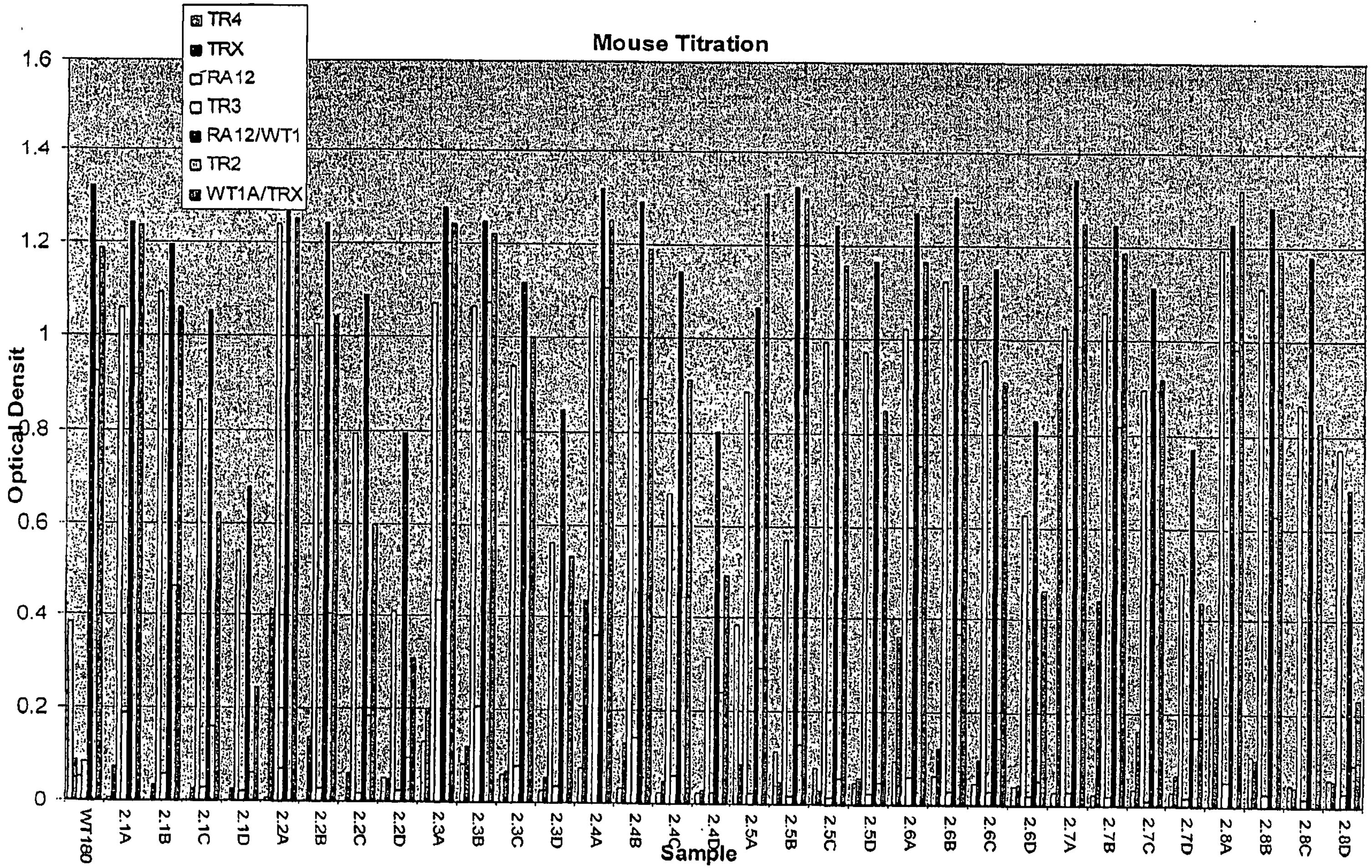


25ug Ra12/WT1+MPL-SE, A: 1;500 Dilution, B: 1:2000, C: 1: 8000, D: 1:16000

FIG. 19B

CID000622 Figure 1c. Ab responses in group 3 (100ug Ra12/WT1)

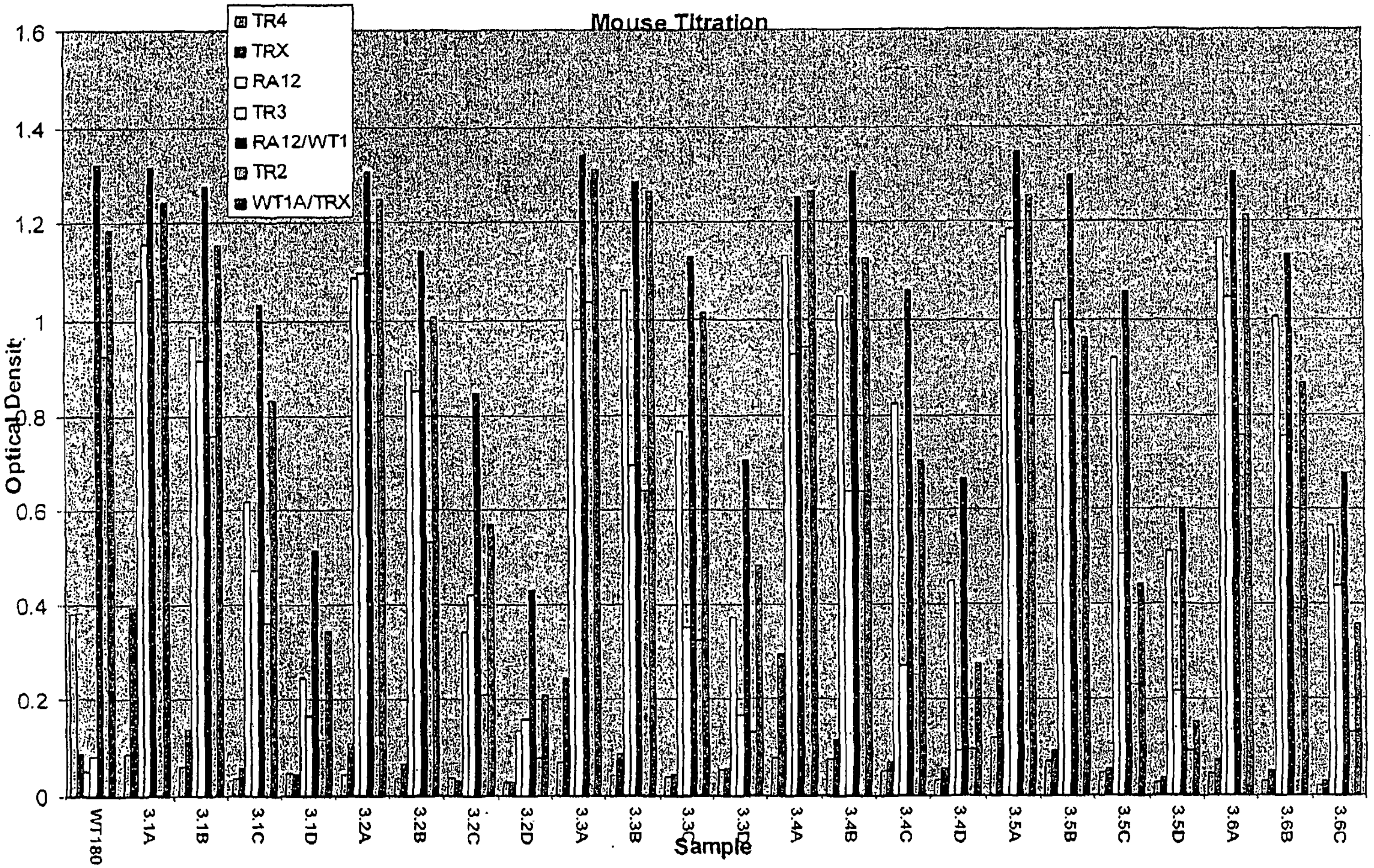


WT1. Dose Titration. Ab responses to WT1. 100ug Ra12-WT1+MPL-SE. A: 1;500 Dilution, B: 1:2000, C: 1: 8000, D: 1:16000

FIG. 19C

CID000622 Figure 1d. Ab responses in groups 3 and 4 (1000ug Ra12/WT1)

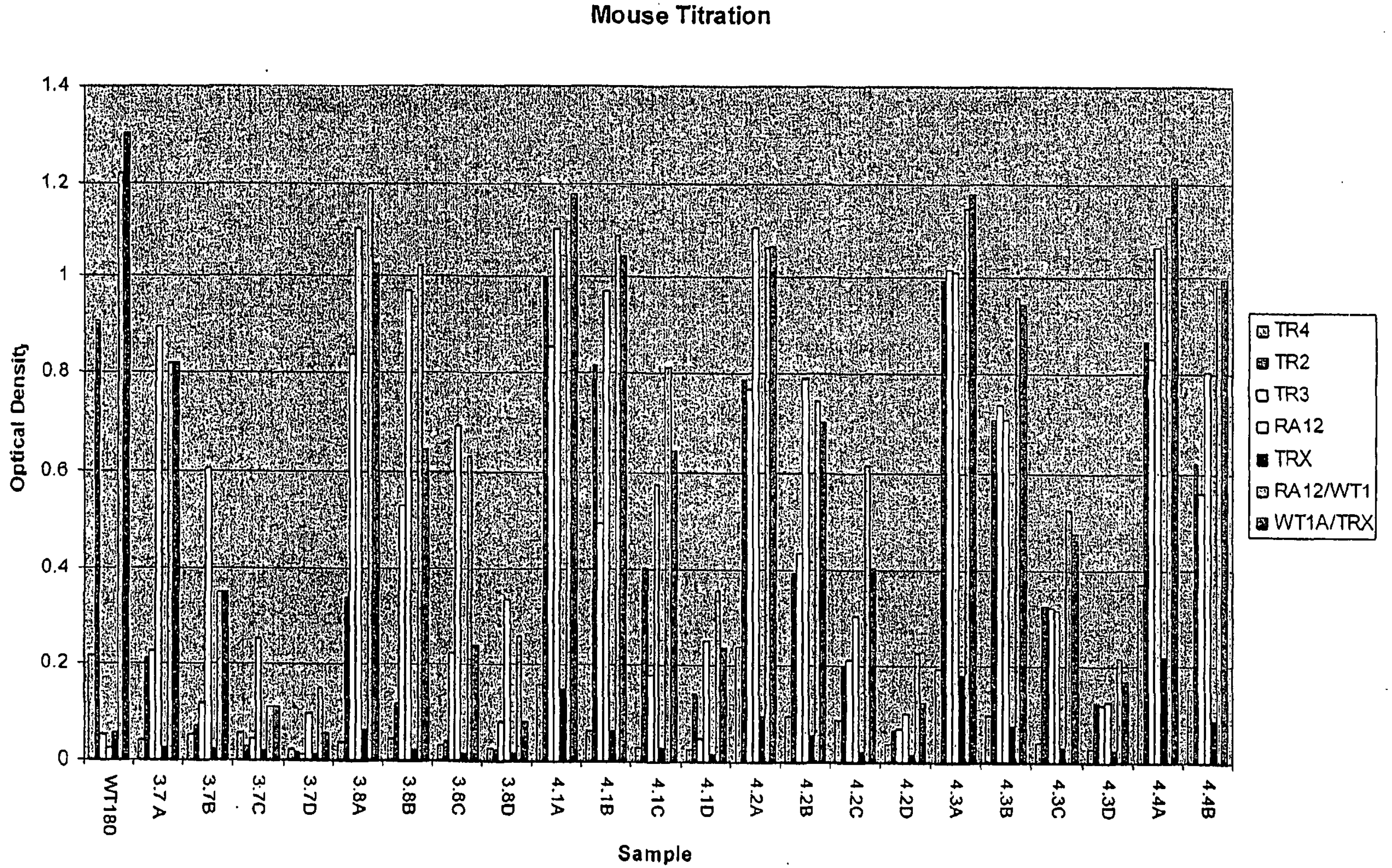


WT1. Dose Titration. Ab responses to WT1. 1000ug Ra12-WT1+MPL-SE. A: 1;500 Dilution, B: 1:2000, C: 1: 8000, D: 1:16000

FIG. 19D

**Figure 1e. Ab responses in group 4 (1000ug Ra12/WT1)**

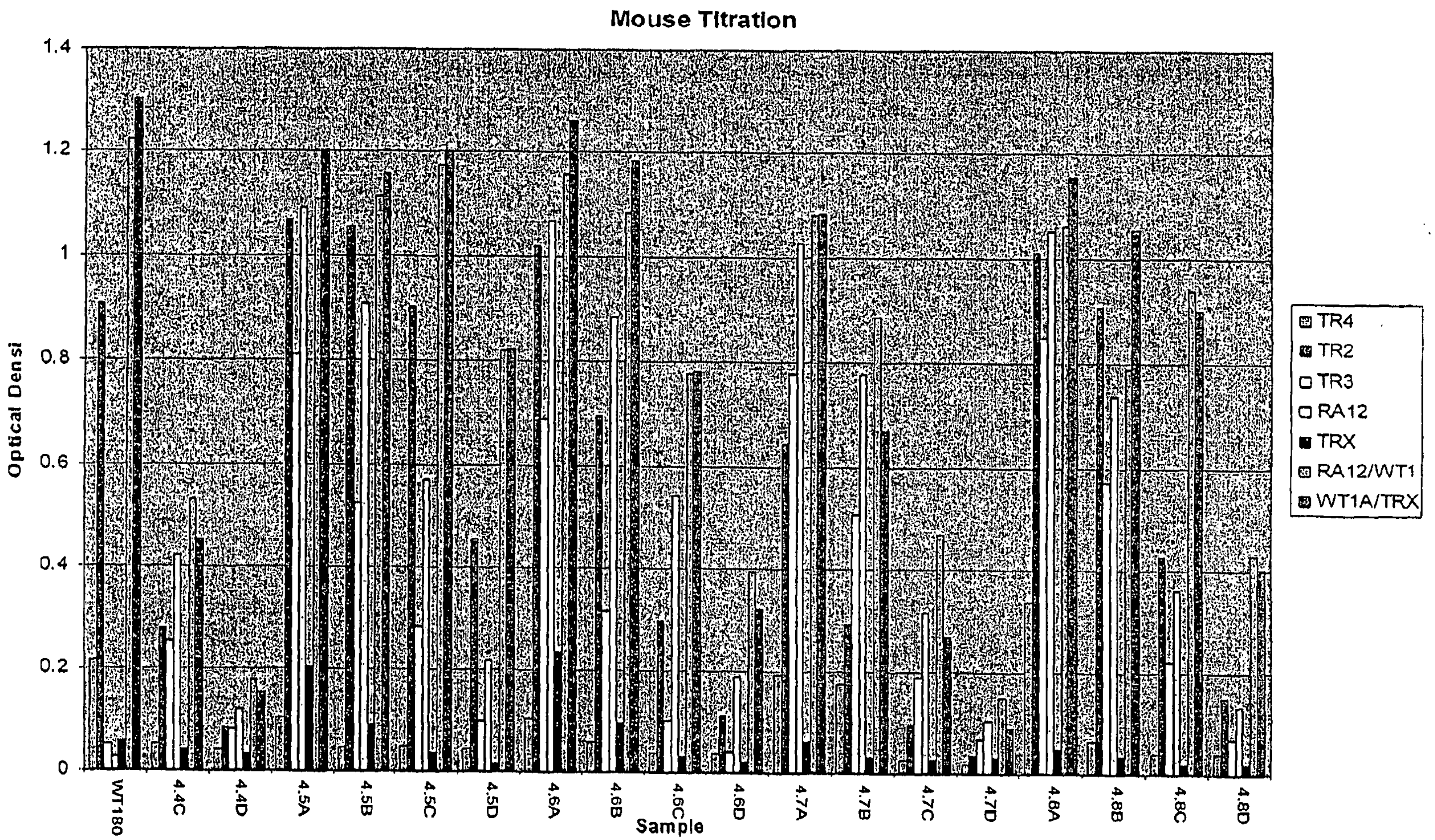


WT1. Dose Titration. Ab responses to WT1. 1000ug Ra12-WT1+MPL-SE. A: 1;500 Dilution, B: 1:2000, C: 1: 8000, D: 1:16000

FIG. 19E

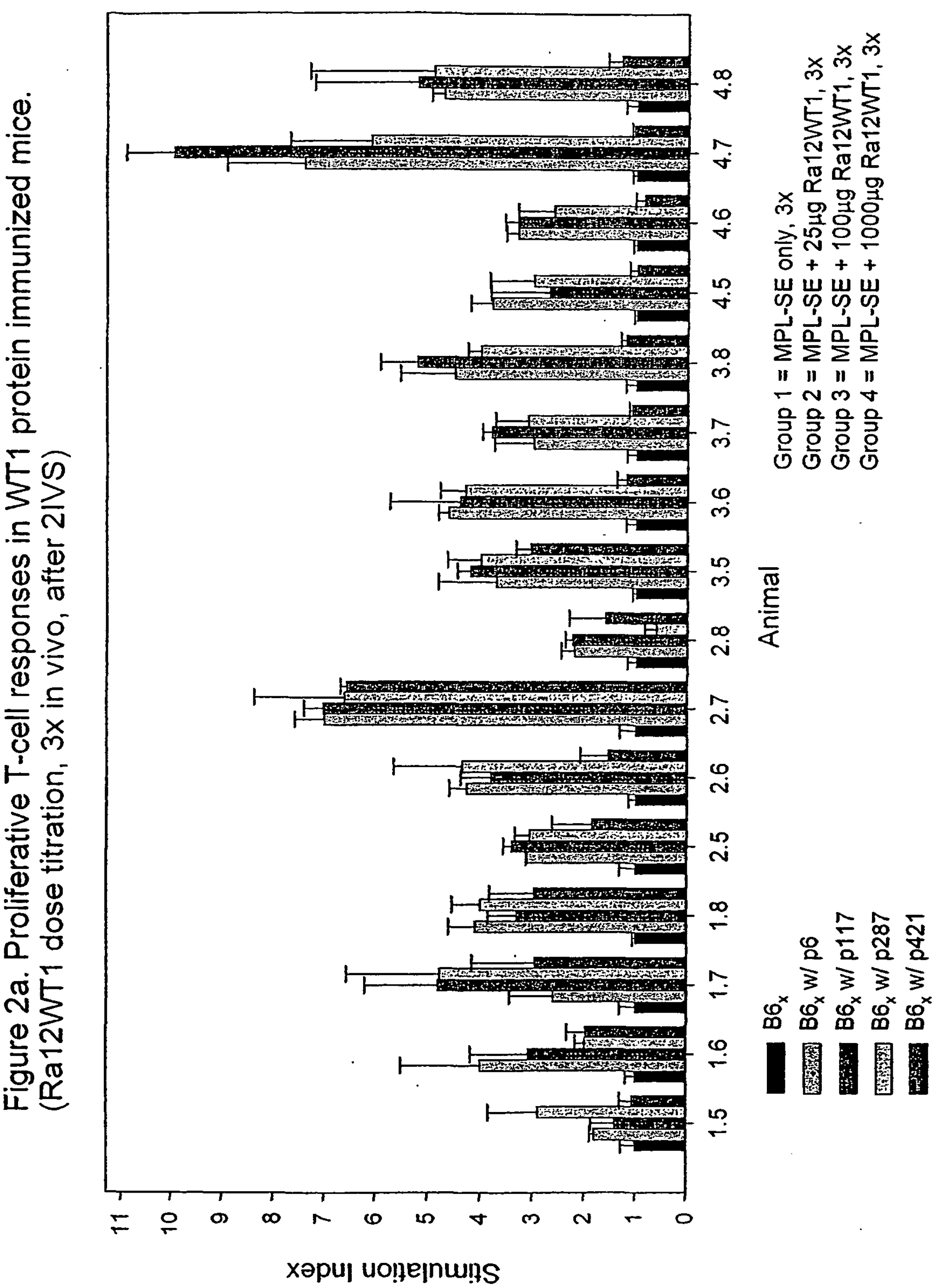
Figure 2a. Proliferative T-cell responses in WT1 protein immunized mice.
(Ra12WT1 dose titration, 3x in vivo, after 2IVS)
Stimulation Index
0
1
2
3
4
5
6
7
8
9
10
11
1.5
1.6
1.7
1.8
2.5
2.6
2.7
2.8
3.5
3.6
3.7
3.8
4.5
4.6
4.7
4.8
Animal
B6x
B6x w/ p6
B6x w/ p117
B6x w/ p287
B6x w/ p421
Group 1 = MPL-SE only, 3x
Group 2 = MPL-SE + 25µg Ra12WT1, 3x
Group 3 = MPL-SE + 100µg Ra12WT1, 3x
Group 4 = MPL-SE + 1000µg Ra12WT1, 3x

FIG. 20A

Figure 2b. Proliferative T-cell responses in WT1 protein immunized mice (Ra12WT1 dose titration, 6x in vivo, after 2IVS)

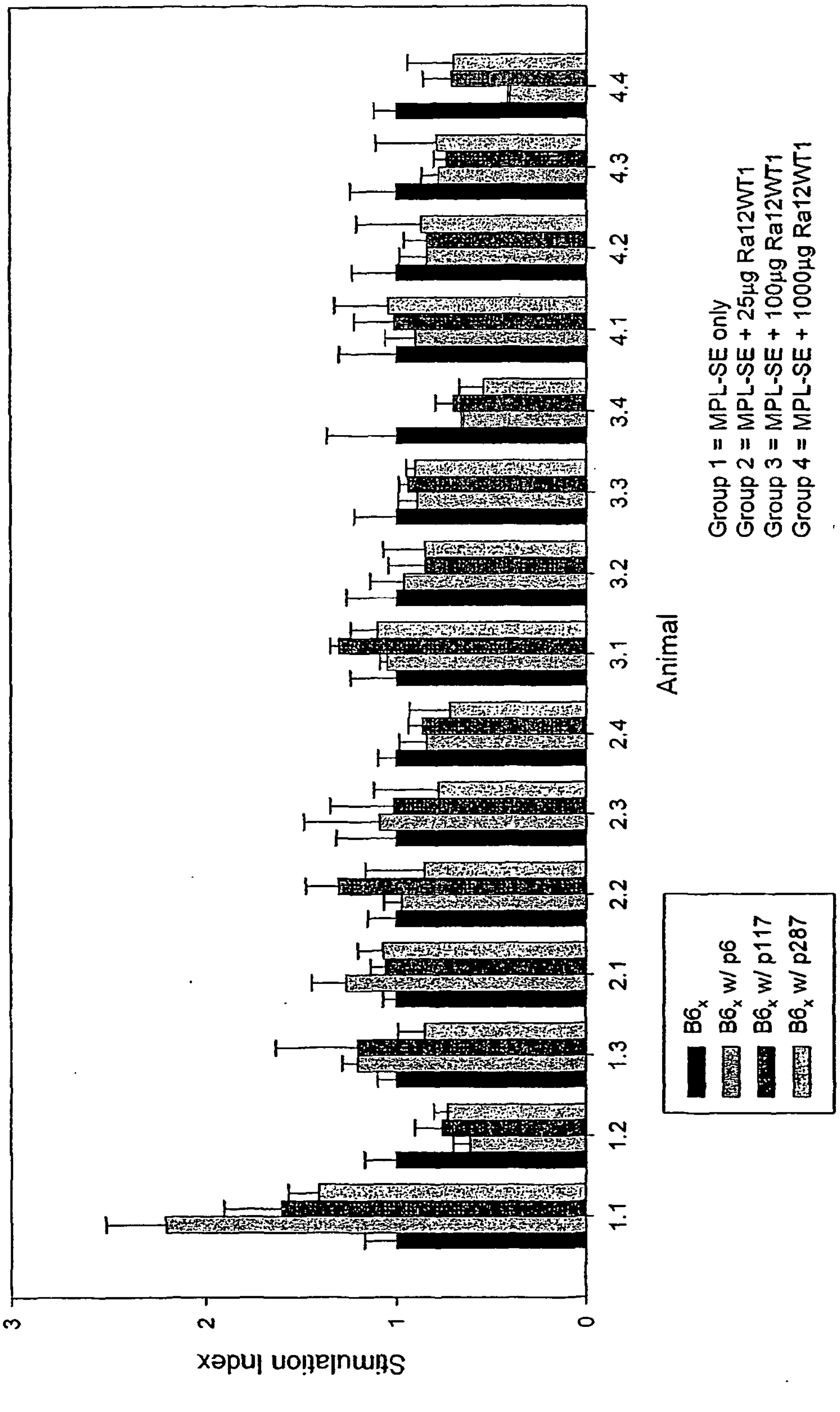


FIG. 20B

FIG. 21

# Figure 1. WT1 expression in human DC followin adeno WT1 and Vaccinia WT1 infection

Control (uninfected human DC

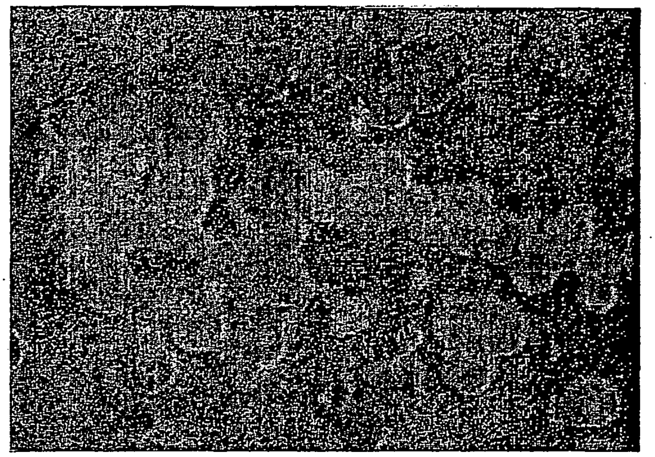

Adeno WT1 infected human DC

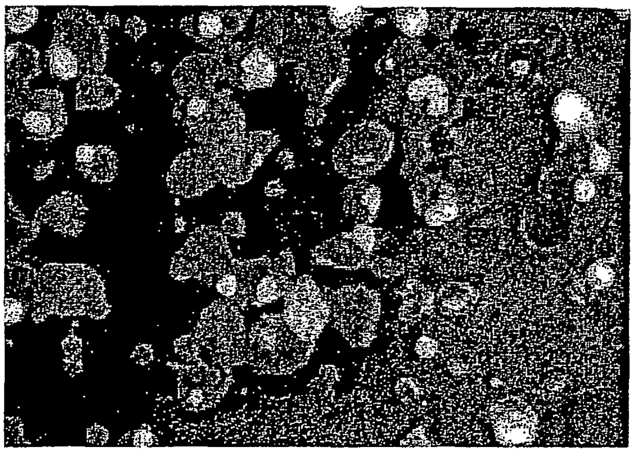

Vaccinia WT1 infected human DC

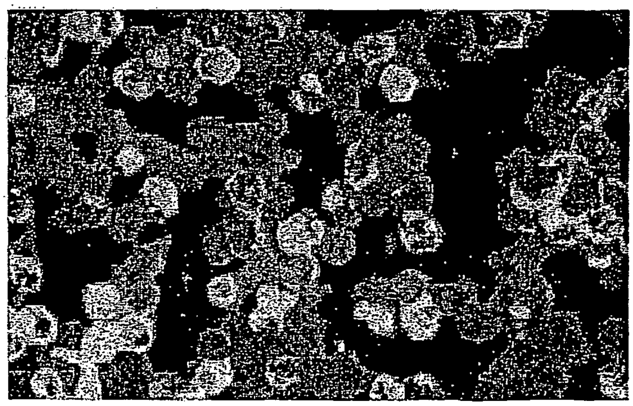

FIG. 22

**Figure 2. WT1 can be expressed reproducible in human DC following adeno WT1 infection and is not induced by a control Adeno infection**

**Control (Adeno EGFP infected human DC)**

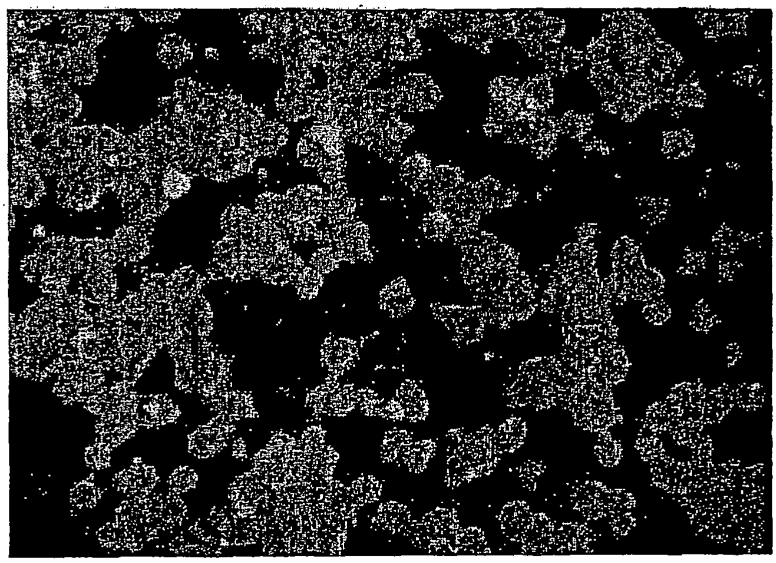

**Adeno WT1 infected human DC**

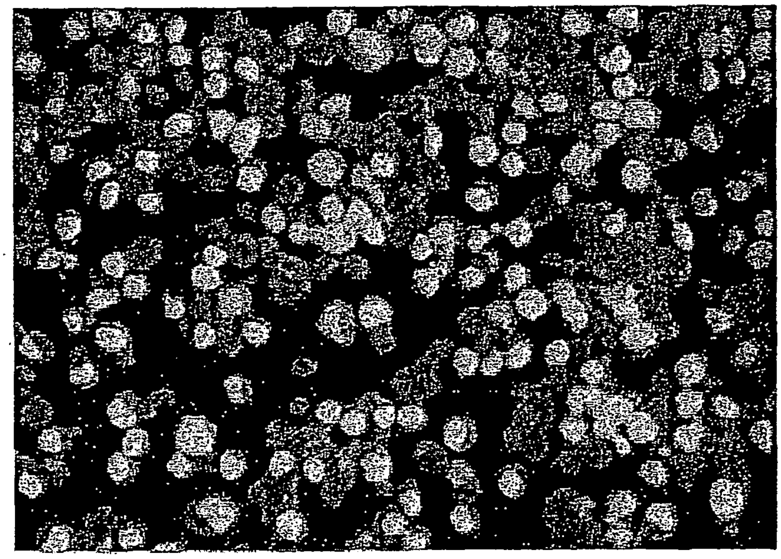

FIG. 23

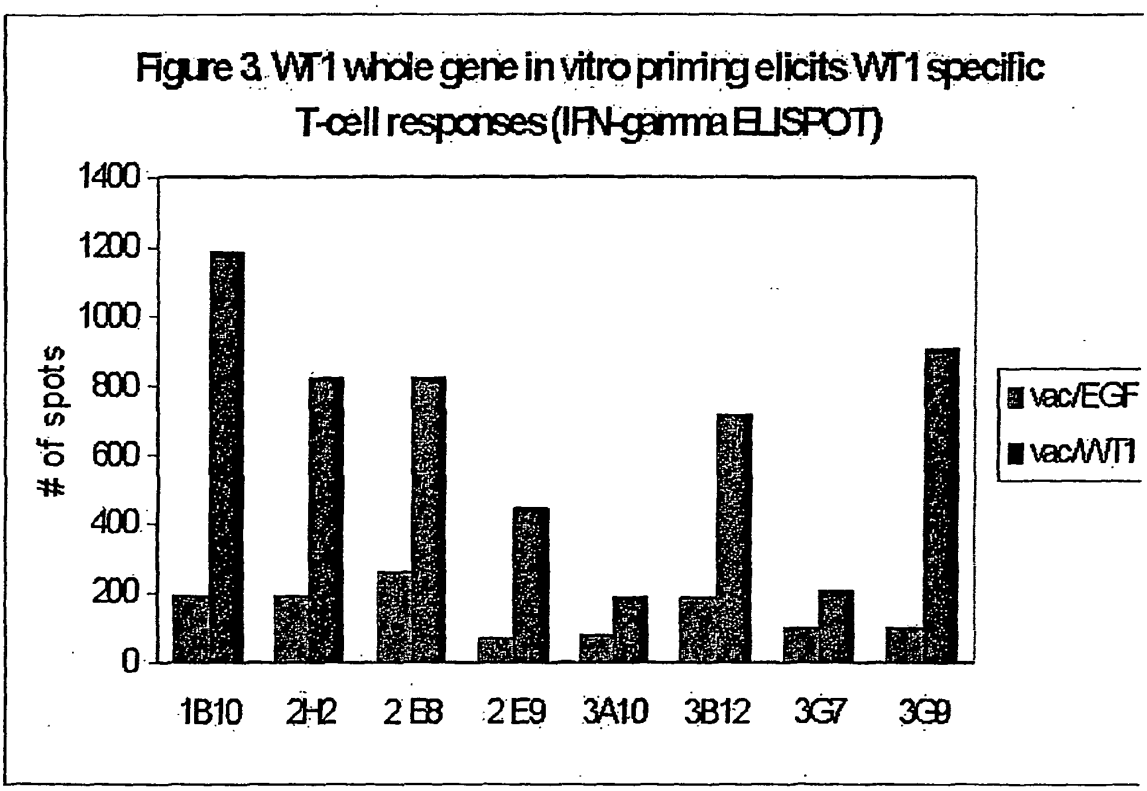
Figure 3. WT1 whole gene in vitro priming elicits WT1 specific T-cell responses (IFN-gamma ELISPOT)
1400
1200
1000
800
600
400
200
0
# of spots
1B10
2H2
2 E8
2 E9
3A10
3B12
3G7
3G9
vac/EGF
vac/WT1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CA 8175201 A1
- US 5350840 A, Call
- US 4935233 A
- US 4751180 A
- US 158585 P
- WO 9118926 A
- US 5633234 A
- US 4671958 A, Rodwell
- US 4489710 A, Spitler
- US 4625014 A, Senter
- US 4638045 A, Kohn
- US 4569789 A, Blattler
- US 4507234 A, Kato
- US 4699784 A, Shih
- US 4429008 A
- US 4873088 A
- US 4735792 A
- US 4673562 A, Davison
- US 5240856 A
- US 5215926 A
- WO 8906280 A
- WO 9116116 A
- WO 9207243 A
- WO 9630516 A
- US 4897268 A
- US 5075109 A
- US 853826 A
- US 09074720 B
- WO 9952549 A1
- WO 9952549 A
- GB 9820956 A
- US 4436727 A
- US 4877611 A
- US 4866034 A
- US 4912094 A
- WO 9602555 A
- WO 9933488 A
- US 6008200 A
- US 5856462 A
- US 4376110 A, David
- US 4452901 A, Gordon
- US 3817827 A
- US 3850752 A
- US 3901654 A
- US 3935074 A
- US 3984533 A
- US 3996345 A
- US 4034074 A
- US 4098876 A
- US 5359681 A


### Non-patent literature cited in the description


- **PARKER et al.** *J. Immunol.,* 1994, vol. 152, 163
- **PAUL.** Fundamental Immunology. Raven Press, 1993, 243-247
- **HARLOW ; LANE.** Antibodies: A Laboratory Manual. Cold Spring Harbor Laboratory, 1988
- **ROTHBARD ; TAYLOR.** *EMBO J.,* 1988, vol. 7, 93-100
- **DEAVIN et al.** *Mol. Immunol.,* 1996, vol. 33, 145-155
- **MERRIFIELD.** *J. Am. Chem. Soc.,* 1963, vol. 85, 2149-2146
- **MARATEA et al.** *Gene,* 1985, vol. 40, 39-46
- **MURPHY et al.** *Proc. Natl. Acad. Sci. USA,* 1986, vol. 83, 8258-8262
- **STOUTE et al.** *New Engl. J. Med.,* 1997, vol. 336, 86-91
- **SKEIKY et al.** *Infection and Immun.,* 1999, vol. 67, 3998-4007
- *Gene,* 1986, vol. 43, 265-292
- *Biotechnology,* 1992, vol. 10, 795-798
- **ADELMAN et al.** *DNA,* 1983, vol. 2, 183
- **KOHLER ; MILSTEIN.** *Eur. J. Immunol.,* 1976, vol. 6, 511-519
- **CHEN et al.** *Cancer Res.,* 1994, vol. 54, 1065-1070
- **COLIGAN et al.** Current Protocols in Immunology. Wiley Interscience, 1998, vol. 1
- **ULMER et al.** *Science,* 1993, vol. 259, 1745-1749
- **COHEN.** *Science,* 1993, vol. 259, 1691-1692
- **REEVES et al.** *Cancer Res.,* 1996, vol. 56, 5672-5677
- **TUTING et al.** *J. Immunol.,* 1998, vol. 160, 1139-1147
- **NAIR et al.** *Nature Biotechnol.,* 1998, vol. 16, 364-369
- **SATO et al.** *Science,* 1996, vol. 273, 352

- **SATOH F. et al.** *Pathol. Int.,* 2000, vol. 50 (6), 458-71
- **CAMPBELL C. E. et al.** *Int. J. Cancer,* 1998, vol. 78 (2), 182-8
- **AMIN, K.M. et al.** *Am. J. Pathol.,* 1995, vol. 146 (2), 344-56
- **HARADA et al.** *Mol. Urol.,* 1999, vol. 3 (4), 357-364
- **NONOMURA et al.** *Hinyokika Kiyo,* 1999, vol. 45 (8), 593-7
- **SHIMIZU et al.** *Int. J. Gynecol. Pathol.,* 2000, vol. 19 (2), 158-63
- **BARNOUD, R. et al.** *Am. J. Surg. Pathol.,* 2000, vol. 24 (6), 830-6
- *Pathol. Res. Pract.,* 1998, vol. 194 (10), 693-700
- **MENSSEN, H.D. et al.** *J. Cancer Res. Clin. Oncol.,* 2000, vol. 126 (4), 226-32
- **SPINSANTI P. et al.** *.Leuk Lymphoma,* 2000, vol. 38 (5-6), 611-9
- *Leukemia,* vol. 14 (9), 1634-4
- **PAN et al.** in vitro IL-12 treatment of peripheral blood mononuclear cells from patients with leukemia or myelodysplastic syndromes, and reported an increase in cytotoxicity and reduction in WT1 gene expression. *Leukemia,* 1999, vol. 13 (6), 891-900
- **PATMASIRIWAT et al.** WT1 and GATA1 expression in myelodysplastic syndrome and acute leukemia. *Leukemia,* 1999, vol. 13 (3), 393-9
- **OJI et al.** *Jpn. J Cancer Res.,* 1999, vol. 90 (2), 194-204
- **WIDE et al.** Radioimmunoassay Methods. E. and S. Livingstone, 1970
- **BROWN et al.** *J. Biol. Chem.,* 1980, vol. 255, 4980-4983
- **RAINES ; ROSS.** *J. Biol. Chem.,* 1982, vol. 257, 5154-5160
- **BROOKS et al.** *Clin. Exp. Immunol.,* 1980, vol. 39, 477
- **BOWEN-POPE et al.** *Proc. Natl. Acad Sci. USA,* 1984, vol. 81, 2396-2400
- **SKEIKY et al.** *Infect Immun.,* 1999, vol. 67, 3998
- **HOCHULI et al.** *Biologically Active Molecules,* 1989, 217
- **GAIGER et al.** *Blood,* 2000, vol. 96, 1334
- **DEAVIN et al.** *Mol. Immarnol.,* 1996, vol. 33, 145-155
- **DEBRUIJN et al.** *Eur. J. Immunol.,* 1991, vol. 21, 2963-2970
- **LJUNGGREN et al.** *Nature,* 1990, vol. 346, 476-480
- **FRAIZER et al.** *Blood,* 1995, vol. 86, 4704-4706
- **KWOK ; HIGUCHI.** *Nature,* 1989, vol. 339, 237-238
- **INOUE et al.** *Blood,* 1996, vol. 88, 2267-2278
- **LOZZIO ; LOZZIO.** *Blood,* 1975, vol. 45, 321-334
- **FOSTER et al.** *Cancer Res.,* 1997, vol. 57, 3325-3330
- *Nature,* 1978, vol. 276, 510-511
- **GILLIS.** *Nature,* 1977, vol. 268, 154-156
- **GLYNN ; FEFER.** *Cancer Res.,* 1968, vol. 28, 434-439
- **AAROSTON.** *J Cell. Physiol.,* 1968, vol. 72, 141-148
- **HORIBATA ; HARRIS.** *Exp. Cell. Res.,* 1970, vol. 60, 61
- **OLD et al.** *Ann. NY Acad. Sci.,* 1962, vol. 101, 80-106
- *Proc. Natl. Acad. Sci. USA,* 1970, vol. 66, 344
- **MELLING et al.** *J. Immunol.,* 1976, vol. 117, 1267-1274
- **SLAVIN ; STROBER.** *Nature,* 1977, vol. 272, 624-626
- **GLYNN et al.** *Cancer Res.,* 1968, vol. 28, 434-439
- *journal of Molecular Biology,* 1996, vol. 255, 589-603